# EUROPEAN PATENT SPECIFICATION

(11) **EP 0 987 254 B1**
(45) Date of publication and mention of the grant of the patent: **22.12.2004**
(21) Application number: 98921809.4
(22) Date of filing: 26.05.1998
(51) Int. Cl.: C07D 233/70, C07D 235/28, C07D 263/58, C07D 277/74, C07D 277/80, C07D 401/12, C07D 413/04, C07D 413/12, C07D 417/12, C07D 498/04, A61K 31/495, A61K 31/55, A61K 31/505

(54) **NOVEL CYCLIC DIAMINE COMPOUNDS AND MEDICINE CONTAINING THE SAME**
NEUE ZYKLISCHE DIAMINVERBINDUNGEN UND HEILMITTEL DAS DIESE ENTHÄLT
NOUVEAUX COMPOSES DE DIAMINE CYCLIQUES ET MEDICAMENT CONTENANT CES COMPOSES

(30) Priority: 26.05.1997 JP 14989297
(43) Date of publication of application: 22.03.2000
(73) Proprietor: Kowa Co., Ltd., Naka-ku, Nagoya-shi, Aichi 460-8625 (JP)
(72) Inventor: SHIBUYA, K., 403, Lions-Hills-Nishitokorozawa, Tokorozawa-shi, Saitama 359-1142 (JP); KAWAMINE, Katsumi, Kowa-Higashimurayama-ryo, Higashimurayama-shi, Tokyo 189-0022 (JP); SATO, Yukihiro, Kowa-Higashimurayama-ryo, Higashimurayama-shi, Tokyo 189-0022 (JP); MIURA, Toru, Omiya-shi, Saitama 331-0062 (JP); OZAKI, Chiyoka, Nerima-ku, Tokyo 177-0045 (JP); EDANO, Toshiyuki, Kawagoe-shi, Saitama 350-1124 (JP); HIRATA, Mitsuteru, Tsurugashima-shi, Saitama 350-2211 (JP)
(74) Representative: Cresswell, Thomas Anthony
(86) International application number: PCT/JP1998/002300
(87) International publication number: WO 1998/054153

(56) References cited:
- EP-A- 0 372 445
- EP-A- 0 807 627
- WO-A-92/07825
- WO-A-98/42680
- WO-A-99/25712
- US-A- 3 629 208
- US-A- 5 596 001
- US-A- 5 721 266

## Description

### Technical Field

The present invention relates to azole compounds having novel cyclic diamine structure and pharmaceutical compositions containing the same. More particularly, the present invention relates to compounds represented by the following formula (I), salt(s) or solvate(s) thereof and also to a pharmaceutical composition consisting of the same. 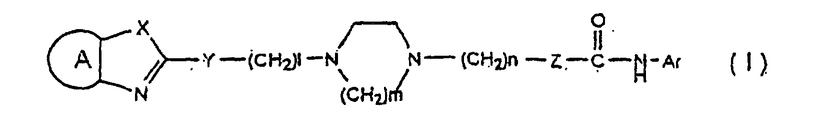 Formula (I) is defined in detail below.

### Background Art

In recent years, as a result of an increase in persons of advanced age and also of changes in daily eating habits to the food of European and American style containing high calories and high cholesterol due to upgrade of the standard of living, there has been a rapid increase in hyperlipemia and arteriosclerotic diseases caused thereby and that is one of the social problems. The pharmacotherapy for hyperlipemia and arteriosclerosis up to now has mostly given its priority to reduce the lipid content in blood which is a cause thereof and has not been a therapy where arteriosclerotic focus *per se* is a target.

Acyl coenzyme A cholesterol acyltransferase (ACAT) is an enzyme which catalyzes the synthesis of cholesterol ester from cholesterol and plays an important role in metabolism and absorption in digestive organs of cholesterol. It is believed that inhibition of ACAT enzyme which esterifies free cholesterol in epithelial cells of small intestine results in inhibition of absorption of cholesterol from intestinal tract, that inhibition of production of cholesterol ester in liver due to ACAT inhibition suppresses the secretion of very low-density lipoprotein (VLDL) from liver into blood and that, as a result thereof, a decrease in cholesterol in blood is resulted. Many of ACAT inhibitors until now have been those which act the ACAT enzyme in small intestine and liver whereby a decrease in cholesterol in blood is expected as antihyperlipemic agents.

For example, 2,2-dimethyl-N-(2,4,6-trimethoxyphenyl)dodecanamide and N'-(2,4-difluorophenyl)-N-[5-(4,5-diphenyl-1H-imidazol-2-ylthio)pentyl]-N-heptylurea are described as ACAT inhibitors in the U. S. Patent No. 4,716,175 and the European Patent No. 372,445, respectively. However, many ACAT inhibitors up to now give a priority on lowering of cholesterol in blood as antihyperlipemic agents and, due to administration in large doses for achieving the action, many side effects such as intestinal bleeding, intestinal disturbance, diarrhea and hepatic disturbance occurred which made their clinical development difficult.

Incidentally, arteriosclerosis is a lesion characterized by thickening of intima and accumulation of lipid in blood vessel and, according to the recent studies, suppression of foaming of macrophage playing a central role in formation of arteriosclerotic lesion is expected to achieve an involution of arteriosclerotic lesion itself. Foam cells (cholesterol ester is stored in the cells as fat droplets) derived from macrophage are in focus of pultaceous arteriosclerosis and it has been said that this foaming of macrophage is closely related to the progress of the focus. It is also reported that the ACAT activity on the blood vessel wall of focus site of arteriosclerosis is high and the cholesterol is accumulated on the blood vessel wall (Gillies, P. J., et al.: Exp. Mole. Pathol., 44, 329-339(1986)).

Inhibition of esterification of cholesterol by an ACAT inhibitor produces free cholesterol in cells and this is then taken out by a high-density lipoprotein (HDL) followed by being transmitted to liver (reverse transmission) to be metabolized and, accordingly, suppression of accumulation of cholesterol at the focus site is expected. It is believed that, as a result thereof, a direct antiarteriosclerotic action is achieved. It is reported that there are two types of ACAT - one is present in small intestine and another is present on blood vessel wall (Kinnunen, P. M., et al.: Biochemistry, 27, 7344-7350(1988)) although many investigations on ACAT inhibitors until now have been conducted using enzymes which are those of a type existing in small intestine and liver (Tomoda, E., et al: J. Antibiotics, 47, 148-153(1994)). Having an idea that the drug which selectively inhibits the ACAT enzyme of a type existing in blood vessel wall can be a therapeutic agent for arteriosclerosis having less side effect, the present inventors have conducted synthesis and investigation on such an inhibitor, found that the compounds represented by the following formula (A) selectively inhibit the ACAT enzyme and filed patent applications (refer to the specifications of the Japanese Patent Applications Hei-09/88660 and Hei-09/90146). 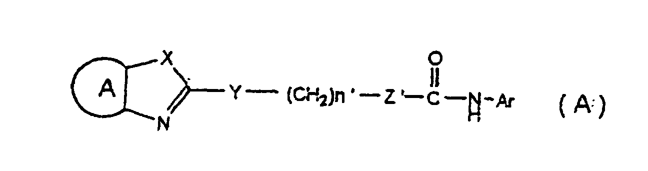 [In the formula, A, Ar, X and Y have the same meanings as those defined for the above-mentioned formula (I) of the present invention; Z' is Z or -CR₄'R₅'-Z- (where Z has the same meaning as that defined for the above-mentioned formula (I) of the present invention and R₄' and R₅' are same or different and are hydrogen atom, lower alkyl group or lower alkoxy group); and n' is an integer of from 0 to 15.]

However, those compounds are highly liposoluble and, therefore, they have disadvantages that oral absorption is not good and that duration in serum is short.

Accordingly, there has been a demand for developing compounds where oral absorption is improved and a high drug concentration in serum is maintained for long time.

### Disclosure of the Invention

In order to achieve the above object, the present inventors have continued the investigation and have found that azole compounds where a cyclic diamine structure is introduced into a molecule are highly soluble in water and exhibit an excellent biological activity. Therefore, to be more specific, the present invention has been accomplished as a result of a finding that the compounds represented by the following formula (I) or salt(s) or solvate(s) thereof have excellent ACAT inhibitory action and also excellent solubility. 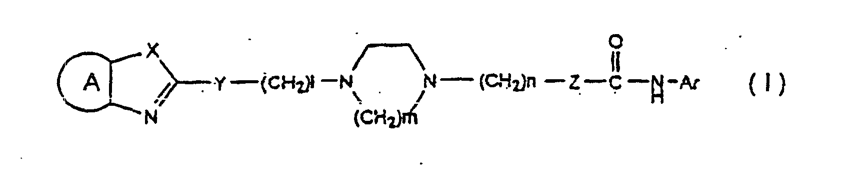 wherein
l is from 1 to 10;
m is 2 or 3;
n is from 1 to 3,
X is -NH-, oxygen atom or sulfur atom;
Y is -NR¹-, oxygen atom, sulfur atom, sulfoxide or sulfone;
Z is a single bond or -NR²-;
R¹ is hydrogen, optionally substituted straight or branched chain C₁₋₈ alkyl, optionally substituted six-membered aromatic hydrocarbon group or five- to seven- membered heterocyclic group containing from one to three oxygen atom(s), nitrogen atom(s) or sulfur atom(s) as heteroatom(s), or optionally substituted silyl alkyl comprising a straight chain or a branched chain alkyl having 1 to 8 carbon atoms;
R² is hydrogen, optionally substituted straight or branched chain C₁₋₃ alkyl, optionally substituted six-membered aromatic hydrocarbon group or five- to seven- membered heterocyclic group containing from one to three oxygen atom(s), nitrogen atom(s) or sulfur atom(s) as heteroatom(s), or optionally substituted silyl alkyl comprising a straight chain or a branched chain alkyl having 1 to 8 carbon atoms; 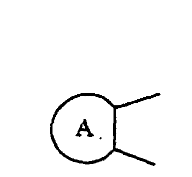 is
(i) a group 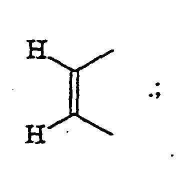
(ii) an unsubstituted divalent residue of benzene, pyridine, cyclohexane or naphthalene; or
(iii) a substituted divalent residue of benzene, pyridine, cyclohexane or naphtalene;
and wherein the optional substituents for the foregoing straight or branched C₁₋₈ alkyl groups are selected from groups W1;
the optional substituents for the foregoing six-membered aromatic hydrocarbon and five- to seven- membered heterocyclic groups are selected from groups W2; and
the substituents for the foregoing substituted divalent residues are selected from groups W3;
each group W1 is hydroxyl, C₁ to C₃ straight or branched alkoxy, C₁ to C₃ straight or branched alkylthio, C₁ to C₃ straight or branched alkoxycarbonyl, C₁ to C₃ straight or branched alkylcarbonyloxy, halogen, amino or nitro;
each group W2 is optionally substituted straight or branched chain C₁ to C₃ alkyl, optionally substituted straight or branched chain C₁ to C₃ alkoxy, optionally substituted straight or branched chain C₁ to C₃ alkylthio, optionally substituted straight or branched chain C₁ to C₃ alkylsulfinyl, optionally substituted straight or branched chain C₁ to C₃ alkylsulfonyl, optionally substituted straight or branched chain C₁ to C₃ alkylsulfonyloxy, optionally substituted straight or branched chain C₁ to C₃ alkylcarbonyl, halogen, hydroxyl, optionally substituted C₁₋₈ alkylcarbonyloxy, nitro, phosphoric acid group, di-(straight or branched chain C₁ to C₈ alkoxy)-phosphoryloxy, sulfonamide, amino, substituted amino and straight or branched chain C₁ to C₈ alkylenedioxy;
each group W3 is optionally substituted straight or branched chain C₁ to C₈ alkyl, optionally substituted straight or branched chain C₁ to C₃ alkoxy, optionally substituted straight or branched chain C₁ to C₈ alkylcarbonyl, straight or branched chain C₁ to C₈ alkylthio, straight or branched chain C₁ to C₈ alkylsulfinyl, straight or branched chain C₁ to C₃ alkylsulfonyl, halogen, hydroxyl carboxyl, straight or branched chain C₁ to C₃ alkoxycarbonyl, hydroxy-straight or branched chain C₁ to C₃ alkyl, phosphoric acid group, cyano, nitro, sulfonamide, optionally substituted amino, optionally substituted amino straight or branched chain C₁ to C₈ alkyl, optionally substituted silyl straight or branched chain C₁ to C₈ alkyl, or an optionally substituted residue of a saturated or unsaturated five to seven membered heterocyclic group containing one to four oxygen atom(s), nitrogen atom(s) or sulfur atom(s) as heteroatom(s); or straight or branched chain C₁ to C₈ alkylenedioxy group;
and wherein the optional substituents for groups W2 and W3 other than substituted amine, substituted aminoalkyl and substituted silylalkyl groups W2 and W3, are selected from groups W1;
the substituents for substituted amino and substituted aminoalkyl groups W2 and W3 are selected from groups W4; and
the substituents for substituted silylalkyl groups W2 and W3 are selected from groups W5;
each group W4 is selected from straight or branched C₁ to C₃ alkyl, optionally substituted six-membered aromatic hydrocarbon group or five- to seven- membered heterocyclic group containing from one to three oxygen atom(s), nitrogen atom(s) or sulfur atom(s) as heteroatom(s) and optionally substituted aralkyl group having 7-20 carbons, the two groups W4 of the amino group may form a five- to seven- membered optionally substituted ring together with the nitrogen atom of the amino group, one or more carbon atom(s) in that ring may be replaced by oxygen atom, sulfur atom or nitrogen atom, optional substituents for groups W4 being selected from straight or branched chain C₁ to C₃ alkyl, straight or branched chain C₁ to C₃ alkoxy, straight or branched chain C₁ to C₈ alkylthio, straight or branched chain C₁ to C₃ alkylsulfinyl, straight or branched chain C₁ to C₃ alkylsulfonyl, straight or branched chain C₁ to C₃ alkylsulfonyloxy, straight or branched chain C₁ to C₃ alkylcarbonyl, halogen, hydroxyl, nitro, phosphoric acid group, di-(straight or branched chain C₁ to C₃ alkoxy)-phosphoryloxy, sulfonamide, amino group and straight or branched chain C₁ to C₈ alkylenedioxy; and
each group W5 is selected from straight or branched chain C₁ to C₃ alkyl; six - membered aromatic hydrocarbon group or five- to seven- membered heterocyclic group containing from one to three oxygen atom(s), nitrogen atam(s) or sulfur atom(s) as heteroatom(s) or aralkyl of 7 to 20 carbons;
and Ar is optionally substituted six-membered aromatic hydrocarbon group or five- to seven- membered heterocyclic group containing from one to three oxygen atom(s), nitrogen atom(s) or sulfur atom(s) as heteroatom(s) wherein optional substituents for Ar are selected from groups W2 as defined above.

The present inventors have found that those azole compounds having intramolecular cyclic diamine structure have organ-selective ACAT inhibitory action and an inhibiting action to intracellular cholesterol transportation and also that they are the compounds where oral absorption is improved and high drug concentration in serum can be maintained for long time. Those compounds of the present invention are particularly useful as antihyperlipemic agents having an excellent cholesterol-lowering action in blood and also as preventive and therapeutic agents for arteriosclerosis, and so on having an suppressing action to foaming of macrophage.

Accordingly, the present invention offers the compounds represented by the above formula (1) and salt(s) or solvate(s) thereof.

The present invention further offers a pharmaceutical composition consisting of therapeutically effective amount of one or more of the compounds represented by the above formula (I) or salt(s) or solvate(s) thereof and a pharmaceutically acceptable carrier.

The present invention furthermore offers the compounds, salts and solvates for use as ACAT inhibitors, agents for inhibiting the transportation of intracellular cholesterol, agents for lowering the cholesterol in blood or agents for suppressing the foaming of macrophage and the use of the compounds, salts and solvates for the preparation of a medicament for use as such an inhibitor or agent.

The present invention further offers the compounds, salts and solvates as agents for therapy and prevention of hyperlipemia, arteriosclerosis, cervical and cerebral arteriosclerosis, cerebrovascular disorder, ischemic cardiopathy, coronary arteriosclerosis, nephrosclerosis, arteriosclerotic nephrosclerosis, arteriocapillary sclerotic nephrosclerosis, malignant nephrosclerosis, ischemic entheropathy, acute occlusion of mesenteric vessel, chronic mesenteric angina, ischemic colitis, aortic aneurysm and arteriosclerosis obliterans (ASO).

Among the compounds of the present invention represented by the above formula (I), more preferred compounds or salts or solvates thereof are those

Where Ar is phenyl, pyridyl or pyrimidyl group which may be substituted with from one to four group(s) selected from optionally-substituted alkyl group, optionally substituted alkoxy group, alkylthio group, alkylsulfinyl group, alkylsulfonyl group, alkylsulfonyloxy grbup, optionally substituted alkyl carbonyl group, halogen atom, hydroxyl group, optionally substituted alkylcarbonyloxy group, nitro group, phosphoric acid group, di-(alkoxy)-phosphoryloxy group, sulfonamide group, optionally substituted amino group and alkylenedioxy group.

More preferred compounds among the compounds of the present invention represented by the above formula (I) or salts or solvates thereof are those wherein 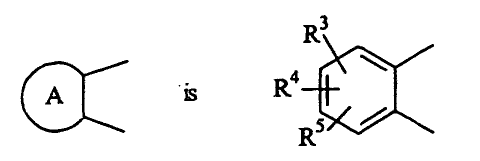 R₃, R₄ and R₅ are same or different and are hydrogen atom, optionally substituted alkyl group, optionally substituted alkoxy group, optionally substituted alkylcarbonyl group, alkylthio group, alkylsulfinyl group, alkylsulfonyl group, halogen atom, hydroxyl group, carboxyl group, alkoxycarbonyl group, hydroxyalkyl group, phosphoric acid group, cyano group, nitro group, sulfonamide group, optionally substituted amino group, optionally substituted aminoalkyl group, optionally substituted silyl alkyl group or heterocyclic residue; or any of two of R₃, R₄ and R₅ form an alkylenedioxy group together.

Additional more preferred compounds or salts or solvates thereof are those wherein 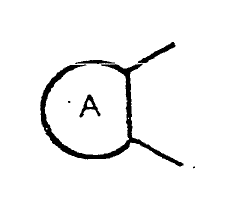 is 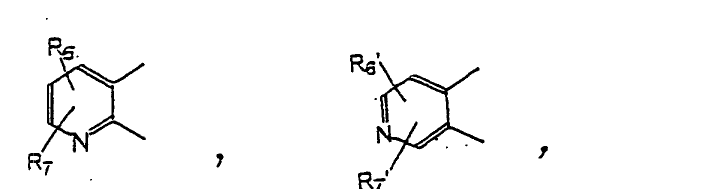 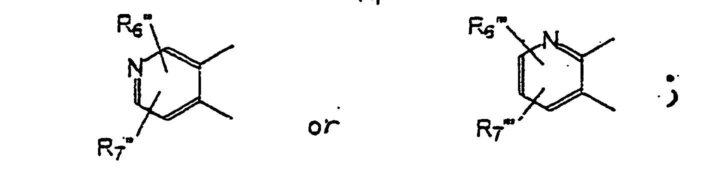 R₆ and R₇, R₆' and R₇', R₆" and R₇", R₆'''and R₇''' are same or different and are hydrogen atom, optionally substituted alkyl group, optionally substituted alkoxy group, halogen atom, hydroxyl group, carboxyl group, alkoxycarbonyl group, hydroxyalkyl group, phosphoric acid group, sulfonamide group, optionally substituted amino group, optionally substituted aminoalkyl group, optionally substituted silyl alkyl group or heterocyclic residue ; or any two of R₆ and R₇; R₆' and R₇'; R₆" and R₇"; R₆'''and R₇''' may form an alkylenedioxy group.

Consequently, the present invention relates to pharmaceutical compositions containing such a preferred compound or salt(s) or solvate(s) thereof, to pharmaceutical compositions containing an effective amount of one or more of the preferred compounds or salt(s) or solvate(s) thereof and a pharmaceutically acceptable carrier, such preferred compounds, salts and solvates for uses as defined above and the use of such preferred compounds, salts and solvates in the preparation of medicaments as defined above.

When 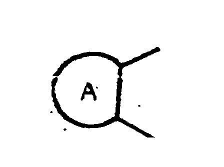 in the above-mentioned formula (I) is optionally substituted divalent residue of benzene, pyridine, cyclohexane or naphthalene, a fused ring system is formed together with the adjacent nitrogen atom and an atom represented by a substituent X as well as carbon atom on the ring. Further, when the group is the following formula, 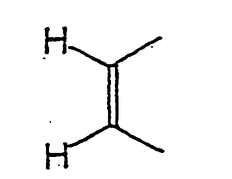 a monocyclic azole is formed.

These divalent residues of benzene, pyridine, cyclohexane or naphthalene may be unsubstituted or may be substituted with from 1 to 3 or, preferably, from 1 to 2 substituent(s). Among those, a divalent residue of benzene or pyridine is preferred.

Among the divalent residues of benzene, a group represented by the following formula is preferred. 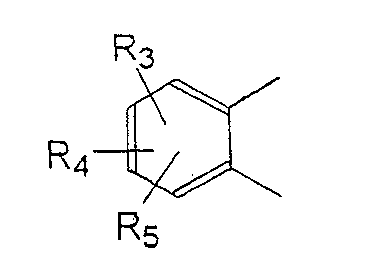 (In the formula, R₃, R₄ and R₅ are same or different and are hydrogen atom, optionally substituted lower alkyl, optionally substituted lower alkoxy, optionally substituted lower acyl, lower alkylthio, lower alkylsulfinyl, lower alkylsulfonyl, halogen atom, hydroxyl group, carboxyl group, alkoxycarbonyl group, hydroxyalkyl group, phosphoric acid group, cyano group, nitro group, sulfonamide group, optionally substituted amino group, optionally substituted aminoalkyl group, optionally substituted silyl lower alkyl group or heterocyclic residue; or any of two of R₃, R₄ and R₅ form an alkylenedioxy group together.)

With respect to the divalent residue of pyridine, a group represented by the following formula is preferred depending upon the selection of the divalent residue of pyridine and upon the connecting position to the adjacent azole ring. 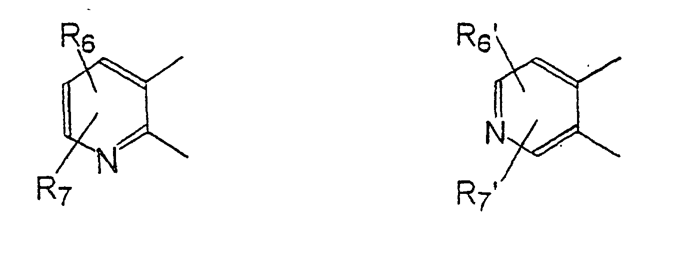 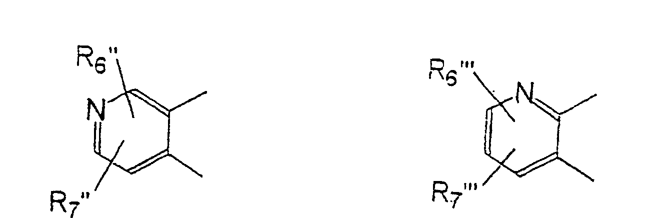 (In the formulae, R₆, R₇, R₆', R₇', R₆'', R₇'', R₆''' and R₇''' are same or different and are hydrogen atom, optionally substituted lower alkyl group, optionally substituted lower alkoxy group, halogen atom, hydroxyl group, carboxyl group, alkoxycarbonyl group, hydroxyalkyl group, phophoric acid group, sulfonamide group, optionally substituted amino group, optionally substituted aminoalkyl group, optionally substituted silyl lower alkyl group or heterocyclic residue; or any two of R₆, R₇, R₆', R₇', R₆'', R₇'', R₆''' and R₇''' may form an alkylenedioxy group.)

The groups used in each of the formulae of the present invention will be further illustrated as hereunder.

"Aryl group" is a group having six-membered aromatic hydrocarbon group or a group having from five- to seven - membered heterocyclic group which may have substituent containing from one to three oxygen atom(s), nitrogen atom(s) or sulfur atom(s) as heteroatom(s) and its examples are phenyl group, naphthyl group, biphenyl group, pyridyl group, furyl group, thienyl group, imidazolyl group and pyrimidyl group. Examples of preferred aryl group are phenyl group, pyridyl group and pyrimidyl group.

The aryl group may be substituted with functional group(s) which will not affect the property of the compound of the present invention. Examples of the substituent for the aryl group are optionally substituted lower alkyl group, optionally substituted lower alkoxy group, optionally substituted lower alkylthio group, optionally substituted lower alkylsulfinyl group, optionally substituted lower alkylsulfonyl group, optionally substituted lower alkylsulfonyloxy group, optionally substituted lower acyl group, halogen atom, hydroxyl group, nitro group, phosphoric acid group, di-(lower alkoxy)-phosphoryloxy group, sulfonamide group, amino group, substituted amino group and alkylenedioxy group. Examples of the particularly preferred group are lower alkyl group, lower alkoxy lower alkoxy group, lower alkoxycarbonyl lower alkoxy group, lower alkylthio group, lower alkylsulfinyl group, lower alkylsulfonyl group, lower alkylsulfonyloxy group, lower acyl group, halogen atom, hydroxyl group, nitro group, phosphoric acid group, sulfonamide group, amino group, substituted amino group and alkylenedioxy group. The aryl group may have 1-4, preferably 1-3 or, more preferably, 1-2 of such substituent(s).

" Lower alkyl group" is preferably a straight chain or a branched chain having 1-8 or, preferably, 1-6 carbon atom(s) and its particularly preferred examples are methyl group, ethyl group, n-propyl group, isopropyl group, n-butyl group, isobutyl group, tert-butyl group, n-pentyl group and n-hexyl group.

"Alkyl group" is preferably a straight chain or a branched chain having 1-15, preferably 1-8 or, more preferably, 1-6 carbon atom(s) and its particularly preferred examples are methyl group, ethyl group, n-propyl group, isopropyl group, n-butyl group, isobutyl group, tert-butyl group, n-pentyl group, n-hexyl group, n-octyl group and n-nonyl group.

" Lower alkoxy group" is preferably an alkoxy group comprising the above-mentioned lower alkyl group and its examples are methoxy group, ethoxy group and n-propoxy group.

"Alkylenedioxy group" has an alkylene group of a straight chain or a branched chain having 1-8, preferably 1-6 or, more preferably, 1-3 carbon atom(s) and its examples are methylenedioxy group, ethylenedioxy group and dimethylmethylenedioxy group.

"Lower alkylthio group" is preferably an alkylthio group comprising the above-mentioned lower alkyl group and its examples are methylthio group, ethylthio group, n-propylthio group and isopropylthio group.

"Lower alkylsulfinyl group" is preferably an alkylsulfinyl group comprising the above-mentioned lower alkyl group and its examples are methylsulfinyl group, ethylsulfinyl group, n-propylsulfinyl group and isopropylsulfinyl group.

"Lower alkylsulfonyl group" is preferably an alkylsulfonyl group comprising the above-mentioned lower alkyl group and its examples are methylsulfonyl group, ethylsulfonyl group, n-propylsulfonyl group and isopropylsulfonyl group.

"Lower alkylsulfonyloxy group" is preferably an alkylsulfonyloxy group comprising the above-mentioned lower alkyl group and its examples are methylsulfonyloxy group, ethylsulfonyloxy group, n-propylsulfonyloxy group and isopropylsulfonyloxy group.

"Lower acyl group" is preferably an alkylcarbonyl group comprising the above-mentioned lower alkyl group and its examples are acetyl group, ethylcarbonyl group, n-propylcarbonyl group and isopropylcarbonyl group.

"Lower acyloxy group" is preferably an alkylcarbonyloxy group comprising the above-mentioned lower alkyl group and its examples are acetyloxy group, ethylcarbonyloxy group, n-propylcarbonyloxy group and isopropylcarbonyloxy group.

"Lower alkoxycarbonyl group" is preferably a group where oxycarbonyl group is bonded to the above-mentioned lower alkyl group and its examples are methoxycarbonyl group, ethoxycarbonyl group and tert-butoxycarbonyl group.

"Di-(lower alkoxy)-phosphoryloxy group" is preferably a di-(lower alkoxy)-phosphoryloxy group comprising the above-mentioned lower alkyl group and its examples are dimethoxyphosphoryloxy group, diethoxyphosphoryloxy group, di-n-propylphosphoryloxy group and di-isopropylphosphoryloxy group.

Hydrogen atom of the lower alkyl group or alkyl group in those groups may be substituted with other substituent if necessary and examples of such a substituent are hydroxyl group, lower alkoxy group, lower alkylthio group, lower alkoxycarbonyl group, lower acyloxy group, halogen atom, amino group and nitro group. Upon substitution with such a substituent, the substituent may be only one or the substitution may be conducted by two or more substituents. Examples of the group having substituent are hydroxyalkyl group, hydroxy lower alkoxy group, lower alkoxy lower alkoxy group, lower alkoxy lower alkyl group, lower alkoxycarbonyl lower alkoxy group, lower alkoxycarbonyl lower alkyl group, halogenated lower alkoxy group, halogenated lower alkyl group and amino lower alkyl group.

Preferred examples of halogen atom are fluorine atom, chlorine atom, bromine atom and iodine atom.

The lower alkyl group in the optionally substituted silyl lower alkyl group is the above-mentioned lower alkyl group and examples of the substituent for the silyl group are the above-mentioned lower alkyl and aryl groups and an aralkyl group having 7-20 carbons or, preferably, 7-12 carbons such as benzyl group and phenethyl group. Trimethylsilylmethyl group and dimethylphenylsilylmethyl group are exemplified.

The optionally substituted amino group may be a free amino group or an amino group which is substituted with one or two substituent(s). Preferred substituent for the amino group are above-mentioned lower alkyl group; the above-mentioned aryl group such as phenyl group and naphthyl group; and an aralkyl group having 7-20 carbons or, preferably, 7-12 carbons such as benzyl group and phenethyl group. Those aromatic ring may be further substituted with the above-mentioned lower alkyl group, lower alkoxy group, and so on Furthermore, the two substituents of the amino group may form a five- to seven-membered ring together with the nitrogen atom of the amino group. One or more carbon atom(s) in the ring may be substituted with oxygen atom, sulfur atom or nitrogen atom. Examples of such an amino group forming a ring are morpholino group, piperazino group and pyrrolidino group. Those rings may be further substituted with other substituent(s).

The alkyl group in the optionally substituted aminoalkyl group is the above-mentioned alkyl group and the amino group therein is the above-mentioned substituted or unsubstituted amino group.

The heterocyclic residue is a residue of a saturated or unsaturated five to seven-membered heterocyclic group containing one to four oxygen atom(s), nitrogen atom(s) or sulfur atom(s) as heteroatom(s) and its example is a tetrazolyl group. Such a heterocyclic residue may have one or more substituent(s) and examples of the substituent are those which were mentioned as the substituents for the above-mentioned alkyl group.

l in the formula (I) is an integer of from 0 to 15, preferably from 1 to 15, more preferably from 1 to 10 or, still more preferably, from 2 to 5 while n is an integer of from 0 to 3 or, preferably, from 1 to 3.

Examples of the acid addition salt(s) of the compound (I) of the present invention are inorganic acid salt(s) such as hydrochloride, sulfate, nitrate and phosphate and organic acid salt(s) such as methanesulfonate, maleate, fumarate and citrate.

The solvate(s) is a product where the solvent used during preparation, purification, and so on such as water and alcohol is added and there is no particular limitation therefor so far as that does not badly affect the ACAT inhibitory action, and so on. Hydrate is preferred as the solvate(s).

### Best Mode for Conducting the Invention.

The compound (I) may be manufactured by various known methods and there is no particular limitation therefor. For example, the compound may be manufactured in accordance with the following reaction steps.

### 1. Preparation of the compound where Z is a single bond.

(1) An amide derivative represented by the formula (VII) is obtained when an arylamine represented by the formula (V) is made to react, in accordance with the following reaction formulae, with a carboxylic acid represented by the formula (VI) or a reactive derivative thereof such as an acid halide. When the resulting compound represented by the formula (VII) is made to react with a cyclic diamine compound represented by the formula (VIII), a primary alcohol derivative represented by the formula (IX) is obtained. The resulting alcohol is subjected to mesylation, tosylation, and so on and then the resulting reactive derivative (X) is made to react with a compound represented by the formula (XI) whereupon the desired compound (I') wherein Z is a single bond is manufactured.

The above reaction routes are shown by the following chemical reaction formulae.

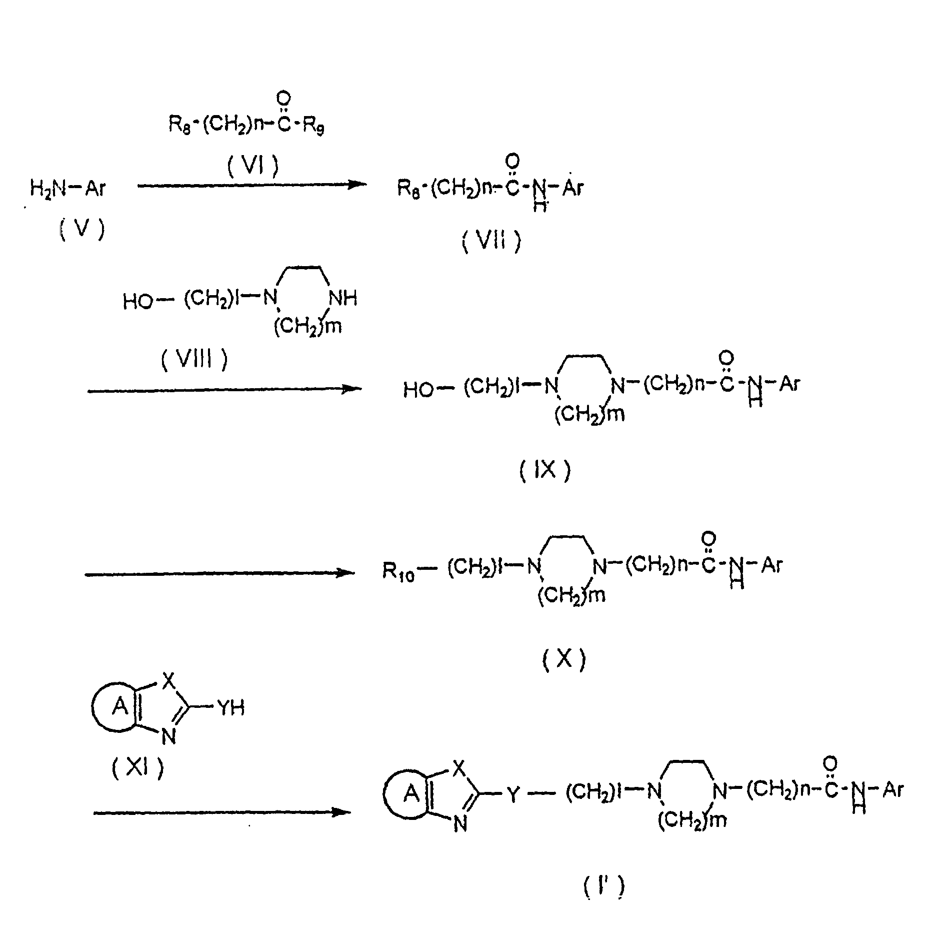

(In the formulae, R₈ and R₁₀ are leaving groups; and R₉ is a residue of a reactive derivative of hydroxyl group or carboxyl group.)

Methods which are used for common peptide synthesis may be applied to the reaction of the compound (V) with the compound (VI). Preferred examples of the leaving group R₈ in the formula (VI)are halogen atoms such as chlorine atom and bromine atom while those of the residue of the reactive derivative of carboxyl group represented by R₉ are acid anhydride residues such as anhydrides of mesylic acid, tosylic acid, acetic acid and pivalic acid. In conducting this reaction, both compounds are made to react, for example, in the presence of a condensing agent whereupon the desired compound is prepared. With regard to a condensing agent, 1-(3'-dimethylaminopropyl)-3-ethyl-carbodimide (WSC), 1,3-dicyclohexylcarbodiimide (DCC), or the like, for example, may be used either solely or jointly together with 1-hydroxybenzotriazole (HOBt), N-hydroxysuccinimide (HOSu), or the like. There is no particular limitation for the solvent and, for example, dimethylformamide, methylene chloride, chloroform, tetrahydrofuran (THF) and toluene may be used either solely or jointly.

The reaction condition may vary depending upon the materials used but, usually, it is completed when the reaction is conducted at 0-100°C or, preferably, at around room temperature for 1-30 hours or, preferably, 10-20 hours. When a carboxylic acid halide having a high reactivity is used as the compound (VI), it is possible, for example, that the compound (V) is made to react by a common method with the compound (VI) in the presence of a base such as triethylamine, 4-dimethylaminopyridine or N-methylmorpholine.

The reaction of the compound (VII) obtained in the above method with the compound (VIII) may be conducted in a solvent in the presence or absence of a base. With regard to a solvent, the above-mentioned one may be used while, with regard to a base, inorganic bases such as alkali metal hydroxide such as sodium hydroxide and potassium hydroxide; alkali metal carbonates such as sodium carbonate and potassium carbonate; alkali metal bicarbonate such as sodium bicarbonate and potassium bicarbonate; organic bases such as pyridine, triethylamine, N,N-diisopropylethylamine, N-methylmorpholine and N, N-dimethylaniline; and the like may be used.

When the resulting compound (IX) is subjected, for example, to sulfonylating reaction such as mesylation or tosylation, a compound (X) is obtained. Common methods may be used for the sulfonylating reaction but a method using, for example, an agent for producing sulfonate such as methanesulfonyl chloride, methanesulfonic acid anhydride, methanesulfonyl fluoride, benzenesulfonyl chloride and p-toluenesulfonyl chloride is preferred. When the compound (IX) is made to react with the agent for producing sulfonate in a solvent in the presence or absence of a base, the compound (X) is prepared. Examples of the solvent are tetrahydrofuran, methylene chloride and chloroform while those of the base are those which were mentioned already.

Reaction of the compound (X) prepared by the above method with a compound (XI) may be conducted by a similar method as mentioned in the above second step.

(2) Alternatively, a compound (XII) wherein hydroxyl group of the compound represented by the formula (VIII) is substituted with a leaving group such as a sulfonyl group is made to react with a compound represented by the formula (XI) to provide a compound represented by the formula (XIII). When the resulting cyclic diamine derivative (XIII) is made to react with a compound represented by the formula (VII), a desired compound (I') where Z is a single bond is obtained. 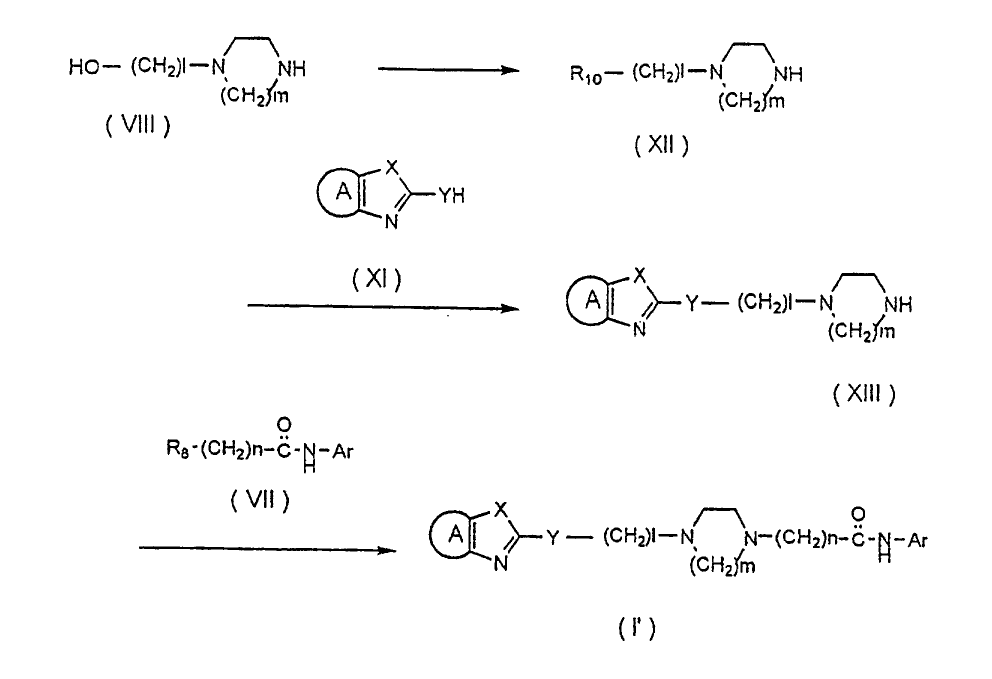 (In the formulae, R₈ and R₁₀ are leaving groups.)

The third step in the above-mentioned (1) may be applied to a step for the preparation of the compound (XII) from the compound (VIII).

The reaction of the resulting compound (XII) with the compound (XI) may be conducted by a similar method as in the final step of the above (1).

The reaction of the resulting compound (XIII) with the compound (VII) may be conducted by a similar method as in the second step of the above-mentioned (1) although it is preferred that the reaction is conducted after converting the compound (XIII) to an acid addition salt(s). With regard to the acid, it is particularly preferred to use organic acid such as acetic acid, trifluoroacetic acid and p-toluenesulfonic acid.

### 2. Preparation of the compound where Z is a single bond and n is 2.

The compound (I'') where Z is a single bond and n is 2 may be manufactured according to the reactions as shown in the following formulae. 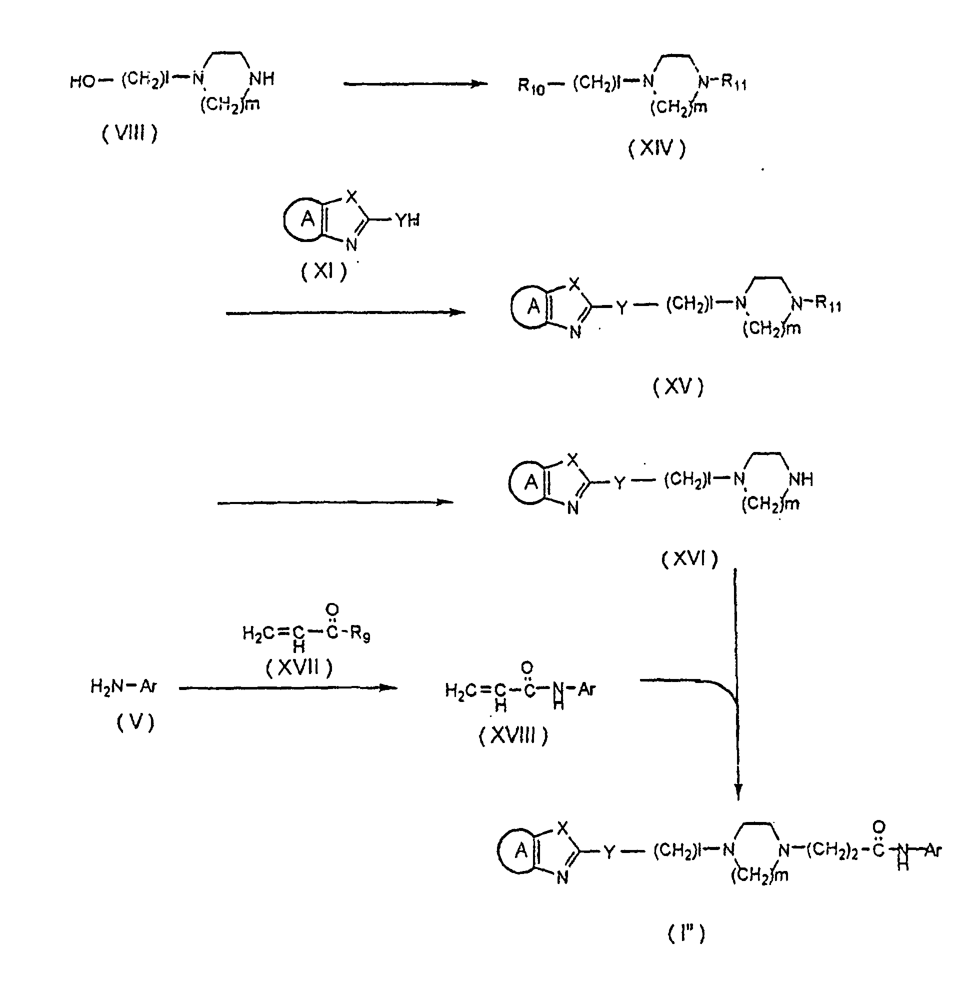 (In the formulae, R₉ is a residue of a reactive derivative of carboxyl group or hydroxyl group; R₁₀ is a leaving group; and R₁₁ is a protective group for amino group.)

After the secondary amine of the compound (VIII) is protected with a group R₁₁, the hydroxyl group is substituted with a leaving group such as a sulfonyl group whereupon the compound (XIV) is obtained. With regard to a protective group for amino group, tert-butoxycarbonyl group is preferred. When a method similar to that for the fourth step in (1) in the above-mentioned 1 is applied to the resulting N-protected compound, a compound (XV) is obtained and, when that is deprotected using, for example, an acid, a compound (XVI) is obtained.

On the other hand, when the compound (V) is made to react with the compound (XVII) or a reactive derivative of carboxylic acid, the compound (XVIII) is obtained. This reaction may be conducted in a similar manner as in the reaction for the first step of (1) of the above-mentioned 1.

When the resulting compound (XVI) and compound (XVIII) are subjected to a Michael reaction in a solvent, the desired compound (I'') is obtained. With regard to a solvent, alcohols such as methanol, ethanol and propanol as well as N,N-dimethylformamide, pyridine, and so on may be used either solely or jointly. The reaction condition may vary depending upon the materials used but, usually, the reaction is conducted at 0-100°C or, preferably, at around the boiling point of the solvent for 1-100 hours or, preferably, 24-72 hours whereupon the desired product can be obtained in a good yield.

### 3. Method for the preparation of a compound (I''') where Z is a group -NR₂-.

The compound represented by the formula (I) where Z is a group -NR₂- may be manufactured by various methods such as by the method as shown in the following reaction formulae. 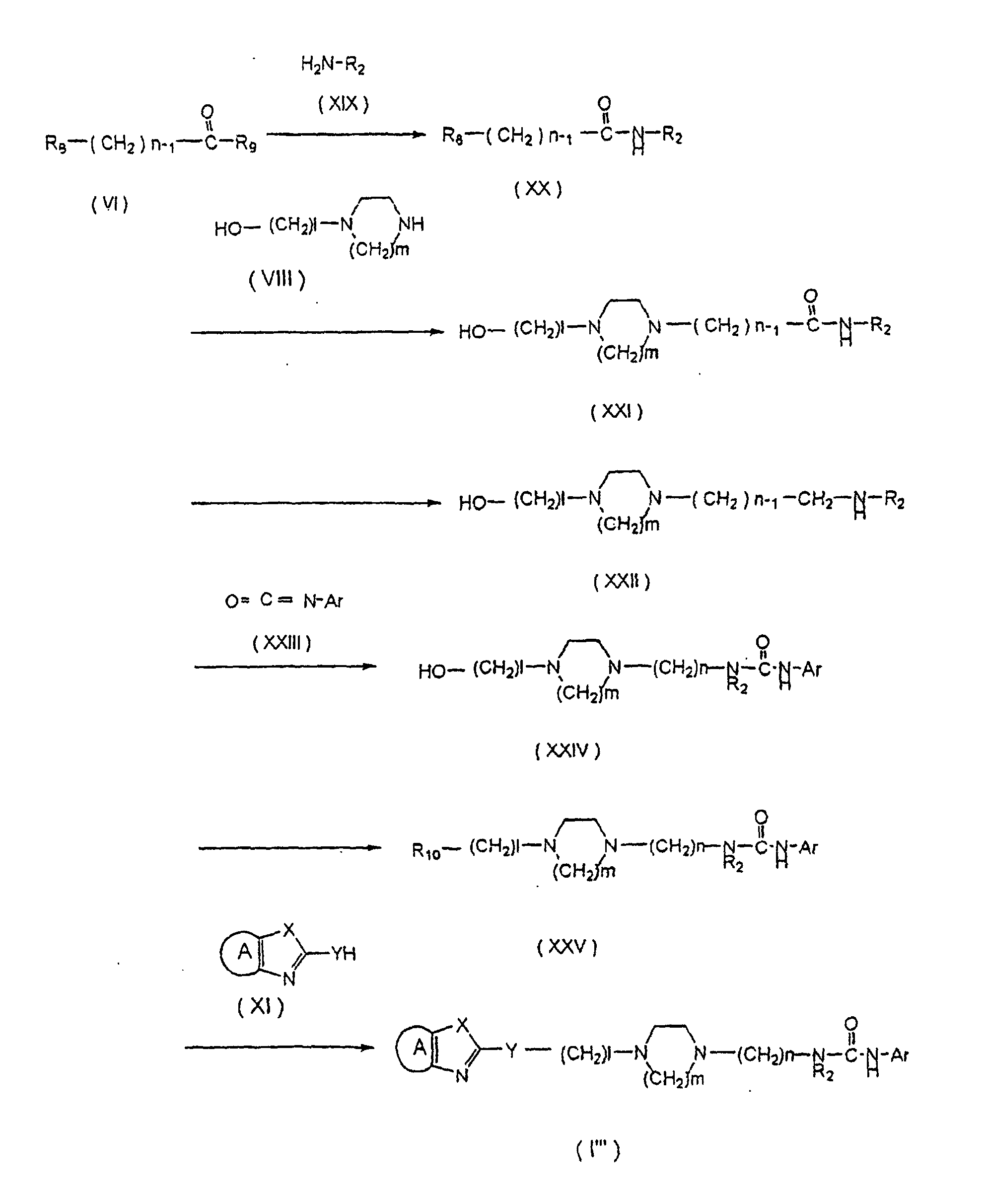 (In the formulae, R₉ is a residue of a reactive derivative of carboxyl group or hydroxyl group; R₈ is a leaving group; and R₁₀ is a leaving group.)

When the compound represented by the formula (XIX) is made to react with a carboxylic acid represented by the formula (VI) or a reactive derivative thereof such as an acid halide, an amide derivative represented by the formula (XX) is obtained. The reaction may be conducted in a similar manner as in the first step of (1) of the above-mentioned 1.

When an amide compound represented by the formula (XX) is made to react with a cyclic diamine-alcohol compound represented by the formula (VIII), an amide derivative represented by the formula (XXI) is obtained. The reaction may be conducted in a solvent in the presence or absence of a base. With regard to the solvent, N,N-dimethylformamide (DMF), acetonitrile, and so on are particularly preferred. With regard to the base, the use of inorganic bases such as alkali metal hydroxides such as sodium hydroxide and potassium hydroxide; alkali metal carbonates such as sodium carbonate and potassium carbonate; alkali metal bicarbonates such as sodium bicarbonate and potassium bicarbonate; and so on is preferred.

When the resulting compound (XXI) is reduced, a compound represented by the formula (XXII) is obtained.

The reaction may be conducted by the use of a hydrogenating reducing agent in a solvent. Examples of the applicable solvent are tetrahydrofuran, ether and toluene while examples of the applicable reducing agent are lithium aluminum hydride, aluminum hydride and sodium aluminum bis(2-methoxyethoxy) hydride.

When the resulting amide compound (XXII) is made to react with an isocyan derivative represented by the formula (XXIII), an urea derivative represented by the formula (XXIV) is obtained. The desired compound (XXIV) is obtained when both compounds are made to react in a solvent at 0-100°C or, preferably, at around room temperature for several minutes to several hours. Examples of the applicable solvent are tetrahydrofuran, ether, hexane, methylene chloride, chloroform, carbon tetrachloride, toluene, N,N-dimethylformamide and acetonitrile.

When the hydroxyl group of the resulting urea derivative represented by the formula (XXIV) is sulfonated and the compound (XXV) obtained thereby is made to react with a compound represented by the formula (XI), the desired compound (I" ') where Z is -NR₂- is obtained. Methods similar to that for the third step and the fourth step of (1) of the above-mentioned 1 can be applied to the reaction.

### 4. Methods for the preparation of the compound (I) having substituent(s) on Ar by other methods.

Besides the above-mentioned methods, there is a method where the main skeleton of the compound of the present invention is manufactured and then each substituent at the side of azole or of aryl group is manufactured. Known methods may be used for producing those substituents and some of them will be exemplified hereunder.

### (1) Method for the preparation of a compound having N-lower alkylamino group on Ar.

Among the compound (I), that having N-alkylamino group on Ar can be manufactured as follows. Thus, a nitro group of the compound (I) having a nitro group on Ar is reduced and the resulting amine compound is subjected to an N-alkylation.

Reduction of nitro group can be conducted by a hydrogenation in an inert solvent such as ethyl acetate or ethanol using palladium-carbon or Raney nickel as a catalyst. Alternatively, reduction using metal such as zinc, tin or iron in an acidic medium such as acetic acid or hydrochloric acid is also able to convert nitro group to amino group.

N-alkylation of the amine compound can be conducted by the reaction of the amine compound with alkyl sulfate, alkyl halide, and so on in a solvent in the presence of a base and each of the resulting N-mono and dialkyl compounds can be isolated from a resulting mixture thereof. Especially in the case of a compound having N,N-dimethylamino group, it can be prepared by subjecting the amino compound to Clarke-Eschweiler reaction. Thus, the amino compound is made to react with formaldehyde, the resulting Schiff's base is reduced, and the resulting N-monomethylamino compound is further made to react with formaldehyde followed by reducing to provide a compound having N,N-dimethylamino group.

Examples of the base which is applicable for the N-lower alkylation are alkali metal bicarbonate such as sodium bicarbonate and potassium bicarbonate; alkali metal carbonates such as sodium carbonate and potassium carbonate; alkali metal hydroxides such as sodium hydroxide and potassium hydroxide; metal hydrides such as sodium hydride; alkali metal alkoxides such as sodium ethoxide and potassium butoxide; and organic bases such as pyridine, triethylamine, N,N-diisopropyl ethylamine, N-methylmorpholine and N,N-dimethylaniline.

With regard to a solvent, acetone, dimethyl sulfoxide, N,N-dimethylformamide, tetrahydrofuran and a mixed solvent thereof are preferred. The reaction is completed for 0.1-20 hours at 20-150°C or, preferably, for 1-5 hours at 50-130°C.

With regard to the solvent used in Clarke-Eschweiler reaction, acetonitrile, N,N-dimethylformamide, formic acid, acetic acid and a mixed solvent thereof are preferred. With regard to a reducing agent, formic acid which acts as a solvent as well may be used or sodium cyanoborohydride may be used in an acidic solvent.

### (2) Method for the preparation of a compound having lower alkylthio group on Ar.

Among the compound (I), that which has alkylthio group on Ar can be manufactured by diazotizing a compound (I) having amino group on Ar followed by subjecting to a reaction with di-alkyl disulfide or thioalkoxide. To be more specific, the corresponding amine compound is diazotized with amyl nitrite or the like in a solution of di-alkyl disulfide followed by decomposing by heating (Japanese Laid-Open Patent Publication Hei-10/025281) or the amine compound is diazotized with sodium nitrite in an acidic solution followed by heating in an aqueous solution of sodium lower thioalkoxide.

### (3) Method for the preparation of a compound having alkylcarbonyloxy group, alkoxy group, alkylsulfonyloxy group or di-alkoxyphosphoryloxy group on Ar.

The compounds (I) having alkylcarbonyloxy group, alkoxy group, alkylsulfonyloxy group or di-alkoxyphosphoryloxy group on Ar can be manufactured as follows. Thus, the corresponding amine compound in the compound (I) is diazotized, heated in an aqueous solution to convert to hydroxyl compound and the compound is then subjected to acylation, alkylation, alkylsulfonylation or di-alkoxyphosphorylation.

Hydroxy compound can be manufactured by diazotizing the corresponding amine compound in a diluted sulfuric acid solution with sodium nitrite followed by pouring the aqueous solution of the resulting diazonium salt (s) into boiling water and by decomposing by heating.

Alkylcarbonyloxy compound can be manufactured by the reaction of the corresponding hydroxy compound with acid halide, acid anhydride, and so on in a solvent in the presence of a base. To be more specific, it can be manufactured by the reaction of a hydroxy compound with acetyl chloride in a pyridine solvent.

Alkoxy compound can be manufactured by the reaction of the corresponding hydroxy compound with alkyl sulfate, alkyl halide, and so on in a solvent in the presence of a base. To be more specific, it can be manufactured by the reaction of a hydroxy compound with sodium hydride in a dimethylformamide solvent followed by subjecting to a reaction with alkyl halide. Alternatively, it can be manufactured by the reaction of a hydroxy compound with diazomethane, trimethylsilyl diazomethane, and so on in a solvent in the presence of a base as well.

Alkylsulfonyloxy compound can be manufactured by the reaction of the corresponding hydroxy compound with alkyl sulfonic acid halide, alkyl sulfonic acid anhydride, and so on in a solvent in the presence of a base. To be more specific, it can be manufactured by the reaction of a hydroxy compound with methanesulfonyl chloride in a solvent in the presence of triethylamine.

Di-alkoxyphosphoryloxy compound can be manufactured by the reaction of the corresponding hydroxy compound with di-alkyl phosphoric acid halide in a solvent in the presence of a base. To be more specific, it can be manufactured by the reaction of a hydroxy compound with sodium hydride in a dimethylformamide solvent followed by subjecting to a reaction with dialkylphosphoric acid chloride.

### (4) Method for the preparation of a compound having hydroxyl group and nitro group on Ar.

The compound (I) having hydroxyl group and nitro group on Ar can be manufactured by the reaction of a hydroxy compound with nitric acid or acetyl nitrate in an inert solvent. To be more specific, it can be manufactured by the reaction of a hydroxy compound in an acetonitrile solvent with acetyl nitrate.

Intermediates and desired compounds obtained in the above reactions can be, if necessary, isolated and purified by means of purifying methods which have been commonly used in organic synthetic chemistry such as filtration, extraction, washing, drying, concentration, recrystallization and various chromatographic means. With regard to intermediates, they may be subjected to the next reaction without particular purification.

The resulting compound (I) can be made into an acid addition salt(s) by a common method. It can also be prepared as solvate(s) with a solvent for the reaction or for the recrystallization, especially as a hydrate.

Specific examples of the compounds obtained by the above-mentioned methods are shown in Tables 1-19.

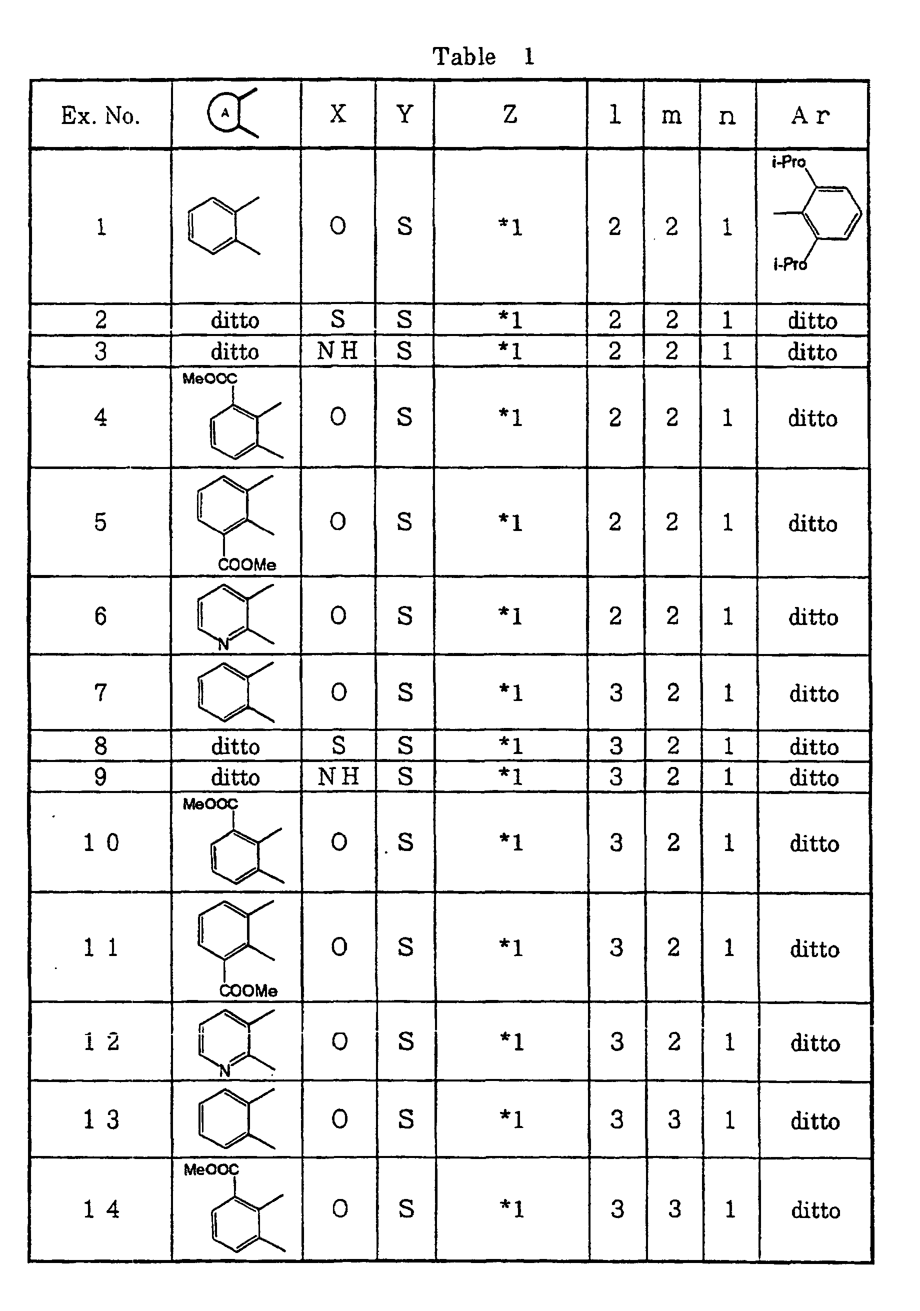

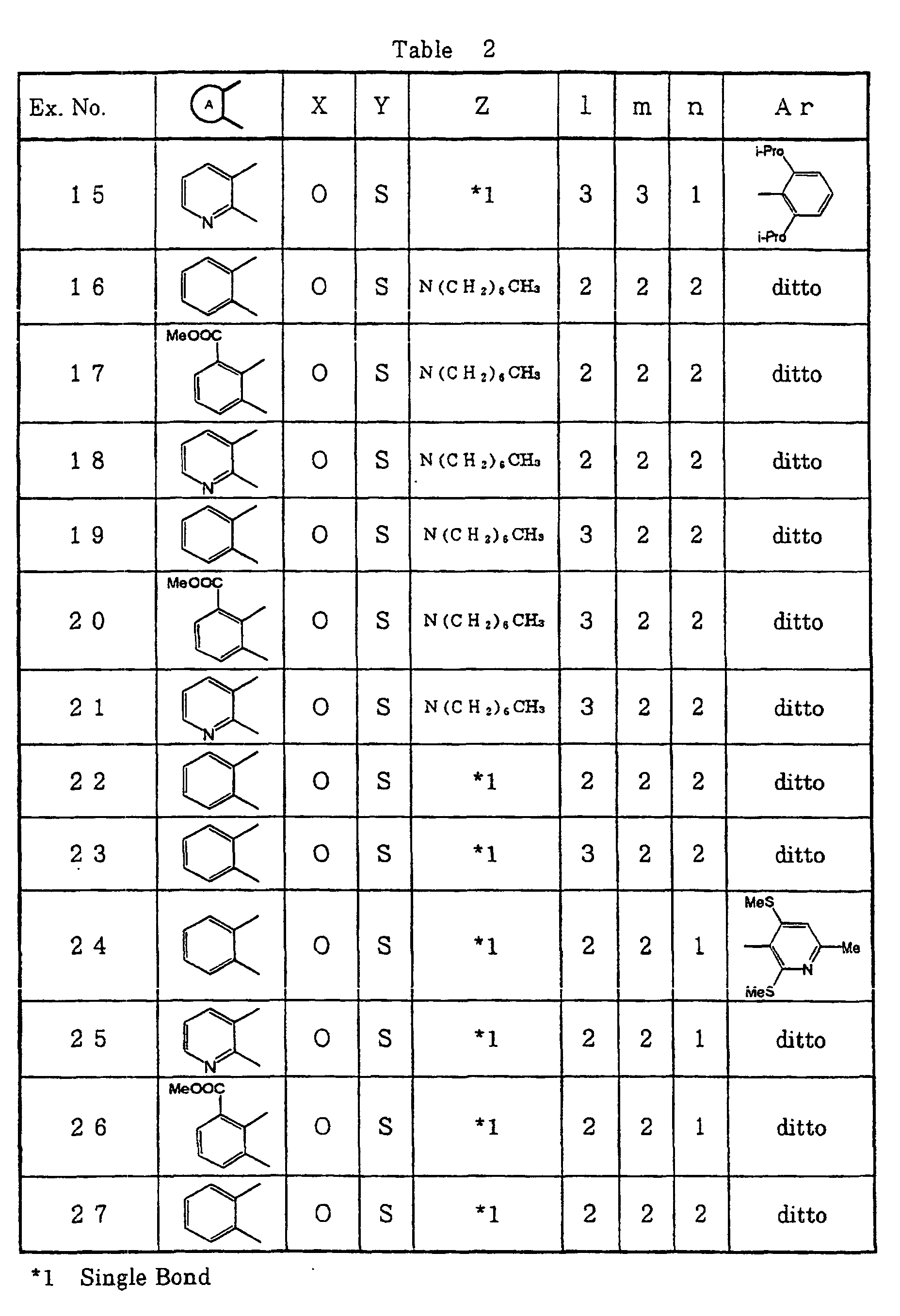

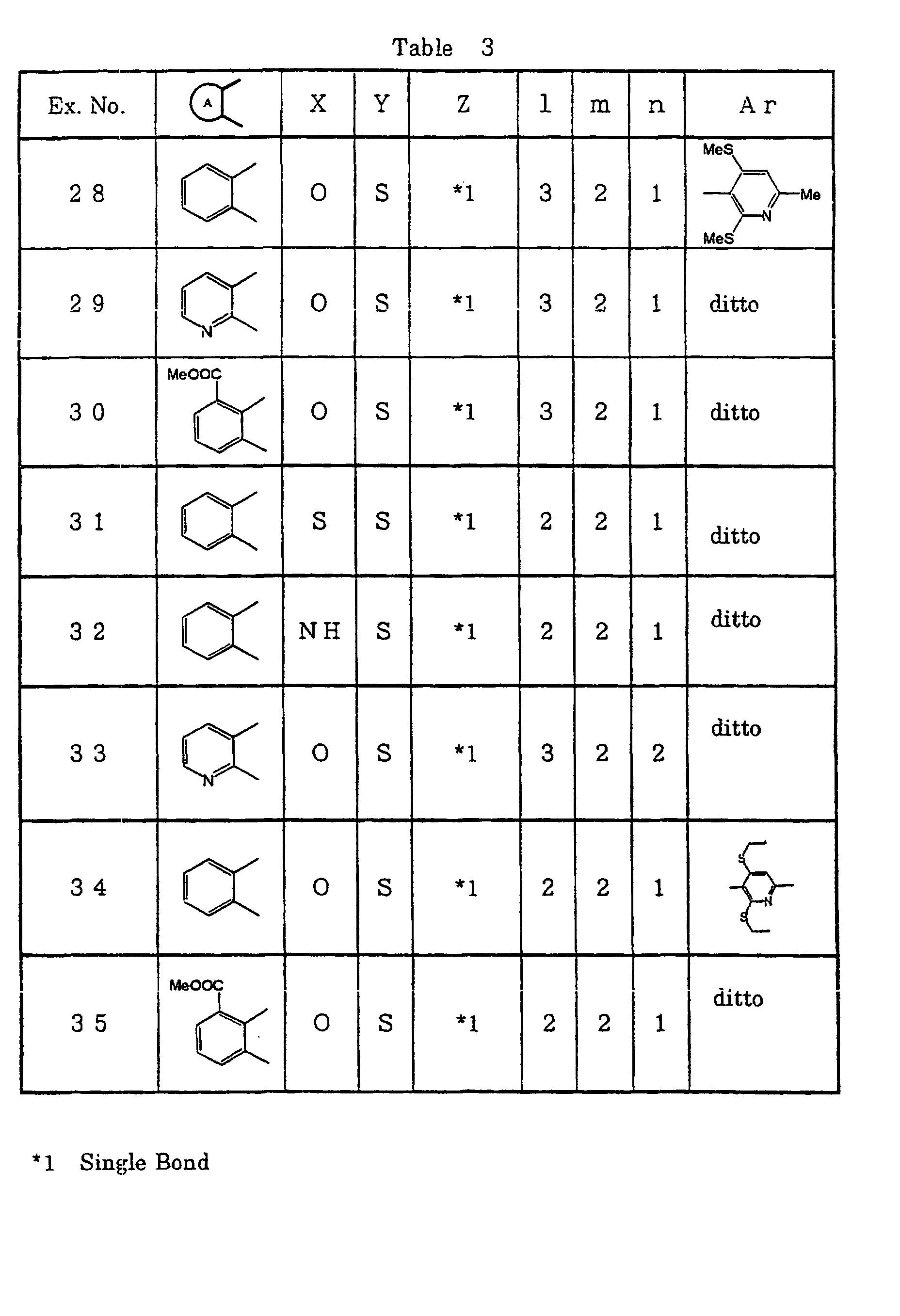

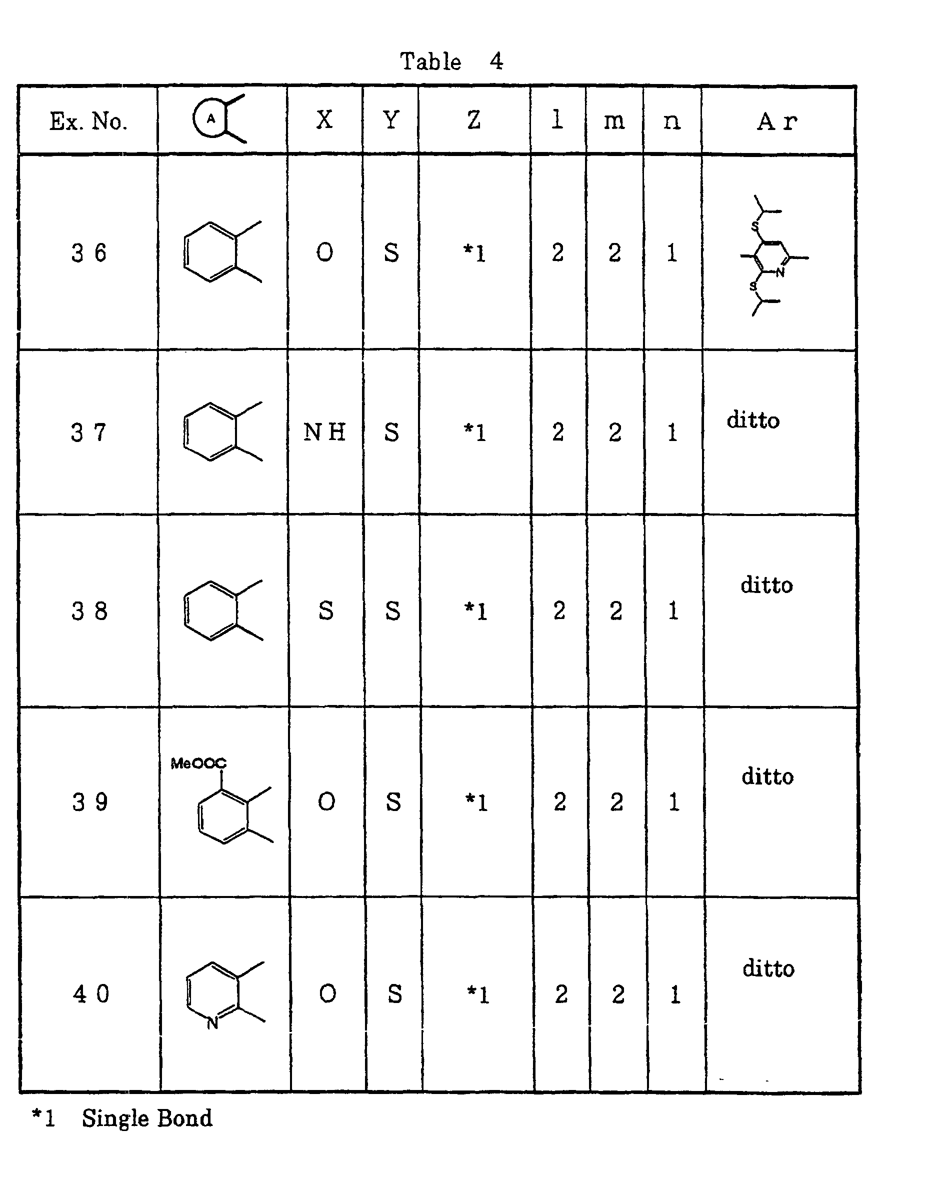

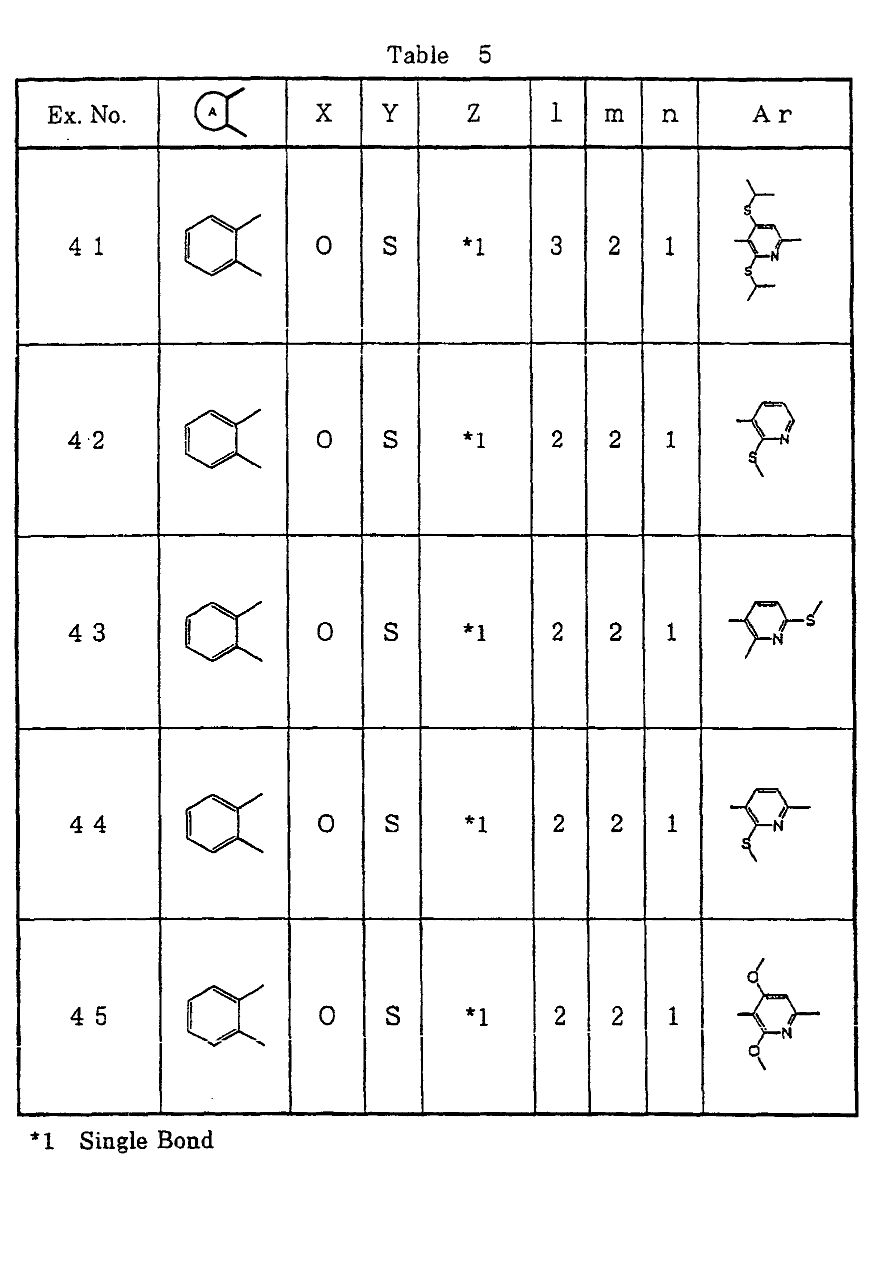

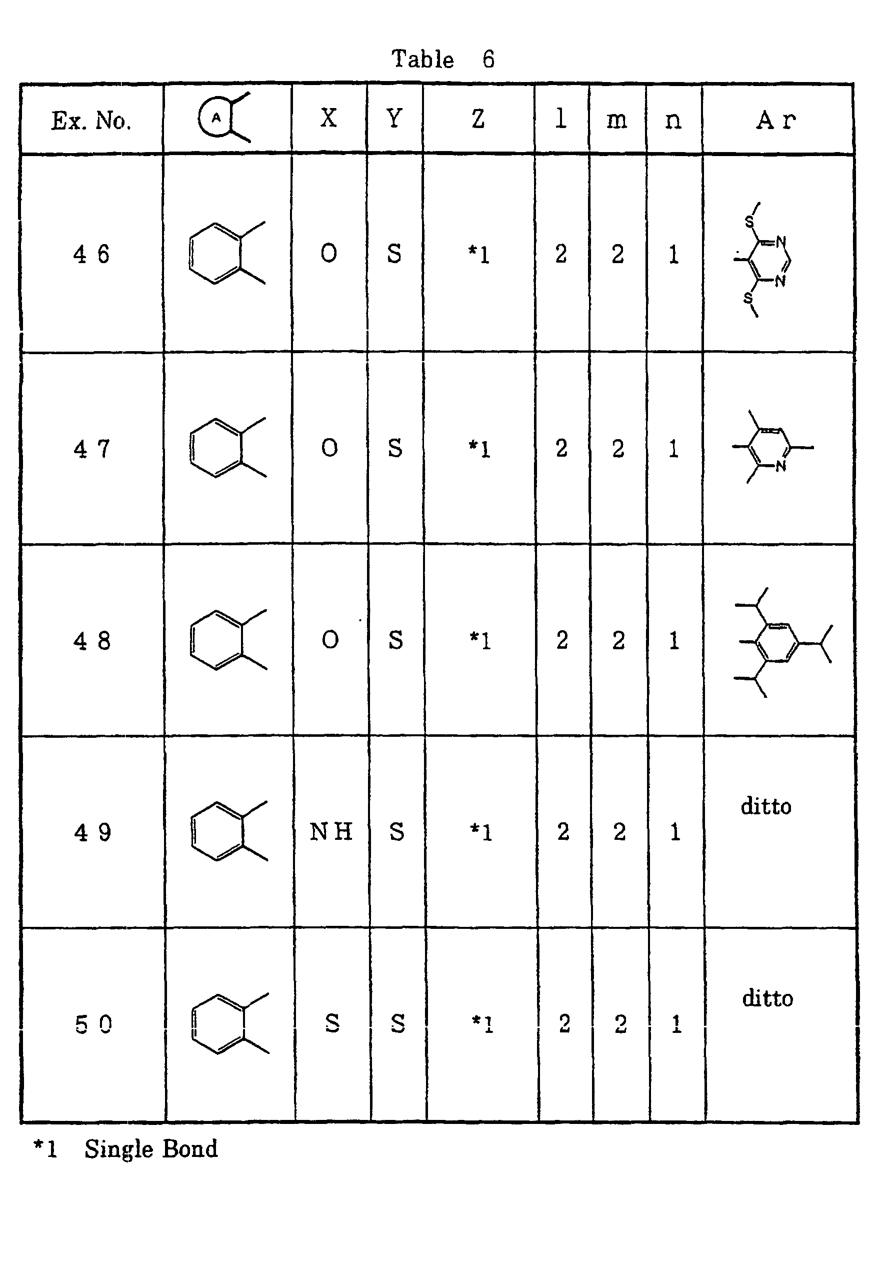

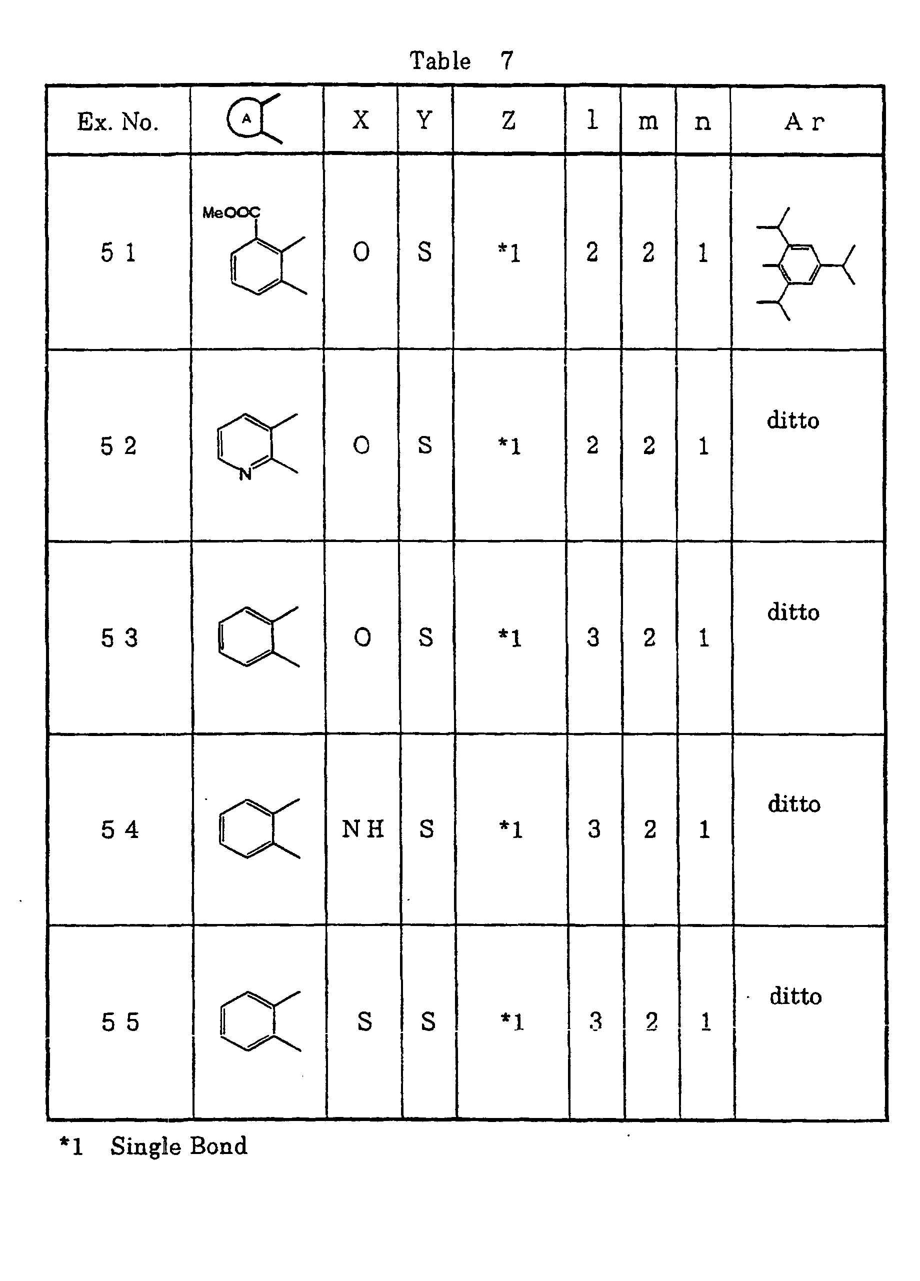

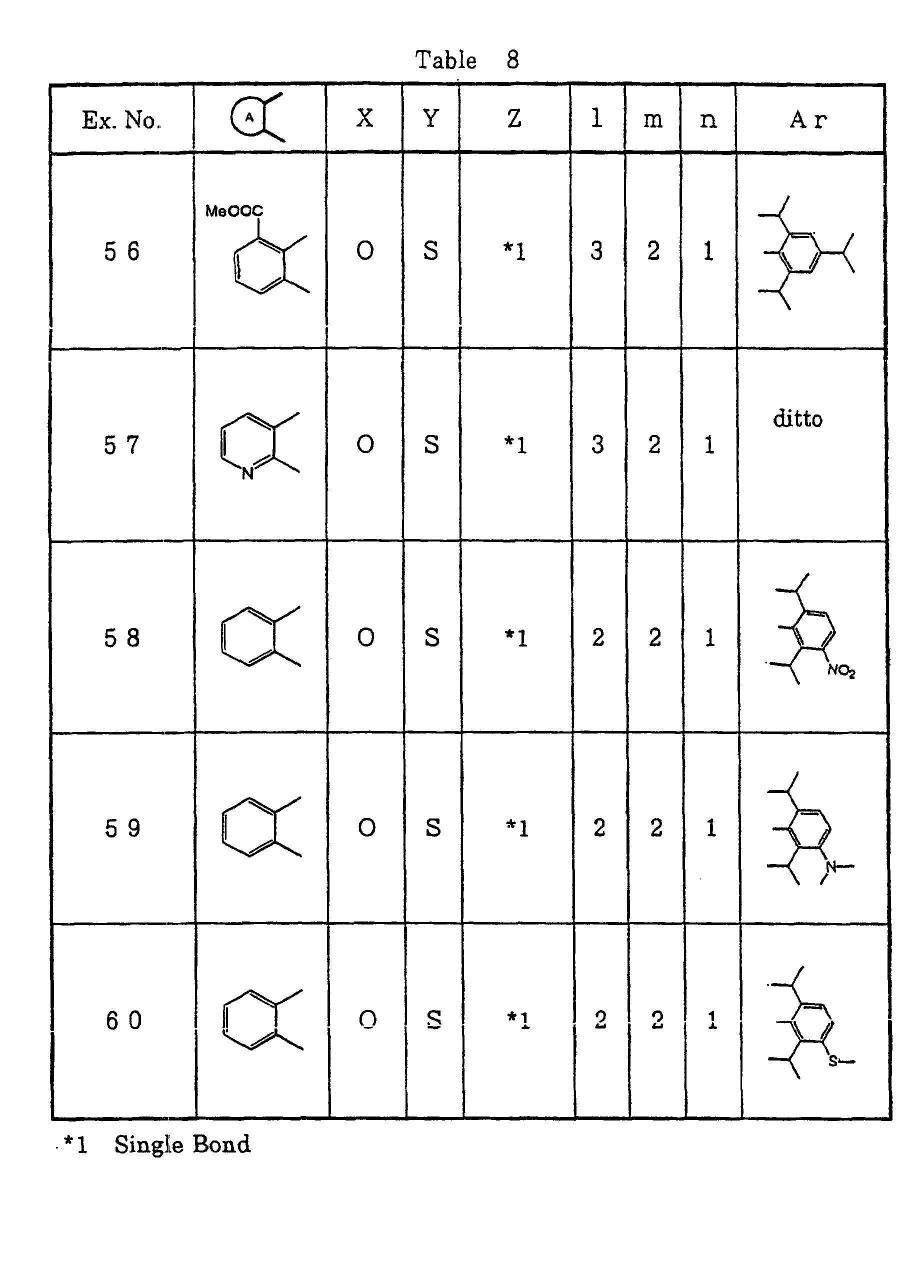

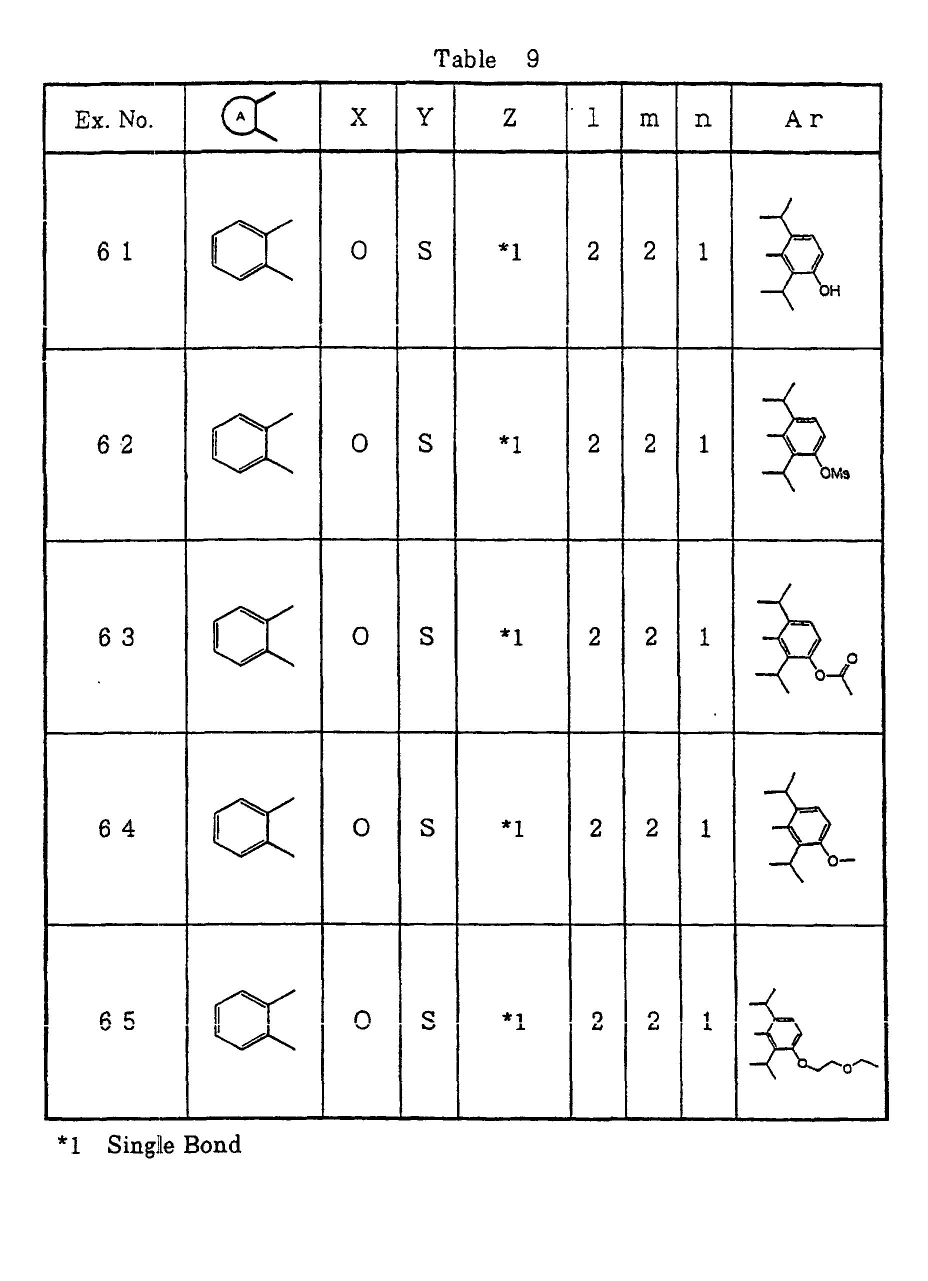

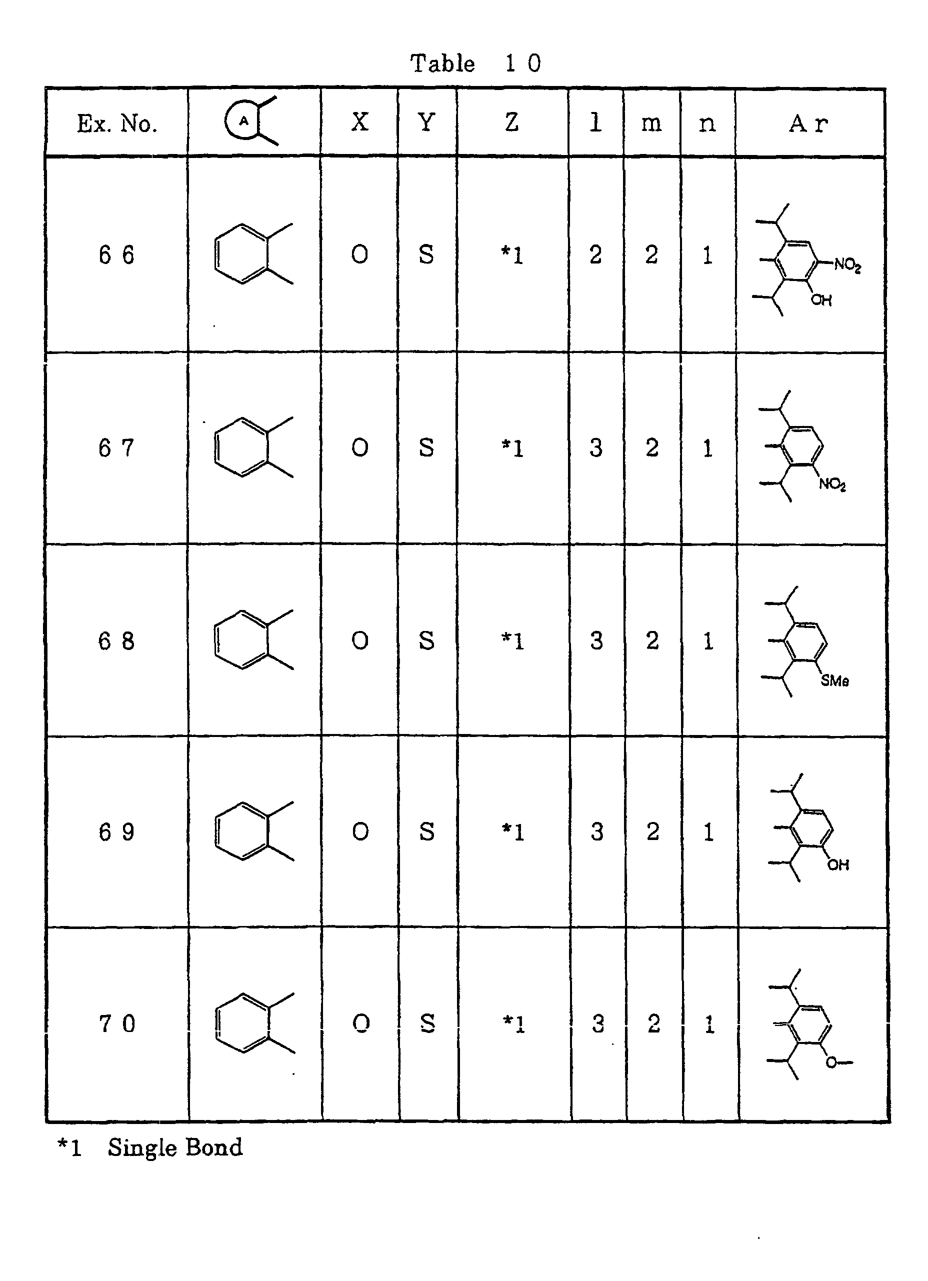

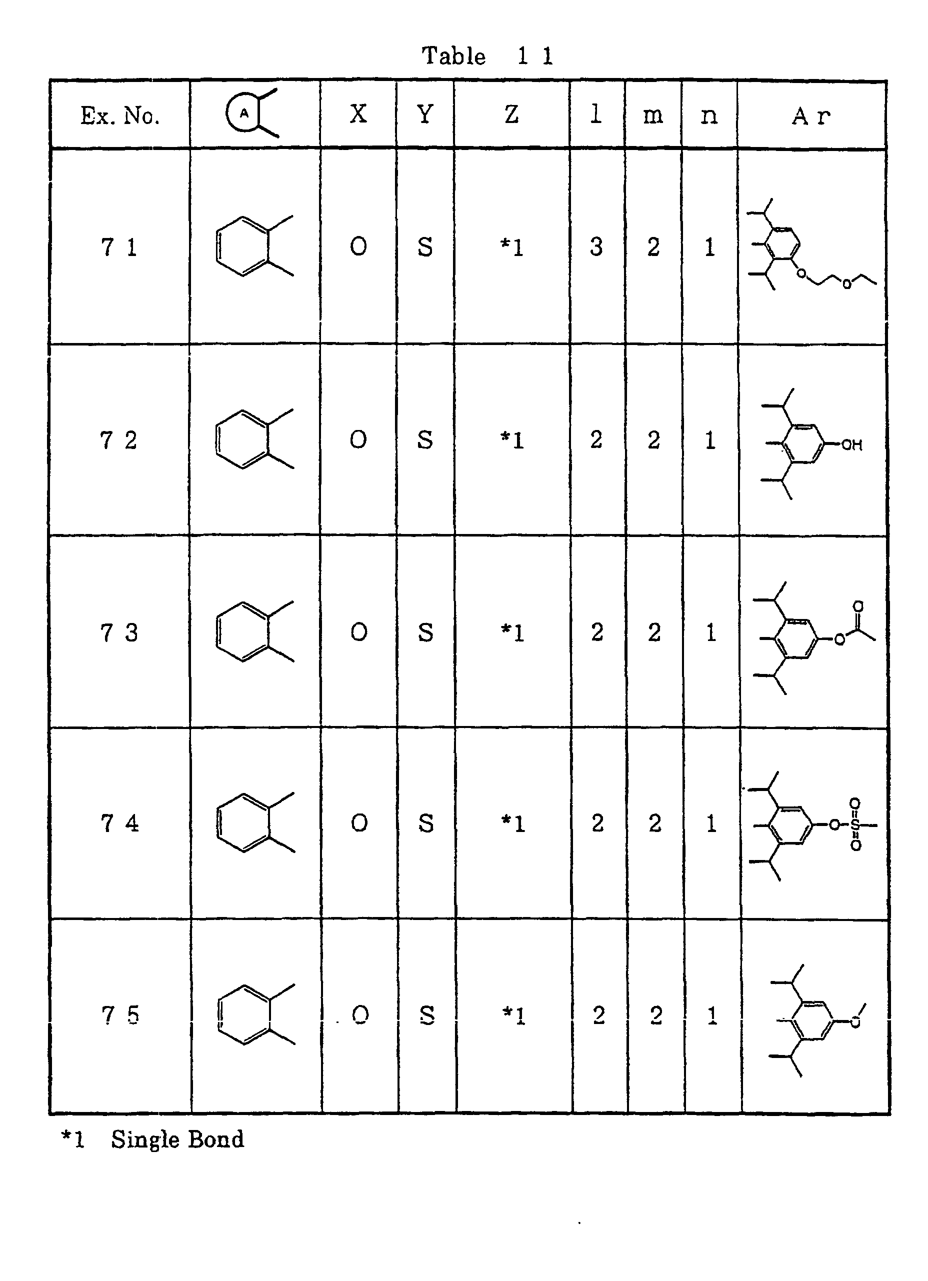

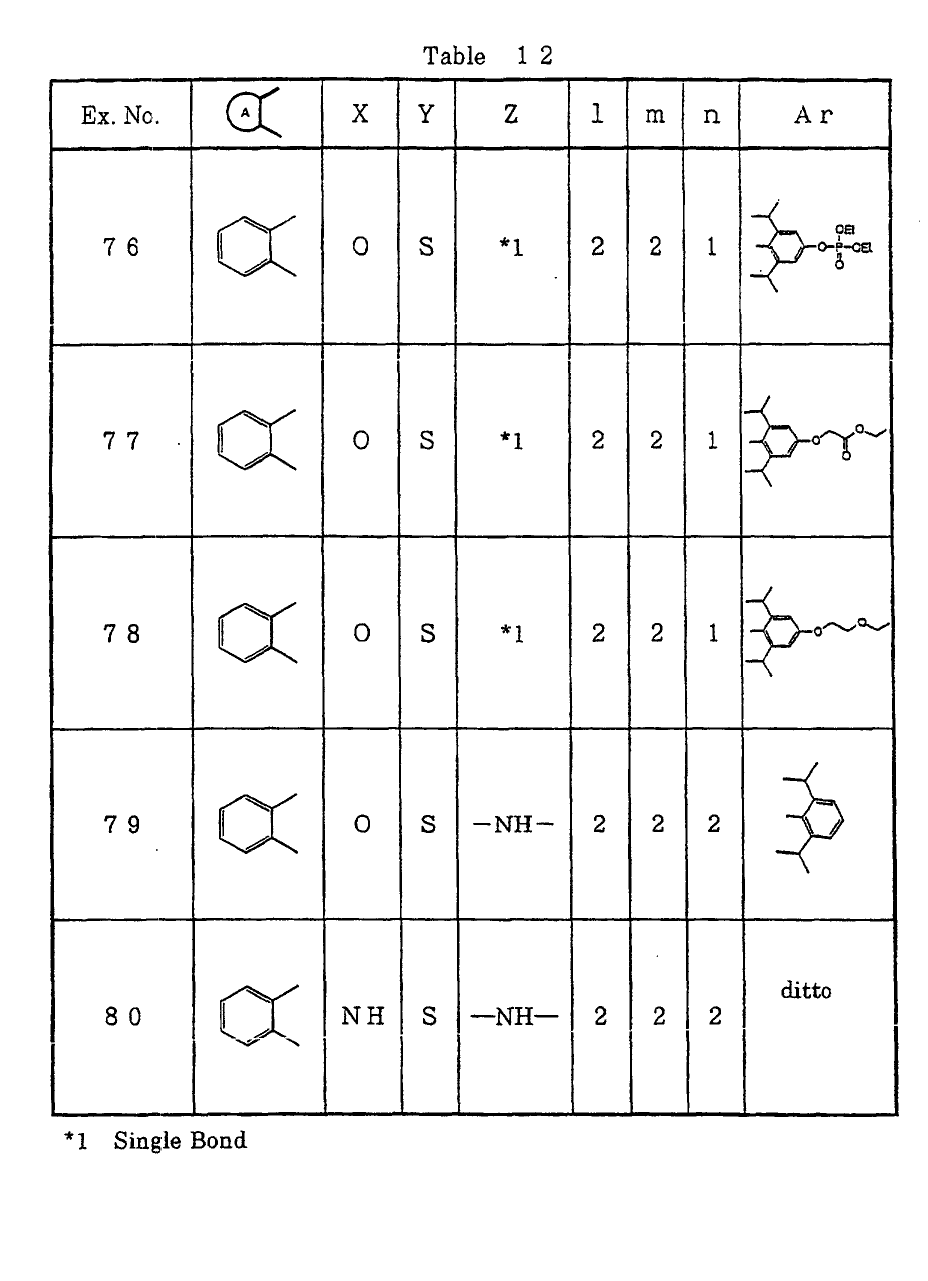

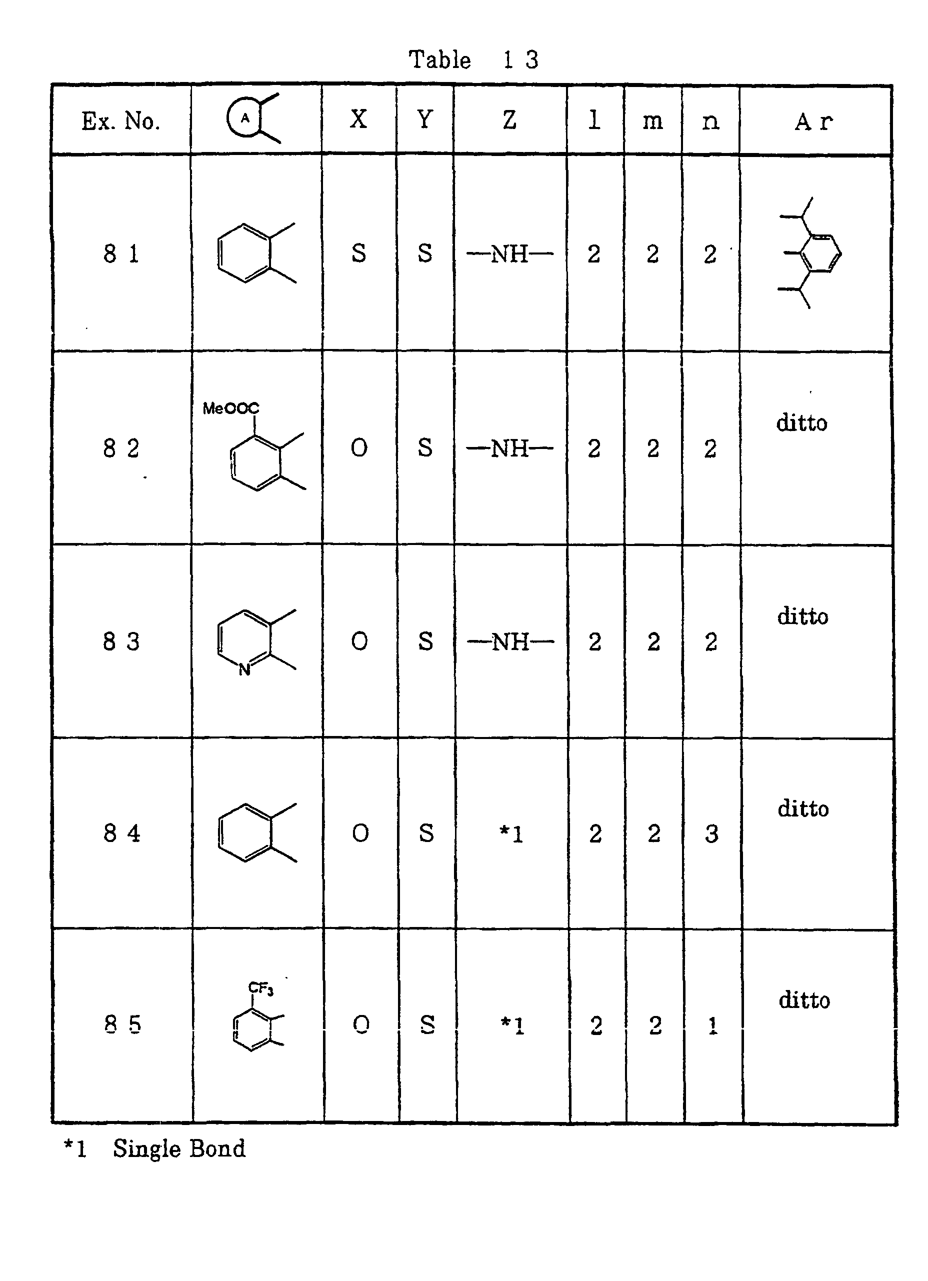

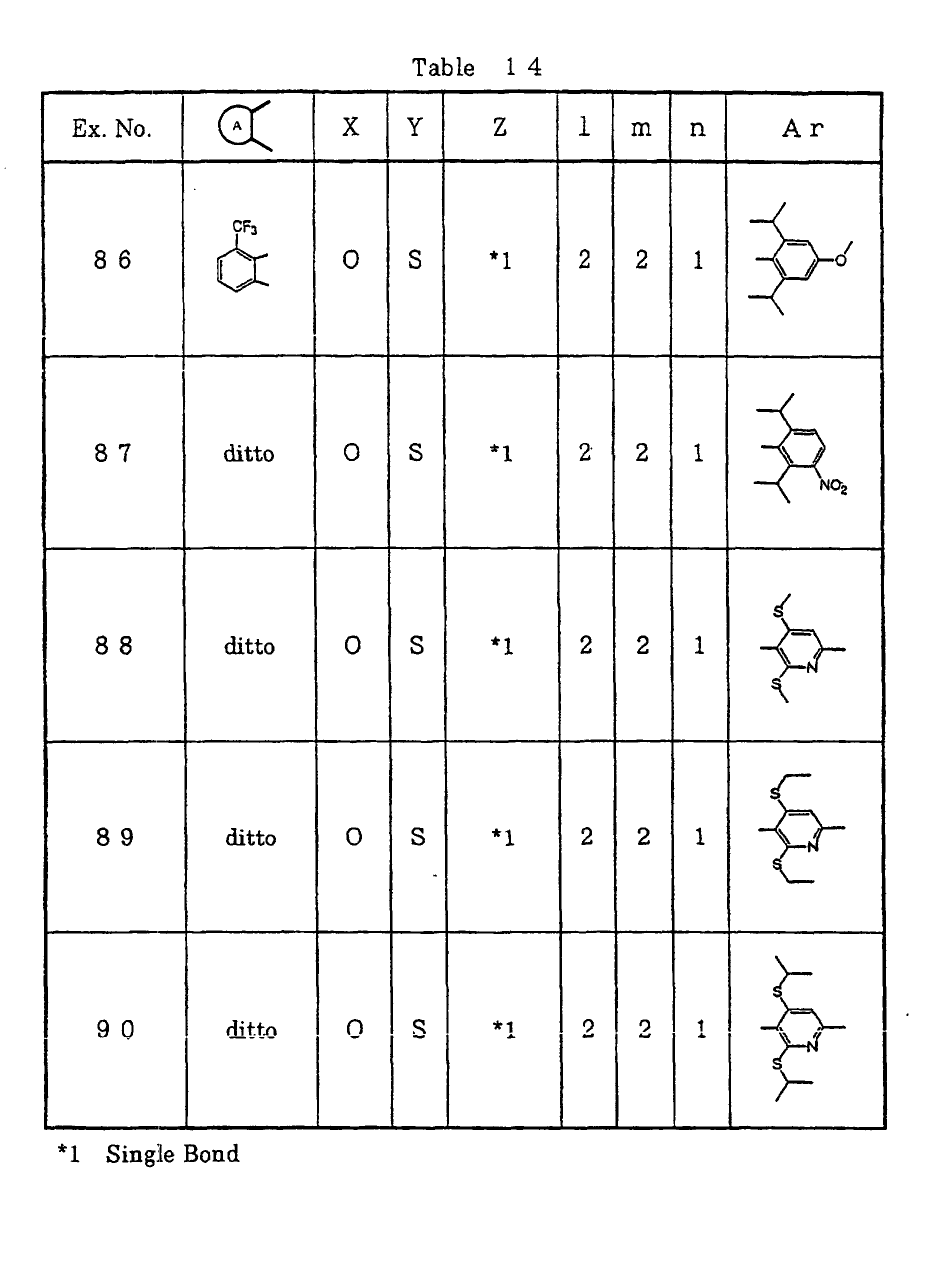

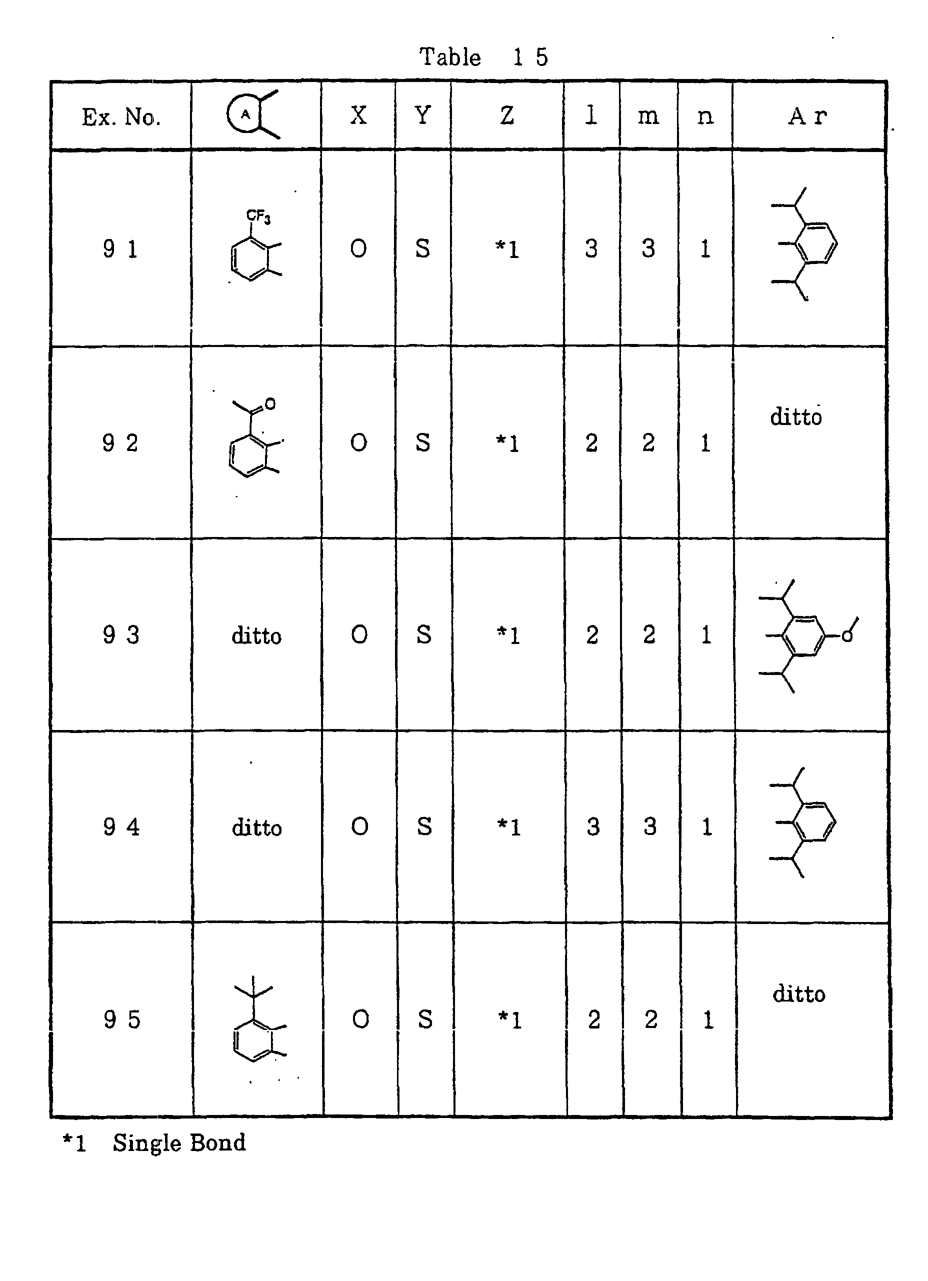

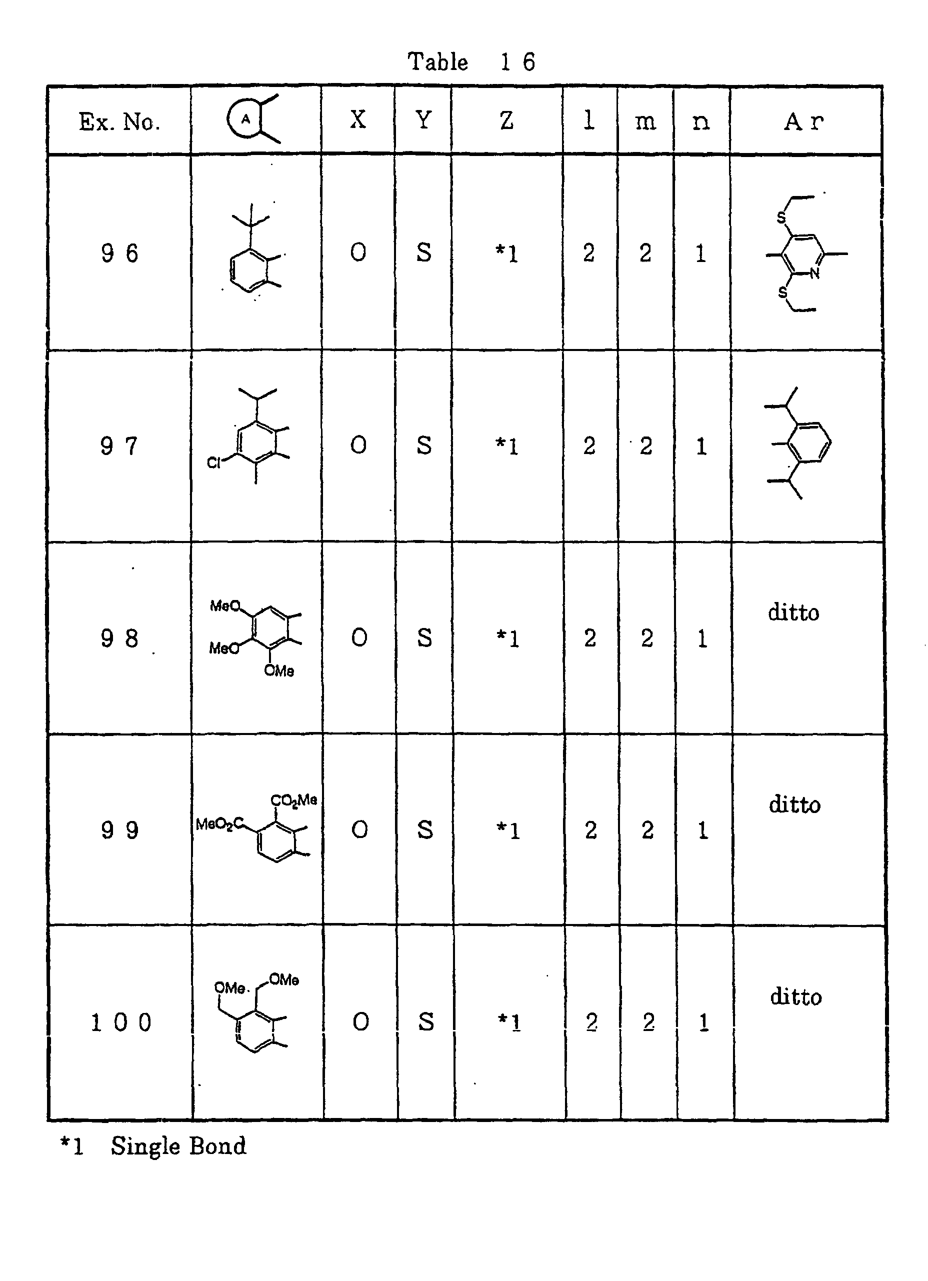

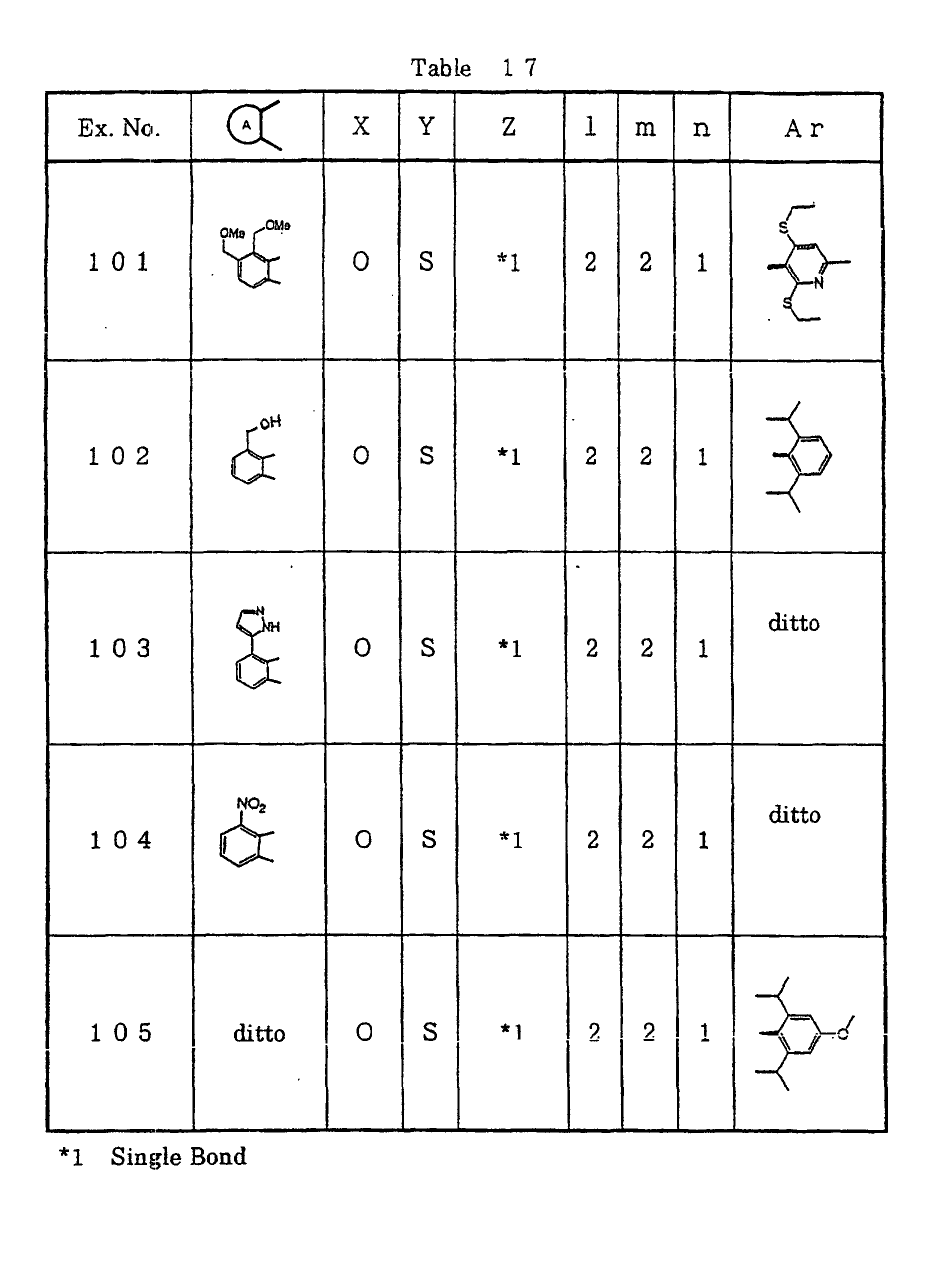

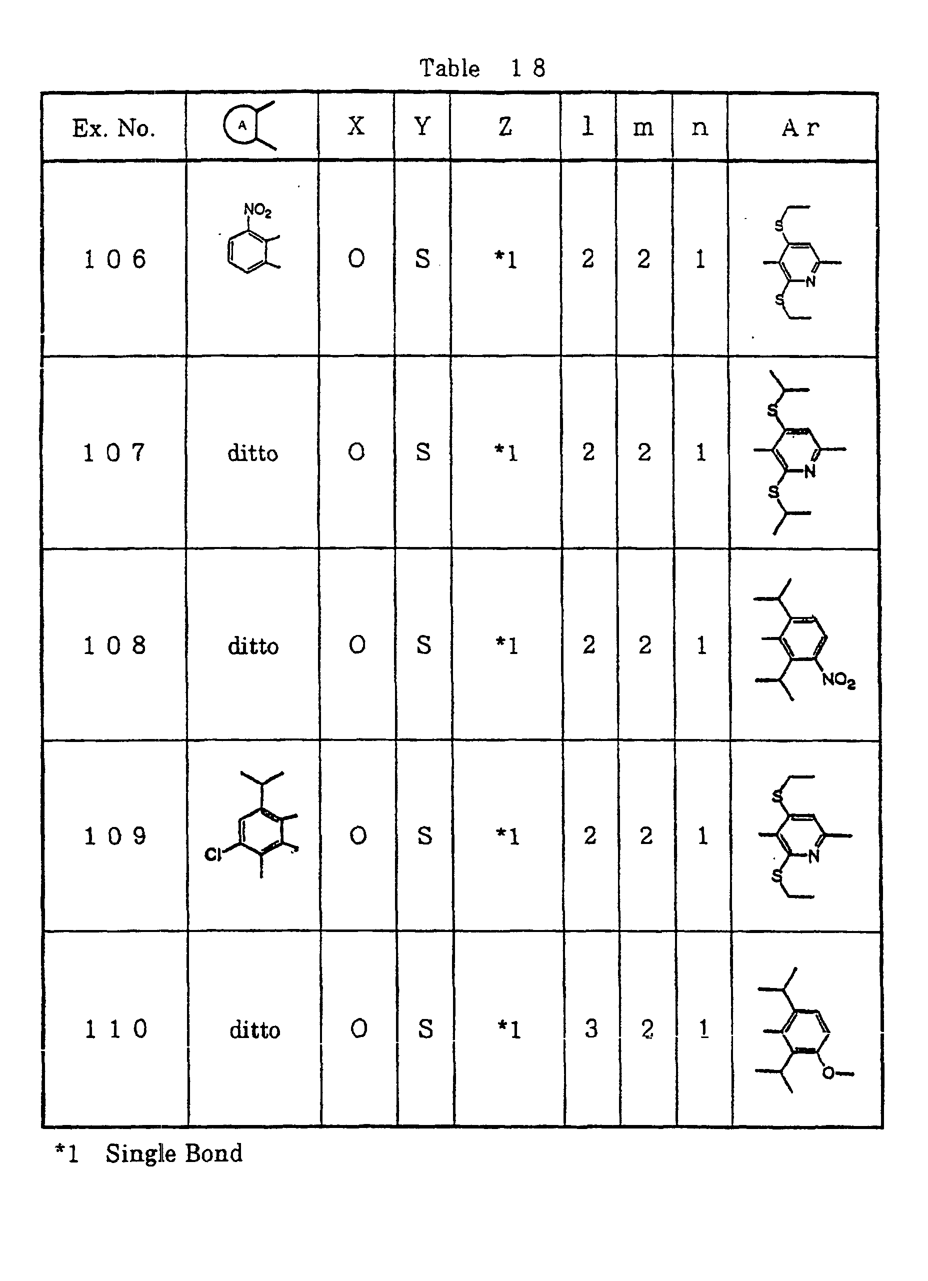

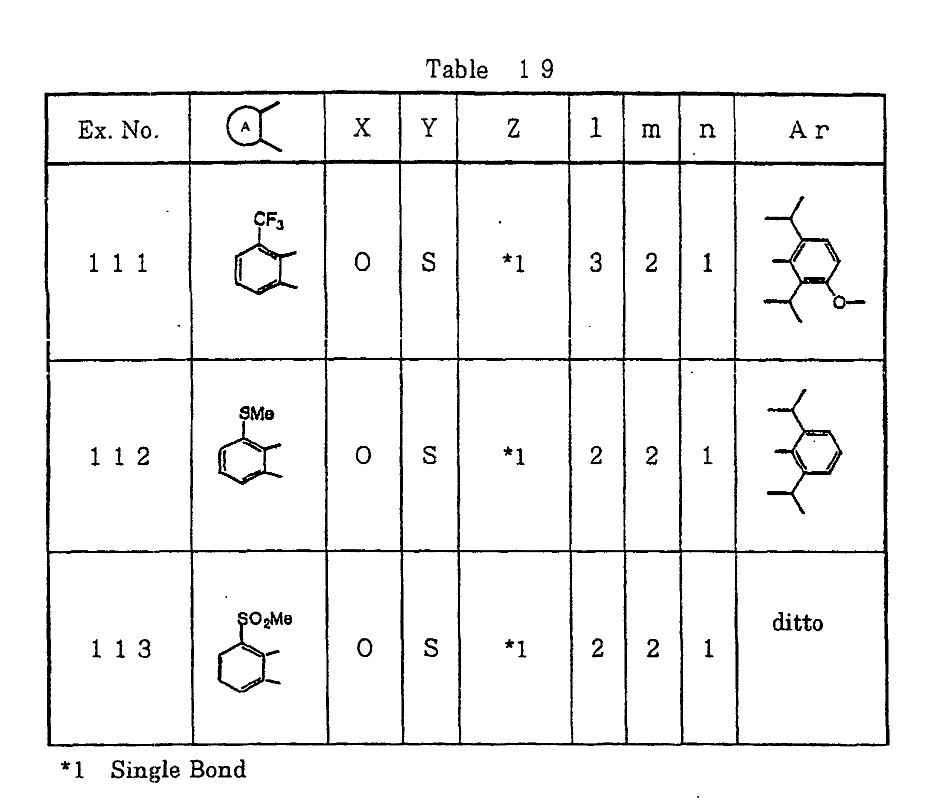

The compounds of the present invention represented by the formula (I) have an ACAT inhibitory action and/or intracellular cholesterol transportation inhibiting action and are useful in a medical field as therapeutic agents for hyperlipemia and for arteriosclerosis. Particularly since the compounds of the present invention show an action of selectively inhibiting the ACAT enzyme of a type existing on blood vessel wall, they have less side effect as compared with nonselective ACAT inhibitors and, further since they are soluble in water, they are expected to have improved oral absorption and are preferred as effective ingredients of pharmaceuticals.

A pharmaceutical composition of the present invention contains the compound represented by the formula (I) or acid addition salt(s) or solvate(s) thereof as an effective ingredient and the effective ingredient can be made into a dosage form such as tablets, capsules, granules, powder, injection and suppositories either as it is or together with other pharmaceutically acceptable fillers, binders, diluents, and so on

Those preparations can be manufactured by a known method. For example, in the preparation of a preparation for oral administration, it can be manufactured by formulating the compound of the formula (I) by means of an appropriate compounding with fillers such as mannitol and lactose; binders such as sodium carboxymethyl cellulose and hydroxypropyl cellulose; disintegrating agents such as crystalline cellulose and calcium carboxymethyl cellulose; lubricants such as talc and magnesium stearate; fluidization improving agents such as light silicic acid anhydride; and so on

The pharmaceutical composition of the present invention can be administered by means of either oral administration or parenteral administration.

Dose of the pharmaceutical composition of the present invention varies depending upon body weight, age, sex, symptom, and so on of the patient and, usually in the case of adults, it is preferred that 1-100 mg/day or, preferably, 5-200 mg/day as a compound represented by the formula (I) is administered once to three times a day.

ACAT inhibitory action, and so on of the compounds of the present invention represented by the formula (I) were tested by the following experimental examples.

### Experimental Example 1.

### ACAT inhibitory Action.

Microsome was prepared by a conventional method from stethartery of rabbits fed for eight weeks with a 1% cholesterol feed and was suspended in a 0.15M phosphate buffer (pH 7.4) to provide an enzyme solution. An enzyme solution derived from small intestine was prepared from small intestine or rabbits fed with normal feed. Measurement of ACAT inhibitory activity was conducted by modifying a method of J. G. Hider (J. Lipid Res., 24, 1127-1134, 1983). Thus, 2 µl of a test compound dissolved in dimethyl sulfoxide (DMSO) was added to 88 µl of a 15M phosphate buffer (pH 7.4) containing ¹⁴C-oleoyl-CoA (40 µM, 60,000 dpm) and bovine serum albumin (2.4 mg/ml) and incubated at 37°C for five minutes. An enzyme solution (10 µl) was added to this liquid and made to react at 37°C for five minutes (or three minutes in the case of small intestine), the reaction was stopped by adding 3 ml of chloroform/methanol (2/1) and 0.5 ml of 0.04N hydrochloric acid and lipid was extracted therefrom. The solvent layer was concentrated to dryness, the residue was dissolved in hexane and the solution was spotted on a TLC plate (manufactured by Merck) followed by developing with hexane : ether : acetic acid (75:25:1). Radioactivity of the resulting cholesterol ester fraction was measured by a BAS 2000 (manufactured by Fuji Photo Film) and IC₅₀ values were determined from a comparative calculation with the control to which only DMSO was added. The result is shown in Table 20.

**Table 20**

| Compound (Ex. No.) | Enzyme from A* IC₅₀(µM) | Enzyme from B* IC₅₀(µM) | IC₅₀ (B) /IC₅₀ (A) |
|---|---|---|---|
| 1 | 0.024 | 0.045 | 1.9 |
| 2 | 0.021 | 0.045 | 2.1 |
| 3 | 0.011 | 0.051 | 4.6 |
| 5 | 0.056 | 0.13 | 2.3 |
| 6 | 0.11 | 0.32 | 2.9 |
| 7 | 0.019 | 0.039 | 2.1 |
| 10 | 0.035 | 0.039 | 1.1 |
| 25 | 0.12 | 0.21 | 1.8 |
| 75 | 0.038 | 0.21 | 5.5 |
| 78 | 0.040 | 0.21 | 5.3 |
| 85 | 0.012 | 0.059 | 4.9 |
| Control 1 | 0.45 | 0.87 | 1.9 |
| Control 2 | 0.047 | 0.13 | 2.8 |
| Control 3 | 0.034 | 0.056 | 1.7 |
| Control 4 | 0.026 | 0.037 | 1.4 |
| Control 5** | 0.004 | 0.021 | 4.8 |

| | | | |
|---|---|---|---|
| ^{*}In table, "A" means the blood vessel wall, and ^{"}B" means the small intestine. | | | |
| **The compound of Control 5 is corresponding to one disclosed in Example 8 of Japanese Patent Application No. 9-88660. | | | |

### Experimental Example 2.

### ACAT inhibitory Action (Anti-Foaming Action) in J774 Cells and HepG2 Cells.

J774 cells or HepG2 cells were planted on a 24-well plate and incubated at 37°C for 24 hours in a 5% CO₂ incubator using DMEM or MEM culture liquid (each containing 10% of fetal calf serum) for J774 cells or HepG cells, respectively. The medium was exchanged with 0.5 ml of each culture liquid containing 10 µg/ml of 25-OH cholesterol and sample followed by incubating for 18 hours more. The medium was removed and, after washing with PBS twice, extraction was conducted with 1.5 ml of hexane : isopropanol (3:2) followed by concentrating to dryness. The extract was dissolved in 0.2 ml of isopropanol containing 10% of 10% Triton X-100 and total cholesterol (TC) and free cholesterol (FC) were determined by a Cholesterol E Test Wako (Wako Pure Chemicals) and a Free Cholesterol E Test Wako (Wako Pure Chemicals), respectively. Residue of the cells after extraction was solubilized with 0.25 ml of 2N NaOH at 30°C for 30 minutes and amount of protein was determined by means of a BCA Protein Assay Reagent (Pierce). From the difference between TC and FC, amount of cholesterol ester per protein was calculated and then IC₅₀ was determined by means of a comparative calculation with the control. The result is shown in Table 21.

**Table 21**

| Compound (Ex. No.) | Enzyme (J774) IC₅₀(µM) | Enzyme (HepG2) IC₅₀(µM) | IC₅₀ (HepG2) /IC₅₀ (J774) |
|---|---|---|---|
| 1 | 0.051 | 0.067 | 5.1 |
| 2 | 0.20 | 2.25 | 11.3 |
| 3 | 0.28 | 9.19 | 32.8 |
| 5 | 0.10 | 1.45 | 14.5 |
| 10 | 0.27 | 4.9 | 18.2 |
| 25 | 0.1 | >9.6 | >96 |
| 75 | 0.10 | >10 | >100 |
| 78 | 0.014 | 0.82 | 58.6 |
| 85 | 0.019 | >1 | >53 |
| Control 1 | 0.56 | 5.3 | 9.5 |
| Control 2 | 0.58 | 1.1 | 1.9 |
| Control 3 | 0.32 | 1.3 | 4.1 |
| Control 4 | 0.12 | 0.75 | 6.3 |
| Control 5 | 0.007 | 0.61 | 87.1 |

Result of the test by the same manner for the following compounds as controls in those tests is given in Tables 20 and 21 as well.
Control compound (1): 5-[2-(2-(4-Fluorophenyl)ethyl)-3-(1-methyl-1H-imidazol-2-yl)-2H-1-benzopyran-6-yl]oxy-2,2-dimethyl-N-(2,6-diisopropylphenyl) -pentanamide (compound mentioned in WO92/09582);
Control compound (2): (+)-(S)-2-[5-(3,5-Dimethylpyrazol-1-yl)pentasulfinyl]-4,5-diphenylimidazole (compound mentioned in European Patent No. 523941);
Control compound (3): N-(2,2,5,5-Tetrametnyl-1,3-dioxan-4-ylcarbonyl)- β -alanine 2(S)-[N'-(2,2-dimethylpropyl-N'-nonylureido)-1(S)-cyclohexyl ester (compound mentioned in European Patent No. 421441);
Control compound (4): [5-(4,5-Diphenyl-1H-imidazol-2-ylthio)pentyl]-N-heptyl-2-benzoxazolamine (compound mentioned in WO93/23392); and
Control compound (5): 6-(Benzoxazol-2-ylthio)-N-(2,6-diisopropylphenyl)nonanamide (compound mentioned in Example 8 of Japanese Patent Application Hei-09/88660).

### Experimental Example 3.

### Disintegration Test.

Compounds of the present invention and the compounds mentioned in the previously-filed Japanese Patent Applications Hei-09/88660 (hereinafter, referred to as "A") and Hei-09/90146 (hereinafter, referred to as "B") as controls were suspended in a liquid No.1 for disintegration test regulated by the Japanese Pharmacopoeia, shaken for two hours, allowed to stand at room temperature for one hour and filtered through a membrane filter of 0.45 µm. Absorbance of a mixture of this filtrate with methanol in the same amounts was measured and, from the previously-determined ε_{1% 1cm} values, solubility was calculated. The result is shown in Table 22.

As the control compounds (5)-(8), the following compounds mentioned in the above A and B were used.
Control compound (5): 6-(Benzoxazol-2-ylthio)-N-(2,6-diisopropylphenyl)nonanamide (compound mentioned in Example 8 of A);
Control compound (6): 6-(Benzoxazol-2-ylthio)-N-(2,6-diisopropylphenyl)hexanamide (compound mentioned in Example 5 of A);
Control compound (7): 6-[5-(N,N-Dimethylaminomethyl)-benzoxazol-2-ylthio]-N-(2,6-diisopropylphenyl)hexanamide (compound mentioned in Example 37 of B); and
Control compound (8): 6-[5-(N,N-Dimethylamino)-benzoxazol-2-ylthio]-N-(2,6-diisopropylphenyl)hexanamide (compound mentioned in Example 38 of B).

**Table 22**

| Compound | Solubility | pH |
|---|---|---|
| Ex. 1 | 14 mg/ml | 1.2 |
| Ex. 6 | 35 mg/ml | 1.2 |
| Ex. 13 | 7.4 mg/ml | 1.2 |
| Ex. 24 | 17 mg/ml | 1.2 |
| Control 5 | 0.05 µg/ml | 1.2 |
| Control 6 | 0.05 µg/ml | 1.2 |
| Control 7 | 360∼400 µg/ml | 1.2 |
| Control 8 | 170∼180 µg/ml | 1.2 |

### Examples

Compounds of the present invention will be specifically mentioned as hereunder although the present invention is not limited to those specific examples.

### Example 1

### Preparation of 2-[4-[2-(benzoxazol-2-ylthio)ethyl]-piperazin-1-yl]-N-(2,6-diisopropylphenyl)acetamide:

Potassium carbonate (2.35 g, 17 mmol) was added to a solution of 1-(2-hydroxyethyl)piperazine (2.21 g, 17 mmol) and 2-bromo-N-(2,6-diisopropylphenyl)acetamide (synthesized by a method mentioned in Example 1 of Japanese Patent Application Hei-08/158743)(5.07 g, 17 mmol) in DMF (30 ml) and stirred at 80°C for 2 hours. The reaction solution was diluted with water and extracted with ethyl acetate. The organic layer was successively washed with water and a saturated sodium chloride solution and dried over anhydrous sodium sulfate and the solvent was evaporated therefrom.

The resulting residue was purified by a silica gel column chromatography (100 g of silica gel; developing solvent, ammonia-saturated methanol:chloroform = 1:20) and the resulting crystals were recrystallized from acetone-hexane to provide 4.72 g (yield 80%) of N-(2,6-diisopropylphenyl)-2-[4-(2-hydroxyethyl)piperazin-1-yl]acetamide as colorless crystals.

To a solution of this alcohol (300 mg, 0.86 mmol) in THF (5 ml) were added triethylamine (172 mg, 1.7 mmol) and 4-dimethylaminopyridine (10 mg, 0.09 mmol), then methanesulfonyl chloride (115 mg, 1.0 mmol) was dropped thereinto with ice-cooling and stirring, and the mixture was stirred for 40 minutes. After that, triethylamine (172 mg, 1.7 mmol) was further added thereto, then methanesulfonyl chloride (115 mg, 1.0 mmol) was dropped thereinto with ice-cooling and stirring, and the mixture was stirred for 20 minutes. The reaction solution was diluted with water and extracted with ethyl acetate. The organic layer was washed with water, an aqueous solution of sodium bicarbonate and a saturated sodium chloride solution successively and dried over anhydrous sodium sulfate, and the solvent was evaporated therefrom. The resulting residue was dissolved in DMF (7 ml), then 2-mercaptobenzooxazole (130 mg, 0.86 mmol), potassium carbonate (180 mg, 1.3 mmol) and 18-crown-6 (21 mg, 0.08 mmol)were added thereto, and the mixture was stirred at 80°C for 1 hour. The reaction solution was diluted with water and extracted with ethyl acetate. The organic layer was washed with water and a saturated sodium chloride solution successively and dried over anhydrous sodium sulfate and the solvent was evaporated therefrom. The residue was purified by a silica gel column chromatography (40 g of silica gel; developing solvent, hexane:acetone = 5:1∼10:3) and the resulting crystals were recrystallized from acetone-hexane to provide 326 mg (yield 79%) of the desired compound as colorless needles.
Melting point: 161-163°C
IR (KBr) cm⁻¹: 3318, 3290, 2961, 1664, 1495.
1H-NMR (CDCl₃) δ:
1.21 (12H, d, J=7.1 Hz), 2.57-2.80 (8H, m), 2.84 (2H, t, J=7.1 Hz),3.02 (2H, sept, J = 7.1 Hz), 3.22 (2H, s), 3.49 (2H, t, J = 7.1 Hz), 7.18 (1H, d, J = 8.3 Hz), 7.19 (1H, d, J = 6.8 Hz), 7.21-7.33 (3H, m), 7.43 (1H, m), 7.59 (1H, m), 8.61 (1H, br s).
EIMS *m*/*z* (relative intensity): 480 (M⁺), 97 (100).
Elementary analysis as C₂₇H₃₆N₄O₂S
Calculated: C 67.47; H 7.55; N 11.66; S 6.67
Found: C 67.47; H 7.52, N 11.58; S 6.65.

### Example 2.

### Preparation of 2-[4-[2-(benzothiazol-2-ylthio)ethyl] -piperazin-1-yl]-N-(2,6-diisopropylphenyl) acetamide:

The same reaction and treatment as in Example 1 were conducted using 2-mercaptobenzothiazole instead of 2-mercaptobenzooxazole to provide the desired compound as colorless needles.
Melting point: 170-171°C
IR (KBr) cm⁻¹: 3435, 3311, 3281, 2961, 1666, 1500.
1H-NMR (d₆-DMSO) δ :
1.14 (12H, d, J = 7.1 Hz), 2.58-2.66 (8H, m), 2.77-2.82 (2H, m), 3.06 (2H, sept, J = 7.1 Hz), 3.12 (2H, s), 3.50 (2H, t, J = 7.0 Hz), 7.11 (1H, d, J = 8.5 Hz), 7.11 (1H, d, J = 6.6 Hz), 7.21 (1H, dd, J = 8.5, 6.6 Hz), 7.31 (1H, td, J = 7.3, 1.2 Hz), 7.42 (1H, td, J = 7.3, 1.2 Hz), 7.80 (1H, ddd, J = 7.3, 1.2, 0.7 Hz), 7.90 (1H, ddd, J = 7.3, 1.2, 0.7 Hz), 8.74 (1H, br s).
EIMS *m*/*z* (relative intensity): 496 (M⁺), 111 (100).
Elementary analysis as C₂₇H₃₆N₄OS₂
Calculated: C 65.29; H 7.30; N 11.28; S 13.04.
Found: C 65.28; H 7.42, N 11.13; S 12.91.

### Example 3

### Preparation of 2-[4-[2-(benzimidazol-2-ylthio)ethyl] - piperazin-1-yl]-N-(2,6-diisopropylphenyl) acetamide:

The same reaction and treatment as in Example 1 were conducted using 2-mercaptobenzimidazole instead of 2-mercaptobenzooxazole to provide the desired compound as colorless needles.
Melting point: 207°C(d)
IR (KBr) cm⁻¹: 3432, 3282, 2961, 1662, 1500.
1H-NMR (d₆-DMSO) δ :
1.14 (12H, d, J = 6.8 Hz), 2.57-2.65 (8H, m), 2.73-2.78 (2H, m),3.05 (2H, sept, J = 6.8 Hz), 3.12 (2H, s), 3.40 (2H, t, J = 7.0 Hz), 7.07 (2H, dd, J = 5.9, 3.2 Hz), 7.11 (1H, d, J = 8.6 Hz), 7.11 (1H, d, J = 6.6 Hz), 7.31 (1H, dd, J = 8.6, 6.6 Hz), 7.40 (2H, dd, J = 5.9, 3.2 Hz), 8.74 (1H, br s).
EIMS *m*/*z* (relative intensity): 479 (M⁺), 316 (100).
Elementary analysis as C₂₇H₃₇N₅OS
Calculated: C 67.61; H 7.77; N 14.60; S 6.68.
Found: C 67.46; H 7.91, N 14.39; S 6.62.

### Example 4

### Preparation of 2-[4-[2-(7-methoxycarbonylbenzoxazol -2-ylthio)ethyl]-piperazin-1-yl]-N-(2,6-diisopropylphenyl) acetamide:

The same reaction and treatment as in Example 1 were conducted using 2-mercapto-7-methoxycarbonylbenzoxazole instead of 2-mercaptobenzooxazole to provide the desired compound as colorless crystals.
Melting point: 159-161°C
IR (KBr) cm⁻¹: 3436, 3291, 2959, 1729, 1657.
1H-NMR (CDCl₃) δ :
1.21 (12H, d, J=6.8 Hz), 2.63-2.76 (8H, m), 2.86 (2H, t, J=6.8 Hz),3.00 (2H, sept, J = 6.8 Hz), 3.21 (2H, s), 3.51 (2H, t, J = 6.8 Hz), 4.00 (3H, s), 7.18 (1H, d, J = 8.3 Hz), 7.18 (1H, d, J = 7.1 Hz),7.29 (1H, dd, J = 8.3, 7.1 Hz), 7.35 (1H, t, J = 7.8 Hz), 7.77 (1H, dd, J = 7.8, 1.2 Hz), 7.88 (1H, dd, J = 7.8, 1.2 Hz),8.60 (1H, br s).
EIMS *m*/*z* (relative intensity): 538 (M⁺), 317 (100).
Elementary analysis as C₂₉H₃₈N₄O₄S
Calculated: C 64.66; H 7.11; N 10.40; S 5.95.
Found: C 64.65; H 7.12, N 10.27; S 5.95.

### Example 5

### Preparation of 2-[4-[2-(4-methoxycarbonylbenzoxazol -2-ylthio)ethyl]-piperazin-1-yl]-N-(2,6-diisopropylphenyl) acetamide:

The same reaction and treatment as in Example 1 were conducted using 2-mercapto-4-methoxycarbonylbenzoxazole instead of 2-mercaptobenzooxazole to provide the desired compound as colorless crystals.
Melting point: 173-175°C
IR (KBr) cm⁻¹: 3428, 3278, 2960, 1710, 1663.
1H-NMR (CDCl₃) δ :
1.21 (12H, d, J=6.8 Hz), 2.63-2.76 (8H, m), 2.86 (2H, t, J=6.8 Hz), 3.00 (2H, sept, J = 6.8 Hz), 3.22 (2H, s), 3.58 (2H, t, J = 6.8 Hz), 3.99 (3H, s), 7.18 (1H, d, J = 8.1 Hz), 7.18 (1H, d, J = 6.8 Hz), 7.29 (1H, dd, J = 8.1, 6.8 Hz), 7.30 (1H, t, J = 8.1 Hz), 7.62 (1H, dd, J = 8.1, 1.0 Hz), 7.94 (1H, dd, J = 8.1, 1.0 Hz), 8.61 (1H, br s).
EIMS *m*/*z* (relative intensity): 538 (M⁺), 317 (100).
Elementary analysis as C₂₉H₃₈N₄O₄S
Calculated: C 64.66; H 7.11; N 10.40; S 5.95.
Found: C 64.63; H 7.24, N 10.34; S 5.91.

### Example 6

### Preparation of 2-[4-[2-(oxazolo[4,5-b]pyridine-2-ylthio)ethyl]-piperazin-1-yl]-N-(2,6-diisopropylphenyl) acetamide:

The same reaction and treatment as in Example 1 were conducted using 2-mercaptooxazolo[4,5-b]pyridine instead of 2-mercaptobenzooxazole to provide the desired compound as colorless needles.
Melting point: 153-154°C
IR (KBr) cm⁻¹: 3433, 3318, 3293, 2961, 1667.
1H-NMR (CDCl₃) δ :
1.21 (12H, d, J=6.9 Hz), 2.64-2.77 (8H, m), 2.87 (2H, t, J=6.8 Hz), 3.00 (2H, sept, J = 6.9 Hz), 3.22 (2H, s), 3.56 (2H, t, J = 6.8 Hz), 7.18 (1H, d, J = 8.6 Hz), 7.18 (1H, d, J = 6.6 Hz), 7.18 (1H, dd, J = 8.1, 4.9 Hz), 7.29 (1H, dd, J = 8.6, 6.6 Hz), 7.70 (1H, dd, J = 8.1, 1.5 Hz), 8.45 (1H, dd, J = 4.9, 1.5 Hz), 8.60 (1H, br s).
EIMS *m*/*z* (relative intensity): 481 (M⁺), 126 (100).
Elementary analysis as C₂₆H₃₅N₅O₂S
Calculated: C 64.84; H 7.32; N 14.54; S 6.66.
Found: C 64.84; H 7.42, N 14.33; S 6.65.

### Example 7

### Preparation of 2-[4-[3-(benzoxazol-2-ylthio)propyl] - piperazin-1-yl]-N-(2,6-diisopropylphenyl)acetamide:

Potassium carbonate (0.76 g, 5.5 mmol) was added to a solution of 1-(3-hydroxypropyl)piperazine (0.71 g, 5.0 mmol) and 2-bromo-N-(2,6-diisopropylphenyl)acetamide (1.49 g, 5.0 mmol) in DMF (10 ml) and the mixture was stirred at 80°C for 2 hours. The reaction solution was diluted with water and extracted with ethyl acetate. The organic layer was successively washed with water and a saturated sodium chloride solution and dried over anhydrous sodium sulfate and the solvent was evaporated therefrom.

The resulting residue was purified by a silica gel column chromatography (30 g of silica gel; developing solvent, ammonia-saturated methanol:chloroform = 1:20) and the resulting crystals were recrystallized from ethyl acetate-chloroform to provide 1.13 g (yield 63%) of 2-[4-(3-hydroxypropyl)piperazin-1-yl]-N-(2,6-diisopropylphenyl)acetamide as colorless crystals.

To a solution of this alcohol (444 mg, 1.22 mmol) in THF (10 ml) were added triethylamine (185 mg, 1.83 mmol) and 4-dimethylaminopyridine (14 mg, 0.06 mmol), then methanesulfonyl chloride (167 mg, 1.46 mmol) was dropped thereinto with ice-cooling and stirring, and the mixture was stirred for 40 minutes. After that, triethylamine (185 mg, 1.83 mmol) was further added thereto, then methanesulfonyl chloride (167 mg, 1.46 mmol) was dropped thereinto with ice-cooling and stirring and the mixture was stirred for 30 minutes. The reaction solution was diluted with water and extracted with ethyl acetate. The organic layer was successively washed with water, an aqueous solution of sodium bicarbonate and a saturated sodium chloride solution and dried over anhydrous sodium sulfate and the solvent was evaporated therefrom. The resulting residue was dissolved in DMF (7 ml), then 2-mercaptobenzooxazole (151 mg, 1.0 mmol), potassium carbonate (166 mg, 1.2 mmol) and 18-crown-6 (13 mg, 0.05 mmol) were added thereto, and the mixture was stirred at 80°C for 1 hour. The reaction solution was diluted with water and extracted with ethyl acetate. The organic layer was washed with water and a saturated sodium chloride solution successively and dried over anhydrous sodium sulfate and the solvent was evaporated therefrom.

The residue was purified by a silica gel column chromatography (40 g of silica gel; developing solvent, hexane: acetone = 5:1∼10:3) and the resulting crystals were recrystallized from acetone-hexane to provide 321 mg (yield 60%) of the desired product as colorless crystals.
Melting point: 123-125°C.
IR (KBr) cm⁻¹: 3317, 2959, 1663, 1499, 1129.
1H-NMR (CDCl₃) δ :
1.21 (12H, d, J = 6.8 Hz), 2.04 (2H, quint J = 6.8 Hz), 2.46-2.80 (8H, m), 2.54 (2H, t, J = 6.8 Hz), 3.00 (2H, sept, J = 6.8 Hz), 3.22 (2H, s), 3.37 (2H, t, J = 6.8 Hz), 7.18 (1H, d, J=8.1 Hz), 7.18 (1H, d, J=6.8 Hz), 7.20-7.32 (3H, m), 7.43 (1H, m), 7.59 (1H, m), 8.62 (1H, br s). EIMS *m*/*z* (relative intensity): 494 (M⁺), 290 (100).
Elementary analysis as C₂₈H₃₈N₄O₂S
Calculated: C 67.98; H 7.74; N 11.33; S 6.48
Found: C 67.84; H 7.78; N 11.22; S 6.43

### Example 8

### Preparation of 2-[4-[3-(benzothiazol-2-ylthio) propyl]piperazin-1-yl]-N-(2,6-diisopropylphenyl)acetamide:

The same reaction and treatment as in Example 7 were conducted using 2-mercaptobenzothiazole instead of 2-mercaptobenzooxazole to provide the desired compound as colorless needles.
Melting point: 113-115°C
IR (KBr) cm⁻¹: 3436, 3299, 2962, 1661, 1502.
1H-NMR (CDCl₃) δ :
1.21 (12H, d, J = 6.8 Hz), 2.03 (2H, quint, J = 7.1 Hz), 2.51-2.58 (6H, m), 2.73-2.77 (4H, m), 3.01 (2H, sept, J = 6.8 Hz), 3.22 (2H, s), 3.41 (2H, t, J = 7.1 Hz), 7.18 (1H, d, J = 8.3 Hz), 7.18 (1H, d, J = 6.8 Hz), 7.29 (1H, dd, J=8.3, 6.8 Hz), 7.29 (1H, td, J = 7.8, 1.2 Hz), 7.41 (1H, td, J = 7.8, 1.2 Hz), 7.76 (1H, dd, J = 7.8, 1.2 Hz), 7.85 (1H, dd, J = 7.8, 1.2 Hz), 8.63 (1H, br s).
EIMS *m*/*z* (relative intensity): 510 (M⁺), 139 (100).
Elementary analysis as C₂₈H₃₈N₄OS₂
Calculated: C 65.85; H 7.50; N 10.97; S 12.55.
Found: C 65.76; H 7.59, N 10.78; S 12.49.

### Example 9

### Preparation of 2-[4-[3-(benzimidazole-2-ylthio) phenyl]piperazin-1-yl]-N-(2,6-diisopropylphenyl)acetamide:

The same reaction and treatment as in Example 7 were conducted using 2-mercaptobenzimidazole instead of 2-mercaptobenzooxazole to provide the desired compound as colorless needles. Melting point: 123-125°C
IR (KBr) cm⁻¹: 3429, 3273, 2961, 1659, 1506.
1H-NMR (d₆-DMSO) δ :
1.13 (12H, d, J = 6.8 Hz), 1.90 (2H, quint, J = 6.8 Hz), 2.40-2.52 (6H, m), 2.61-2.65 (4H, m), 3.05 (2H, sept, J = 6.8 Hz), 3.12 (2H, s), 3.29 (2H, t, J = 6.8 Hz), 7.06-7.10 (2H, m) 7.12 (2H, d, J = 7.6 Hz), 7.22 (1H, t, J=7.6 Hz), 7.38-7.42 (2H, m), 8.76 (1H, br s).
EIMS *m*/*z* (relative intensity): 493 (M⁺), 139 (100).

### Example 10

### Preparation of 2-[4-[3-(7-methoxycarbonylbenzoxazol - 2-ylthio)propyl]piperazin-1-yl]-N-(2,6-diisopropylphenyl) acetamide:

The same reaction and treatment as in Example 7 were conducted using 2-mercapto-7-methoxycarbonylbenzoxazole instead of 2-mercaptobenzooxazole to provide the desired compound as colorless crystals.
Melting point: 135-136°C
IR (KBr) cm⁻¹: 3429, 3340, 2961, 1720, 1663.
1H-NMR (CDCl₃) δ :
1.21 (12H, d, J = 6.8 Hz), 2.07 (2H, quint, J = 7.0 Hz), 2.52-2.57 (6H, m), 2.73-2.76 (4H, m), 3.01 (2H, sept, J = 6.8 Hz), 3.22 (2H, s), 3.40 (2H, t, J = 7.0 Hz), 4.00 (3H, s), 7.18 (1H, d, J = 8.3 Hz), 7.18 (1H, d, J = 7.1 Hz), 7.29 (1H, dd, J = 8.3, 7.1 Hz), 7.35 (1H, t, J = 7.8 Hz), 7.77 (1H, dd, J = 7.8, 1.2 Hz), 7.88 (1H, dd, J = 7.8, 1.2 Hz), 8.63 (1H, br s).
EIMS *m*/*z* (relative intensity): 552 (M⁺, 100).
Elementary analysis as C₃₀H₄₀N₄O₄S
Calculated: C 65.19; H 7.29; N 10.14; S 5.80.
Found: C 65.31; H 7.57, N 10.02; S 5.78.

### Example 11

### Preparation of 2-[4-[3-(4-methoxycarbonylbenzoxazol - 2-ylthio)propyl]piperazin-1-yl]-N-(2,6-diisopropylphenyl) acetamide:

The same reaction and treatment as in Example 7 were conducted using 2-mercapto-4-methoxycarbonylbenzoxazole instead of 2-mercaptobenzooxazole to provide the desired compound as faint orange crystals.
Melting point: 132-133°C
IR (KBr) cm⁻¹: 3422, 3239, 2958, 1717, 1660.
1H-NMR (CDCl₃) δ :
1.21 (12H, d, J = 6.8 Hz), 2.06 (2H, quint, J = 7.0 Hz), 2.52-2.57 (6H, m), 2.74-2.77 (4H, m), 3.01 (2H, sept, J = 6.8 Hz), 3.23 (2H, s), 3.45 (2H, t, J = 7.0 Hz), 4.00 (3H, s), 7.18 (1H, d, J = 8.1 Hz), 7.19 (1H, d, J = 6.8 Hz), 7.29 (1H, dd, J = 8.1, 6.8 Hz), 7.30 (1H, t, J = 7.8 Hz), 7.62 (1H, dd, J = 7.8, 1.0 Hz), 7.94(1H, dd, J = 7.8, 1.0 Hz), 8.64 (1H, br s).
EIMS *m*/*z* (relative intensity): 552 (M⁺, 100).
Elementary analysis as C₃₀H₄₀N₄O₄S
Calculated: C 65.19; H 7.29; N 10.14; S 5.80.
Found: C 65.18; H 7.39, N 9.90; S 5.84.

### Example 12

### Preparation of 2-[4-[3-(oxazolo[4,5-b]pyridine-2-ylthio)propyl]piperazin-1-yl]-N-(2,6-diisopropylphenyl) acetamide:

The same reaction and treatment as in Example 7 were conducted using 2-mercaptooxazolo[4,5-b]pyridine instead of 2-mercaptobenzooxazole to provide the desired compound as colorless needles.
Melting point: 125-127°C
IR (KBr) cm⁻¹: 3431, 3241, 2959, 1664, 1496.
1H-NMR (CDCl₃) δ :
1.21 (12H, d, J = 6.8 Hz), 2.09 (2H, quint, J = 7.2 Hz), 2.52-2.59 (6H, m), 2.73-2.77 (4H,m), 3.01 (2H, sept, J = 6.8 Hz), 3.22 (2H,s), 3.44 (2H, t, J = 7.2 Hz), 7.18 (1H, d, J = 8.3 Hz) 7.18 (1H, d, J = 6.6 Hz), 7.18 (1H, dd, J = 8.1, 5.1 Hz), 7.29 (1H, dd, J = 8.3, 6.6 Hz), 7.69 (1H, dd, J = 8.1, 1.5 Hz), 8.45 (1H, dd, J = 5.1, 1.5 Hz), 8.63 (1H, br s).
EIMS *m*/*z* (relative intensity): 495 (M⁺), 302 (100).
Elementary analysis as C₂₇H₃₇N₅O₂S
Calculated: C 65.42; H 7.52; N 14.13; S 6.47.
Found: C 65.57; H 7.63, N 13.84; S 6.38.

### Example 13

### Preparation of 2-[4-[3-(benzoxazol-2-ylthio)propyl] - homopiperazin-1-yl]-N-(2,6-diisopropylphenyl)acetamide:

To a solution of 3-hydroxypropyl-1-homopiperazine (158 mg, 1 mmol) in acetonitrile (5 ml) were added potassium carbonate (152 mg, 1.1 mmol) and 2-bromo-N-(2,6-diisopropylphenyl)acetamide (298 mg, 1 mmol) and the mixture was stirred at room temperature for 3 hours. The reaction solution was concentrated and the residue was extracted with ethyl acetate. The organic layer was extracted with water and a saturated sodium chloride solution successively and dried over anhydrous sodium sulfate, and the solid resulted after evaporating the solvent was crystallized from hexane-ether-acetone to provide 327 mg (yield 87%) of 2-[4-[(3-hydroxypropyl)homopiperazin-1-yl]-N-(2,6-diisopropylphenyl)acetamide as pale red needles.

To a solution of the resulting alcohol (130 mg, 0.34 mmol) in methylene chloride (3 ml) were added triethylamine (52 mg, 0.52 mmol) and 4-dimethylaminopyridine (6 mg, 0.05 mmol) and then methanesulfonyl chloride (59 mg, 0.52 mmol) was gradually dropped thereinto with ice-cooling and stirring. The mixture was stirred at room temperature for 30 minutes and then the reaction solution was extracted with ethyl acetate. The organic layer was washed with water and a saturated sodium chloride solution successively and dried over anhydrous sodium sulfate and the solvent was evaporated therefrom.

The resulting residue was dissolved in DMF (2 ml), then 2-mercaptobenzooxazole (51 mg, 0.34 ml), potassium carbonate (51 mg, 0.37 mmol) and 18-crown-6 (11 mg, 0.04 mmol) were added thereto and the mixture was stirred at 80°C for 2 hours. The reaction solution was diluted with water and extracted with ethyl acetate. The organic layer was successively washed with water and a saturated sodium chloride solution and dried over anhydrous sodium sulfate and the residue obtained by evaporating of the solvent was purified by a preparative thin layer chromatography (developing solvent, hexane:acetone = 1:1) followed by recrystallizing from hexane-acetone to provide 140 mg (yield 81%) of the desired compound as colorless needles.
Melting point: 109-111°C.
IR (KBr) cm⁻¹: 3429, 3275, 1661, 1500, 1453.
1H-NMR (CDCl₃) δ :
1.21 (12H, d, J = 6.8 Hz), 1.89 (2H, quint, J = 5.8 Hz), 2.00 (2H, quint, J = 6.8 Hz), 2.67 (2H, t, J = 6.8 Hz), 2.73-2.78 (4H,m), 2.91-2.96 (4H, m), 3.03 (2H, sept, J = 6.8 Hz), 3.35 (2H,s), 3.37 (2H, t, J = 6.8 Hz), 7.18 (2H, d, J = 7.6 Hz), 7.21-7.31 (3H, m), 7.43 (1H, m), 7.58 (1H, m), 8.77 (1H, br s).
EIMS *m*/*z* (relative intensity): 508 (M⁺, 100).
Elementary analysis as C₂₉H₄₀N₄O₂S.
Calculated: C 68.47; H 7.92; N 11.01; S 6.30
Found: C 68.19; H 8.03; N 10.79; S 6.28.

### Example 14

### Preparation of 2-[4-[3-(7-methoxycarbonylbenzoxazol - 2-ylthio)propyl]homopiperazin-1-yl]-N-(2,6-diisopropylphenyl)acetamide:

The same reaction and treatment as in Example 13 were conducted using 2-mercapto-7-methoxycarbonylbenzoxazole instead of 2-mercaptobenzooxazole to provide the desired compound as colorless needles.
Melting point: 83-85°C
IR (KBr) cm⁻¹: 3425, 3250, 1735, 1719, 1660.
1H-NMR (CDCl₃) δ :
1.21 (12H, d, J = 6.8 Hz), 1.89 (2H, quint, J = 5.8 Hz), 2.02 (2H, quint, J = 6.8 Hz), 2.68 (2H, t, J = 6.8 Hz), 2.73-2.79 (4H, m), 2.91-2.96 (4H, m), 3.02 (2H, sept, J = 6.8 Hz), 3.35 (2H, s), 3.40 (2H, t, J = 6.8 Hz), 4.00 (3H, s) 7.18 (2H, d, J = 7.6 Hz), 7.28 (1H, t, J = 7.6 Hz), 7.34 (1H, t, J = 7.8 Hz), 7.76 (1H, dd, J = 7.8, 1.2 Hz), 7.87 (1H, dd, J = 7.8, 1.2 Hz), 8.77 (1H, br s).
EIMS *m*/*z* (relative intensity): 566 (M⁺), 153 (100).
Elementary analysis as C₃₁H₄₂N₄O₄S
Calculated: C 65.70; H 7.47; N 9.89; S 5.66.
Found: C 65.81; H 7.56, N 9.79; S 5.65.

### Example 15

### Preparation of 2-[4-[3-(oxazolo[4,5-b]pyridin-2-ylthio)propyl]homopiperazin-1-yl]-N-(2,6-diisopropylphenyl)acetamide:

The same reaction and treatment as in Example 13 were conducted using 2-mercaptooxazolo[4,5-b]pyridine instead of 2-mercaptobenzooxazole to provide the desired compound as colorless needles.
Melting point: 73-75°C
IR (KBr) cm⁻¹: 3435, 3240, 1660, 1497, 1403.
1H-NMR (CDCl₃) δ :
1.21 (12H, d, J=6.8 Hz), 1.87-1.95 (2H, m), 2.01-2.10 (2H, m), 2.67-2.73 (2H, m), 2.75-2.82 (4H,m), 2.92-2.96 (4H,m), 3.03 (2H, sept, J=6.8 Hz), 3.36 (2H,s), 3.43 (2H, t, J=6.8 Hz), 7.18 (1H, dd, J = 8.0, 5.0 Hz), 7.18 (2H, d, J = 7.6 Hz), 7.28 (1H, t, J = 7.6 Hz), 7.69 (1H, dd, J = 8.0, 1.5 Hz), 8.45 (1H, dd, J = 5.0, 1.5 Hz), 8.78 (1H, br s).
EIMS *m*/*z* (relative intensity): 509 (M⁺), 316 (100).
Elementary analysis as C₂₈H₃₉N₅O₂S·0.2H₂O
Calculated: C 65.52; H 7.74; N 13.64; S 6.25.
Found: C 65.52; H 7.71, N 13.44; S 6.31.

### Example 16

### Preparation of N-[2-[4-[2-(benzoxazol-2-ylthio)-ethyl]piperazin-1-yl]ethyl]-N'-(2,6-diisopropylphenyl)-N-heptylurea:

To a solution of n-heptylamine (2.30 g, 20 mmol) in THF (20 ml) was dropped a solution of bromoacetyl bromide (2.02 g, 10 mmol) in THF (10 ml) with ice-cooling and stirring and the mixture was stirred at 0°C for 1 hour. The reaction solution was concentrated *in vacuo*, water was added to the residue and the mixture was extracted with ether. The organic layer was washed with water and a saturated sodium chloride solution successively and dried over anhydrous sodium sulfate and the solvent was evaporated therefrom to provide 2.36 g (yield 99%) of crude 2-bromo-N-heptylacetamide as oil.

Potassium carbonate (1.52 g, 11 mmol) was added to a solution of this amide (2.36 g, 10 mmol) and 1-(2-hydroxyethyl)piperazine (1.30 g, 10 mmol) in acetonitrile (40 ml) and the mixture was stirred at room temperature for 12 hours. The reaction solution was concentrated and the residue was extracted with ethyl acetate. The organic layer was washed with water and a saturated sodium chloride solution successively and dried over anhydrous sodium sulfate and the residue obtained by evaporation of the solvent was purified by a silica gel column chromatography (75 g of silica gel; developing solvent, ammonia-saturated methanol:chloroform = 1:20) to provide 2.39 g (yield 83%) of N-heptyl-2-[4-(2-hydroxyethyl)piperazin-1-yl]acetamide as colorless oil.

Lithium aluminum hydride (380 mg, 10 mmol) was added to a solution of this amide (1.69 g, 5.92 mmol) in THF (40 ml) with ice-cooling and stirring and the mixture was stirred for 15 minutes after returning to room temperature and heated to reflux for 2 hours. Saturated aqueous solution of ammonium chloride was added gradually thereto with ice-cooling and stirring until the reaction solution became turbid, the separated matters were filtered off with celite, the filtrate was dried over anhydrous sodium carbonate and the solvent was evaporated therefrom. The resulting residue was purified by a silica gel column chromatography (75 g of silica gel; developing solvent, ammonia-saturated methanol:chloroform = 1:20) to provide 694 mg (yield 43%) of 2-[4-(2-heptylaminoethyl)-piperazin-1-yl]ethanol as colorless oil.

To a solution of this aminoalcohol (271 mg, 1 mmol) in chloroform (5 ml) was added 2,6-diisopropylphenyl isocyanate (204 mg, 1 mmol) and the mixture was stirred for 15 minutes. The reaction solution was concentrated, the residue was purified by a silica gel column chromatography (10 g of silica gel; developing solvent being ammonia-saturated methanol: chloroform = 3:97) and the resulting crystals were recrystallized from acetone-hexane to provide 340 mg (yield 71%) of N'-(2,6-diisopropylphenyl)-N-heptyl-N-[2-[4-(2-hydroxyethyl)piperazin-1-yl]ethyl]urea as colorless needles.

To a solution of this alcohol (338 mg, 0.71 mmol) in THF (5 ml) were added triethylamine (93 mg, 0.92 mmol) and 4-dimethylaminopyridine (9 mg, 0.07 mmol), then methanesulfonyl chloride (89 mg, 0.78 mmol) was dropped thereinto with ice-cooling and stirring and the mixture was stirred for 30 minutes. After that, triethylamine (93 mg, 0.92 mmol) was added thereto, methanesulfonyl chloride (89 mg, 0.78 mmol) was dropped thereinto with ice-cooling and stirring and the mixture was stirred for 20 minutes. The reaction solution was diluted with water and extracted with ethyl acetate. The organic layer was washed with water, aqueous solution of sodium bicarbonate and saturated sodium chloride solution successively and dried over anhydrous sodium sulfate and the solvent was evaporated therefrom.

The resulting residue was dissolved in DMF (3 ml), then 2-mercaptobenzooxazole (91 mg, 0.6 mmol), potassium carbonate (104mg, 0.75 mmol) and 18-crown-6 (16 mg, 0.06 mmol) were added thereto and the mixture was stirred at 80°C for 1 hour. The reaction solution was diluted with water and extracted with ethyl acetate. The organic layer was washed with water and saturated sodium chloride solution successively and the solvent was evaporated therefrom. The residue was purified by a silica gel column chromatography (40 g of silica gel; developing solvent, hexane:acetone = 5:1∼10:3) and the resulting crystals were recrystallized from acetone-hexane to provide 243 mg (yield 57%) of the desired compound as colorless needles.
Melting point: 110-111°C.
IR (KBr) cm⁻¹: 3326, 2956, 1627, 1498, 1130.
1H-NMR (CDCl₃) δ :
0.88 (3H, t, J = 7.1 Hz), 1.14-1.36 (22H, m), 2.39-2.61 (8H, m), 2.63 (2H, t, J = 5.1 Hz), 2.69 (2H, t, J = 6.8 Hz), 3.16 (2H, sept, J = 7.1 Hz), 3.34 (2H, t, J = 7.1 Hz), 3.39 (2H, t, J = 6.8 Hz), 3.46 (2H, t, J = 5.1 Hz), 7.13 (1H, d, J=8.3 Hz), 7.13 (1H, d, J=7.1 Hz), 7.19-7.30 (3H, m), 7.41 (1H, dd, J = 7.6, 1.4 Hz), 7.56 (1H, dd, J = 7.6, 1.4 Hz), 8.10 (1H, br s).
EIMS *m*/*z* (relative intensity): 607 (M⁺), 254 (100).
Elementary analysis as C₃₅H₅₃N₅O₂S
Calculated: C 69.15; H 8.79, N 11.52; S 5.27
Found: C 69.27; H 8.93; N 11.29; S 5.32.

### Example 17

### Preparation of N'-(2,6-diisopropylphenyl)-N-heptyl-N-[2-[4-[2-(7-methoxycarbonylbenzoxazol-2-ylthio)-ethyl]piperazin-1-yl]ethyl]urea:

The same reaction and treatment as in Example 16 were conducted using 7-methoxycarbonyl-2-mercaptobenzooxazole instead of 2-mercaptobenzooxazole to provide the desired compound as colorless crystals.
Melting point: 125-126°C
IR (KBr) cm⁻¹: 3425, 3304, 2957, 1725, 1628.
1H-NMR (CDCl₃) δ :
0.88 (3H, t, J = 6.7 Hz), 1.20 (12H, d, J = 6.8 Hz), 1.25-1.33 (8H, m), 1.51-1.59 (2H, m), 2.43-2.51 (4H,m), 2.54-2.65 (6H, m), 2.71 (2H, t, J = 6.8 Hz), 3.16 (2H, sept, J = 6.8 Hz), 3.34 (2H, t, J = 7.3 Hz), 3.40-3.47 (4H, m), 3.98 (3H, s), 7.13 (1H, d, J = 8.8 Hz), 7.13 (1H, d, J = 6.4 Hz), 7.23 (1H, dd, J = 8.8, 6.4 Hz), 7.34 (1H, t, J = 7.8 Hz), 7.74 (1H, dd, J = 7.8, 1.2 Hz), 7.87 (1H, dd, J = 7.8, 1.2 Hz), 8.10 (1H, br s).
EIMS *m*/*z* (relative intensity): 665 (M⁺), 265 (100).
Elementary analysis as C₃₇H₅₅N₅O₄S
Calculated: C 66.73; H 8.32; N 10.52; S 4.81.
Found: C 66.77; H 8.24, N 10.45; S 4.79.

### Example 18

### Preparation of N'-(2,6-diisopropylphenyl)-N-heptyl-N-[2-[4-[2-(oxazolo[4,5-b]pyridin)-2-ylthio)ethyl] piperazin-1-yl]ethyl]urea:

The same reaction and treatment as in Example 16 were conducted using 2-mercaptooxazolo[4,5-b]pyridine instead of 2-mercaptobenzooxazole to provide the desired compound as colorless crystals.
Melting point: 106-108°C
IR (KBr) cm⁻¹: 3420, 3331, 2958, 1628, 1495.
1H-NMR (CDCl₃) δ :
0.88 (3H, t, J=6.9 Hz), 1.20 (12H, d, J=6.9 Hz), 1.26-1.33 (8H, m), 1.53-1.59 (2H, m), 2.43-2.51 (4H, m), 2.55-2.66 (6H, m), 2.73 (2H, t, J = 6.7 Hz), 3.16 (2H, sept, J = 6.9 Hz), 3.34 (2H, t, J = 7.3 Hz), 3.44-3.50 (4H, m), 7.13 (1H, d, J=8.5 Hz), 7.13 (1H, d, J = 6.4 Hz), 7.17 (1H, dd, J = 8.1, 4.9 Hz), 7.23 (1H, dd, J = 8.5, 6.4 Hz), 7.68 (1H, dd, J = 8.1, 1.5 Hz), 8.06 (1H, br s), 8.44 (1H, dd, J = 4.9, 1.5 Hz).
EIMS *m*/*z* (relative intensity): 608 (M⁺), 188 (100).
Elementary analysis as C₃₄H₅₂N₆O₂S
Calculated: C 67.07; H 8.61; N 13.80; S 5.27.
Found: C 67.06; H 8.52, N 13.66; S 5.27.

### Example 19

### Preparation of N-[2-[4-[3-(benzoxazol-2-ylthio)-propyl]piperazin-1-yl]ethyl]-N'-(2,6-diisopropylphenyl)-N-heptylurea:

Potassium carbonate (2.49 g, 18 mmol) was added to a solution of 2-bromo-N-heptylacetamide (3.30 g, 15 mmol) obtained in Example 16 and 1-(3-hydroxypropyl)piperazine (2.16 g, 15 mmol) in acetonitrile (60 ml) and the mixture was stirred at room temperature for 15 hours. The reaction solution was concentrated and the residue was extracted with ethyl acetate. The organic layer was washed with water and saturated sodium chloride solution successively and dried over anhydrous sodium carbonate and the residue obtained after evaporation of the solvent was purified by a silica gel column chromatography (75 g of silica gel; developing solvent, ammonia-saturated methanol: chloroform = 1:20) to provide 4.38 g (yield 97%) of N-heptyl-2-[4-[3-hydroxypropyl]piperazin-1-yl]acetamide as colorless oil.

Lithium aluminum hydride (380 mg, 10 mmol) was added to a solution of this amide (1.50 g, 5.0 mmol) in THF (50 ml) with ice-cooling and stirring and the mixture was returned to room temperature and stirred for 15 minutes and then heated to reflux for 2 hours. Under ice-cooling and stirring, a saturated ammonium chloride solution was gradually added thereto until the reaction solution became turbid, the separated matters were filtered off through celite, the filtrate was dried over anhydrous sodium carbonate and the solvent was evaporated therefrom. The resulting residue was purified by a silica gel column chromatography (75 g of silica gel; developing solvent, ammonia-saturated methanol:chloroform = 1:20) to provide 586 mg (yield 41%) of 3-[4-(2-heptylaminoethyl)piperazin-1-yl]propanol as colorless oil.

To a solution of this aminoalcohol (586 mg, 2.05 mmol) in chloroform (5 ml) was added 2,6-diisopropylphenyl isocyanate (408 mg, 2 mmol) and the mixture was stirred for 15 minutes. The reaction solution was concentrated, the residue was purified by a silica gel column chromatography (20 g of silica gel; developing solvent, ammonia-saturated methanol:chloroform = 3:97) and the resulting crystals were recrystallized from acetone-hexane to provide 340 mg (yield 71%) of N'-(2,6-diisopropylphenyl)-N-heptyl-N-[2-[4-(3-hydroxypropyl)-piperazin-1-yl]ethyl]urea as colorless needles.

To a solution of this alcohol (147 mg, 0.3 mmol) in THF (3 ml) were added triethylamine (39 mg, 0.39 mmol) and 4-dimethylaminopyridine (3.7 mg, 0.03 mmol), then methanesulfonyl chloride (38 mg, 0.33 mmol) was dropped thereino with ice-cooling and stirring and the mixture was stirred for 30 minutes. Then triethylamine (39 mg, 0.39 mmol) was added thereto, methanesulfonyl chloride (38 mg, 0.33 mmol) was dropped thereinto and the mixture was stirred for 30 minutes. The reaction solution was diluted with water and extracted with ethyl acetate. The organic layer was washed with water, aqueous solution of sodium bicarbonate and saturated sodium chloride solution successively and dried over anhydrous sodium sulfate and the solvent was evaporated therefrom. The resulting residue was dissolved in DMF (3 ml), then 2-mercaptobenzooxazole (45 mg, 0.3 mol), potassium carbonate (62 mg, 0.45 mmol) and 18-crown-6 (8 mg, 0.03 mmol) were added thereto, and the mixture was stirred at 80°C for 1 hour. The reaction solution was diluted with water and extracted with ethyl acetate. The organic layer was washed with water and saturated sodium chloride solution successively and dried over anhydrous sodium sulfate and the solvent was evaporated therefrom. The residue was purified by a silica gel column chromatography (25 g of silica gel; developing solvent, hexane:acetone = 5:1∼5:2) and the resulting crystals were recrystallized from ether-pentane to provide 78 mg (yield 42%) of the desired compound as colorless crystals.
Melting point: 93-94°C.
IR (KBr) cm⁻¹: 3430, 3313, 2959, 2931, 1627, 1502.
1H-NMR (CDCl₃) δ :
0.88 (3H, t, J=6.7 Hz), 1.20 (12H, d, J=6.8 Hz), 1.25-1.35(8H, m), 1.53-1.59 (2H, m), 1.95 (2H, quint, J = 7.0 Hz), 2.34-2.42 (6H,m), 2.54-2.66 (6H,m), 3.16 (2H, sept, J = 6.8 Hz), 3.31 (2H, t, J = 7.0 Hz), 3.34 (2H, t, J = 7.0 Hz), 3.46 (2H, t, J = 5.0 Hz), 7.13 (1H, d, J = 8.5 Hz), 7.13 (1H, d, J = 6.8 Hz), 7.20-7.31 (3H, m), 7.42 (1H, m), 7.57 (1H, m), 8.17 (1H, br s).
EIMS *m*/*z* (relative intensity): 621 (M⁺), 188 (100).
Elementary analysis as C₃₆H₅₅N₅O₂S
Calculated: C 69.53; H 8.91; N 11.26
Found: C 69.51; H 9.02; N 11.12.

### Example 20

### Preparation of N'-(2,6-diisopropylphenyl)-N-heptyl-N-[2-[4-[3-(7-methoxycarbonylbenzoxazol-2-ylthio)-propyl]piperazin-1-yl]ethyl]urea:

The same reaction and treatment as in Example 19 were conducted using 7-methoxycarbonyl-2-mercaptobenzooxazole instead of 2-mercaptobenzooxazole to provide the desired compound as colorless crystals.
Melting point: 97-99°C
IR (KBr) cm⁻¹: 3428, 3318, 2958, 1728, 1628.
1H-NMR (CDCl₃) δ :
0.88 (3H, t, J=6.7 Hz), 1.20 (12H, d, J=6.8 Hz), 1.25-1.34 (8H, m), 1.54-1.59 (2H, m), 1.97 (2H, quint; J = 7.1 Hz), 2.35-2.43 (6H, m), 2.55-2.66 (6H,m), 3.16 (2H, sept, J = 6.8 Hz), 3.33 (2H, t, J = 7.1 Hz), 3.34 (2H, t, J = 7.1 Hz), 3.34 (2H, t, J = 4.9 Hz), 3.99 (3H, s), 7.13 (1H, d, J = 8.8 Hz), 7.13 (1H, d, J = 6.6 Hz), 7.23 (1H, dd, J = 8.8, 6.6 Hz), 7.14 (1H, t, J = 7.8 Hz), 7.75 (1H, dd, J = 7.8, 1.2 Hz), 7.87 (1H, dd, J = 7.8, 1.2 Hz),
8.17 (1H, br s).
EIMS *m*/*z* (relative intensity): 476 (M⁺ - 203), 97 (100).
Elementary analysis as C₃₈H₅₇N₅O₄S
Calculated: C 67.12; H 8.45; N 10.30.
Found: C 66.90; H 8.48, N 10.12.

### Example 21

### Preparation of N'-(2,6-diisopropylphenyl)-N-heptyl N-[2-[4-[3-(oxazolo[4,5-b]pyridin)-2-ylthio)propyl]-piperazin-1-yl]ethyl]urea:

The same reaction and treatment as in Example 19 were conducted using 2-mercaptooxazolo[4,5-b]pyridine instead of 2-mercaptobenzooxazole to provide the desired compound as colorless crystals.
Melting point: 90-92°C
IR (KBr) cm⁻¹: 3434, 3310, 2958, 1626, 1515.
1H-NMR (CDCl₃) δ :
0.88 (3H, t, J=6.8 Hz), 1.20 (12H, d, J=6.8 Hz), 1.25-1.34 (8H, m), 1.54-1.59 (2H, m), 1.98 (2H, quint, J = 7.1 Hz), 2.34-2.42 (6H,m), 2.55-2.66 (6H, m), 3.16 (2H, sept, J = 6.8 Hz), 3.34 (2H, t, J = 7.1 Hz), 3.37 (2H, t, J = 7.1 Hz), 3.46 (2H, t, J = 4.9 Hz), 7.13 (1H, d, J = 8.8 Hz), 7.13 (1H, d, J = 6.6 Hz), 7.17 (1H, dd, J = 8.3, 5.1 Hz), 7.23 (1H, dd, J = 8.8, 6.6 Hz), 7.68 (1H, dd, J = 8.3, 1.5 Hz), 8.16 (1H, br s), 8.46 (1H, dd, J = 5.1, 1.5 Hz).
EIMS *m*/*z* (relative intensity): 622 (M⁺), 98 (100).
Elementary analysis as C₃₅H₅₄N₆O₂S
Calculated: C 67.49; H 8.74; N 13.49.
Found: C 67.36; H 8.76, N 13.25.

### Example 22

### Preparation of 3-[4-[2-(benzoxazol-2-ylthio)-ethyl]piperazin-1-yl]-N-(2,6-iisopropylphenyl)propanamide:

Di-tert-butyl dicarbonate (5.2 g, 2.4 mmol) was added to a solution of 1-(2-hydroxyethyl)piperazine (2.6 g, 20 mmol) in methylene chloride (50 ml) with ice-cooling and the mixture was stirred for 3 hours. The reaction solution was concentrated and the resulting residue was purified by a silica gel column chromatography (75 g of silica gel; developing solvent, chloroform:methanol = 20:1) to provide 5.5 g (yield 100%) of 1-tert-butoxycarbonyl-4-(2-hydroxyethyl)piperazine as colorless needles.

To a solution of 1-tert-butoxycarbonyl-4-(2-hydroxyethyl)piperazine (1.15 g, 5 mmol) in THF (20 ml) were added triethylamine (607 mg, 6 mmol) and 4-dimethylaminopyridine (73 mg, 0.6 mmol), then methanesulfonyl chloride (687 mg, 6 mmol) was gradually dropped thereinto with ice-cooling and the mixture was stirred for 30 minutes. The reaction solution was filtered to remove triethylamine hydrochloride and the filtrate was concentrated.

To a solution of the resulting residue in DMF (30 ml) were added 2-mercaptobenzooxazole (756 mg, 5 mmol), potassium carbonate (760 mg, 5.5 mmol) and 18-crown-6 (132 mg, 0.5 mmol) and the mixture was stirred at 80°C for 2 hours. The reaction solution was diluted with water and extracted with ethyl acetate. The organic layer was washed with water and saturated sodium chloride solution successively and dried over anhydrous sodium sulfate and the solvent was evaporated therefrom. The residue was purified by a silica gel column chromatography (75 g of silica gel; developing solvent, hexane:acetone = 8:1) and the resulting crystals were recrystallized from hexane-ether to provide 1.02 g (yield 56%) of 1-tert-butoxycarbonyl-4-[2-(benzoxazol-2-ylthio)ethyl]piperazine as colorless needles.

To this tert-butoxy carbonyl compound (364 mg, 1 mmol) was added trifluoroacetic acid (1.8 ml), the mixture was stirred for 5 minutes and the reaction solution was concentrated. Crystallization from ether gave 492 mg (yield 100%) of 1-[2-(benzoxazol-2-ylthio)ethyl]piperazine ditrifluoroacetate.

To a solution of 2,6-diisopropylaniline (1.77 g, 10 mmol) in chloroform (30 ml) was added triethylamine (1.11 g, 11 mmol), then acryloyl acid chloride (905 mg, 10 mmol) was gradually dropped thereinto with ice-cooling and the mixture was stirred for 1 hour. The reaction solution was concentrated, diluted with water and extracted with ethyl acetate. The organic layer was washed with diluted hydrochloric acid, saturated aqueous solution of sodium bicarbonate, water and saturated sodium chloride solution successively and dried over anhydrous sodium sulfate, the solvent was evaporated therefrom and the resulting crystals were recrystallized from hexane-ether to provide 1.9 g (yield 82%) of N-(2,6-diisopropylphenyl)acrylamide as colorless needles.

Triethylamine (142 mg, 1.4 mmol) was added to a solution of 1-[2-(benzoxazol-2-ylthio)ethyl]-piperazine ditrifluoroacetate (344 mg, 0.7 mmol) as prepared in the above into ethanol (10 ml), then N-(2,6-diisopropylphenyl)acrylamide (162 mg, 0.7 mmol) was added thereto and the mixture was heated to reflux for 3 days. The reaction solution was concentrated, diluted with water and extracted with ethyl acetate. The organic layer was washed with aqueous solution of sodium bicarbonate, water and saturated sodium chloride solution successively and dried over anhydrous sodium sulfate and the solvent was evaporated therefrom. The residue was purified by a silica gel column chromatography (25 g of silica gel; developing solvent, hexane:acetone = 5:1∼1:1) and the resulting crystals were recrystallized from hexane-ether-acetone to provide 165 mg (yield 48%) of the desired compound as colorless needles.
Melting point: 125-127°C.
IR (KBr) cm⁻¹: 3433, 3253, 1647, 1500, 1455.
1H-NMR (d₆-DMSO) δ :
1.12 (12H, d, J = 6.8 Hz), 2.40-2.55 (10H, m), 2.65 (2H, t, J = 6.8 Hz), 2.76 (2H, t, J = 6.8 Hz), 3.13 (2H, sept, J = 6.8 Hz), 3.46 (2H, t, J = 6.8 Hz), 7.10 (2H, d, J = 7.8 Hz), 7.20 (1H, t, J = 7.8 Hz), 7.25-7.32 (2H, m), 7.54-7.59 (2H, m), 8.88 (1H, br s).
EIMS *m*/*z* (relative intensity): 494 (M⁺), 344 (100).
Elementary analysis as C₂₈H₃₈N₄O₂S
Calculated: C 67.98; H 7.74; N 11.33; S 6.48
Found: C 68.05; H 7.69; N 11.23; S 6.45.

### Example 23

### Preparation of 3-[4-[3-(benzoxazol-2-ylthio)-propyl]piperazin-1-yl]-N-(2,6-diisopropylphenyl) propanamide:

The same reaction and treatment as in Example 22 were conducted using 1-(3-hydroxypropyl)piperazine instead of 1-(2-hydroxyethyl)piperazine to provide the desired compound as colorless needles.
Melting point: 93-95°C
IR (KBr) cm⁻¹: 3418, 3229, 1645, 1504, 1454.
1H-NMR (d₆-DMSO) δ : 1.12 (12H, d, J = 6.8 Hz), 1.95 (2H, quint, J = 6.8 Hz), 2.40-2.51 (12H, m), 2.67 (2H, t, J = 6.8 Hz), 3.13 (2H, sept, J = 6.8 Hz), 3.36 (2H, t, J = 6.8 Hz), 7.10 (2H, d, J = 7.6 Hz), 7.20 (1H, t, J = 7.6 Hz), 7.25-7.33 (2H, m), 7.54-7.59 (2H, m), 8.90 (1H, br s).
EIMS *m*/*z* (relative intensity): 508 (M⁺, 100).
Elementary analysis as C₂₉H₄₀N₄O₂S
Calculated: C 68.47; H 7.92; N 11.01; S 6.30.
Found: C 68.51; H 7.90, N 10.85; S 6.30.

### Example 24.

### Preparation of 2-[4-[2-(benzoxazol-2-ylthio)-ethyl]piperazin-1-yl]-N-(2,4-bis(methylthio)-6-methyl-3-pyridyl)acetamide.

To a solution of N-[2,4-bis(methylthio)-6-methyl-pyridin-3-yl]-2-bromoacetamide (synthesized by a method according the description in U. S. Patent No. 5,583,147)(130 mg, 0.40 mmol)in DMF (2.5 ml) were added 1-[2-(benzoxazol-2-ylthio)ethyl]piperazine ditrifluoroacetate (199 mg, 0.40 mmol), potassium carbonate (224 mg, 1.62 mmol) and 18-crown-6 (53 mg, 0.20 mmol) and the mixture was stirred at 80°C for 4 hours. The reaction solution was diluted with water and extracted with ethyl acetate. The organic layer was washed with water and dried over anhydrous magnesium sulfate and the solvent was evaporated therefrom. The residue was purified by a preparative thin layer chromatography (developing solvent, chloroform:methanol = 20:1) and the resulting crystals were recrystallized from ethyl acetate-hexane to provide 169 mg (yield 83%) of the desired compound as colorless needles.
Melting point: 140-141°C.
IR (KBr) cm⁻¹: 3440, 3308, 2824, 1695, 1480.
1H-NMR (d₆-DMSO) δ :
2.34 (3H, s), 2.36 (3H, s), 2.38 (3H,s), 2.50-2.58 (8H, m), 2.72 (2H, t, J = 6.8 Hz), 3.00 (2H, s), 3.40 (2H, t, J = 6.8 Hz), 6.80 (1H,s), 7.19-7.26 (2H, m), 7.46-7.54 (2H, m), 8.66 (1H, br s).
EIMS *m*/*z* (relative intensity): 504 (M⁺), 179 (100).
Elemewtary analysis as C₂₃H₂₉N₅O₂S₃
Calculated: C 54.85; H 5.80; N 13.90
Found: C 54.92; H 5.83; N 13.64.

### Example 25

### Preparation of N-[2,4-bis(methylthio)-6-methyl-3-pyridyl]-2-[4-[2-(oxazolo[4,5-b]pyridin-2-ylthio)ethyl] piperazin-1-yl]acetamide:

The same reaction and treatment as in Example 24 were conducted using 1-[2-(oxazolo[4,5-b]pyridin-2-ylthio)ethyl] piperazine ditrifluoroacetate instead of 1-[2-(benzoxazol-2-ylthio)ethyl]piperazine ditrifluoroacetate to provide the desired compound as colorless amorphous.
IR (KBr) cm⁻¹: 3448, 3274, 2816, 1699, 1493.
1H-NMR (d₆-DMSO) δ :
2.47 (3H, s), 2.49 (3H, s), 2.50 (3H, s), 2.64-2.72 (8H, m), 2.87 (2H, t, J = 6.7 Hz), 3.13 (2H, s), 3.58 (2H, t, J = 6.7 Hz), 6.93 (1H, s), 7.34 (1H, dt, J = 8.1, 4.9 Hz), 8.01 (1H, dt, J = 8.1, 1.5 Hz), 8.46 (1H, dt, J = 4.9, 1.5 Hz), 8.81 (1H, br s).
EIMS *m*/*z* (relative intensity): 503 (M⁺), 97 (100).
Elementary analysis as C₂₂H₂₈N₆O₂S₃
Calculated: C 52.36; H 5.59; N 16.65.
Found: C 52.34; H 5.73, N 16.39.

### Example 26

### Preparation of N-[2,4-bis(methylthio)-6-methyl-3-pyridyl]-2-[4-2-[7-methoxycarbonylbenzoxazol-2-ylthio)ethyl]piperazin-1-yl]acetamide:

The same reaction and treatment as in Example 24 were conducted using 1-[2-(7-methoxycarbonyrbenzoxazol-2 ylthio)ethyl] piperazine ditrifluoroacetate instead of 1-[2-(benzoxazol-2-ylthio)ethyl]piperazine ditrifluoroacetate to provide the desired compound as colorless crystals.
Melting point: 125-127°C.
IR (KBr) cm⁻¹: 3434, 3303, 1724, 1702, 1482.
1H-NMR (CDCl₃) δ :
2.42 (3H, s), 2.50 (3H, s), 2.52 (3H, s), 2.63-2.85 (8H, m), 2.87 (2H, t, J = 6.8 Hz), 3.20 (2H, s), 3.52 (2H, t, J = 6.8 Hz), 4.00 (3H, s), 6.67 (1H, s), 7.35 (1H, t, J = 7.8 Hz), 7.77 (1H, dd, J = 7.8, 1.2 Hz), 7.88 (1H, dd, J = 7.8, 1.2 Hz), 8.55 (1H, br s).
EIMS *m*/*z* (relative intensity): 561 (M⁺), 334 (100).
Elementary analysis as C₂₅H₃₁N₅O₄S₃
Calculated: C 53.46; H 5.56; N 12.47.
Found: C 53.41; H 5.49, N 12.32.

### Example 27

### Preparation of 3-[4-[2-(benzoxazol-2-ylthio)ethyl] piperazin-1-yl]-N-[2,4-bis(methylthio)-6-methyl-3-pyridyl]propanamide:

The same reaction and treatment as in Example 22 were conducted using 3-amino-2,4-bis(methylthio)-6-methylpyridine instead of 2,6-diisopropylaniline to provide the desired compound as colorless powdery crystals.
Melting point: 110-112°C.
IR (KBr) cm⁻¹: 3439, 3242, 2814, 1648, 1500.
1H-NMR (CDCl₃) δ :
1.54-1.64 (2H, m), 2.40 (3H, s), 2.50 (3H, s), 2.51 (3H, s), 2.46-2.82 (12H, m), 3.47 (2H, t, J = 6.8 Hz), 6.65 (1H, s), 7.21-7.30 (2H, m), 7.42 (1H, dd, J = 7.6, 1.0 Hz), 7.57 (1H, dd, J = 7.0, 0.8 Hz), 10.35 (1H, br s).
Elementary analysis as C₂₄H₃₁N₅O₂S₃
Calculated: C 55.68; H 6.04; N 13.53.
Found: C 55.76; H 5.99, N 13.39.

### Example 28

### Preparation of 2-[4-[3-(benzoxazol-2-ylthio)propyl] piperazin-1-yl]-N-[2,4-bis(methylthio)-6-methyl-3-pyridyl] acetamide:

The same reaction and treatment as in Example 24 were conducted using 1-[3-(benzoxazol-2-ylthio)propyl] piperazine ditrifluoroacetate instead of 1-[2-(benzoxazol-2-ylthio)ethyl]piperazine ditrifluoroacetate to provide the desired compound as colorless powdery crystals.
Melting point: 160-161°C.
IR (KBr) cm⁻¹: 3441, 3312, 2809, 1699, 1482.
1H-NMR (d₆-DMSO) δ :
1.89 (2H, t, J = 7.1 Hz), 2.33 (3H, s), 2.36 (3H, s), 2.38 (3H, s), 2.39-2.45 (6H, m), 2.54-2.60 (4H, m), 3.01 (2H, s), 3.29 (2H, t, J = 7.1 Hz), 6.80 (1H, s), 7.19-7.25 (2H, m), 7.48-7.52 (2H, m), 8.67 (1H, br s).
Elementary analysis as C₂₄H₃₁N₅O₂S₃
Calculated: C 55.68; H 6.04; N 13.53.
Found: C 55.83; H 6.10, N 13.17.

### Example 29

### Preparation of N-[2,4-bis(methylthio)-6-methyl-3-pyridyl]-2-[4-[3-(oxazolo[4,5-b]pyridin)-2-ylthio)-propyl] piperazin-1-yl]acetamide:

The same reaction and treatment as in Example 24 were conducted using 1-[3-(oxazolo[4,5-b]pyridin-2-ylthio)propyl]piperazine ditrifluoroacetate instead of 1-[2-(benzoxazol-2-ylthio)ethyl]piperazine ditrifluoroacetate to provide the desired compound as colorless powdery crystals.
Melting point: 79-82°C.
IR (KBr) cm⁻¹: 3433, 3291, 2818, 1701, 1493.
1H-NMR (CDCl₃) δ :
2.07-2.17 (2H, m), 2.42 (3H, s), 2.49 (3H, s), 2.52 (3H, br s), 2.52-2.66 (8H, m), 2.72-2.87 (2H, m), 3.22 (2H, s), 3.44 (2H, t, J = 7.1 Hz), 6.67 (1H, s), 7.18 (1H, dd, J = 8.1, 5.1 Hz), 7.69 (1H, dd, J = 8.1, 1.5 Hz), 8.46 (1H, dd, J = 5.1, 1.5 Hz), 8.54 (1H, br s).

### Example 30

### Preparation of N-[2,4-bis(methylthio)-6-methyl-3-pyridyl)-2-[4-[3-(7-methoxycarbonylbenzoxazol-2-ylthio)-propyl]piperazin-1-yl]acetamide:

The same reaction and treatment as in Example 24 were conducted using 1-[3-(7-methoxycarbonylbenzoxazol-2-ylthio)propyl]piperazine ditrifluoroacetate instead of 1-[2-(benzoxazol-2-ylthio)ethyl]piperazine ditrifluoroacetate to provide the desired compound as colorless powdery crystals.
Melting point: 76-79°C.
IR (KBr) cm⁻¹: 3430, 3305, 2819, 1725, 1694.
1H-NMR (d₆-DMSO) δ :
1.97-2.04 (2H, m), 2.42 (3H, s), 2.44 (3H, s), 2.46 (3H, s), 2.48-2.53 (6H, m), 2.61-2.69 (4H, m), 3.06-3.11 (2H, m), 3.41 (2H, t, J = 7.1 Hz), 3.95 (3H, s), 6.89 (1H, s), 7.43 (1H, t, J = 7.8 Hz), 7.81 (1H, dd, J = 7.8, 1.2 Hz), 7.84 (1H, dd, J = 7.8, 1.2 Hz), 8.72 (1H, br s).
Elementary analysis as C₂₆H₃₃N₅O₄S₃
Calculated: C 54.24; H 5.78; N 12.16.
Found: C 54.44; H 6.01, N 11.79.

### Example 31

### Preparation of 2-[4-[2-(benzothiazol-2-ylthio)-ethyl]piperazin-1-yl]-N-(2,4-bis(methylthio)-6-methyl-3-pyridyl)acetamide:

The same reaction and treatment as in Example 24 were conducted using 1-[2-(benzothiazol-2-ylthio)ethyl] piperazine ditrifluoroacetate instead of 1-[2-(benzoxazol-2-ylthio)ethyl]piperazine ditrifluoroacetate to provide the desired compound as colorless powdery crystals.
Melting point: 136-139°C. IR (KBr) cm⁻¹: 3444, 2923, 1696, 1480, 1427.
1H-NMR (d₆-CDCl₃) δ :
2.42 (3H, s), 2.50 (3H, s), 2.52 (3H, s), 2.60-2.96 (10H, m), 3.18-3.27 (2H, m), 3.48-3.65 (2H, m), 6.67 (1H, s), 7.30 (1H, m), 7.41 (1H,m), 7.75 (1H, d, J = 8.1 Hz), 7.84 (1H, d, J = 8.1 Hz), 8.53 (1H, br s).
EIMS *m*/*z* (relative intensity): 519 (M⁺), 352 (100).
Elementary analysis as C₂₃H₂₉N₅OS₄
Calculated: C 53.15; H 5.62; N 13.47; S 24.67.
Found: C 53.17; H 5.67, N 13.24; S 24.52.

### Example 32

### Preparation of 2-[4-[2-(benzimidazol-2-ylthio)-ethyl]piperazin-1-yl]-N-[2,4-bis(methylthio)-6-methyl-3-pyridyl] acetamide dihydrochloride:

The same reaction and treatment as in Example 24 were conducted using 1-[2-(benzimidazol-2-ylthio)ethyl] piperazine ditrifluoroacetate instead of 1-[2-(benzoxazol-2-ylthio)ethyl]piperazine ditrifluoroacetate, and further reaction and treatment are performed to the obtained 2-[4-[2-(benzimidazol-2-ylthio)ethyl]piperazin-1-yl]-N-[2,4-bis(methylthio)-6-methyl-3-pyridyl] acetamide in order to provide dihydrochloride, then the compound as colorless powdery crystals was obtained.
Melting point: 214-218°C.
IR (KBr) cm⁻¹: 3240, 2923, 1679, 1485, 1438.
1H-NMR (d₆-DMSO) δ :
2.40 (3H, s), 2.41 (3H, s), 2.45 (3H, s), 3.00-3.81 (15H, m), 6.93 (1H, s), 7.13-7.23 (2H, m), 7.46-7.57 (2H, m), 9.65 (1H, br s).
EIMS *m*/*z* (relative intensity): 519 (M⁺), 352 (100).

### Example 33

### Preparation of N-[2,4-bis(methylthio)-6-methyl-3-pyridyl]-3-[4-[3-(oxazolo[4,5-b]pyridin-2-ylthio)propyl]-piperazin-1-yl]propaneamide dihydrochloride:

Triethylamine (277 mg, 2.75 mmol) was added to a solution of 3-amino-2,4-bis(methylthio)-6-methylpyridine (500 mg, 2.50 mmol) in THF (10 ml), then a solution of acryloyl acid chloride (225 mg, 2.50 mmol) in THF (3 ml) was gradually dropped thereinto and the mixture was stirred for 14 hours. The reaction solution was diluted with water and extracted with ethyl acetate. The organic layer was washed with a sodium chloride solution and dried over anhydrous sodium sulfate, the solvent was evaporated therefrom and the resulting crystals were recrystallized from chloroform-ethyl acetate-hexane to provide 276 mg (yield 44%) of N-[2,4-bis(methylthio)-6-methyl-3-pyridyl]acrylamide as colorless powdery crystals.

Triethylamine (79 mg, 0.78 mmol) was added to a solution of 1-[3-(oxazolo[4,5-b]pyridin-2-ylthio)propyl]piperazine ditrifluoroacetate ( 199 mg, 0.39 mmol) in ethanol (10 ml) , then the above-prepared N-[2,4-bis(methylthio)-6-methyl-3-pyridyl]acrylamide (100 mg, 0.39 mmol) was added thereto and the mixture was heated to reflux for four days. The reaction solution was concentrated, diluted with water and extracted with ethyl acetate. The organic layer was washed with a saturated sodium chloride solution and dried over anhydrous sodium sulfate and the solvent was evaporated therefrom. The residue was purified by a silica gel column chromatography (developing solvent, chloroform:methanol = 20:1) to provide N-[2,4-bis(methylthio)-6-methyl-3-pyridyl]-3-[4-[3-(oxazolo[4,5-b]pyridin-2-ylthio)propyl]piperazin-1-yl] propanamide. The resulting crystals were made into a dihydrochloride to provide 193 mg (yield 81%) of a desired compound as colorless powdery crystals.
Melting point: 224-227°C.
IR (KBr) cm⁻¹: 3413, 2922, 2424, 1683, 1404.
1H-NMR (CD₃OD) δ :
2.38-2.47 (2H, m), 2.57 (3H, s), 2.63 (3H, s), 2.64 (3H, s), 3.05 (2H, t, J=7.1 Hz), 3.42 (2H, t, J=7.1 Hz), 3.47-3.74 (12H, m), 7.24 (1H, s), 7.40 (1H, dd, J=8.2, 5.1 Hz), 8.04 (1H, dd, J=8.2, 1.3 Hz), 8.44 (2H, dd, J=5.1, 1.3 Hz).
EIMS *m*/*z* (relative intensity): 532 (M⁺), 55 (100).

### Example 34

### Preparation of 2-[4-[2-(benzoxazol-2-ylthio)ethyl]-piperazin-1-yl]-N-[2,4-bis(ethylthio)-6-methyl-3-pyridyl]-acetamide:

Ethanethiol(1.55 g, 25 mmol) was dropped into a solution of sodium ethoxide (1.27 g, 25 mmol) in ethanol (50 ml) with ice-cooling and the mixture was stirred for 30 minutes. With ice-cooling, a solution of 2,4-dichloro-6-methyl-3-nitropyridine (2.1 g, 10 mmol) in DMF (40 ml) was gradually dropped thereinto. After stirring for 2 hours, the reaction mixture was extracted with ethyl acetate. The organic layer was washed with water and a saturated sodium chloride solution successively and dried over anhydrous sodium sulfate and the solvent was evaporated therefrom to provide 2.45 g (yield 95%) of 2,4-bis(ethylthio)-6-methyl-3-nitropyridine as yellow needles. The nitropyridine (775 mg, 3 mmol) was dissolved in a mixed solvent of acetic acid (30 ml) and concentrated hydrochloric acid (1.5 ml) and zinc (4 g, 60 mmol) was added little by little thereto with ice-cooling. After stirring for 10 minutes, the reaction mixture was filtered off and the filtrate was neutralized with an aqueous solution of sodium hydroxide and extracted with ethyl acetate. The organic layer was washed with water and a saturated sodium chloride solution successively and dried over anhydrous sodium sulfate and the solvent was evaporated therefrom to provide 590 mg (yield 86%) of 3-amino-2,4-bis(ethylthio)-6-methylpyridine as yellow oil.

Triethylamine (304 mg, 3 mmol) was added to a solution of the aminopyridine (590 mg, 2.6 mmol) in THF (10 ml), then bromoacetyl bromide (606 mg, 3 mmol) was gradually dropped thereinto with ice-cooling and the mixture was stirred at room temperature for 1 hour. The reaction mixture was filtered, the filtrate was concentrated and the residue was purified by a silica gel column chromatography (60 g of silica gel; developing solvent, hexane:acetone = 10:1 → 5:1) to provide 410 mg (yield 45%) of 2-bromo-N-[2,4-bis(ethylthio)-6-methyl-3-pyridyl]-acetamide as pale brown needles. Potassium carbonate (166 mg, 1.2 mmol) was added to a solution of the amide (105 mg, 0.3 mmol) and 1-[2-(benzoxazol-2-ylthio)ethyl]piperazine ditrifluoroacetate (147mg, 0.3 mmol) in acetonitrile (8 ml) and the mixture was stirred at room temperature for 3 hours. The reaction mixture was extracted with ethyl acetate, the organic layer was washed with water and a saturated sodium chloride solution successively and dried over sodium sulfate and the solvent was evaporated therefrom. The residue was purified by a silica gel column chromatography (25 g of silica gel; developing solvents, hexane:acetone = 3:1 → chloroform:methanol = 20:1) and the resulting crude crystals were recrystallized from acetone-hexane to provide 140 mg (yield 88%) of the desired compound as colorless crystals.
Melting point: 108-109°C.
IR (KBr) cm⁻¹: 3433, 3304, 1697 1500, 1482.
1H-NMR (d₆-DMSO) d:
1.32 (3H, t, J=7.3 Hz), 1.35 (3H, t, J=7.3 Hz), 2.47 (3H, s), 2.64-2.70 (4H, m), 2.74-2.81 (4H, m), 2.85 (2H, t, J=6.8 Hz), 2.93 (2H, q, J=7.3 Hz), 3.16 (2H, q, J=7.3 Hz), 3.20 (2H, s), 3.49 (2H, t, J = 6.8 Hz), 6.70 (1H, s), 7.22-7.30 (2H, m), 7.44 (1H, m), 7.59 (1H, m), 8.53 (1H, br s).
EIMS *m*/*z* (relative intensity): 531 (M⁺), 381 (100).
Elementary analysis as C₂₅H₃₃N₅O₂S₃
Calculated: C 56.47; H 6.25; N 13.17; S 18.09
Found: C 56.73; H 6.23; N 13.08; S 18.20.

### Example 35

### Preparation of -2-[4-[2-(7-methoxycarbonylbenzoxazol - 2-ylthio)ethyl]piperazin-1-yl]-N-[2,4-bis(ethylthio)-6-methyl-3-pyridyl) acetamide:

The same reaction and treatment as in Example 34 were conducted using 1-[2-(7-methoxycarbonylbenzoxazol-2-ylthio)ethyl]piperazine ditrifluoroacetate instead of 1-[2-(benzoxazol-2-ylthio)ethyl]piperazine ditrifluoroacetate to provide the desired compound as colorless crystals.
Melting point: 118-119°C.
IR (KBr) cm⁻¹: 3424, 3350, 1718, 1505.
1H-NMR (CDCl₃) δ:
1.32 (3H, t, J=7.4 Hz), 1.36 (3H, t, J=7.4 Hz), 2.47 (3H, s), 2.66-2.70 (4H, m), 2.74-2.78 (4H, m), 2.87 (2H, t, J=6.8 Hz), 2.93 (2H, q, J=7.4 Hz), 3.15 (2H, q, J=7.4 Hz), 3.19 (2H, s) 3.52 (2H, t, J = 6.8 Hz), 4.00 (3H, s), 6.70 (1H, s), 7.34 (1H, dd, J = 8.1, 7.8 Hz), 7.77 (1H, dd, J = 8.1, 1.2 Hz), 7.88 (1H, dd, J = 7.8, 1.2 Hz), 8.53 (1H, br s).
EIMS *m*/*z* (relative intensity): 589 (M⁺), 380 (100).
Elementary analysis as C₂₇H₃₅N₅O₄S₃
Calculated: C 54.99; H 5.98; N 11.87; S 16.31.
Found: C 54.98; H 5.96, N 11.75; S 16.26.

### Example 36

### Preparation of 2-[4-[2-(benzoxazol-2-ylthio)ethyl]-piperazin-1-yl]-N-[2,4-bis(isopropylthio)-6-methyl-3-pyridyl]acetamide dihydrochloride:

2-Propanethiol (1.90 g, 25 mmol) was dropped into a solution of sodium isopropoxide (2.05 g, 25 mmol) in 2-propanol (50 ml) with ice-cooling and the mixture was stirred for 30 minutes. With ice-cooling, a solution of 2,4-dichloro-6-methyl-3-nitropyridine (2.07 g, 10 mmol) in DMF (40 ml) was gradually dropped thereinto. After stirring for 2 hours, the reaction mixture was extracted with ethyl acetate. The organic layer was washed with water and a saturated sodium chloride solution successively and dried over sodium sulfate and the solvent was evaporated therefrom to provide 2.77 g (yield 97%) of 2,4-bis(isopropylthio)-6-methyl-3-nitropyridine as yellow needles. The nitropyridine (1.08 g, 3.77 mmol) was dissolved in a mixed solvent of acetic acid (35 ml) and concentrated hydrochloric acid (1.6 ml) and then zinc (2.96 g, 45.25 mmol) was added little by little thereto with ice-cooling. After stirring for 1 hour, the reaction mixture was filtered and the filtrate was neutralized with an aqueous solution of sodium bicarbonate and extracted with chloroform. The organic layer was washed with water and a saturated sodium chloride solution successively and the residue obtained by evaporation of the solvent therefrom was purified by a silica gel column chromatography (developing solvent, hexane:ethyl acetate = 30:1 → 10:1) to provide 774 mg (yield 80%) of 3-amino-2,4-bis(isopropylthio)-6-methylpyridine as yellow oil.

Triethylamine (336 mg, 3.32 mmol) was added to a solution of the aminopyridine (774 mg, 3.02 mmol) inn THF (10 ml), then bromoacetic acid bromide (732 mg, 3.62 mmol) was gradually dropped thereinto with ice-cooling and the mixture was stirred for 17 hours. The reaction mixture was filtered, the filtrate was concentrated and the residue was purified by a silica gel column chromatography (developing solvent, hexane:ethyl acetate = 10:1) to provide 595 mg (yield 52%) of N-[2,4-bis(isopropylthio)-6-methyl-3-pyridyl]-2-bromoacetamide as colorless powdery crystals.

After that, the same reaction and treatment as in Example 24 were conducted using N-[2,4-bis(isopropylthio)-6-methyl-3-pyridyl]-2-bromoacetamide instead of N-[2,4-bis (methylthio)-6-methyl-3-pyridyl]-2-bromoacetamide and the resulting 2-[4-[2-(benzoxazol-2-ylthio)-ethyl]piperazin-1-yl]-N-[2,4-bis(isopropylthio)-6-methyl-3-pyridyl]acetamide was converted to a dihydrochloride to provide a desired compound as colorless powdery crystals.
Melting point: 159-164°C.
IR (KBr) cm⁻¹: 3421, 2965, 1695, 1502, 1454.
1H-NMR (d₆-DMSO) δ:
1.29-1.35 (12H, m), 2.46 (3H, s), 2.93-3.33 (12H, m), 3.52(2H, t, J=7.0 Hz), 3.61 (1H, m), 3.94 (1H, m), 7.00 (1H, m), 7.30-7.35 (2H, m), 7.57-7.63 (2H, m), 8.16 (1H, s).
EIMS *m*/*z* (relative intensity): 559 (M⁺), 125 (100).

### Example 37

### Preparation of 2-[4-[2-(benzimidazol-2-ylthio)-ethyl]piperazin-1-yl]-N-(2,4-bis(isopropylthio)-6-methyl-3-pyridyl)acetamide:

1-[2-(Benzimidazol-2-ylthio)ethyl]piperazine ditrifluoroacetate was obtained as colorless powdery crystals according to Example 22 using 2-mercaptobenzimidazole instead of 2-mercaptobenzooxazole.

Potassium carbonate (146 mg, 1.08 mmol) was added to a solution of the above-prepared 1-[2-(benzimidazol-2-ylthio)ethyl]piperazine ditrifluoroacetate (160 mg, 0.27 mmol) and N-[2,4-bis(isopropylthio)-6-methyl-3-pyridyl]-2-bromoacetamide (100 mg, 0.27 mmol) in acetonitrile (5 ml) and the mixture was stirred at room temperature for 3 hours. The reaction solution was diluted with water and extracted with ethyl acetate. The organic layer was washed with a sodium chloride solution and dried over anhydrous sodium sulfate and the solvent was evaporated therefrom. The residue was purified by a silica gel column chromatography (developing solvent, chloroform: methanol = 20:1) and the resulting crystals were recrystallized from ether-hexane to provide 104 mg (yield 70%) of the desired compound as colorless powdery crystals.
Melting point: 186-188°C.
IR (KBr) cm⁻¹: 3197, 2963, 2816, 1660, 1518, 1491.
1H-NMR (CDCl₃) δ :
1.35 (6H, d, J=6.8 Hz), 1.36 (6H, d, J = 6.8 Hz), 2.47 (3H, s), 2.88-3.05 (8H, m), 3.07 (2H, t, J = 5.4 Hz), 3.30 (2H, t, J = 5.4 Hz), 3.34 (2H, s), 3.51 (1H, sept, J = 6.8 Hz), 4.04 (1H, sept, J = 6.8 Hz), 6.76 (1H, s), 7.19-7.23 (2H, m), 7.51-7.56 (2H, m), 8.34 (1H, br s).
EIMS *m*/*z* (relative intensity): 558 (M⁺), 125 (100).

### Example 38

### Preparation of 2-[4-[2-(benzothiazol-2-ylthio)-ethyl]piperazin-1-yl]-N-[2,4-bis(isopropylthio)-6-methyl-3-pyridyl] acetamide dihydrochloride:

The same reaction and treatment as in Example 37 were conducted using 2-mercaptobenzothiazole instead of 2-mercaptobenzimidazole to provide the desired compound as colorless powdery crystals.
Melting point: 139-142°C.
IR (KBr) cm⁻¹: 3424, 2962, 1690, 1456, 1428.
1H-NMR (d₆-DMSO) δ :
1.31 (6H, d, J = 6.6 Hz), 1.34 (6H, d, J = 6.6 Hz), 2.45 (3H, s), 3.05-3.37 (10H, m), 3.46-3.52 (2H, m), 3.61 (1H, sept, J = 6.6 Hz), 3.74 (2H, t, J = 7.2 Hz), 3.93 (1H, sept, J = 6.6 Hz), 6.99 (1H,s), 7.37 (1H, m), 7.47 (1H, m), 7.86 (1H, d, J = 8.4 Hz), 7.96 (1H, d, J = 7.8 Hz), 8.53 (1H, br s).
EIMS *m*/*z* (relative intensity): 575 (M⁺), 125 (100).

### Example 39

### Preparation of N-[2,4-bis(isopropylthio)-6-methyl-3-pyridyl]-2-[4-[2-(7-methoxycarbonylbenzoxazol-2-ylthio)-ethyl]piperazin-1-yl]acetamide:

The same reaction and treatment as in Example 37 were conducted using 7-methoxycarbonyl-2-mercaptobenzooxazole instead of 2-mercaptobenzimidazole to provide the desired compound as colorless amorphous.
Melting point: 60-63°C.
IR (KBr) cm⁻¹: 3302, 2960, 1726, 1702, 1482.
1H-NMR (CDCl₃) δ :
1.35 (6H, d, J = 6.6 Hz), 1.36 (6H, d, J = 6.6 Hz), 2.46 (3H,s), 2.62-2.93 (10H, m), 3.14-3.24 (2H, m), 3.46-3.57 (2H, m), 3.96-4.06 (2H, m), 4.00 (3H, s), 6.76 (1H, s), 7.35 (1H, m), 7.76 (1H, d, J = 7.8 Hz), 7.88 (1H, d, J = 7.5 Hz), 8.50 (1H, br s).
EIMS *m*/*z* (relative intensity): 617 (M⁺), 334 (100).
Elementary analysis as C₂₉H₃₉N₅O₄S₃
Calculated: C 56.38; H 6.36; N 11.34; S 15.57.
Found: C 56.30; H 6.25, N 11.21; S 15.50.

### Example 40

### Preparation of N-[2,4-bis(isopropylthio)-6-methyl-3-pyridyl]-2-[4-[2-(oxazolo[4,5-b]pyridin-2-ylthio)-ethyl]piperazin-1-yl] acetamide dihydrochloride:

The same reaction and treatment as in Example 37 were conducted using 2-mercaptooxazolo[4,5-b]pyridine instead of 2-mercaptobenzimidazole to provide the desired compound as pale yellow powdery crystals.
Melting point: 170-172°C.
IR (KBr) cm⁻¹: 3416, 2967, 1699, 1615, 1496.
1H-NMR (d₆-DMSO) δ : 1.31 (6H, d, J = 6.7 Hz), 1.34 (6H, d, J = 6.7 Hz), 2.45 (3H,s), 3.00-3.38 (10H, m), 3.56-3.65 (3H, m), 3.74 (2H, t, J = 7.1 Hz), 3.94 (1H, sept, J = 6.7 Hz), 6.99 (1H, s), 7.33 (1H, dd, J = 8.0, 4.9 Hz), 8.00 (1H, dd, J = 8.0, 1.4 Hz), 8.44 (1H, dd, J = 4.9, 1.4 Hz), 9.30 (1H, br s).
EIMS *m*/*z* (relative intensity): 560 (M⁺), 277 (100).

### Example 41

### Preparation of 2-[4-[3-(benzoxazol-2-ylthio)-propyl]piperazin-1-yl]-N-(2,4-bis(isopropylthio)-6-methyl-3-pyridyl)acetamide dihydrochloride:

Potassium carbonate (120 mg, 0.88 mmol) was added to a solution of 1-[3-(benzoxazol-2-ylthio)propyl]piperazine ditrifluoroacetate (110 mg, 0.22 mmol) and N-[2,4-bis(isopropylthio)-6-methyl-3-pyridyl]-2-bromoacetamide (82 mg, 0.22 mmol) in acetonitrile (4 ml) and the mixture was stirred for 3 hours. The reaction solution was diluted with water and extracted with ethyl acetate. The organic layer was washed with a sodium chloride solution and dried over anhydrous sodium sulfate and the solvent was evaporated therefrom. The residue was purified by a preparative thin layer chromatography (developing solvent, chloroform:methanol = 20:1) and the resulting 2-[4-[3-(benzoxazol-2-ylthio)-propyl]piperazin-1-yl]-N-(2,4-bis(isopropylthio)-6-methyl-3-pyridyl)acetamide was converted to a dihydrochloride to provide 71 mg (yield 51%) of the desired compound as colorless powdery crystals.
Melting point: 178-181°C.
IR (KBr) cm⁻¹: 3424, 2964, 1691, 1499, 1454.
1H-NMR (d₆-DMSO) δ :
1.22 (6H, d, J = 6.6 Hz), 1.25 (6H, d, J = 6.6 Hz), 2.17-2.25 (2H, m), 2.37 (3H, s), 2.83-3.30 (12H, m), 3.38 (2H, t, J = 7.1 Hz), 3.51 (1H, sept, J = 6.6 Hz), 3.84 (1H, sept, J = 6.6 Hz), 6.90 (1H, s), 7.21-7.28 (2H, m), 7.49-7.54 (2H, m), 8.94 (1H, br s).
EIMS *m*/*z* (relative intensity): 573 (M⁺), 111 (100).

### Example 42

### Preparation of 2-[4-[2-(benzoxazol-2-ylthio)-ethyl]-piperazin-1-yl]-N-(2-methylthio-3-pyridyl)acetamide dihydrochloride:

Triethylamine (197 mg, 1.95 mmol) was added to a solution of 3-amino-2-(methylthio)pyridine (248 mg, 1.77 mmol) in THF (5 ml), then a solution of bromoacetyl bromide (428 mg, 2.12 mmol) in THF (1 ml) was dropped thereinto and the mixture was stirred for 17 hours. The reaction solution was diluted with water and extracted with ethyl acetate. The organic layer was washed with a saturated sodium chloride solution and dried over anhydrous sodium sulfate, the solvent was evaporated therefrom and the residue was purified by a silica gel column chromatography (developing solvent, hexane:ethyl acetate = 5:1) to provide 104 mg (yield 22%) of N-(2-methylthio-3-pyridyl)-2-bromoacetamide as colorless powdery crystals.

Potassium carbonate (214 mg, 1.55 mmol) was added to a solution of 1-[2-(benzoxazol-2-ylthio)ethyl]piperazine ditrifluoroacetate (190 mg, 0.39 mmol) and N-(2-methylthio-3-pyridyl)-2-bromoacetamide (101 mg, 0.39 mmol) in acetonitrile (5 ml) and the mixture was stirred for 3 hours. The reaction solution was diluted with water and extracted with ethyl acetate. The organic layer was washed with a saturated sodium chloride solution and dried over anhydrous sodium sulfate and the solvent was evaporated therefrom. The residue was purified by a preparative thin layer chromatography (developing solvent, chloroform:methanol = 20:1) to provide 147 mg (yield 74%) of 2-[4-[2-(benzoxazol-2-ylthio)-ethyl]piperazin-1-yl]-N-(2-methylthio-3-pyridyl)acetamide. The resulting crystals were converted to a dihydrochloride to provide the desired compound as colorless powdery crystals.
Melting point: 186-189°C.
IR (KBr) cm⁻¹: 3424, 2926, 2553, 1702, 1504, 1453.
1H-NMR (CD₃OD) δ :
2.65 (3H, s), 3.26-3.37 (5H, m), 3.60-3.80 (7H, m), 3.79 (2H, s), 7.31-7.38 (3H, m), 7.55 (1H, m), 7.61 (1H, m), 8.13 (1H, m), 8.38 (1H, m).
EIMS *m*/*z* (relative intensity): 443 (M⁺), 125 (100).

### Example 43

### Preparation of 2-[4-[2-(benzoxazol-2-ylthio)-ethyl]piperazin-1-yl]-N-(2-methyl-6-methylthio-3-pyridyl) acetamide:

The same reaction and treatment as in Example 42 were conducted using 3-amino-2-methyl-6-(methylthio)pyridine instead of 3-amino-2-(methylthio)pyridine to provide the desired compound as colorless powdery crystals.
Melting point: 116-117°C.
IR (KBr) cm⁻¹: 3265, 2944, 1670, 1497, 1453.
1H-NMR (CDCl₃) δ :
2.49 (3H, s), 2.54 (3H, s), 2.60-2.80 (8H, s), 2.82-2.95 (8H, m), 3.12-3.24 (2H, m), 3.43-3.57 (2H, m), 7.04 (1H, d, J = 8.6 Hz), 7.22-7.32 (2H, m), 7.44 (1H, d, J=7.3 Hz), 7.58 (1H, d, J = 7.3 Hz), 8.30 (1H, d, J = 8.6 Hz), 9.20 (1H, br s).
EIMS *m*/*z* (relative intensity): 457 (M⁺), 125 (100).
Elementary analysis as C₂₂H₂₇N₅O₂S₂·0.4H₂O
Calculated: C 56.85; H 6.03; N 15.07; S 13.80.
Found: C 56.94; H 5.90, N 14.94; S 13.65.

### Example 44

### Preparation of 2-[4-[2-(benzoxazol-2-ylthio)-ethyl]piperazin-1-yl]-N-(6-methyl-2-methylthio-3-pyridyl) acetamide dihydrochloride:

The same reaction and treatment as in Example 42 were conducted using 3-amino-6-methyl-2-(methylthio)pyridine instead of 3-amino-2-(methylthio)pyridine to provide the desired compound as colorless powdery crystals.
Melting point: 200-203°C.
IR (KBr) cm⁻¹: 3416, 2924, 1698, 1507, 1455.
1H-NMR (d₆-DMSO) δ :
2.42 (3H, s), 2.49 (3H, s), 3.05-3.13 (4H, m), 3.22-3.30 (4H, m), 3.38 (2H, t, J = 7.4 Hz), 3.49 (2H, s), 3.70 (2H, t, J = 7.4 Hz), 6.94 (1H, d, J = 7.9 Hz), 7.25-7.31 (2H, m), 7.52-7.60 (2H, m), 7.68 (1H, d, J = 7.9 Hz), 9.32 (1H, br s).
EIMS *m*/*z* (relative intensity): 457 (M⁺), 125 (100).

### Example 45

### Preparation of 2-[4-[2-(benzoxazol-2-ylthio)-ethyl]piperazin-1-yl]-N-[2,4-dimethoxy-6-methyl-3-pyridyl) acetamide:

The same reaction and treatment as in Example 42 were conducted using 3-amino-2,4-dimethoxy-6-methylpyridine instead of 3-amino-2-(methylthio)pyridine to provide the desired compound as colorless powdery crystalsy crystals.
Melting point: 113-115°C.
IR (KBr) cm⁻¹: 3326, 2944, 1698, 1600, 1504.
1H-NMR (CDCl₃) δ :
2.42 (3H, s), 2.57-2.97 (10H, m), 3.13-3.22 (2H, m), 3.45-3.57 (2H,m), 3.84 (3H, s), 3.91 (3H, s), 6.42 (1H, s), 7.22-7.31 (2H, m), 7.44 (1H, m), 7.58 (1H, m), 8.22 (1H, br s).
EIMS *m*/*z* (relative intensity): 471 (M⁺), 307 (100).
Elementary analysis as C₂₃H₂₉N₅O₄S
Calculated: C 58.58; H 6.20; N 14.85; S 6.80.
Found: C 58.54; H 6.24, N 14.88; S 6.79.

### Example 46

### Preparation of 2-[4-[2-(benzoxazol-2-ylthio)-ethyl]piperazin-1-yl]-N-[4,6-bis(methylthio)-5-pyrimidyl)-acetamide:

Triethylamine (78 mg, 0.8 mmol) was added to a solution of 4,6-bis(methylthio)-5-aminopyrimidine (120 mg, 0.7 mmol) in THF (2 ml) at room temperature, then bromoacetyl bromide (141 mg, 0.7 mmol) was dropped thereinto and the mixture was stirred for 1 hour. Then the same amounts of triethylamine and bromoacetyl bromide were added followed by stirring for 1 hour. The reaction solution was diluted with water and extracted with ether. The organic layer was washed with water and a saturated sodium chloride solution successively and dried over magnesium sulfate and the solvent was evaporated therefrom. The residue was purified by a silica gel column chromatography (20 g of silica gel; developing solvent, hexane:acetone = 5:1) to provide 78 mg (yield 40%) of N-[4,6-bis(methylthio)-5-pyrimidyl]-2-bromoacetamide as pale yellow crystals.

Potassium carbonate (104 mg, 0.75 mmol) was added to a solution of 1-[2-(benzoxazol-2-ylthio)ethyl]piperazine ditrifluoroacetate (123 mg, 0.25 mmol) in acetonitrile (3 ml), then the above-prepared amide (78 mg, 0.25 mmol) was added and the mixture was stirred at 50°C for 1 hour. The reaction solution was extracted with ether. The organic layer was washed with water and a saturated sodium chloride solution successively and dried over sodium sulfate and the solvent was evaporated therefrom. The residue was purified by a preparative thin layer chromatography (developing solvent, chloroform:methanol = 50:1) to provide 70 mg (yield 57%) of the desired compound as pale yellow needles.
Melting point: 171-172°C.
IR (KBr) cm⁻¹: 3441, 3280, 1699, 1528, 1412.
1H-NMR (CDCl₃) δ :
2.54 (6H, s), 2.63-2.80 (8H, m), 2.86 (2H, t, J = 6.8 Hz), 3.21 (2H, s), 3.49 (2H, t, J = 6.8 Hz), 7.22-7.32 (2H, m), 7.44 (1H, m), 7.59 (1H, m), 8.67 (1H, s), 8.67 (1H, br s).
EIMS *m*/*z* (relative intensity): 489 (M⁺-1), 339 (100).
Elementary analysis as C₂₁H₂₆N₆O₂S₃
Calculated: C 51.41; H 5.34; N 17.13; S 19.60
Found: C 51.42; H 5.45; N 16.90; S 19.41.

### Example 47

### Preparation of 2-[4-[2-(benzoxazol-2-ylthio)-ethyl]piperazin-1-yl]-N-(2,4,6-trimethyl-3-pyridyl)-acetamide:

The same reaction and treatment as in Example 42 were conducted using 3-amino-2,4,6-trimethylpyridine instead of 3-amino-2-(methylthio)pyridine to provide the desired compound as colorless powdery crystals.
Melting point: 159-160°C.
IR (KBr) cm⁻¹: 3262, 2943, 1666, 1500, 1453.
1H-NMR (CDCl₃) δ :
2.21 (3H, s), 2.46 (3H, s), 2.51 (3H,s), 2.65-2.80 (8H, m), 2.89 (2H, t, J = 6.9 Hz), 3.22 (2H, s), 3.51 (2H, t, J = 6.9 Hz), 6.94 (1H, s), 7.22-7.31 (2H, m), 7.44 (1H, m), 7.59 (1H, m), 8.66 (1H, br s).
EIMS *m*/*z* (relative intensity): 439 (M⁺), 163 (100).
Elementary analysis as C₂₃H₂₉N₅O₂S
Calculated: C 62.33; H 6.69; N 15.80; S 7.24.
Found: C 62.26; H 6.68; N 15.62; S 7.16.

### Examples 48

### Preparation of 2-[4-[2-(benzoxazol-2-ylthio)-ethyl]piperazin-1-yl]-N-(2,4,6-triisopropylphenyl)-acetamide:

Triethylamine (111 mg, 1.1 mmol) was added to a solution of 2,4,6-triisopropylaniline (219 mg, 1.0 mmol) in chloroform (3 ml), then bromoacetyl bromide (222 mg, 1.1 mmol) was gradually dropped thereinto with ice-cooling and the mixture was srirred for 1 hour. The reaction mixture was concentrated and the residue was extracted with ethyl acetate. The organic layer was washed with diluted hydrochloric acid, an aqueous solution of sodium bicarbonate and a saturated sodium chloride solution successively and dried over magnesium sulfate and the solvent was evaporated therefrom. The residue was purified by a silica gel column chromatography (15 g of silica gel; developing solvent, hexane:acetone = 5:1) followed by recrystallizing from hexane-ether to provide 275 mg (yield 81%) of 2-Bromo-N-(2,4,6-triisopropylphenyl)acetamide as colorless needles.

Potassium carbonate (124 mg, 0.9 mmol) was added to a solution of 1-[2-(benzoxazol-2-ylthio)ethyl]piperazine ditrifluoroacetate (147 mg, 0.3mmol) in acetonitrile (5 ml) at room temperature, then the above-prepared anilide (102 mg, 0.3 mmol) was added and the mixture was stirred for 4 hours. The reaction solution was diluted with water and extracted with ethyl acetate. The organic layer was washed with water and a saturated sodium chloride solution successively and dried over sodium sulfate and the solvent was evaporated therefrom. The residue was purified by a silica gel column chromatography (12 g of silica gel; developing solvent, hexane:acetone = 5:1) and then recrystallized from hexane-acetone to provide 75 mg (yield 48%) of the desired compound as colorless needles.
Melting point: 160-163°C.
IR (KBr) cm⁻¹: 3433, 3239, 1666, 1498, 1455.
1H-NMR (CDCl₃) δ : 1
1.21 (12H, d, J = 6.8 Hz), 1.24 (6H, d, J = 6.0 Hz), 2.60-2.76 (8H,m), 2.84 (2H, t, J = 6.8 H), 2.89 (1H, sept, J = 6.0 Hz), 2.98 (2H, sept, J = 6.8 Hz), 3.21 (2H, s), 3.49 (2H, t, J = 6.8 Hz), 7.03 (2H, s), 7.22-7.31 (2H, m), 7.44 (1H, m), 7.58 (1H, m), 8.67 (1H, br s).
EIMS *m*/*z* (relative intensity): 522 (M⁺, 100).
Elementary analysis as C₃₀H₄₂N₄O₂S
Calculated: C 68.93; H 8.10; N 10.72; S 6.13
Found: C 68.89; H 8.05; N 10.64; S 6.11.

### Example 49

### Preparation of 2-[4-[2-(benzimidazol-2-ylthio)-ethyl]piperazin-1-yl]-N-(2,4,6-triisopropylphenyl)-acetamide:

The same reaction and treatment as in Example 48 were conducted using 2-mercaptobenzimidazole instead of 2-mercaptobenzooxazole to provide the desired compound as colorless crystals.
Melting point: 217-218°C.
IR (KBr) cm⁻¹: 3440, 3292, 2959, 1670, 1498.
1H-NMR (CDCl₃) δ :
1.22 (12H, d, J = 7.1 Hz), 1.25 (6H, d, J = 7.1 Hz), 2.74-2.97 (11H, m), 2.99 (2H, sept, J = 7.1 Hz), 3.24-3.27 (2H, m), 3.35 (2H, s), 7.04 (2H, s), 7.19-7.24 (2H, m), 7.37 (1H, m), 7.65 (1H, m), 8.43 (1H, br s).
EIMS *m*/*z* (relative intensity): 521 (M⁺), 372 (100).
Elementary analysis as C₃₀H₄₃N₅OS
Calculated: C 69.06; H 8.31; N 13.41; S 6.14.
Found: C 69.18; H 8.31; N 13.16; S 6.14.

### Example 50

### Preparation of 2-[4-[2-(benzothiazol-2-ylthio)-ethyl]piperazin-1-yl]-N-(2,4-6-triisopropylphenyl)-acetamide:

The same reaction and treatment as in Example 48 were conducted using 2-mercaptobenzothiazole instead of 2-mercaptobenoxazole to provide the desired compound as colorless crystals.
Melting point: 117-118°C.
IR (KBr) cm⁻¹: 3435, 3263, 1683, 1668, 1493.
1H-NMR (CDCl₃) δ :
1.21 (12H, d, J = 7.1 Hz), 2.24 (6H, d, J = 7.1 Hz), 2.60-2.77 (8H, m), 2.83 (2H, t, J = 7.3 Hz), 2.89 (1H, sept, J = 7.1 Hz), 2.98 (2H, sept, J = 7.1 Hz), 3.22 (2H, s), 3.54 (2H, t, J = 7.3 Hz), 7.03 (2H, s), 7.29 (1H, m), 7.41 (1H, m), 7.76 (1H, m), 7.85 (1H, m), 8.56 (1H, br s).
EIMS *m*/*z* (relative intensity): 538 (M⁺), 359 (100).
Elementary analysis as C₃₀H₄₂N₄OS₂
Calculated: C 66.88; H 7.86; N 10.40; S 11.90.
Found: C 66.65; H 7.79; N 10.15; S 11.79.

### Example 51

### Preparation of 2-[4-[2-(7-methoxycarbonylbenzoxazol-2-ylthio)ethyl]piperazin-1-yl]-N-(2,4-6-triisopropylphenyl)acetamide:

The same reaction and treatment as in Example 48 were conducted using 7-methoxycarbonyl-2-mercaptobenzooxazole instead of 2-mercaptobenzooxazole to provide the desired compound as colorless crystals.
Melting point: 153-155°C.
IR (KBr) cm⁻¹: 3427, 3248, 1723, 1664, 1501.
1H-NMR (CDCl₃) δ :
1.21 (12H, d, J = 6.8 Hz), 1.24 (6H, d, J = 6.8 Hz), 2.60-2.74 (8H, m), 2.85 (2H, t, J = 6.8 Hz), 2.86 (1H, sept, J = 6.8 Hz), 2.98 (2H, sept, J = 6.8 Hz), 3.21 (2H, s), 3.51 (2H, t, J=6.8 Hz), 4.00 (3H, s), 7.03 (2H, s), 7.35 (1H, t, J = 7.8 Hz), 7.77 (1H, dd, J = 7.8, 1.2 Hz), 7.88 (1H, dd, J = 7.8, 1.2 Hz), 8.56 (1H, br s).
EIMS *m*/*z* (relative intensity): 580 (M⁺), 373 (100).
Elementary analysis as C₃₂H₄₄N₄O₄S
Calculated: C 66.18; H 7.64; N 9.65; S 5.58.
Found: C 66.27; H 7.63; N 9.46; S 5.52.

### Example 52

### Preparation of 2-[4-[2-(oxazolo[4,5-b]pyridin-2-ylthio)ethyl]piperazin-1-yl]-N-(2,4-6-triisopropylphenyl)-acetamide:

The same reaction and treatment as in Example 48 were conducted using 2-mercaptooxazolo[4,5-b]pyridine instead of 2-mercaptobenzooxazole to provide the desired compound as colorless needles.
Melting point: 144-145°C.
IR (KBr) cm⁻¹: 3434, 3247, 2959, 1668, 1490.
1H-NMR (CDCl₃) δ :
1.21 (12H, d, J = 6.8 Hz), 1.24 (6H, d, J = 6.8 Hz), 2.62-2.76 (8H, m), 2.84-2.94 (3H, m), 2.98 (2H, sept, J = 6.8 Hz), 3.22 (2H, s), 3.56 (2H, t, J = 6.7 Hz), 7.03 (2H, s), 7.19 (1H, dd, J = 8.1, 4.9 Hz), 7.70 (1H, dd, J = 8.1, 1.5 Hz), 8.46 (1H, dd, J = 4.9, 1.5 Hz), 8.56 (1H, br s).
EIMS *m*/*z* (relative intensity): 523 (M⁺), 372 (100).
Elementary analysis as C₂₉H₄₁N₅O₂S
Calculated: C 66.51; H 7.89; N 13.37; S 6.12.
Found: C 66.55; H 7.94; N 13.21; S 6.13.

### Example 53

### Preparation of 2-[4-[3-(benzoxazol-2-ylthio)-propyl]piperazin-1-yl]-N-(2,4-6-triisopropylphenyl)-acetamide:

The manner according to Example 22 were conducted using 1-(3-hydroxypropyl)piperazine instead of 1-(2-hydroxyethyl) piperazine to provide 1-[3-(benzoxazol-2-ylthio) propyl]piperazine ditrifluoroacetate, and the same reaction and treatment as in Example 48 were conducted to provide the desired compound as colorless crystals.
Melting point: 125-127°C.
IR (KBr) cm⁻¹: 3429, 3234, 2958, 1663, 1503.
1H-NMR (CDCl₃) δ :
1.21 (12H, d, J = 6.8 Hz), 1.24 (6H, d, J = 6.8 Hz), 2.04 (2H, quint, J = 7.1 Hz), 2.51-2.56 (6H, m), 2.72-2.76 (4H, m), 2.89 (1H, sept, J = 6.8 Hz), 2.98 (2H, sept, J = 6.8 Hz), 3.22 (2H, s), 3.38 (2H, t, J = 7.1 Hz), 7.03 (2H, s), 7.21-7.32 (2H, m), 7.44 (1H, m), 7.59 (1H, m), 8.58 (1H, br s).
EIMS *m*/*z* (relative intensity): 536 (M⁺, 100).
Elementary analysis as C₃₁H₄₄N₄O₂S
Calculated: C 69.37; H 8.26; N 10.44; S 5.97.
Found: C 69.28; H 8.28; N 10.43; S 5.98.

### Example 54

### Preparation of 2-[4-[3-(benzimidazol-2-ylthio)-propyl]piperazin-1-yl]-N-(2,4-6-triisopropylphenyl)-acetamide:

The same reaction and treatment as in Example 53 were conducted using 2-mercaptobenzimidazole instead of 2-mercaptobenzooxazole to provide the desired compound as colorless crystals.
Melting point: 229-231°C.(d)
IR (KBr) cm⁻¹: 3433, 3261, 2961, 1654.
1H-NMR (CDCl₃) δ :
1.22 (12H, d, J = 6.8 Hz), 1.25 (6H, d, J = 6.8 Hz), 2.02 (2H, quint, J = 6.5 Hz), 2.58-2.68 (6H, m), 2.84-2.92 (5H, m), 2.99 (2H, sept, J = 6.8 Hz), 3.31 (2H, t, J = 6.5 Hz), 3.32 (2H, s), 7.04 (2H, s), 7.17-7.24 (2H, m), 7.38 (1H, m), 7.65 (1H, m), 8.50 (1H, br s).
EIMS *m*/*z* (relative intensity): 535 (M⁺), 139 (100).
Elementary analysis as C₃₁H₄₅N₅OS
Calculated: C 69.49; H 8.47; N 13.07; S 5.98.
Found: C 69.41; H 8.44; N 12.82; S 5.90.

### Example 55

### Preparation of 2-[4-[3-(benzothiazol-2-ylthio)-propyl]piperazin-1-yl]-N-(2,4,6-triisopropylphenyl)-acetamide:

The same reaction and treatment as in Example 53 were conducted using 2-mercaptobenzothiazole instead of 2-mercaptobenzooxazole to provide the desired compound as colorless crystals.
Melting point: 107-108°C.
IR (KBr) cm⁻¹: 3436, 3227, 2956, 1669.
1H-NMR (CDCl₃) δ :
1.21 (12H, d, J = 6.8 Hz), 1.24 (6H, d, J = 6.8 Hz), 2.03 (2H, quint, J = 7.1 Hz), 2.50-2.55 (6H, m), 2.72-2.76 (4H, m), 2.89 (1H, sept, J = 6.8 Hz), 2.99 (2H, sept, J = 6.8 Hz), 3.22 (2H, s), 3.41 (2H, t, J = 7.1 Hz), 7.03 (2H, s), 7.29 (1H, m), 7.41 (1H, m), 7.76 (1H, m), 7.85 (1H, m), 8.59 (1H, br s).
EIMS *m*/*z* (relative intensity): 552 (M⁺), 385 (100).
Elementary analysis as C₃₁H₄₄N₄OS₂
Calculated: C 67.35; H 8.02; N 10.13; S 11.60.
Found: C 67.20; H 8.08; N 10.01; S 11.59.

### Example 56

### Preparation of 2-[4-[3-(7-methoxycarbonylbenzoxazol-2-ylthio)propyl]piperazin-1-yl]-N-(2,4-6-triisopropylphenyl)acetamide:

The same reaction and treatment as in Example 53 were conducted using 7-methoxycarbonyl-2-mercaptobenzooxazole instead of 2-mercaptobenzooxazole to provide the desired compound as colorless needles.
Melting point: 137-139°C.
IR (KBr) cm⁻¹: 3433, 3260, 1727, 1661, 1505.
1H-NMR (CDCl₃) δ :
1.21 (12H, d, J = 6.8 Hz), 1.24 (6H, d, J = 6.8 Hz), 2.06 (2H, quint, J = 7.1 Hz), 2.52-2.57 (6H, m), 2.73-2.76 (4H, m), 2.89 (1H, sept, J = 6.8 Hz), 2.99 (2H, sept, J = 6.8 Hz), 3.22 (2H, s), 3.40 (2H, t, J = 7.1 Hz), 4.00 (3H, s), 7.03 (2H, s), 7.35 (1H, t, J = 7.8 Hz), 7.77 (1H, dd, J = 7.8, 1.2 Hz), 7.88 (1H, dd, J = 7.8, 1.2 Hz), 8.58 (1H, br s).
EIMS *m*/*z* (relative intensity): 594 (M⁺), 348 (100).
Elementary analysis as C₃₃H₄₆N₄O₄S
Calculated: C 66.64; H 7.79; N 9.42; S 5.39.
Found: C 66.49; H 7.84; N 9.12; S 5.27.

### Example 57

### Preparation of 2-[4-[3-(oxazolo[4,5-b]pyridin-2-ylthio)propyl]piperazin-1-yl]-N-(2,4-6-triisopropylphenyl) acetamide:

The same reaction and treatment as in Example 53 were conducted using 2-mercaptooxazolo[4,5-b]pyridine instead of 2-mercaptobenzooxazole to provide the desired compound as colorless crystals.
Melting point: 156-157°C.
IR (KBr) cm⁻¹: 3433, 3248, 2958, 1662, 1496.
1H-NMR (CDCl₃) δ :
1.21 (12H, d, J = 7.1 Hz), 1.24 (6H, d, J = 7.1 Hz), 2.08 (2H, quint, J = 7.1 Hz), 2.50-2.56 (6H, m), 2.72-2.76 (4H, m), 2.89 (1H, sept, J = 7.1 Hz), 2.99 (2H, sept, J = 7.1 Hz), 3.22 (2H, s), 3.43 (2H, t, J = 7.1 Hz), 7.03 (2H, s), 7.18 (1H, dd, J = 8.1, 5.1 Hz), 7.69 (1H, dd, J = 8.1 1.5 Hz), 8.45 (1H, dd, J = 5.1, 1.5 Hz), 8.59 (1H, br s).
EIMS *m*/*z* (relative intensity): 537 (M⁺), 139 (100).
Elementary analysis as C₃₀H₄₃N₅O₂S
Calculated: C 67.01; H 8.06; N 13.02; S 5.96.
Found: C 67.13; H 8.12; N 12.88; S 6.02.

### Example 58

### Preparation of 2-[4-[2-(benzoxazol-2-ylthio)-ethyl]piperazin-1-yl]-N-(2,6-diisopropyl-3-nitrophenyl)acetamide:

2-Bromo-N-(2,6-diisopropylphenyl)acetamide (5.96 g, 20 mmol) was dissolved in concentrated sulfuric acid (100 ml) with ice-cooling, a solution of fuming nitric acid (1.51 g, 24 mmol) dissolved in concentrated sulfuric acid (10 ml) was dropped thereinto and the mixture was stirred for 10 minutes. The reaction solution was poured onto ice water, the separated matters were filtered off and the extraction with ethyl acetate was conducted. The organic layer was washed with water and a saturated sodium chloride solution successively and dried over sodium sulfate and the solvent was evaporated therefrom. The residue was recrystallized from acetone-hexane to provide 6.52 g (yield 95%) of 2-bromo-N-(2,6-diisopropyl-3-nitrophenyl)acetamide as pale yellow needles.

The same reaction and treatment as in Example 1 were conducted using 2-bromo-N-(2,6-diisopropyl-3-nitrophenyl)-acetamide instead of 2-bromo-N-(2,6-diisopropylphenyl)-acetamide to provide the desired compound as colorless crystals.
Melting point: 143-145°C.
IR (KBr) cm⁻¹: 3432, 3293, 1663, 1527, 1496.
1H-NMR (CDCl₃) δ :
1.21 (6H, d, J = 6.9 Hz), 1.33 (6H, d, J = 7.2 Hz), 2.63-2.70 (4H, m), 2.74-2.78 (4H, m), 2.85 (2H, t, J = 6.8 Hz), 2.99 (1H, sept, J=6.9 Hz), 3.23 (2H, s), 3.25 (1H, sept, J=7.2 Hz), 3.49 (2H, t, J=6.8 Hz), 7.22-7.31 (2H, m), 7.30 (1H, d, J = 8.5 Hz), 7.44 (1H, m), 7.48 (1H, d, J = 8.5 Hz), 7.59 (1H, m), 8.81 (1H, br s).
EIMS *m*/*z* (relative intensity): 525 (M⁺), 375 (100).
Elementary analysis as C₂₇H₃₅N₅O₄S
Calculated: C 61.69; H 6.71; N 13.32; S 6.10
Found: C 61.62; H 6.70; N 13.15; S 6.14.

### Example 59

### Preparation of 2-[4-[2-(benzoxazol-2-ylthio)-ethyl]piperazin-1-yl]-N-(2,6-diisopropyl-3-dimethylaminophenyl)acetamide:

Zinc (8.37 g, 128 mmol) was added to a solution of 2-[4-[2-(benzoxazol-2-ylthio)ethyl]piperazin-1-yl]-N-(2,6-diisopropyl-3-nitrophenyl)acetamide (3.36 g, 6.4 mmol) in acetic acid (35 ml) with ice-cooling and the mixture was stirred at room temperature for 5 minutes. The reaction solution was diluted with ethyl acetate and filtered off through celite and the filtrate was concentrated. The residue was diluted with water, adjusted to pH 10 with potassium carbonate and extracted with methylene chloride. The organic layer was washed with a saturated sodium chloride solution and dried over potassium carbonate, the solvent was evaporated therefrom and the resulting crude crystals were recrystallized from acetone-hexane to provide 2.90 g (yield 91%) of 2-[4-[2-(benzoxazol-2-ylthio)ethyl]piperazin-1-yl]-N-(3-amino-2,6-diisopropyl)acetamide as colorless needles.

To a solution of the acetamide (248 mg, 0.5 mmol) in acetonitrile (2 ml) were added at room temperature, a solution of a 37% aqueous solution of formaldehyde (405 mg, 5.0 mmol) in acetonitrile (1 ml), a solution of sodium cyanoborohydride (126 mg, 2.0 mmol) in acetonitrile (2 ml), and acetic acid (0.1 ml) successively followed by stirring for 1 hour. The reaction solution was concentrated, diluted with water and extracted with ethyl acetate. The organic layer was washed with water and a saturated sodium chloride solution successively and dried over sodium sulfate and the solvent was evaporated therefrom. The residue was purified by a preparative thin layer chromatography (developing solvent, chloroform:methanol = 20:1) and the resulting crude crystals were recrystallized from acetone-hexane to provide 100 mg (yield 38%) of the desired compound as colorless needles.
Melting point: 159-161°C.
IR (KBr) cm⁻¹: 3432, 3302, 2936, 1667, 1500.
1H-NMR (CDCl₃) δ :
1.18 (6H, d, J = 6.8 Hz), 1.30 (6H, d, J = 6.8 Hz), 2.61 (6H,s), 2.63-2.67 (4H, m), 2.74-2.78 (4H, m), 2.85 (2H, t, J = 6.8 Hz), 2.92 (1H, sept, J = 6.8 Hz), 3.21 (2H, s), 3.49 (2H, t, J = 6.8 Hz), 3.77 (1H, sept, J = 6.8 Hz), 7.16 (2H, s) 7.21-7.30 (2H, m),7.44 (1H, m), 7.59 (1H, m), 8.74 (1H, br s).
EIMS *m*/*z* (relative intensity): 523 (M⁺), 323 (100).
Elementary analysis as C₂₉H₄₁N₅O₂S
Calculated: C 66.51; H 7.89; N 13.37; S 6.12
Found: C 66.28; H 7.95; N 13.35; S 6.11.

### Example 60

### Preparation of 2-[4-[2-(benzoxazol-2-ylthio)-ethyl]piperazin-1-yl]-N-[2,6-diisopropyl-3-(methylthio)phenyl]acetamide:

Isoamyl nitrite (1 ml) was slowly dropped into a solution of N-[3-amino-2,6-diisopropylphenyl]-2-[4-(2-hydroxyethyl)piperazin-1-yl]acetamide (500 mg) in dimethyl disulfide (10 ml) with heating and stirring at 110°C followed by stirring at the same temperature for 20 minutes. The reaction solution was allowed to cool and concentrated. The resulting residue was purified by a silica gel column chromatography (developing solvent, chloroform:methanol = 20:1) to provide 370 mg (yield 68%) of N-[2,6-diisopropyl-3-(methylthio)phenyl]-2-[4-(2-hydroxyethyl)piperazin-1-yl]acetamide.

After that, the same reaction and treatment as in Example 1 were conducted using N-[2,6-diisopropyl-3-(methylthio)phenyl]-2-[4-(2-hydroxyethyl)piperazin-1-yl]acetamide instead of N-(2,6-diisopropylphenyl)-2-[4-(2-hydroxyethyl)piperazin-1-yl]acetamide to provide a desired compound as pale yellow powdery crystals.
Melting point: 148-150°C.
IR (KBr) cm⁻¹: 3286, 2960, 2817, 1664, 1499, 1455.
1H-NMR (CDCl₃) δ :
1.12-1.27 (6H, m), 1.28-1.44 (6H, m), 2.43 (3H, s), 2.59-2.79 (9H, m), 2.81-2.88 (2H, m), 2.92 (1H, sept, J = 6.8 Hz), 3.21 (2H, s), 3.49 (2H, t, J = 6.6 Hz), 7.16-7.30 (4H, m), 7.43 (1H, d, J = 7.3 Hz), 7.58 (1H, d, J = 7.3 Hz), 8.73 (1H, br s).
EIMS *m*/*z* (relative intensity): 526 (M⁺), 56 (100).
Elementary analysis as C₂₈H₃₈N₄O₂S₂·0.4H₂O
Calculated: C 62.98; H 7.32; N 10.49
Found: C 62.79; H 7.32; N 10.76.

### Example 61

### Preparation of 2-[4-[2-(benzoxazol-2-ylthio)ethyl]-piperazin-1-yl]-N-(2,6-diisopropyl-3-hydroxyphenyl)-acetamide:

2-[4-[2-(Benzoxazol-2-ylthio)ethyl]piperazin-1-yl]-N-(3-amino-2,6-diisopropylphenyl)acetamide (198 mg, 0.4 mmol) was dissolved in 6% aqueous solution of sulfuric acid (3.4 ml), an aqueous solution (0.8 ml) of sodium nitrite (35 mg, 0.5 mmol) was added thereto at 0°C and the mixture was stirred at that temperature for 30 minutes. The reaction solution was slowly dropped into a boiling water (40 ml) which was heating and stirring at an external temperature of 140°C. After 10 minutes from the dropping, the reaction solution was allowed to cool, neutralized with sodium bicarbonate and extracted with chloroform. The organic layer was washed with water and a saturated sodium chloride solution successively and dried over anhydrous magnesium sulfate and the solvent was evaporated. The residue was purified by a preparative thin layer chromatography (developing solvent, chloroform:ammonia-saturated methanol = 20:1) to provide 178 mg (yield 89%) of the desired compound, which was recrystallized from ethanol- hexane to provide colorless needles.
Meltiing point: 96-98°C.
IR (KBr) cm⁻¹: 3282, 2958, 1667, 1499, 1454.
1H-NMR (CDCl₃) δ :
1.16 (6H, d, J = 7.1 Hz), 1.34 (6H, d, J = 7.1 Hz), 2.60-2.78 (8H, m), 2.84 (2H, t, J = 6.8 Hz), 2.89 (2H, sept, J = 7.1 Hz), 3.14 (2H, t, J = 6.8 Hz) 3.20 (2H, s), 3.49 (2H, t, J = 6.8 Hz), 5.31 (1H, br s), 6.65 (1H, d, J = 8.3 Hz), 6.99 (1H, d, J = 8.3 Hz), 7.24 (1H, td, J = 8.5, 1.4 Hz), 7.28 (1H, td, J = 8.5, 1.4 Hz), 7.43 (1H, dd, J = 8.5, 1.4 Hz), 7.58 (1H, dd, J = 8.5, 1.4 Hz), 8.70 (1H, br s).
EIMS *m*/*z* (relative intensity): 496 (M⁺), 125 (100).
Elementary analysis as C₂₇H₃₆N₄O₂S
Calculated: C 65.29; N 7.31; N 11.28; S 6.46
Found: C 64.65; N 7.32; N 11.16; S 6.36.

### Example 62

### Preparation of 2-[4-[2-(benzoxazol-2-ylthio)ethyl]-piperazin-1-yl]-N-(2,6-diisopropyl-3-mesyloxyphenyl)-acetamide:

Methanesulfonyl chloride (103 mg, 0.9 mmol) was added to a solution of 2-[4-[2-(benzoxazol-2-ylthio)ethyl]-piperazin-1-yl]-N-(2,6-diisopropyl-3-hydroxyphenyl)-acetamide (149 mg, 0.3 mmol) and triethylamine (91 mg, 0.9 mmol) in THF (2 ml) with ice-cooling and the mixture was stirred for 30 minutes. Then triethylamine (46 mg, 0.45 mmol) and methanesulfonyl chloride (52 mg, 0.45 mmol) were further added thereto and the mixture was stirred for 20 minutes. The reaction solution was diluted with water and extracted with ethyl acetate. The organic layer was washed with water and a saturated sodium chloride solution successively and dried over sodium sulfate and the solvent was evaporated therefrom. The residue was purified by a silica gel column chromatography (20 g of silica gel; developing solvent, chloroform:methanol = 20:1) and the resulting crude crystals were recrystallized from acetone and hexane to provide 120 mg (yield 70%) of the desired compound as colorless crystals.
Melting point: 164-166°C (decomposition)
IR (KBr) cm⁻¹: 3433, 3273, 1668, 1455, 1450.
1H-NMR (CDCl₃) δ :
1.19 (6H, d, J = 6.8 Hz), 1.33 (6H, d, J = 7.3 Hz), 2.65-2.81 (8H, m), 2.86-2.90 (2H, m), 2.94 (1H, sept, J = 6.8 Hz), 3.22 (5H, s), 3.34 (1H, sept, J = 7.3 Hz), 3.51 (2H, t, J = 6.7 Hz), 7.21 (1H, d, J=8.5 Hz), 7.23-7.31 (2H, m), 7.39 (1H, d, J = 8.5 Hz), 7.44 (1H, m), 7.58 (1H, m), 8.72 (1H, br s).
EIMS *m*/*z* (relative intensity): 574 (M⁺), 410 (100).
Elementary analysis as C₂₈H₃₈N₄O₅S₂·0.2H₂O
Calculated: C 58.15; H 6.69; N 9.69; S 11.09
Found: C 58.18; H 6.63; N 9.74; S 11.05.

### Example 63

### Preparation of 2-[4-[2-(benzoxazol-2-ylthio)ethyl]-piperazin-1-yl]-N-(2,6-diisopropyl-3-acetyloxyphenyl)-acetamide:

Acetic anhydride (2 ml) was added to a solution of 2-[4-[2-(benzoxazol-2-ylthio)ethyl]piperazin-1-yl]-N-(2,6-diisopropyl-3-hydroxyphenyl)acetamide (176 mg, 0.354 mmol) in pyridine (1 ml) and the mixture was stirred at room temperature for 90 minutes. The reaction solution was neutralized with an aqueous solution of sodium bicarbonate and extracted with ethyl acetate. The organic layer was washed with water and a saturated sodium chloride solution successively and dried over sodium sulfate and the solvent was evaporated therefrom. The residue was purified by a silica gel column chromatography (20 g of silica gel; developing solvent, chloroform:methanol = 30:1 → 20:1) and the resulting crude crystals were recrystallized from acetone-hexane to provide 140 mg (yield 73%) of the desired compound as colorless needles.
Melting point: 129-131°C.
IR (KBr) cm⁻¹: 3436, 3291, 1760, 1665, 1499.
1H-NMR (CDCl₃) δ :
1.20 (6H, d, J = 6.8 Hz), 1.26 (6H, d, J = 7.1 Hz), 2.32 (3H,s), 2.64-2.81 (8H, m), 2.86-2.92 (2H, m), 2.95 (1H, sept, J = 6.8 Hz), 3.11 (1H, sept, J = 7.1 Hz), 3.22 (2H, s), 3.51 (2H, t, J = 7.0 Hz), 6.98 (1H, d, J = 8.5 Hz), 7.18 (1H, d, J = 8.5 Hz), 7.23-7.33 (2H, m), 7.44 (1H, m), 7.59 (1H, m), 8.60 (1H, br s).
EIMS *m*/*z* (relative intensity): 538 (M⁺), 388 (100).
Elementary analysis as C₂₉H₃₈N₄O₂S·0.4H₂O
Calculated: C 64.02; H 7.15; N 10.30; S 5.89
Found: C 63.64; H 7.10; N 10.23; S 5.92.

### Example 64

### Preparation of 2-[4-[2-(benzoxazol-2-ylthio)ethyl]-piperazin-1-yl]-N-(2,6-diisopropyl-3-methoxyphenyl)-acetamide dihydrochloride:

Sodium hydride (21 mg, 0.48 mmol) was added to a solution of 2-[4-[2-(benzoxazol-2-ylthio)ethyl]piperazin-1-yl]-N-(2,6-diisopropyl-3-hydroxyphenyl)acetamide (200 mg, 0.40 mmol) in DMF (2 ml), the mixture was stirred at 40°C for 10 minutes and iodomethane (68 mg, 0.48 mmol) was added thereto followed by stirring for 1 hour. The reaction solution was diluted with water and extracted with ethyl acetate. The organic layer was washed with a saturated sodium chloride solution and dried over sodium sulfate and the solvent was evaporated therefrom. The residue was purified by a silica gel column chromatography (developing solvent, chloroform:methanol = 50:1) to provide 47 mg (yield 23%) of 2-[4-[2-(benzoxazol-2-ylthio)ethyl]-piperazin-1-yl]-N-(2,6-diisopropyl-3-methoxyphenyl)acetamide. This was made into a dihydrochloride and recrystallized to provide the desired compound as colorless powdery crystals.
Melting point: 218-222°C.
IR (KBr) cm⁻¹: 3432, 2963, 1669, 1506, 1454.
1H-NMR (CD₃OD) δ :
1.17 (6H, d, J = 6.8 Hz), 1.29 (6H, d, J = 6.8 Hz), 2.96 (1H, sept, J = 6.8 Hz), 3.17 (1H, sept, J = 6.8 Hz), 3.47-3.64 (10H, m), 3.67-3.75 (2H, m), 3.81 (2H, s), 4.11 (2H, s), 6.97 (1H, d, J = 8.8 Hz), 7.17 (1H, d, J = 8.8 Hz), 7.28-7.37 (2H, m), 7.53-7.63 (2H, m)
EIMS *m*/*z* (relative intensity): 510 (M⁺), 360 (100).
Elementary analysis as C₂₈H₃₈N₄O₃S₂·2HCl·0.6H₂O
Calculated: C 56.58; H 6.99; N 9.43; Cl 11.93
Found: C 56.88; H 6.94; N 9.47; Cl 11.64.

### Example 65

### Preparation of 2-[4-[2-(benzoxazol-2-ylthio)ethyl]-piperazin-1-yl]-N-[2,6-diisopropyl-3-(2-ethoxyethyloxy)-phenyl)acetamide:

2-Bromoethyl ethyl ether (2 ml) and potassium fluoride catalyst carried on alumina (40wt%, 225 mg, 1.51 mmol) were added to a solution of 2-[4-[2-(benzoxazol-2-ylthio)ethyl]piperazin-1-yl]-N-(2,6-diisopropyl-3-hydroxyphenyl)acetamide (150 mg, 0.30 mmol) in acetonitrile (3 ml) followed by stirring for 41 hours. The catalyst was filtered off and the filtrate was diluted with water and extracted with ethyl acetate. The organic layer was washed with a sodium chloride solution and dried over anhydrous sodium sulfate and the solvent was evaporated therefrom. The residue was purified by a preparative thin layer chromatography (developing solvent, chloroform:ammonia-saturated methanol = 20:1) and the resulting crude crystals were recrystallized from ethyl acetate-hexane to provide 120 mg (yield 70%) of the desired compound as colorless powdery crystals.
Melting point: 100-103°C
IR (KBr) cm⁻¹: 3282, 2960, 1661, 1498, 1454.
1H-NMR (CDCl₃) δ :
1.17 (6H, d, J = 6.9 Hz), 1.23 (3H, t, J = 7.0 Hz), 1.32 (6H, d, J = 6.9 Hz), 2.62-2.79 (8H, m), 2.86 (2H, t, J = 6.2 Hz), 2.93 (1H, sept, J = 6.9 Hz), 3.19 (1H, sept, J = 6.9 Hz), 3.21 (2H, s), 3.50 (2H, t, J = 6.2 Hz), 3.59 (2H, q, J = 7.0 Hz), 3.81 (2H, t, J = 5.1 Hz), 4.09 (2H, t, J = 5.9 Hz), 6.83 (1H, d, J = 8.7 Hz), 7.09 (1H, d, J = 8.7 Hz), 7.22-7.30 (2H, m), 7.43 (1H, m), 7.58 (1H, m), 8.56 (1H, br s).
EIMS *m*/*z* (relative intensity): 568 (M⁺), 276 (100).
Elementary analysis as C₃₁H₄₄N₄O₄S
Calculated: C 65.46; H 7.80; N 9.85; S 5.64
Found: C 65.16; H 7.75; N 9.81; S 5.70.

### Example 66

### Preparation of 2-[4-[2-(benzoxazol-2-ylthio)ethyl]-piperazin-1-yl]-N-(2,6-diisopropyl-3-hydroxy-4-nitrophenyl)acetamide:

Acetyl nitrate (145 mg, 0.75 mmol) was added to a solution of 2-[4-[2-(benzoxazol-2-ylthio)ethyl]piperazin-1-yl]-N-(2,6-diisopropyl-3-hydroxyphenyl)acetamide (107 mg, 0.22 mmol) in acetonitrile (3 ml) with ice cooling followed by stirring for 10 minutes. The reaction solution was diluted with water, made alkaline by adding an aqueous solution of sodium bicarbonate thereto and extracted with chloroform twice. The organic layer was washed with a saturated sodium chloride solution and dried over anhydrous sodium sulfate and the solvent was evaporated therefrom. The residue was purified by a preparative thin layer chromatography (developing solvent, chloroform:methanol = 20:1) and the resulting crude crystals were recrystallized from chloroform-ethyl acetate-hexane to provide 60 mg (yield 51%) of the desired compound as yellow powdery crystals.
Melting point: 139-141°C
IR (KBr) cm⁻¹: 3256, 2962, 1690, 1480, 1454.
1H-NMR (CDCl₃) δ :
1.21 (6H, d, J = 7.0 Hz), 1.38 (6H, d, J = 7.0 Hz), 2.63-2.73 (4H, m), 2.73-2.79 (4H, m), 2.87 (4H, t, J = 6.9 Hz), 2.92 (1H, sept, J = 7.0 Hz), 3.20 (1H, sept, J = 7.0 Hz), 3.22 (2H, s), 3.50 (2H, t, J = 6.9 Hz), 7.23-7.32 (2H, m), 7.44 (1H, m), 7.58 (1H, m), 7.93 (1H, s), 8.83 (1H, br s), 11.10 (1H, br s).
EIMS *m*/*z* (relative intensity): 541 (M⁺), 377 (100).
Elementary analysis as C₂₇H₃₅N₅O₅S
Calculated: C 59.87; H 6.51; N 12.93; S 5.92
Found: C 59.81; H 6.64; N 12.94; S 5.84.

### Example 67

### Preparation of 2-[4-[3-(benzoxazol-2-ylthio)propyl]piperazin-1-yl]-N-(2,6-diisopropyl-3-nitrophenyl)acetamide:

Potassium carbonate (226 mg, 1.63 mmol) was added to a solution of 1-[3-(benzoxazol-2-ylthio)propyl]piperazine ditrifluoroacetate (206 mg, 0.41 mmol) and N-(2,6-diisopropyl-3-nitrophenyl)-2-bromoacetamide (140 mg, 0.41 mmol) in acetonitrile (5 ml) followed by stirring for 2 hours. The reaction solution was diluted with water and extracted with ethyl acetate. The organic layer was washed with a saturated sodium chloride solution and dried over anhydrous sodium sulfate and the solvent was evaporated therefrom. The resulting crude crystals were recrystallized from ethyl acetate-hexane to provide 179 mg (yield 81%) of the desired product as colorless powdery crystals.
Melting point: 156-158°C
IR (KBr) cm⁻¹: 3277, 2936, 1665, 1499, 1455.
1H-NMR (CDCl₃) δ :
1.21 (6H, d, J = 6.9 Hz), 1.32 (6H, d, J = 6.9 Hz), 2.05 (2H, quint, J = 6.9 Hz), 2.51-2.62 (4H, m), 2.55 (2H, t, J = 6.9 Hz), 2.73-2.79 (4H, m), 2.99 (1H, sept, J = 6.9 Hz), 3.24 (2H, s), 3.25 (1H, sept, J = 6.9 Hz), 3.38 (2H, t, J = 6.9 Hz), 7.24-7.31 (2H, m), 7.30 (1H, d, J = 8.7 Hz), 7.43 (1H, m), 7.47 (1H, d, J = 8.7 Hz), 7.58 (1H, m), 8.83 (1H, br s).
EIMS *m*/*z* (relative intensity): 539 (M⁺), 193 (100).
Elementary analysis as C₂₈H₃₇N₅O₄S
Calculated: C 62.31; H 6.91; N 12.98
Found: C 62.23; H 6.94; N 12.85.

### Example 68

### Preparation of 2-[4-[3-(benzoxazol-2-ylthio)propyl]piperazin-1-yl]-N-[2,6-diisopropyl-3-(methylthio)phenyl]acetamide:

The same reaction and treatment as in Example 60 were conducted using N-[3-amino-2,6-diisopropylphenyl]-2-[4-(3-hydroxypropyl)piperazin-1-yl]acetamide instead of N-[3-amino-2,6-diisopropylphenyl]-2-[4-(2-hydroxyethyl)piperazin-1-yl]acetamide to provide the desired compound as pale yellow powdery crystals.
Melting point: 126-127°C.
IR (KBr) cm⁻¹: 3271, 2961, 1662, 1499, 1454.
1H-NMR (CDCl₃) δ :
1.13-1.22 (6H, m), 1.30-1.39 (6H, m), 2.04 (2H, quint, J = 6.9 Hz), 2.43 (3H, s), 2.51-2.57 (4H, m), 2.54 (2H, t, J = 6.9 Hz), 2.72-2.80 (5H, m), 2.93 (1H, sept, J = 6.9 Hz), 3.21 (2H, s), 3.38 (2H, t, J = 6.9 Hz), 7.16-7.31 (4H, m), 7.43 (1H, m), 7.59 (1H, m), 8.76 (1H, br s).
EIMS *m*/*z* (relative intensity): 540 (M⁺), 70 (100).
Elementary analysis as C₂₉H₄₀N₄O₂S₂
Calculated: C 64.41; H 7.46; N 10.36
Found: C 64.46; H 7.48; N 10.55.

### Example 69

### Preparation of 2-[4-[3-(benzoxazol-2-ylthio)propyl]piperazin-1-yl]-N-(2,6-diisopropyl-3-hydroxy)phenyl]acetamide:

The same reaction and treatment as in Example 61 were conducted using 2-[4-(3-benzoxazole-2-ylthio) propyl]piperazin-1-yl]-N-(2,6-diisopropyl-3-nitrophenyl]-acetamide instead of 2-[4-[2-benzoxazole-2-ylthio)ethyl]piperazin-1-yl]-N-(2,6-diisopropyl-3-nitrophenyl]acetamide to provide the desired compound as colorless powdery crystals.
Melting point: 176-178°C.
IR (KBr) cm⁻¹: 3263, 2960, 1665, 1496, 1454.
1H-NMR (CDCl₃) δ :
1.16 (6H, d, J = 6.9 Hz), 1.34 (6H, d, J = 6.9 Hz), 2.05 (2H, quint, J = 6.9 Hz), 2.51-2.60 (4H, m), 2.54 (2H, t, J = 6.9 Hz), 2.70-2.77 (4H, m), 2.91 (1H, sept, J = 6.9 Hz), 3.16 (1H, sept, J = 6.9 Hz), 3.21 (2H, s), 3.38 (2H, t, J = 6.9 Hz), 4.80 (1H, br s), 6.66 (1H, d, J = 8.4 Hz), 7.01 (1H, d, J = 8.4 Hz), 7.22-7.30 (2H, m), 7.43 (1H, m), 7.59 (1H, m) 8.60 (1H, br s).
EIMS *m*/*z* (relative intensity): 510 (M⁺), 70 (100).
Elementary analysis as C₂₈H₃₈N₄O₃S
Calculated: C 65.85; H 7.50; N 10.97.
Found: C 65.66; H 7.52; N 10.80.

### Example 70

### Preparation of 2-[4-[3-(benzoxazol-2-ylthio)propyl]piperazin-1-yl]-N-(2,6-diisopropyl-3-methoxyphenyl)acetamide:

2-[4-[3-(Benzoxazol-2-ylthio)propyl]piperazin-1-yl]-N-(2,6-diisopropyl-3-hydroxyphenyl)acetamide (150 mg, 0.29 mmol) was dissolved in methanol (2 ml) and acetonitrile (3 ml), then N,N-diisopropylethylamine (227 mg, 1.76 mmol) and a solution of trimethylsilyl diazomethane in hexane (2.0M, 0.88 ml, 1.76 mmol) were added thereto and the mixture was stirred for 14 hours. The reaction solution was concentrated, made alkaline with an aqueous solution of sodium bicarbonate and extracted with ethyl acetate. The organic layer was washed with water and a saturated sodium chloride solution successively and dried over anhydrous sodium sulfate and the solvent was evaporated therefrom. The residue was purified by a silica gel column chromatography (developing solvent, hexane:acetone = 7:3) and the resulting crude crystals were recrystallized from ethyl acetate and hexane to provide 31 mg (yield 20%) of the desired compound as colorless powdery crystals.
Melting point: 105-107°C
IR (KBr) cm⁻¹: 3289, 2959, 1663, 1501, 1454.
1H-NMR (CDCl₃) δ :
1.17 (6H, d, J = 6.9 Hz), 1.29 (6H, d, J = 6.9 Hz), 2.04 (2H, quint, J = 6.9 Hz), 2.51-2.59 (4H, m), 2.54 (2H, t, J = 6.9 Hz), 2.71-2.78 (4H, m), 2.92 (1H, sept, J = 6.9 Hz), 3.19 (1H, sept, J = 6.9 Hz), 3.21 (2H, s), 3.38 (2H, t, J = 6.9 Hz), 3.80 (3H, s), 6.84 (1H, d, J = 8.6 Hz), 7.12 (1H, d, J = 8.6 Hz), 7.22-7.31 (2H, m), 7.43 (1H, m), 7.59 (1H, m), 8.60 (1H, br s).
EIMS *m*/*z* (relative intensity): 524 (M⁺), 290 (100).

### Example 71

### Preparation of 2-[4-[3-(benzoxazol-2-ylthio)propyl]piperazin-1-yl]-N-[2,6-diisopropyl-3-(2-ethoxyethyloxy)phenyl]acetamide:

To a solution of N-(2,6-diisopropyl-3-hydroxyphenyl)-2-[4-(3-hydroxypropyl)piperazin-1-yl]acetamide (180 mg, 0.61 mmol) in DMF (3 ml) were added 2-bromoethyl ethyl ether (2 ml) and potassium fluoride catalyst carried on alumina (40wt%, 355 mg, 2.39 mmol) followed by stirring at 50°C for 3 hours. After the catalyst was filtered off, the filtrate was diluted with water and extracted with ethyl acetate. The organic layer was washed with a saturated sodium chloride solution and dried over anhydrous sodium sulfate and the solvent was evaporated therefrom. The residue was purified by a silica gel column chromatography (developing solvent, chloroform:methanol = 20:1) to provide 90 mg (yield 42%) of N-(2,6-diisopropyl-3-(2-ethoxyethyloxy)phenyl)-2-[4-(3-hydroxypropyl)piperazin-1-yl]acetamide as colorless powdery crystals.

After that, the same reaction and treatment as Example 1 were conducted using N-(2,6-diisopropyl-3-(2-ethoxyethyloxy)phenyl)-2-[4-(3-hydroxypropyl)piperazin-1-yl]acetamide instead of N-(2,6-diisopropylphenyl)-2-[4-(2-hydroxyethyl)piperazin-1-yl]acetamide to provide a desired compound as colorless powdery crystals.
Melting point: 99-100°C
IR (KBr) cm⁻¹: 3267, 2962, 1664, 1501, 1455.
1H-NMR (CDCl₃) δ :
1.17 (6H, d, J = 6.9 Hz), 1.23 (3H, t, J = 7.0 Hz), 1.32 (6H, d, J = 6.9 Hz), 2.04 (2H, quint, J = 6.9 Hz), 2.51-2.59 (4H, m), 2.54 (2H, t, J = 6.9 Hz), 2.70-2.77 (4H, m), 2.91 (1H, sept, J = 6.9 Hz), 3.16 (1H, sept, J = 6.9 Hz), 3.21 (2H, s), 3.38 (2H, t, J = 7.0 HZ), 3.59 (2H, q, J = 7.0 Hz), 3.81 (2H, t, J = 5.1 Hz), 4.09 (2H, t, J = 5.1 Hz), 6.83 (1H, d, J = 8.8 Hz), 7.09 (1H, d, J = 8.8 Hz), 7.22-7.31 (2H, m), 7.43 (1H, m), 7.59 (1H, m), 8.59 (1H, br s).
EIMS *m*/*z* (relative intensity): 582 (M⁺), 139 (100).

### Example 72

### Preparation of 2-[4-[2-(benzoxazol-2-ylthio)ethyl]-piperazin-1-yl]-N-(2,6-diisopropyl-4-hydroxyphenyl)-acetamide:

To a solution of sodium carbonate (51 mg, 0.5 mmol) in water (1 ml) was added sulfanilic acid (167 mg, 1.0 mmol), the mixture was heated to dissolve, then sodium nitrite (73 mg, 1.1 mmol) was added with ice cooling and concentrated hydrochloric acid (0.25 ml) was dropped thereinto to provide a colorless suspension.

3,5-Diisopropylphenol (172 mg, 1.0 mmol) was added to a solution of sodium hydroxide (212 mg, 5.5 mmol) in water (1.2 ml), the mixture was heated to dissolve, the previously-prepared suspension was slowly dropped thereinto with ice cooling and the mixture was stirred at room temperature for 2 hours. Sodium hydrosulfite was added thereto at 50°C until the red color of the reaction solution almost disappeared and stirred at 80°C for 2 hours. The reaction mixture was allowed to cool and the separated matter was collected by filtration and dried by heating *in vacuo* to provide 107 mg (yield 58%) of 4-amino-3,5-diisopropylphenol as purple needles.

Then the same reaction and treatment as in Example 48 were conducted using 4-amino-3,5-diisopropylphenol instead of 2,4,6-triisopropylaniline to provide the desired compound as colorless needles.
Melting point: 162-164°C
IR (KBr) cm⁻¹: 3307, 2961, 1665, 1499, 1455.
1H-NMR (CDCl₃) δ :
1.17 (12H, d, J = 6.8 Hz), 2.60-2.76 (8H, m), 2.85 (2H, t, J = 6.8 Hz), 2.93 (2H, sept, J = 6.8 Hz), 3.20 (2H,s), 3.49 (2H, t, J = 6.8 Hz), 5.59 (1H, br s), 6.62 (2H, s), 7.22-7.31 (2H, m), 7.44 (1H, m), 7.58 (1H, m), 8.47 (1H, br s).
EIMS *m*/*z* (relative intensity): 496 (M⁺), 97 (100).
Elementary analysis as C₂₇H₃₆N₄O₃S
Calculated: C 65.29; H 7.31; N 11.28; S 6.46
Found: C 65.35; H 7.42; N 11.12; S 6.41.

### Example 73

### Preparation of 2-[4-[2-(benzoxazol-2-ylthio)ethyl]-piperazin-1-yl]-N-(4-acetoxy-2,6-diisopropylphenyl)-acetamide:

Acetic anhydride (2 ml) was dropped into a solution of 2-[4-[2-(benzoxazol-2-ylthio)ethyl]piperazin-1-yl]-N-(2,6-diisopropyl-4-hydroxyphenyl)acetamide (149 mg, 0.3 mmol) in pyridine (1 ml) with ice cooling and the mixture was stirred at room temperature for 3 hours. The reaction solution was neutralized by adding a saturated aqueous solution of sodium bicarbonate thereto and extracted with ethyl acetate. The organic layer was washed with water and a saturated sodium chloride solution successively and dried over sodium sulfate and the solvent was evaporated therefrom. The residue was purified by a silica gel column chromatography (developing solvent, chloroform:methanol = 20:1) to provide 166 mg (yield 100%) of the desired compound as colorless needles.
Melting point: 126-129°C
IR (KBr) cm⁻¹: 3440, 3275, 1762, 1664, 1498.
1H-NMR (CDCl₃) δ :
1.20 (12H, d, J = 6.8 Hz), 2.30 (3H, s), 2.60-2.78 (8H, m), 2.86 (2H, t, J= 6.8 Hz), 2.99 (2H, sept, J = 6.8 Hz), 3.22 (2H, s), 3.49 (2H, t, J = 6.8 Hz), 6.89 (2H, s), 7.22-7.31 (2H, m), 7.44 (1H, m), 7.59 (1H, m), 8.60 (1H, br s).
EIMS *m*/*z* (relative intensity): 538 (M⁺), 276 (100).
Elementary analysis as C₂₉H₃₈N₄O₄S·0.2H₂O
Calculated: C 64.23; H 7.14; N 10.33
Found: C 64.22; H 7.08; N 10.27.

### Example 74

### Preparation of 2-[4-[2-(benzoxazol-2-ylthio)ethyl]-piperazin-1-yl]-N-(2,6-diisopropyl-4-mesyloxyphenyl)-acetamide:

Triethylamine (30 mg, 0.3 mmol) was added to a solution of 2-[4-[2-(benzoxazol-2-ylthio)ethyl]piperazin-1-yl]-N-(2,6-diisopropyl-4-hydroxyphenyl)acetamide (50 mg, 0.1 mmol) in THF (1 ml), then methanesulfonyl chloride (34 mg, 0.3 mmol) was dropped thereinto with ice cooling and the mixture was stirred at room temperature for 2 hours. The reaction solution was diluted with water and extracted with ethyl acetate. The organic layer was washed with water and a saturated sodium chloride solution successively and dried over sodium sulfate and the solvent was evaporated therefrom. The residue was purified by a preparative thin layer chromatography (developing solvent, chloroform:methanol = 19:1) to provide 47 mg (yield 82%) of the desired compound as colorless needles.
Melting point: 115-117°C
IR (KBr) cm⁻¹: 3436, 3222, 1666, 1497, 1367.
1H-NMR (CDCl₃) δ :
1.21 (12H, d, J = 6.8 Hz), 2.60-2.77 (8H, m), 2.85 (2H, t, J= 6.8 Hz), 3.00 (2H, sept, J = 6.8 Hz), 3.14 (3H, s), 3.22 (2H, s), 3.49 (2H, t, J = 6.8 Hz), 7.08 (2H, s), 7.22-7.31 (2H, m), 7.44 (1H, m), 7.59 (1H, m), 8.63 (1H, br s).
EIMS *m*/*z* (relative intensity): 574 (M⁺), 125 (100).
Elementary analysis as C₂₈H₃₈N₄O₅S₂·0.3H₂O
Calculated: C 57.97; H 6.71; N 9.66
Found: C 58.06; H 6.63; N 9.56.

### Example 75

### Preparation of 2-[4-[2-(benzoxazol-2-ylthio)ethyl]-piperazin-1-yl]-N-(2,6-diisopropyl-4-methoxyphenyl)-acetamide:

Sodium hydride (7 mg, 0.3 mmol) was added to a solution of 2-[4-[2-(benzoxazol-2-ylthio)ethyl]piperazin-1-yl]-N-(2,6-diisopropyl-4-hydroxyphenyl)acetamide (99 mg, 0.2 mmol) in DMF (2 ml), the mixture was heated at 60°C for 10 minutes, and iodomethane (43 mg, 0.3 mmol) was dropped thereinto followed stirring for 30 minutes. The reaction solution was diluted with water and extracted with ethyl acetate. The organic layer was washed with water and a saturated sodium chloride solution successively and dried over sodium sulfate and the solvent was evaporated therefrom. The residue was purified by a preparative thin layer chromatography (developing solvent, hexane:acetone = 5:3) to provide 44 mg (yield 43%) of the desired compound as colorless needles.
Melting point: 115-117°C
IR (KBr) cm⁻¹: 3432, 3238, 1662, 1500, 1455.
1H-NMR (CDCl₃) δ :
1.20 (12H, d, J = 6.8 Hz), 2.60-2.78 (8H, m), 2.86 (2H, t, J= 6.8 Hz), 2.97 (2H, sept, J = 6.8 Hz), 3.21 (2H, s), 3.50 (2H, t, J = 6.8 Hz), 3.81 (3H, s), 6.71 (2H, s), 7.22-7.31 (2H, m), 7.44 (1H, m), 7.58 (1H, m), 8.45 (1H, br s).
EIMS *m*/*z* (relative intensity): 510 (M⁺), 276 (100).
Elementary analysis as C₂₈H₃₈N₄O₃S
Calculated: C 65.85; H 7.50; N 10.97; S 6.28
Found: C 65.80; H 7.63; N 10.71; S 6.05.

### Example 76

### Preparation of 2-[4-[2-(benzoxazol-2-ylthio)ethyl]piperazin-1-yl]-N-(4-diethoxyphosphoryloxy-2,6-diisopropylphenyl)acetamide:

The same reaction and treatment as in Example 75 were conducted using diethylphosphoric chloride instead of iodomethane to provide the desired compound as colorless needles.
Melting point: 108-109°C.
IR (KBr) cm⁻¹: 3440, 3276, 1673, 1497, 1455.
1H-NMR (CDCl₃) δ :
1.19 (12H, d, J = 6.8 Hz), 1.36 (6H, m), 2.60-2.78 (8H, m), 2.85 (2H, t, J = 6.8 Hz), 2.97 (2H, sept, J = 6.8 Hz), 3.21 (2H, s), 3.49 (2H, t, J = 6.8 Hz), 4.22 (4H, m), 7.02 (2H, s), 7.22-7.31 (2H, m), 7.44 (1H, m), 7.58 (1H, m), 8.53 (1H, br s).
EIMS *m*/*z* (relative intensity): 632 (M⁺), 482 (100).
Elementary analysis as C₃₁H₄₅N₄O₆PS
Calculated: C 58.84; H 7.17; N 8.85.
Found: C 59.00; H 7.22; N 8.79.

### Example 77

### Preparation of 2-[4-[2-(benzoxazol-2-ylthio)ethyl]piperazin-1-yl]-N-[2,6-diisopropyl-4-ethoxycarbonylmethyloxyphenyl)acetamide:

The same reaction and treatment as in Example 75 were conducted using bromoethylacetate instead of iodomethane to provide the desired compound as colorless needles.
Melting point: 118-120°C.
IR (KBr) cm⁻¹: 3330, 2939, 1766, 1662, 1499.
1H-NMR (CDCl₃) δ :
1.18 (12H, d, J = 6.8 Hz), 1.32 (3H, t, J = 7.2 Hz), 2.60-2.78 (8H, m), 2.84 (2H, t, J = 6.8 Hz), 2.97 (2H,sept, J = 6.8 Hz), 3.20 (2H, s), 3.49 (2H, t, J = 6.8 Hz), 4.29 (2H, q, J = 7.2 Hz), 4.61 (2H, s), 6.73 (2H, s), 7.22-7.31 (2H, m), 7.44 (1H, m), 7.58 (1H, m), 8.48 (1H, br s).
EIMS *m*/*z* (relative intensity): 582 (M⁺), 363 (100).
Elementary analysis as C₃₁H₄₂N₄O₅S
Calculated: C 63.89; H 7.26; N 9.61; S 5.50.
Found: C 63.94; H 7.33; N 9.57; S 5.54.

### Example 78

### Preparation of 2-[4-[2-(benzoxazol-2-ylthio)ethyl]piperazin-1-yl]-N-[2,6-diisopropyl-4-(2-ethoxyethyl)oxyphenyl)acetamide:

The same reaction and treatment as in Example 75 were conducted using chloroethyl ethyl ether instead of iodomethane to provide the desired compound as colorless needles.
Melting point: 92-95°C.
IR (KBr) cm⁻¹: 3429, 3296, 1664, 1501, 1455.
1H-NMR (CDCl₃) δ:
1.18 (12H, d, J = 6.8 Hz), 1.25 (3H, t, J = 7.0 Hz), 2.60-2.78 (8H, m), 2.85 (2H, t, J = 6.8 Hz), 2.96 (2H, sept, J = 6.8 Hz), 3.20 (2H, s), 3.49 (2H, t, J = 6.8 Hz), 3.61 (2H, q, J = 7.0 Hz), 3.79 (2H, t, J = 5.5 Hz), 4.13 (2H, t, J = 5.5 Hz), 6.74 (2H, s), 7.22-7.31 (2H, m), 7.44 (1H, m), 7.58 (1H, m), 8.46 (1H, br s).
EIMS *m*/*z* (relative intensity): 568 (M⁺), 405 (100).
Elementary analysis as C₃₁H₄₄N₄O₄S
Calculated: C 65.46; H 7.80; N 9.85; S 5.64.
Found: C 65.42; H 7.75; N 9.73; S 5.68.

### Example 79

### Preparation of N-[2-[4-[2-(benzoxazol-2-ylthio)-ethyl]piperazin-1-yl]ethyl]-N'-(2,6-diisopropylphenyl)-urea:

To a solution of 1-(2-hydroxyethyl)piperazine (2.60 g, 20 mmol) in acetonitrile (35 ml) was added potassium carbonate (3.04 g, 22 mmol) and chloroacetonitrile (1.51 g, 20 mmol) was dropped thereinto with ice cooling. The mixture was stirred at room temperature for 30 minutes and then stirred at 45°C for 30 minutes. This reaction mixture was filtered off, the filtrate was concentrated *in vacuo* and the residue was purified by a silica gel column chromatography (100 g of silica gel; developing solvent, chloroform:ammonia-saturated methanol = 20:1 → 10:1) to provide 3.20 g (yield 95%) of 4-(cyanomethyl)piperazine-1-ethanol.

A solution of 4-(cyanomethyl)piperazine-1-ethanol (1.69 g, 10 mmol) in THF (20 ml) was added to a solution of lithium aluminum hydride in THF (20 ml, 20 mmol) in an argon stream with ice cooling, the mixture was stirred at room temperature for 5 minutes and heated to reflux for 90 minutes. The reaction solution was allowed to cool, diluted with ethanol with ice cooling and stirred at room temperature for 15 minutes after adding an aqueous 1N sodium hydroxide solution thereto. This was filtered off through celite and the filtrate was concentrated *in vacuo* to provide 4-(aminoethyl)piperazine-1-ethanol.

Into a solution of 4-(aminoethyl)piperazine-1-ethanol in chloroform (20 ml) was dropped a solution of 2,6-diisopropylphenyl isocyanate (2.03 g, 10 mmol) in chloroform (20 ml) followed by stirring for 5 minutes. The reaction solution was concentrated *in vacuo* and the residue was purified by a silica gel column chromatography (100 g of silica gel; developing solvents, chloroform → chloroform:ammonia-saturated methanol = 20:1) to provide 2.03 g (yield 54%) of N-[2-[4-(2-hydroxyethyl)piperazin-1-yl]ethyl]-N'-(2,6-diisopropylphenyl)urea.

Then the same reaction and treatment as in Example 1 were conducted using N-[2-[4-(2-hydroxyethyl)-piperazin-1-yl]ethyl]-N'-(2,6-diisopropylphenyl)urea instead of N-(2,6-diisopropylphenyl)-2-[4-(2-hydroxyethyl)-piperazin-1-yl]acetamide to provide the desired compound as colorless needles.
Melting point: 152-153°C
IR (KBr) cm⁻¹: 3345, 3276, 1633, 1500.
1H-NMR (CDCl₃) δ :
1.19 (12H, d, J = 6.8 Hz), 2.22-2.38 (10H, m), 2.70 (2H, t, J = 7.1 Hz), 3.25 (2H, q, J = 5.6 Hz), 3.30 (2H, sept, J = 6.8 Hz), 3.41 (2H, t, J = 7.1 Hz), 4.90 (1H, t, J = 5.6 Hz), 5.68 (1H, br s), 7.19-7.35 (5H, m), 7.43 (1H, m), 7.59 (1H, m).
EIMS *m*/*z* (relative intensity): 509 (M⁺), 227 (100).
Elementary analysis as C₂₈H₃₉N₅O₂S
Calculated: C 65.98; H 7.71; N 13.74; S 6.29
Found: C 65.98; H 7.63; N 13.60; S 6.24.

### Example 80

### Preparation of N-[2-[4-[2-(benzimidazol-2-ylthio)-ethyl]piperazin-1-yl]ethyl]-N'-(2,6-diisopropylphenyl)-urea:

The same reaction and treatment as in Example 79 were conducted using 2-mercaptobenzimidazole instead of 2-mercaptobenzooxazole to provide the desired compound as colorless crystals.
Melting point: 120-122°C.
IR (KBr) cm⁻¹: 3329, 3280, 1632, 1567.
1H-NMR (CDCl₃) δ:
1.20 (12H, d, J = 6.1 Hz), 2.49-2.53 (10H, m), 2.86-2.89 (2H, m), 3.11-3.15 (2H, m), 3.24-3.39 (4H, m), 4.81 (1H, t, J = 5.0 Hz), 5.70 (1H, br s), 7.14-7.20 (2H, m), 7.23 (1H, d, J = 8.6 Hz), 7.23 (1H, d, J = 6.8 Hz), 7.35 (1H, dd, J = 8.6, 6.8 Hz), 7.43-7.56 (2H, m).
EIMS *m*/*z* (relative intensity): 508 (M⁺), 156 (100).
Elementary analysis as C₂₈H₄₀N₆OS
Calculated: C 66.11; H 7.92; N 16.52; S 6.30.
Found: C 65.87; H 8.02; N 16.32; S 6.26.

### Example 81

### Preparation of N-[2-[4-[2-(benzothiazole-2-ylthio)-ethyl]piperazin-1-yl]ethyl]-N'-(2,6-diisopropylphenyl)-urea:

The same reaction and treatment as in Example 79 were conducted using 2-mercaptobenzothiazole instead of 2-mercaptobenzooxazole to provide the desired compound as colorless needles.
Melting point: 147-149°C.
IR (KBr) cm⁻¹: 3327, 3260, 1632, 1567.
1H-NMR (CDCl₃) δ :
1.18 (12H, d, J = 6.8 Hz), 2.25-2.38 (10H, m), 2.70 (2H, d, J = 7.1 Hz), 3.25 (2H, q, J = 5.9 Hz), 3.30 (2H, sept, J = 6.8 Hz), 3.46 (2H, t, J = 7.1 Hz), 4.91 (1H, t, 5.9 Hz), 5.67 (1H, br s), 7.20 (1H, d, J = 8.4 Hz), 7.20 (1H, d, J = 7.3 Hz), 7.26-7.34 (2H, m), 7.42 (1H, td, J = 8.1, 0.8 Hz), 7.76 (1H, dd, J = 8.1, 0.8 Hz), 7.86 (1H, dd, J = 8.1, 0.8 Hz).
EIMS *m*/*z* (relative intensity): 525 (M⁺), 293 (100).
Elementary analysis as C₂₈H₃₉N₅OS₂
Calculated: C 63.96; H 7.48; N 13.32; S 12.20.
Found: C 63.82; H 7.51; N 13.14; S 12.27.

### Example 82

### Preparation of N-[2-[4-[2-(7-methoxycarbonylbenzoxazol-2-ylthio)ethyl]piperazin-1-yl]ethyl]-N'-(2,6-diisopropylphenyl)urea:

The same reaction and treatment as in Example 79 were conducted using 7-methoxycarbonyl-2-mercaptobenzooxazole instead of 2-mercaptobenzooxazole to provide the desired compound as colorless crystals.
Melting point: 186-188°C.
IR (KBr) cm⁻¹: 3414, 3349, 1718, 1668, 1508.
1H-NMR (CDCl₃) δ :
1.18 (12H, d, J = 6.8 Hz), 2.24-2.37 (10H, m), 2.72 (2H, t, J = 7.0 Hz), 3.25 (2H, q, J = 5.4 Hz), 3.30 (2H, sept, J = 6.8 Hz), 3.44 (2H, t, J = 7.0 Hz), 3.99 (3H, s), 4.88 (1H, t, J = 5.4 Hz), 5.67 (1H, br s), 7.20 (1H, d, J = 7.1 Hz), 7.20 (1H, d, J = 8.3 Hz), 7.32 (1H, dd, J = 8.3, 7.1 Hz), 7.35 (1H, t, J = 7.8 Hz), 7.77 (1H, dd, J = 7.8, 1.2 Hz), 7.88 (1H, dd, J = 7.8, 1.2 Hz).
EIMS *m*/*z* (relative intensity): 567 (M⁺), 146 (100).
Elementary analysis as C₃₀H₄₁N₅O₄S
Calculated: C 63.47; H 7.28; N 12.34; S 5.65.
Found: C 63.53; H 7.25; N 12.10; S 5.59.

### Example 83

### Preparation of N-[2-[4-[2-(oxazolo[4,5-b]pyridine-2-ylthio)ethyl]piperazin-1-yl]ethyl]-N'-(2,6-diisopropylphenyl)urea:

The same reaction and treatment as in Example 79 were conducted using 2-mercaptooxazolo[4,5-b]pyridine instead of 2-mercaptobenzooxazole to provide the desired compound as colorless needles.
Melting point: 175-176°C.
IR (KBr) cm⁻¹: 3385, 3313, 1660, 1541.
1H-NMR (CDCl₃) δ :
1.19 (12H, d, J = 6.8 Hz), 2.25-2.38 (10H, m), 2.73 (2H, t, J = 7.1 Hz), 3.25 (2H, q, J = 6.1 Hz), 3.30 (2H, sept, J = 6.08 Hz), 3.49 (2H, t, J = 7.1 Hz), 4.90 (1H, t, J = 6.1 Hz), 5.69 (1H, br s), 7.18 (1H, dd, J = 8.1, 4.9 Hz), 7.21 (1H, d, J = 8.6 Hz), 7.21 (1H, d, J = 6.8 Hz), 7.33 (1H, dd, J = 8.6, 6.8 Hz), 7.69 (1H, dd, J = 8.1, 1.5 Hz), 8.46 (1H, dd, J = 4.9, 1.5 Hz).
EIMS *m*/*z* (relative intensity): 510 (M⁺), 97 (100).
Elementary analysis as C₂₇H₃₈N₆O₂S
Calculated: C 63.50; H 7.50; N 16.46; S 6.28.
Found: C 63.63; H 7.50; N 16.16; S 6.21.

### Example 84

### Preparation of 4-[4-[2-(benzoxazol-2-ylthio)-ethyl]piperazin-1-yl]ethyl]-N-(2,6-diisopropylphenyl)-butyramide:

To a solution of 2,6-diisopropylaniline (1.77 g, 10 mmol) in chloroform (30 ml) was added triethylamine (1.11 g, 11 mmole), then 4-bromobutyryl bromide (1.95 mg, 10.5 mmol) was slowly dropped thereinto with ice cooling and the mixture was made to react for 20 minutes. The reaction solution was diluted with water and extracted with chloroform. The organic layer was washed with 0.5N HCl, water, aqueous solution of sodium bicarbonate and saturated sodium chloride solution successively and dried over magnesium sulfate and the solvent was evaporated therefrom. The residue was purified by a silica gel column chromatography (120 g of silica gel; developing solvent, hexane:acetone = 7:1 → 5:1) and the resulting crude crystals were recrystallized from hexane-acetone to provide 2.06 g (yield 63%) of 4-bromo-N-(2,6-diisopropylphenyl)butyramide as colorless needles.

Potassium carbonate (1.11 g, 8 mmol) was added to a solution of the amide (655 mg, 2 mmol) and 1-[2-(benzoxazol-2-ylthio)ethyl]piperazine ditrifluoroacetate (983 mg, 2 mmol) in acetonitrile (15 ml) and the mixture was stirred at room temperature for 7 hours. The reaction solution was filtered and the filtrate was concentrated *in vacuo*. The residue was diluted with ethyl acetate. The organic layer was washed with water and a saturated sodium chloride solution successively and dried over sodium sulfate and the solvent was evaporated therefrom. The residue was purified by a silica gel column chromatography (75 g of silica gel; developing solvents, chloroform:methanol = 25:1 → chloroform:ammonia-saturated methanol = 10:1) and the resulting crude crystals were recrystallized from acetone-ether-hexane to provide 117 mg (yield 12%) of the desired compound as colorless crystals.
Melting point: 134-136°C
IR (KBr) cm⁻¹: 3432, 3290, 1652, 1500.
1H-NMR (CDCl₃) δ :
1.12 (12H, d, J = 6.8 Hz), 1.77-1.82 (2H, m), 2.34-2.41 (6H, m), 2.50-2.52 (6H, m), 2.75 (2H, t, J = 6.8 Hz), 3.08 (2H, sept, J = 6.8 Hz), 3.46 (2H, t, J = 6.8 Hz), 7.10 (2H, d, J = 7.6 Hz), 7.20 (1H, t, J = 7.6 Hz), 7.25-7.32 (2H, m), 7.53-7.58 (2H, m), 8.72 (1H, br s).
EIMS *m*/*z* (relative intensity): 508 (M⁺, 100).
Elementary analysis as C₂₉H₄₀N₄O₂S
Calculated: C 68.47; H 7.92; N 11.01; S 6.30
Found: C 68.31; H 8.03; N 11.25; S 6.26.

### Example 85

### Preparation of 2-[4-[2-(7-trifluoromethylbenzoxazol-2-ylthio)ethyl]piperazin-1-yl]-N-(2,6-diisopropylphenyl)acetamide:

Acetyl nitrate obtained mixing acetyl anhydride (3.30 g, 33 mmol) with fuming nitric acid (2.05 g, 33 mmol) at 0°C was dropped into a solution of 2-trifluoromethylphenol (4.86 mg, 30 mmol) in acetonitrile (60 ml) at 0°C followed by stirring for 10 minutes. The reaction solution was diluted with water and extracted with ether. The organic layer was washed with water and a saturated sodium chloride solution successively and dried and the solvent was evaporated therefrom. The residue was purified by a silica gel column chromatography (60 g of silica gel; developing solvent, hexane:methylene chloride = 5:1) to provide 2.1 g (yield 33%) of 2-nitro-6-trifluoromethylphenol as pale yellow crystals.

A 10% palladium carbon catalyst (1.0 g) was added to a solution of the resulting nitro compound (2.0 g, 9.65 mmol) in ethanol (60 ml) and the mixture was stirred under hydrogen atmosphere at room temperature for 5 hours. After completion of the reaction, the reaction solution was filtered off through celite and the filtrate was concentrated to provide 1.70 g (yield 99%) of 2-amino-6-trifluoromethylphenol as pale yellow crystals.

O-Ethyl potassium dithiocarbonate (1.68 g, 11 mmol) was added to a solution of the aminophenol (1.70g, 11 mmol) in ethanol (30 ml), the mixture was heated to reflux for 16 hours and the solvent was evaporated therefrom. The residue was dissolved in water, the solution was adjusted to pH 3-4 by adding 2N hydrochloric acid thereto and extracted with ethyl acetate. The organic layer was washed with water and a saturated sodium chloride solution successively and dried over anhydrous magnesium sulfate and the solvent was evaporated therefrom. The residue was purified by a silica gel column chromatography (60 g of silica gel; developing solvents, hexane:acetone = 5:1) to provide 1.78 g (yield 81%) of 2-mercapto-7-trifluoromethylbenzoxazole as pale brown crystals.

The same reaction and treatment as in Example 1 were conducted using 2-mercapto-7-trifluoromethylbenzoxazole instead of 2-mercaptobenzooxazole to provide the desired compound as colorless needles.
Melting point: 135-137°C (decomposition)
IR (KBr) cm⁻¹: 3433, 3229, 1664, 1505.
1H-NMR (CDCl₃) δ :
1.21 (12H, d, J = 6.8 Hz), 2.64-2.68 (4H, m), 2.71-2.75 (4H, m), 2.86 (2H, t, J = 6.8 Hz), 3.00 (2H, sept, J = 6.8 Hz), 3.21 (2H, s), 3.50 (2H, t, J = 6.8 Hz), 7.18 (1H, d, J = 8.3 Hz), 7.18 (1H, d, J = 7.1 Hz), 7.29 (1H, dd, J = 8.3, 7.1 Hz), 7.38 (1H, t, J = 7.8 Hz), 7.48 (1H, d, J = 7.8 Hz), 7.76 (1H, d, J = 7.8 Hz), 8.59 (1H, br s).
EIMS *m*/*z* (relative intensity): 548 (M⁺), 261 (100).
Elementary analysis as C₂₈H₃₅F₃N₄O₂S
Calculated: C 61.30; H 6.43; N 10.21; F 10.39
Found: C 61.31; H 6.41; N 10.15; F 10.16.

### Example 86

### Preparation of 2-[4-[2-(7-trifluoromethylbenzoxazol -2-ylthio)ethyl]piperazin-1-yl]-N-(2,6-diisopropyl-4-methoxyphenyl)acetamide:

Potassium carbonate (1.52 g, 11 mmol) was added to a solution of 1-(2-hydroxyethyl)piperazine (1.43 g, 11 mmol) and 2-bromo-N-(2,6-diisopropyl-4-hydroxyphenyl)acetamide in acetonitrile (50 ml) and the mixture was stirred at room temperature for 12 hours. The reaction solution was diluted with water and extracted with chloroform. The organic layer was washed with water and a saturated sodium chloride solution successively and dried oven anhydrous sodium sulfate and the solvent was evaporated therefrom. The crude crystals were recrystallized from acetone-hexane to provide 2.5 g (yield 69%) of 2-[4-(2-hydroxyethyl)piperazin-1-yl]-N-(2,6-diisopropyl-4-hydroxyphenyl)acetamide as colorless needles.

Into a solution of the acetamide (640 mg, 1.76 mmol) in a mixed solvent (10 ml) of methanol and acetonitrile (1:4) were dropped N,N-diisopropylethylamine (0.43 ml, 2.46 mmol) and trimethylsilyl diazomethane (1.23 ml, 2.46 mmol) and the mixture was stirred for 12 hours. The residue obtained by evaporation of the solvent was made alkaline by adding 2N sodium hydroxide and then extracted with chloroform. The organic layer was washed with water and a saturated sodium chloride solution successively and dried over anhydrous sodium sulfate and the solvent was evaporated therefrom to provide 550 mg (yield 83%) of 2-[4-(2-hydroxyethyl)piperazin-1-yl]-N-(2,6-diisopropyl-4-methoxyphenyl)acetamide as colorless crystals.

The same reaction and treatment as in Example 85 were conducted using the acetamide instead of 2-[4-(2-hydroxyethyl)piperazin-1-yl]-N-(2,6-diisopropylphenyl)-acetamide to provide the desired compound as colorless needles.
Melting point: 122-123°C
IR (KBr) cm⁻¹: 3471, 3266, 2961, 1633, 1603.
1H-NMR (CDCl₃) δ :
1.18 (12H, d, J = 7.0 Hz), 2.64-2.69 (4H, m), 2.69-2.74 (4H, m), 2.86 (2H, t, J = 7.0 Hz), 2.97 (2H, sept, J = 7.0 Hz), 3.19 (2H, s), 3.50 (2H, t, J = 7.0 Hz), 3.81 (3H, s), 6.71 (2H, s), 7.38 (1H, t, J = 7.8 Hz), 7.47 (1H, d, J = 7.8 Hz), 7.75 (1H, d, J = 7.8 Hz), 8.45 (1H, br s).
EIMS *m*/*z* (relative intensity): 578 (M⁺), 111 (100).
Elementary analysis as C₂₉H₃₇F₃N₄O₃S
Calculated: C 60.19; H 6.44; N 9.68; F 9.85
Found: C 60.43; H 6.49; N 9.63; F 9.57.

### Example 87

### Preparation of 2-[4-[2-(7-trifluoromethylbenzoxazol-2-ylthio)ethyl]piperazin-1-yl]-N-(2,6-diisopropyl-3-nitrophenyl)acetamide:

The same reaction and treatment as in Example 85 were conducted using 2-bromo-N-(2,6-diisopropyl-3-nitrophenyl) acetamide instead of 2-bromo-N-(2,6-diisopropylphenyl) acetamide to provide the desired compound as colorless needles.
Melting point: 115-117°C.
IR (KBr) cm⁻¹: 3441, 3294, 1665, 1526, 1506.
1H-NMR (CDCl₃) δ :
1.21 (6H, d, J = 6.9 Hz), 1.33 (6H, d, J = 7.1 Hz), 2.46-2.69 (4H, s), 2.72-2.76 (4H, m), 2.86 (2H, t, J = 6.8 Hz), 2.99 (1H, sept, J = 6.9 Hz), 3.22 (2H, s), 3.25 (1H, sept, J = 7.1 Hz), 3.50 (2H, t, J = 6.8 Hz), 7.30 (1H, d, J = 8.5 Hz), 7.38 (1H, t, J = 7.8 Hz), 7.47 (1H, d, J = 8.5 Hz), 7.48 (1H, d, J = 7.8 Hz), 7.76 (1H, d, J = 7.8 Hz), 8.80 (1H, br s).
EIMS *m*/*z* (relative intensity): 593 (M⁺), 375 (100).
Elementary analysis as C₂₈H₃₄F₃N₅O₄S
Calculated: C 56.65; H 5.77; N 11.80.
Found: C 56.66; H 5.85; N 11.75.

### Example 88

### Preparation of 2-[4-[2-(7-trifluoromethylbenzoxazol -2-ylthio)ethyl]piperazin-1-yl]-N-(2,4-bis(methylthio-6-methyl-3-pyridyl)acetamide:

The same reaction and treatment as in Example 85 were conducted using 2-bromo-N-[2,4-bis(methylthio)-6-methyl-pyridine-3-yl]acetamide instead of 2-bromo-N-(2,6-diisopropylphenyl)acetamide to provide the desired compound as colorless needles.
Melting point: 153-155°C.
IR (KBr) cm⁻¹: 3437, 3280, 1653, 1505.
1H-NMR (CDCl₃) δ :
2.42 (3H, s), 2.50 (3H, m), 2.52 (3H, s), 2.65-2.70 (4H, m), 2.73-2.78 (4H, m), 2.86 (2H, t, J = 7.0 Hz), 3.19 (2H, s), 3.50 (2H, t, J = 7.0 Hz), 6.67 (1H, s), 7.37 (1H, t, J = 7.8 Hz), 7.47 (1H, d, J= 7.8 Hz), 7.76 (1H, d, J = 7.8 Hz), 8.55 (1H, br s).
EIMS *m*/*z* (relative intensity): 571 (M⁺), 354 (100).
Elementary analysis as C₂₄H₂₈F₃N₅O₂S₃
Calculated: C 50.42; H 4.94; N 12.25.
Found: C 50.49; H 4.98; N 12.14.

### Example 89

### Preparation of 2-[4-[2-(7-trifluoromethylbenzoxazol-2-ylthio)ethyl]piperazin-1-yl]-N-[2,4-bis(ethylthio)-6-methyl-3-pyridyl]acetamide:

The same reaction and treatment as in Example 85 were conducted using 2-bromo-N-[2,4-bis(ethylthio)-6-methyl-3-pyridyl]acetamide instead of 2-bromo-N-(2,6-diisopropylphenyl)acetamide to provide the desired compound as colorless needles.
Melting point: 107-108°C.
IR (KBr) cm⁻¹: 3438, 3298, 1702, 1505.
1H-NMR (CDCl₃) δ :
1.32 (3H, t, J = 7.3 Hz), 1.35 (3H, t, J = 7.3 Hz), 2.47 (3H,s), 2.64-2.71 (4H, m), 2.73-2.79 (4H, m), 2.86 (2H, t, J = 6.8 Hz), 2.93 (2H, q, J = 7.3 Hz), 3.16 (2H, q, J = 7.3 Hz), 3.19 (2H, s), 3.51 (2H, t, J = 6.8 Hz), 6.70 (1H, s), 7.37 (1H, t, J = 7.8 Hz), 7.47 (1H, d, J = 7.8 Hz), 7.76 (1H, d, J = 7.8 Hz), 8.52 (1H, br s).
EIMS *m*/*z* (relative intensity): 599 (M⁺), 538 (100).
Elementary analysis as C₂₆H₃₂F₃N₅O₂S₃
Calculated: C 52.07; H 5.38; N 11.68.
Found: C 52.16; H 5.43; N 11.59.

### Example 90

### Preparation of 2-[4-[2-(7-trifluoromethylbenzoxazol-2-ylthio)ethyl]piperazin-1-yl]-N-[2,4-bis(isopropylthio)-6-methyl-3-pyridyl]acetamide:

The same reaction and treatment as in Example 85 were conducted using 2-bromo-N-[2,4-bis(isopropylthio)-6-methyl-3-pyridyl]acetamide instead of 2-bromo-N-(2,6-isopropylphenyl)acetamide to provide the desired compound as pale amorphous.
IR (KBr) cm⁻¹: 3434, 3312, 1702, 1506.
1H-NMR (CDCl₃) δ :
1.35 (6H, d, J = 6.8 Hz), 1.36 (6H, d, J = 6.8 Hz), 2.46 (3H, s), 2.65-2.71 (4H, m), 2.73-2.80 (4H, m), 2.87 (2H, t, J = 7.0 Hz), 3.18 (2H, s), 3.50(1H, sept, J = 6.8 Hz), 3.51 (2H, t, J= 7.0 Hz), 4.02 (1H, sept, J = 6.8 Hz), 6.75 (1H, s), 7.37 (1H, t, J = 7.8 Hz), 7.47 (1H, d, J = 7.8 Hz), 7.76 (1H, d, J = 7.8 Hz), 8.51 (1H, br s).
EIMS *m*/*z* (relative intensity): 627 (M⁺), 111 (100).

### Example 91

### Preparation of 2-[4-[3-(7-trifluoromethylbenzoxazol-2-ylthio)propyl]homopiperazin-1-yl]-N-(2,6-diisopropylphenyl)acetamide:

The same reaction and treatment as in Example 13 were conducted using 2-mercapto-7-trifuluorobenzoxazole instead of 2-mercaptobenzooxazole to provide the desired compound as pale yellow crystals.
Melting point: 77-79°C.
IR (KBr) cm⁻¹: 3447, 3276, 1661, 1503.
1H-NMR (CDCl₃) δ:
1.21 (12H, d, J = 6.8 Hz), 1.89 (2H, quint, J = 5.9 Hz), 2.02 (2H, quint, J = 6.8 Hz), 2.68 (2H, t, J = 6.8 Hz), 2.74-2.78 (4H, m), 2.92-2.96 (4H, m), 3.02 (2H, sept, J = 6.8 Hz), 3.35 (2H, s), 3.39 (2H, t, J = 6.8 Hz), 7.18 (2H, d, J = 8.1 Hz), 7.28 (1H, t, J = 8.1 Hz), 7.37 (1H, t, J = 7.8 Hz), 7.47 (1H, d, J = 7.8 Hz), 7.75 (1H, d, J = 7.8 Hz), 8.77 (1H, br s).
EIMS *m*/*z* (relative intensity): 576 (M⁺), 153 (100).
Elementary analysis as C₃₀H₃₉F₃N₄O₂S
Calculated: C 62.48; H 6.82; N 9.71; F 9.88.
Found: C 62.56; H 6.85; N 9.69; F 9.71.

### Example 92

### Preparation of 2-[4-[2-(7-acetylbenzoxazol-2-ylthio)-ethyl]piperazin-1-yl]ethyl]-N-(2,6-diisopropylphenyl)-acetamide:

Potassium O-ethyl dithiocarbonate (241 mg, 1.5 mmol) was added to a solution of 3-amino-2-hydroxyacetophenone (113 mg, 0.75 mmol) in ethanol (10 ml) and the mixture was heated to reflux for 16 hours. The reaction was concentrated, water (20 ml) was added thereto and the mixture was adjusted to pH 3-4 with diluted hydrochloric acid. The separated matter was collected by filtration and dried by heating *in vacuo* to provide 134 mg (yield 92%) of 7-acetyl-2-mercaptobenzooxazole as a dark solid.

After that, the same reaction and treatment as in Example 1 were conducted using 7-acetyl-2-mercaptobenzooxazole instead of 2-mercaptobenzooxazole to provide the desired compound as colorless needles.
Melting point: 137-139°C
IR (KBr) cm⁻¹: 3432, 3291, 2961, 1688, 1505.
1H-NMR (CDCl₃) δ :
1.21 (12H, d, J = 6.8 Hz), 2.64-2.69 (4H, m), 2.73-2.77 (4H, m), 2.78 (3H, s), 2.87 (2H, t, J = 6.8 Hz), 3.01 (2H, sept, J = 6.8 Hz), 3.23 (2H, s), 3.53 (2H, t, J = 6.8 Hz), 7.19 (2H, d, J = 7.8 Hz), 7.29 (1H, t, J = 7.8 Hz), 7.37 (1H, t, J = 7.8 Hz), 7.77 (1H, dd, J = 7.8, 1.2 Hz), 7.82 (1H, dd, J = 7.8, 1.2 Hz), 8.60 (1H, br s).
EIMS *m*/*z* (relative intensity): 522 (M⁺), 314 (100).
Elementary analysis as C₂₉H₃₈N₄O₃S
Calculated: C 66.64; H 7.33; N 10.72; S 6.13
Found: C 66.57; H 7.34; N 10.70; S 6.19.

### Example 93

### Preparation of 2-[4-[2-(7-acetylbenzoxazol-2-ylthio) ethyl]piperazin-1-yl]-N-(2,6-diisopropyl-4-methoxyphenyl)acetamide:

The same reaction and treatment as in Example 92 were conducted using 2-[4-(2-hydroxyethyl)piperazin-1-yl]-N-(2,6-diisopropyl-4-methoxyphenyl)acetamide instead of 2-[4-(2-hydroxyethyl)piperazin-1-yl]-N-(2,6-diisopropylphenyl)acetamide to provide the desired compound as pale yellow needles.
Melting point: 185-186°C.
IR (KBr) cm⁻¹: 3454, 3270, 2961, 1686, 1657.
1H-NMR (CDCl₃) δ :
1.19 (12H, d, J = 7.0 Hz), 1.43 (3H, s), 2.65-2.69 (4H, m), 2.72-2.77 (4H, m), 2.87 (2H, t, J = 6.7 Hz), 2.98 (2H, sept, J = 7.0 Hz), 3.21 (2H, s), 3.53 (2H, t, J = 6.7 Hz), 3.81 (3H, s), 6.71 (2H, s), 7.37 (1H, dd, J = 8.0, 7.8 Hz), 7.77 (1H, d, J = 8.0 Hz), 7.82 (1H, d, J = 7.8 Hz), 8.46 (1H, br s).
EIMS m/z (relative intensity): 552 (M⁺), 318 (100).

### Example 94

### Preparation of 2-[4-[3-(7-acetylbenzoxazol-2-ylthio) propyl]homopiperazin-1-yl]-N-(2,6-diisopropylphenyl)acetamide:

The same reaction and treatment as in Example 13 were conducted using 7-acetyl-2-mercaptobenzooxazole instead of 2-mercaptobenzooxazole to provide the desired compound as colorless crystals.
Melting point: 86-88°C.
IR (KBr) cm⁻¹: 3425, 3303, 2960, 1687, 1658.
1H-NMR (CDCl₃) δ :
1.21 (12H, d, J = 6.8 Hz), 1.87-1.93 (2H, m), 2.00-2.06 (2H, m), 2.67-2.70 (2H, m), 2.46-2.78 (7H, m), 2.92-2.96 (4H, m), 3.03 (2H, sept, J = 6.8 Hz), 3.35 (2H, s), 3.41 (2H, t, J = 7.0 Hz), 7.18 (2H, d, J = 7.6 Hz), 7.28 (1H, t, J = 7.6 Hz), 7.37 (1H, t, J = 7.8 Hz), 7.77 (1H, dd, J = 7.8, 1.2 Hz), 7.82 (1H, dd, J = 7.8, 1.2 Hz), 8.75 (1H, br s).
EIMS *m*/*z* (relative intensity): 550 (M⁺), 84 (100).
Elementary analysis as C₃₁H₄₂N₄O₃S
Calculated: C 67.61; H 7.69; N 10.17; S 5.82
Found: C 67.37; H 7.62; N 10.18; S 5.73.

### Example 95

### Preparation of 2-[4-[2-(7-tert-butylbenzoxazol-2-ylthio)ethyl]piperazin-1-yl]-N-(2,6-diisopropylphenyl)-acetamide:

Acetyl nitrate obtained by mixing acetyl anhydride (1.35 g, 13.3 mmol) with fuming nitric acid (13.3 mmol) at 0°C was dropped into a solution of 2-tert-butylphenol (2.00 g, 13.3 mmol) in acetonitrile (30 ml) at -20°C followed by stirring for 5 minutes. The reaction solution was diluted with water and extracted with ether. The organic layer was washed with water and a saturated sodium chloride solution successively and dried over anhydrous magnesium sulfate and the solvent was evaporated therefrom. The residue was purified by a silica gel column chromatography (60 g of silica gel; developing solvent, hexane:acetone = 3:1) to provide 600 mg (yield 23%) of 2-tert-butyl-6-nitrophenol as yellow crystals.

A 10% palladium carbon catalyst (250 mg) was added to a solution of the nitrophenol (316 mg, 1.62 mmol) in ethanol (20 ml) and stirred under hydrogen atmosphere at room temperature for 12 hours. The reaction solution was filtered off through celite and the filtrate was evaporated *in vacuo* to provide 260 mg (yield 97%) of 2-amino-6-tert-butylphenol as red crystals. Potassium O-ethyl dithiocarbonate (242 mg, 1.51 mmol) was added to a solution of the aminophenol (227 mg, 1.37 mmol) in ethanol (10 ml) followed by heating to reflux for eight hours. After allowing to cool, the solvent was evaporated *in vacuo* and the resulting residue was dissolved in water, acidified with concentrated hydrochloric acid and extracted with ethyl acetate. The organic layer was washed with water and a saturated sodium chloride solution successively and dried over anhydrous sodium sulfate and the solvent was evaporated therefrom. The residue was purified by a silica gel column chromatography (20 g of silica gel; developing solvent, hexane:acetone = 4:1) to provide 124 mg (yield 44%) of 7-tert-butyl-2-mercaptobenzooxazole as colorless crystals.

After that, the same reaction and treatment as in Example 1 were conducted using 7-tert-butyl-2-mercaptobenzooxazole instead of 2-mercaptobenzooxazole to provide the desired compound as colorless needles.
Melting point: 138-140°C
IR (KBr) cm⁻¹: 3431, 3286, 2961, 1664, 1503.
1H-NMR (CDCl₃) δ :
1.21 (12H, d, J = 6.8 Hz), 1.46 (9H, s), 2.63-2.68 (4H, m), 2.73-2.77 (4H, m), 2.86 (2H, t, J = 7.0 Hz), 3.00 (2H, sept, J = 6.8 Hz), 3.22 (2H, s), 3.49 (2H, t, J = 7.0 Hz), 7.14 (1H, dd, J = 7.8, 1.2 Hz), 7.18-7.23 (3H, m), 7.29 (1H, t, J = 7.7 Hz), 7.44 (1H, dd, J = 7.8, 1.2 Hz), 8.61 (1H, br s).
EIMS *m*/*z* (relative intensity): 536 (M⁺), 263 (100).
Elementary analysis as C₃₁H₄₄N₄O₂S
Calculated: C 69.37; H 8.26; N 10.44
Found: C 60.53; H 8.21; N 10.41.

### Example 96

### Preparation of 2-[4-[2-(7-tert-buthylbenzoxazol-2-ylthio)ethyl]piperazin-1-yl]-N-(2,4-bis(ethylthio)-6-methyl-3-pyridyl)acetamide:

The same reaction and treatment as in Example 89 were conducted using 7-tert-buthyl-2-mercaptobenzooxazole instead of 2-mercapto-7-trifluoromethylbenzoxazole to provide the desired compound as colorless needles.
Melting point: 115-117°C.
IR (KBr) cm⁻¹: 3430, 3327, 1699, 1504, 1479.
1H-NMR (CDCl₃) δ:
1.32 (3H, t, J = 7.3 Hz), 1.35 (3H, t, J = 7.3 Hz), 1.46 (9H, s), 2.47 (3H, s), 2.65-2.70 (4H, m), 2.76-2.81 (4H, m), 2.87 (2H, t, J = 7.0 Hz), 2.93 (2H, q, J = 7.3 Hz), 3.16 (2H, q, J = 7.3 Hz), 3.20 (2H, s), 3.50 (2H, t, J = 7.0 Hz), 6.70 (1H, s), 7.16 (1H, dd, J = 7.8, 1.2 Hz), 7.21 (1H, t, J = 7.8 Hz), 7.44 (1H, dd, J = 7.8, 1.2 Hz), 8.54 (1H, br s).
EIMS *m*/*z* (relative intensity): 587 (M⁺), 381 (100).

### Example 97

### Preparation of 2-[4-[2-(5-chloro-7-isopropyl-4-methylbenzoxazol-2-ylthio)ethyl]piperazin-1-yl]-N-(2,6-diisopropylphenyl)acetamide:

Acetyl nitrate obtained by mixing acetyl anhydride (1.12 g, 11 mmol) with fuming nitric acid (693 mg, 11 mmol) at 0 °C was dropped into a solution of 4-chloro-2-isopropyl-5-methylphenol (1.84 g, 10 mmol) in acetonitrile (20 ml) at 0°C followed by stirring for 50 minutes. The reaction solution was diluted with water and extracted with ether. The organic layer was washed with water and a saturated sodium chloride solution successively and dried over anhydrous magnesium sulfate and the solvent was evaporated therefrom. The residue was purified by a silica gel column chromatography (80 g of silica gel; developing solvent, hexane:methylene chloride = 30:1 → 10:1) to provide 1.88 g (yield 83%) of 4-chloro-6-isopropyl-3-methyl-2-nitrophenol as pale yellow crystals.

Zinc (6.4 g, 98 mmol) was added little by little to a solution of the nitro compound (1.88 g, 8.18 mmol) in acetic acid (30 ml) with ice cooling. After stirring for 1 hour, the reaction solution was diluted by adding ethyl acetate thereto and the mixture was filtered off. The filtrate was neutralized with an aqueous solution of sodium carbonate and extracted with ethyl acetate. The organic layer was washed with water and a saturated sodium chloride solution successively and dried over magnesium sulfate and the solvent was evaporated therefrom to provide 1.63 g (yield 99%) of 2-amino-4-chloro-6-isopropyl-3-methylphenol as pale yellow oil.

Potassium O-ethyl dithiocarbonate (1.60 g, 10 mmol) was added to a solution of the aminophenol (1.60 g, 8.0 mmol) in ethanol (30 ml), the mixture was heated to reflux for 16 hours and the solvent was evaporated. The resulting residue was dissolved in water, acidified to pH 3-4 by adding 2N hydrochloric acid and extracted with ethyl acetate. The organic layer was washed with water and a saturated sodium chloride solution successively and dried over anhydrous magnesium sulfate and the solvent was evaporated therefrom. The residue was purified by a silica gel column chromatography (70 g of silica gel; developing solvent, hexane:acetone = 7:1) to provide 1.28g (yield 66%) of 5-chloro-7-isopropyl-2-mercapto-4-methyl-benzoxazole as colorless needles.

After that, the same reaction and treatment as in Example 1 were conducted using 5-chloro-7-isopropyl-2-mercapto-4-methylbenzoxazole instead of 2-mercaptobenzooxazole to provide the desired compound as colorless needles.
Melting point: 162-163°C
IR (KBr) cm⁻¹: 3436, 3290, 2963, 1660, 1505.
1H-NMR (CDCl₃) δ :
1.21 (12H, d, J = 6.9 Hz), 1.33 (6H, d, J = 6.9 Hz), 2.52 (3H, s), 2.64-2.70 (4H, m), 2.73-2.77 (4H, m), 2.85 (2H, t, J = 7.1 Hz), 3.01 (2H, sept, J = 6.9 Hz), 3.22 (1H, sept, J = 6.9 Hz), 3.23 (2H, s), 3.48 (2H, t, J = 7.1 Hz), 7.07 (1H, s), 7.19 (2H, d, J = 7.8 Hz), 7.29 (1H, t, J = 7.8 Hz), 8.61 (1H, br s).
EIMS *m*/*z* (relative intensity): 570 (M⁺), 330 (100).
Elementary analysis as C₃₁H₄₃ClN₄O₂S
Calculated: C 65.18; H 7.59; N 9.81
Found: C 65.19; H 7.59; N 9.83.

### Example 98

### Preparation of 2-[4-[2-(4,5,6-trimethoxybenzoxazol-2-ylthio)ethyl]piperazin-1-yl]-N-(2,6-diisopropylphenyl)-acetamide:

Sulfanilic acid (1.0 g, 6.0 mmol) was added to a solution of sodium carbonate (318 mg, 3.0 mmol) in water (5 ml)and dissolved by heating, a solution of sodium nitrite (414 mg, 6.0 mmol) in water (1 ml) was added thereto with ice cooling and concentrated hydrochloric acid (1.25 ml) was dropped thereinto to prepare a colorless suspension.

3,4,5-Trimethoxyphenol (921 mg, 6.0 mmol) was added to a solution of sodium hydroxide (1.1 g, 27.5 mmol) in water (6 ml) and dissolved by heating, then the above-prepared suspension was slowly dropped thereinto with ice cooling and the mixture was stirred at room temperature for 1.5 hours. Sodium hydrosulfite was added thereto at 50°C until the red color of the reaction solution almost disappeared. The reaction solution was allowed to cool and extracted with ether three times and with ethyl acetate once. The organic layer was washed with water and a saturated sodium chloride solution successively and dried over magnesium sulfate and the solvent was evaporated therefrom to provide 640 mg (yield 64%) of crude 2-amino-3,4,5-trimethoxyphenol.

Potassium O-ethyl dithiocarbonate (321 mg, 2.0 mmol) was added to a solution of the aminophenol (199 mg, 1.0 mmol) in ethanol (5 ml) and the mixture was heated to reflux for 16 hours. The reaction solution was concentrated, water (30 ml) was added thereto and the mixture was adjusted to pH 3-4 with diluted hydrochloric acid. the separated matter was collected by filtration, dried by heating *in vacuo* and recrystallized from methanol, ether and hexane to provide 155 mg (yield 64%) of 2-mercapto-4,5,6-trimethoxybenzoxazole as reddish purple needles.

After that, the same reaction and treatment as in Example 1 were conducted using 2-mercapto-4,5,6-trimethoxybenzoxazole instead of 2-mercaptobenzooxazole to provide the desired compound as colorless crystals.
Melting point: 126-129°C (decomposition)
IR (KBr) cm⁻¹: 3433, 3254, 2960, 1663, 1486.
1H-NMR (CDCl₃) δ :
1.21 (12H, d, J = 6.8 Hz), 2.62-2.67 (4H, m), 2.72-2.77 (4H, m), 2.83 (2H, t, J = 7.0 Hz), 3.00 (2H, sept, J = 6.8 Hz), 3.22 (2H, s), 3.43 (2H, t, J = 7.0 Hz), 3.84 (3H, s), 3.88 (3H, s), 4.32 (3H, s), 6.71 (1H, s), 7.18 (2H, d, J = 7.6 Hz), 7.29 (1H, t, J = 7.6 Hz), 8.59 (1H, br s).
EIMS *m*/*z* (relative intensity): 570 (M⁺), 126 (100).
Elementary analysis as C₃₀H₄₂N₄O₅S
Calculated: C 63.13; H 7.42; N 9.82; S 5.62
Found: C 63.01; H 7.35; N 9.64; S 5.51.

### Example 99

### Preparation of 2-[4-[2-(6,7-bis(methoxycarbonyl)-benzoxazol-2-ylthio]ethyl]piperazin-1-yl]-N-(2,6-diisopropylphenyl)acetamide:

Acetyl nitrate obtained by mixing acetic anhydride (3.6 g, 36 mmol) with fuming nitric acid (2.16 g, 36 mmol) at 0°C was dropped into a solution of dimethyl 3-hydroxyphthalate (3.8 g, 18 mmol) in acetonitrile (60 ml) at 0°C followed by stirring for 40 minutes. The reaction solution was diluted with water and extracted with ether. The organic layer was washed with water and a saturated sodium chloride solution successively and dried over anhydrous magnesium sulfate and the solvent was evaporated therefrom. A 3:2 mixture (4.34 g; yield 94%) of dimethyl 3-hydroxy-4-nitrophthalate and dimethyl 3-hydroxy-6-nitrophthalate was obtained as a yellow solid. This was used in the next reaction without separation and purification.

A 10% palladium carbon catalyst (2.5 g) was added to a solution of the nitro compound mixture (4.3 g, 16.8 mmol) in ethyl acetate (60 ml) and stirred under hydrogen atmosphere at room temperature for 3 hours. The reaction solution was filtered off through celite and the filtrate was concentrated. The residue was dissolved in methanol (50 ml), potassium O-ethyl dithiocarbonate (1.76 g, 11.0 mmol) was added thereto and the mixture was heated to reflux for 16 hours. After cooling, the solvent was evaporated *in vacuo*. The residue was diluted and acidified with 2N hydrochloric acid and extracted with ethyl acetate. The organic layer was washed with water and a saturated sodium chloride solution successively and dried over anhydrous magnesium sulfate and the solvent was evaporated therefrom. The residue was crystallized from ether to provide 1.61 g (yield 60%) of 6,7-bis(methoxycarbonyl)-2-mercaptobenzooxazole as yellow crystals.

After that, the same reaction and treatment as in Example 1 were conducted using 6,7-bis(methoxycarbonyl)-2-mercaptobenzooxazole instead of 2-mercaptobenzooxazole to provide the desired compound as colorless needles.
Melting point: 186-187°C
IR (KBr) cm⁻¹: 3312, 2963, 1733, 1718, 1660.
1H-NMR (CDCl₃) δ :
1.21 (12H, d, J = 6.8 Hz), 2.60-2.78 (8H, m), 2.84 (2H, t, J = 6.8 Hz), 3.00 (2H, sept, J = 6.8 Hz), 3.21 (2H, s), 3.50 (2H, t, J = 6.8 Hz), 3.92 (3H, s), 4.01 (3H, s), 7.18 (2H, d, J = 7.8 Hz), 7.28 (1H, t, J = 8.3 Hz), 7.63 (1H, d, J = 8.3 Hz), 7.89 (1H, d, J = 8.3 Hz), 8.59 (1H, br s).
EIMS *m*/*z* (relative intensity): 596 (M⁺), 330 (100).
Elementary analysis as C₃₁H₄₀N₄O₆S
Calculated: C 62.40; H 6.76; N 9.39; S 5.37
Found: C 62.21; H 6.76; N 9.37; S 5.40.

### Example 100

### Preparation of 2-[4-[2-(6,7-bis(methoxymethyl)-benzoxazol-2-ylthio]ethyl]piperazin-1-yl]-N-(2,6-diisopropylphenyl)acetamide:

N,N-Diisopropylethylamine (4.39 g, 34.0 mmol) was added to a solution of 3-hydroxyphthalic anhydride (5.0 g, 30.5 mmol) in dichloroethane (60 ml), then chloromethyl methyl ether (2.57 g, 32.0 mmol) was dropped thereinto under cooling with ice water and the mixture was returned to room temperature and stirred for 1 hour. Then N,N-diisopropylethylamine (2.20 g, 17.0 mmol) and chloromethyl methyl ether (1.28 g, 16.0 mmol) were further added thereto followed by stirring for 1 hour. After the reaction, the solvent was evaporated and the residue was diluted with water followed by extracting with ether. The organic layer was washed with water and a saturated sodium chloride solution successively and dried over anhydrous magnesium sulfate and the solvent was evaporated therefrom to provide 6.3 g (yield 99%) of 3-methoxymethyloxyphthalic anhydride as colorless oil.

After that, lithium aluminum hydride (1.14 g, 30.0 mmol) was added little by little to a solution of 3-methoxymethyloxyphthalic anhydride (3.0 g, 14.4 mmol) in anhydrous tetrahydrofuran (100 ml) under cooling with ice water and the mixture was returned to room temperature followed by stirring for 12 hours. The reaction solution was diluted with ether (300 ml) and a saturated aqueous solution (3 ml) of ammonium chloride was added thereto followed by stirring for 1 hour. The reaction solution was dried over anhydrous magnesium sulfate, filtered off through celite and the filtrate was concentrated to provide 1.71 g (yield 60%) of 3-methoxymethyloxy-1,2-benzenedimethanol as colorless oil.

Sodium hydride (384 mg, 8.0 mmol) was added to a solution of the above-prepared diol (714 mg, 3.6 mmol) in dimethyl formamide (10 ml) under cooling with ice water followed by stirring for 15 minutes. Then iodomethane (1.13 g, 8.0 mmol) was added thereto and the mixture was returned to room temperature and stirred for 1 hour. The reaction solution was diluted with a saturated ammonium chloride solution and extracted with ether. The organic layer was washed with water and a saturated sodium chloride solution successively and dried over anhydrous magnesium sulfate and the solvent was evaporated therefrom to provide 810 mg (yield 99%) of 1,2-bis(methoxymethyl)-3-methoxymethyloxybenzene as colorless oil.

Then 2N hydrochloric acid (8 ml) was added to a solution of the above-prepared methoxymethyl ether (810 mg) in tetrahydrofuran (12 ml) and stirred at room temperature for 12 hours. The reaction solution was diluted with water and extracted with ether. The organic layer was washed with a saturated sodium chloride solution and dried over anhydrous magnesium sulfate and the solvent was evaporated therefrom. The residue was purified by a silica gel column chromatography (20 g of silica gel; developing solvent, hexane:acetone = 10:1) to provide 480 mg (yield 73%) of 2,3-bis(methoxymethyl)phenol as colorless oil.

Acetyl nitrate obtained by mixing acetic anhydride (306 mg, 3.0 mmol) with fuming nitric acid (189 mg, 3.0 mmol) at 0 °C was dropped into a solution of 2,3-bis(methoxymethyl)phenol (483 mg, 2.65 mmol) in acetonitrile (5 ml) at 0°C followed by stirring for 40 minutes. The reaction solution was diluted with water and extracted with ether. The organic layer was washed with water and a saturated sodium chloride solution successively and dried over anhydrous magnesium sulfate and the solvent was evaporated therefrom to provide a 2:1 mixture (329 mg; yield 54%) of 2,3-bis(methoxymethyl)-6-nitrophenol and 2,3-bis(methoxymethyl)-4-nitrophenol as yellow solid. This was used in the next reaction without separation and purification.

Zinc (1.13 g, 17 mmol) was added little by little to a solution of the nitro compound (329 mg, 1.44 mmol) in acetic acid (5 ml) under cooling with water. After stirring for 40 minutes, the reaction solution was diluted by adding ethyl acetate thereto and the mixture was filtered. The filtrate was neutralized with an aqueous solution of sodium carbonate and extracted with ethyl acetate. The organic layer was washed with water and a saturated sodium chloride solution successively and dried over magnesium sulfate and the solvent was evaporated therefrom to provide 276 mg (yield 89%) of a 2:1 mixture of 2-amino-5,6-bis(methoxymethyl)phenol and 4-amino-2,3-bis(methoxymethyl)phenol as oil. This was used to the next reaction without separation and purification.

Thus, the aminophenol (276 mg, 1.29 mmol) was dissolved in ethanol (10 ml) and potassium O-ethyl dithiocarbonate (228 mg, 1.42 mmol) was added thereto followed by heating to reflux for 16 hours. After cooling, the solvent was evaporated therefrom *in vacuo*. The residue was acidified by adding 2N hydrochloric acid thereto and extracted with ethyl acetate. The organic layer was washed with water and a saturated sodium chloride solution successively and dried over anhydrous magnesium sulfate and the solvent was evaporated therefrom. The residue was purified by a preparative thin layer chromatography (developing solvent, hexane:acetone = 5:3) to provide 182 mg (yield 59%) of 6,7-bis(methoxymethyl)-2-mercaptobenzooxazole as pale brown solid.

After that, the same reaction and treatment as in Example 1 were conducted using the above-prepared 6,7-bis(methoxymethyl)-2-mercaptobenzooxazole instead of 2-mercaptobenzooxazole to provide the desired compound as colorless needles.
Melting point: 96-97°C
IR (KBr) cm⁻¹: 3290, 2961, 1662, 1506, 1125.
1H-NMR (CDCl₃) δ :
1.20 (12H, d, J = 6.8 Hz), 2.60-2.78 (8H, m), 2.84 (2H, t, J = 6.8 Hz), 3.00 (2H, sept, J = 6.8 Hz), 3.22 (2H, s), 3.40 (6H, s), 3.48 (2H, t, J = 6.8 Hz), 4.63 (2H, s), 4.74 (2H, s), 7.18 (2H, d, J = 7.6 Hz), 7.29 (1H, d, J = 7.6 Hz), 7.34 (1H, d, J = 8.0 Hz), 7.49 (1H, d, J = 8.0 Hz), 8.60 (1H, br s).
EIMS *m*/*z* (relative intensity): 568 (M⁺), 330 (100).
Elementary analysis as C₃₁H₄₄N₄O₄S
Calculated: C 65.46; H 7.80; N 9.85
Found: C 65.41; H 7.75; N 9.71.

### Example 101

### Preparation of 2-[4-[2-(6,7-bis(methoxymethyl)benzoxazol-2-ylthio)ethyl]piperazin-1-yl]-N-(2,4-bis(ethylthio)-6-methyl-3-pyridyl)acetamide:

The same reaction and treatment as in Example 89 were conducted using 6,7-bis(methoxyxethyl)-2-mercaptobenzooxazole instead of 2-mercapto-7-trifluoromethylbenzoxazole to provide the desired compound as colorless needles.
Melting point: 118-120°C
IR (KBr) cm⁻¹: 3334, 2926, 1699, 1561, 1501.
1H-NMR (CDCl₃) δ :
1.32 (3H, t, J = 7.4 Hz), 1.35 (3H, t, J = 7.4 Hz), 2.47 (3H, s), 2.64-2.69 (4H, m), 2.75-2.88 (4H, m), 2.85 (2H, t, J = 7.0 Hz), 2.93 (2H, q, J = 7.4 Hz), 3.15 (2H, q, J = 7.4 Hz), 3.20 (2H, s), 3.40 (3H, s), 3.41 (3H, s), 3.49 (2H, t, J = 7.0 Hz), 4.64 (2H, s), 4.77 (2H, s), 6.70 (1H, s), 7.34 (1H, d, J = 8.3 Hz), 7.50 (1H, d, J = 8.3 Hz), 8.53 (1H, br s).
EIMS *m*/*z* (relative intensity): 619 (M⁺), 381 (100).
Elementary analysis as C₂₉H₄₁N₅O₄S₃
Calculated: C 56.19; H 6.67; N 11.30.
Found: C 56.27; H 6.67; N 11.19.

### Example 102

### Preparation of 2-[4-[2-(7-hydroxymethylbenzoxazol-2-ylthio]ethyl]piperazin-1-yl]-N-(2,6-diisopropylphenyl)acetamide:

A 1.0M solution of diisobutyl aluminum hydride in toluene (10 ml) was slowly dropped, under argon atmosphere at -78 °C , into a solution of 2-mercapto-7-methoxycarbonylbenzoxazole (1.1 g, 5.0 mmol) in THF (20 ml) followed by stirring for 30 minutes. At that temperature, a 1.0M solution of diisobutyl aluminum hydride in toluene (5 ml) was slowly dropped thereinto followed by stirring for 30 minutes. After cooling, diluted hydrochloric acid was added to the reaction mixture to decompose the excessive diisobutyl aluminum hydride, after then extracted with ethyl acetate. The organic layer was washed with diluted hydrochloric acid solution and a saturated sodium chloride solution, dried over sodium sulfate and the solvent was evaporated therefrom. The resulting crude product was recrystallized from hexane-acetone-methanol to provide 848 mg (yield 94%) of 7-hydroxymethyl-2-mercaptobenzooxazole as colorless needles.

After that, the same reaction and treatment as in Example 1 were conducted using 7-hydroxymethyl-2-mercaptobenzooxazole instead of 2-mercaptobenzooxazole to provide the desired compound as colorless crystals.
Melting point: 138-139°C
IR (KBR) cm⁻¹: 3331, 2962, 1657, 1507, 1427.
1H-NMR (d₆-DMSO) δ :
1.13 (12H, d, J=6.8 Hz), 2.55-2.65 (8H, m), 2.79 (2H, t, J=6.8 Hz), 3.05 (2H, sept, J = 6.8 Hz), 3.11 (2H, s), 3.47 (2H, t, J = 6.8 Hz), 4.74 (2H, d, J = 5.5 Hz), 4.90 (1H, t, J = 5.5 Hz), 7.12 (2H, d, J = 7.6 Hz), 7.22 (1H, t, J = 7.6 Hz), 7.26 (1H, t, J = 7.2 Hz), 7.29 (1H, dd, J = 7.2, 2.0 Hz), 7.45 (1H, dd, J = 7.2, 2.0 Hz), 8.77 (1H, br s),
EIMS *m*/*z* (relative intensity): 510 (M⁺), 316 (100).
Elementary analysis as C₂₈H₃₈N₄O₃S
Calculated: C 65.85; H 7.50; N 10.97
Found : C 65.77; H 7.64; N 10.84.

### Example 103

### Preparation of 2-[4-[2-[7-(pyrazol-3-yl)benzoxazol-2-ylthio]ethyl]piperazin-1-yl]-N-(2,6-diisopropylphenyl)acetamide:

Dimethylformamide dimethylacetal (146 mg, 1.2 mmol) was dropped into a solution of 2-[4-[2-(7-acetylbenzoxazol-2-ylthio)ethyl]piperazin-1-yl]-N-(2,6-diisopropylphenyl)acetamide (214 mg, 0.4 mmol) in DMF (15 ml), followed by stirring at 80°C for 4 hours. The reaction solution was allowed to cool and extracted with ethyl acetate. The organic layer was washed with water and diluted hydrochloric acid solution successively and dried over sodium sulfate to provide 245 mg of crude 2-[4-[2-[7-(3-dimethylaminoacryloyl)benzoxazol-2-ylthio]ethyl]piperazin-1-yl]-N-(2,6-diisopropylphenyl)acetamide.

To a solution of the enamine (245 mg, 0.4 mmol) in methanol (6 ml) were added acetic acid (123 mg, 2.05 mmol) and hydrazine monohydrate (102 mg, 2.05 mmol) followed by stirring at room temperature for 15 hours. The reaction solution was concentrated and extracted with ethyl acetate. The organic layer was washed with water and a saturated sodium chloride solution, dried over sodium sulfate and the solvent was evaporated therefrom. The residue was purified by a preparative thin layer chromatography (developing solvent, hexane : acetone = 5:3) to provide 129 mg (yield 58%) of the desired compound as colorless needles.
Melting point: 181-183°C
IR (KBR) cm⁻¹: 3262, 2960, 2360, 1655, 1500.
1H -NMR (CDCl₃) δ :
1.21 (12H, d, J = 6.8 Hz), 2.60-2.76 (8H, m), 2.87 (2H, t, J = 6.8 Hz), 3.01 (2H, sept, J=6.8 Hz), 3.23 (2H, s), 3.53 (2H, t, J = 6.8 Hz), 6.89 (1H, d, J = 2.2 Hz), 7.19 (2H, d, J = 7.6 Hz), 7.29 (1H, t, J = 7.6 Hz), 7.34 (1H, t, J = 8.0 Hz), 7.55 (1H, dd, J = 8.0, 1.2 Hz), 7.68 (1H, d, J = 2.2 Hz), 7.71 (1H, dd, J = 8.0, 1.2 Hz), 8.62 (1H, br s).
EIMS *m*/*z* (relative intensity): 546 (M⁺), 342 (100).
Elementary analysis as C₃₀H₃₈N₆O₂S
Calculated: C 65.91; H 7.01; N 15.37
Found: C 65.89; H 7.06; N 15.22.

### Example 104

### Preparation of 2-[4-[2-(7-nitrobenzoxazol-2-ylthio)ethyl]-piperazin-1-yl]-N-(2,6-diisopropylphenyl)acetamide:

Potassium carbonate (16.6 g, 120.1 mmol) was added to a solution of 3-nitrosalicylic acid (10 g, 54.6 mmol) in DMF (100 ml) and benzyl bromide (14.3 ml, 120.1 mmol) was dropped thereinto. The mixture was stirred at 80°C for 12 hours and the reaction solution was diluted with water and extracted with ether. The organic layer was washed with water and a saturated sodium chloride solution successively, dried over anhydrous sodium sulfate and the solvent was evaporated therefrom to provide 16.1 g (yield 81%) of benzyl 2-benzyloxy-3-nitrobenzoate as brown oil.

To a suspension of the benzyl ester compound (4.42 g, 12.2 mmol) in ethanol (30 ml) was added an aqueous solution (30 ml) of potassium hydroxide (1.37 g, 24.4 mmol) followed by stirring at 50°C for 2 hours. The reaction solution was diluted with water, washed with ether, acidified with 2N hydrochloric acid and extracted with ether. The organic layer was washed with water and a saturated sodium chloride solution successively, dried over anhydrous sodium sulfate and the solvent was evaporated therefrom. The residue was purified by a silica gel column chromatography (90 g of silica gel; developing solvent, hexane : acetone : acetic acid = 25 : 25 : 1) and the resulting crude crystals were recrystallized from acetone-hexane to provide 2.1 g (yield 63%) of 2-benzyloxy-3-nitrobenzoic acid as colorless crystals.

Into a solution of the benzoic acid (2.1 g, 7.69 mmol) in tert-butanol (70 ml) were dropped triethylamine (3.2 ml, 23.1 mmol) and diphenylphosphoryl azide (1.7 ml, 7.69 mmol) with ice cooling and the mixture was heated to reflux for 1 hour. The reaction solution was diluted with water and extracted with ethyl acetate. The organic layer was successively washed with a 2N aqueous solution of sodium hydroxide, 2N hydrochloric acid, water and a saturated sodium chloride solution, dried over anhydrous sodium sulfate and the solvent was evaporated therefrom. The residue was purified by a silica gel column chromatography (90 g of silica gel; developing solvent, hexane : acetone = 5 : 1) to provide 1.61 g (yield 61%) of N-tert-butoxycarbonyl-2-benzyloxy-3-nitroaniline as pale yellow oil.

Into a solution of the aniline compound (1.41 g, 4.1 mmol) in trifuoroacetic acid (30 ml) was dropped thioanisole (4.8 ml, 4 mmol) and the mixture was stirred at room temperature for 15 minutes. The reaction solution was diluted with water, neutralized with a 2N aqueous solution of sodium hydroxide and a saturated sodium bicarbonate solution and extracted with ethyl acetate. The organic layer was washed with water and a saturated sodium chloride solution successively, dried over anhydrous sodium sulfate and the solvent was evaporated therefrom. The residue was purified by a silica gel column chromatography (30 g of silica gel; developing solvent, hexane : acetone = 3 : 1) to provide 430 mg (yield 68%) of 2-amino-6-nitrophenol as reddish purple crystals.

To a solution of the phenol compound (430 mg, 2.8 mmol) in ethanol (30 ml) was added potassium O-ethyl dithiocarbonate (497 mg, 3.1 mmol) and the mixture was heated to reflux for 12 hours. The reaction mixture was diluted with water, acidified with 2N hydrochloric acid and extracted with ether. The organic layer was washed with water and a saturated sodium chloride solution successively, dried over anhydrous sodium sulfate and the solvent was evaporated therefrom. The residue was purified by a silica gel column chromatography (30 g of silica gel; developing solvent, chloroform : methanol = 10 : 1) to provide 381 mg (yield 69%) of 2-mercapto-7-nitrobenzoxazole as yellow crystals.

The same reaction and treatment as in Example 1 were conducted using 2-mercapto-7-nitrobenzoxazole instead of 2-mercaptobenzooxazole to provide the desired compound as pale yellow needles.
Melting point: 153-155°C
IR (KBr) cm⁻¹: 3437, 3226, 1662, 1532, 1505.
1H-NMR (CDCl₃) δ :
1.21 (12H, d, J = 6.8 Hz), 2.65-2.70 (4H, m), 2.72-2.76 (4H, m), 2.88 (2H, t, J = 6.8 Hz), 3.00 (2H, sept, J = 6.8 Hz), 3.22 (2H, s), 3.54 (2H, t, J = 6.8 Hz), 7.19 (2H, d, J = 7.8 Hz), 7.29 (1H, t, J = 7.8 Hz), 7.43 (1H, t, J = 8.3 Hz), 7.88 (1H, dd, J = 8.3, 1.0 Hz), 8.07 (1H, dd, J = 8.3, 1.0 Hz), 8.60 (1H, br s).
EIMS *m*/*z* (relative intensity): 525 (M⁺), 125 (100).
Elemenltary analysis as C₂₇H₃₅N₅O₄S
Calculated : C 61.69; H 6.71; N 13.32
Found : C 61.77, H 6.79; N 13.16.

### Example 105

### Preparation of 2-[4-[2-(7-nitrobenzoxazol-2-ylthio)ethyl]-piperazin-1-yl]-N-(2,6-diisopropyl-4-methoxyphenyl)acetamide:

The same reaction and treatment as in Example 104 were conducted using 2-[4-(2-hydroxyethyl)piperazin-1-yl]-N-(2,6-diisopropyl-4-methoxyphenyl)acetamide instead of 2-[4-(2-hydroxyethyl)piperazin-1-yl]-N-(2,6-diisopropylphenyl)acetamide to provide the desired compound as pale yellow needles.
Melting point: 165-166°C
IR (KBr) cm⁻¹: 3271, 2963, 1659, 1600, 1534.
1H-NMR (CDCl₃) δ :
1.19 (12H, d, J = 6.8 Hz), 2.65 - 2.71 (4H, m), 2.71-2.76 (4H, m), 2.88 (2H, t, J = 6.8 Hz), 2.97 (2H, sept, J = 6.8 Hz), 3.20 (2H, s), 3.54 (2H, t, J = 6.8 Hz), 3.81 (3H, s), 6.71 (2H, s), 7.43 (1H, dd, J = 8.3, 7.9 Hz), 7.88 (1H, d, J = 7.9 Hz), 8.07 (1H, d, J = 8.3 Hz), 8.45 (1H, br s).
EIMS *m*/*z* (relative intensity): 555 (M⁺), 70 (100).
Elementary analysis as C₂₈H₃₇N₅O₅S
Calculated : C 60.52; H 6.71; N 12.60.
Found : C 60.49; H 6.71; N 12.58.

### Example 106

### Preparation of 2-[4-[2-(7-nitrobenzoxazol-2-ylthio)ethyl]-piperazin-1-yl]-N-[2,4-bis(ethylthio)-6-methyl-3-pyridyl]acetamide:

The same reaction and treatment as in Example 104 were conducted using 2-[4-(2-hydroxyethyl)piperazin-1-yl]-N-[2,4-bis(ethylthio)-6-methyl-3-pyridyl]acetamide instead of 2-[4-(2-hydroxyethyl)piperazin-1-yl]-N-(2,6-diisopropylphenyl)acetamide to provide the desired compound as pale yellow amorphous.
Melting point: 50-52°C
IR (KBr) cm⁻¹: 3292, 2929, 2817, 1699, 1532.
1H-NMR (CDCl₃) δ :
1.33 (3H, t, J = 7.3 Hz), 1.36 (3H, t, J = 7.4 Hz), 2.47 (3H, s), 2.64-2.70 (4H, m), 2.72-2.77 (4H, m), 2.88 (2H, t, J = 6.8 Hz), 2.93 (2H, q, J = 7.3 Hz), 3.54 (2H, t, J = 6.8 HZ), 3.16 (2H, q, J = 7.4 Hz), 3.19 (2H, s), 6.70 (1H, s), 7.43 (1H, dd, J = 8.4, 7.9 Hz), 7.88 (1H, d, J = 7.9 HZ), 8.06 (1H, d, J = 8.4 Hz), 8.52 (1H, br s).

### Example 107

### Preparation of 2-[4-[2-(7-nitrobenzoxazol-2-ylthio)ethyl]-piperazin-1-yl]-N-[2,4-bis(isopropylthio)-6-methyl-3-pyridyl]-acetamide:

The same reaction and treatment as in Example 104 were conducted using 2-[4-(2-hydroxyethyl)piperazin-1-yl]-N-[2,4-bis(isopropylthio)-6-methyl-3-pyridyl]acetamide instead of 2-[4-(2-hydroxyethyl)piperazin-1-yl]-N-(2,6-diisopropylphenyl)acetamide to provide the desired compound as pale yellow amorphous.
Melting point: 57-59°C
IR (KBr) cm⁻¹: 3299, 2962, 2818, 1702, 1559.
1H-NMR (CDCl₃) δ :
1.35 (6H, d, J = 6.7 Hz), 1.36 (6H, d, J = 6.8 Hz), 2.46 (3H, s), 2.64-2.69 (4H, m), 2.72-2.76 (4H, m), 2.89 (2H, t, J = 6.9 Hz), 3.18 (2H, s), 3.50 (1H, sept, J = 6.7 Hz), 3.55 (2H, t, J = 6.9 Hz), 4.02 (1H, sept, J = 6.8 Hz), 6.75 (1H, s), 7.43 (1H, dd, J = 8.3, 7.9 Hz), 7.88 (1H, d, J = 7.9 Hz), 8.06 (1H, d, J = 8.3 Hz), 8.50 (1H, br s).
EIMS *m*/*z* (relative intensity): 604 (M⁺), 409 (100).

### Example 108

### Preparation of 2-[4-[2-(7-nitrobenzoxazol-2-ylthio)ethyl]-piperazin-1-yl]-N-(2,6-diisopropyl-3-nitrophenyl)acetamide:

The same reaction and treatment as in Example 104 were conducted using 2-[4-(2-hydroxyethyl )piperazin - 1 - yl ] -N-(2,6-diisopropyl-3-nitrophenyl)acetamide instead of 2-[4-(2-hydroxyethyl)piperazin-1-yl]-N-(2,6-diisopropylphenyl)acetamide to provide the desired compound as pale yellow amorphous.
Melting point: 70-72°C
IR (KBr) cm⁻¹: 3290, 2966, 2820, 1683, 1530.
1H-NMR (CDCl₃) δ :
1.22 (6H, d, J=6.5 Hz), 1.32 (6H, d, J=7.2 Hz), 2.60-2.85 (8H, m), 2.89 (2H, t, J=6.8 Hz), 2.99 (1H, sept, J = 6.5 HZ), 3.23 (2H, s), 3.26 (1H, sept, J = 7.2 Hz), 3.54 (2H, t, J = 6.8 Hz), 7.30 (1H, d, J = 8.5 Hz), 7.44 (1H, dd, J = 8.3, 8.0 Hz), 7.48 (1H, d, J = 8.5 Hz), 7.88 (1H, d, J = 8.0 Hz), 8.07 (1H, d, J = 8.3 Hz), 8.79 (1H, br s).
EIMS *m*/*z* (relative intensity): 570 (M⁺), 321 (100).

### Example 109

### Preparation of 2-[4-[2-(5-chloro-7-isopropyl-4-methylbenzoxazol-2-ylthio)ethyl]piperazin-1-yl]-N-[2,4-bis(ethylthio)-6-methyl-3-pyridyl]acetamide:

The same reaction and treatment as in Example 97 were conducted using 2-[4-(2-hydroxyethyl)piperazin-1-yl]-N-[2,4-bis(ethylthio)-6-methyl-3-pyridyl]acetamide instead of 2-[4-(2-hydroxyethyl)piperazin-1-yl]-N-(2,6-diisopropylphenyl)acetamide to provide the desired compound as colorless needles.
Melting point: 120-122°C
IR (KBr) cm⁻¹: 3301, 2968, 1690, 1481, 1216.
1H-NMR (CDCl₃) δ :
1.32 (3H, t, J = 7.3 Hz), 1.33 (6H, d, J = 6.8 Hz), 1.35 (3H, t, J = 7.3 Hz), 2.47 (3H, s), 2.66-2.71 (4H, m), 2.75-2.81 (4H, m), 2.86 (2H, t, J = 7.1 Hz), 2.93 (2H, q, J = 7.3 Hz), 3.15 (2H, q, J = 7.3 Hz), 3.20 (2H, s), 3.22 (1H, sept, J = 6.8 Hz), 3.49 (2H, t, J = 7.1 Hz), 6.70 (1H, s), 7.07 (1H, s), 8.53 (1H, br s).
EIMS *m*/*z* (relative intensity): 621 (M⁺), 368 (100).
Elementary analysis as C₂₉H₄₀ClN₅O₂S₃
Calculated : C 55.97; H 6.48; N 11.25.
Found C 56.26; H 6.40; N 11.17.

### Example 110

### Preparation of 2-[4-[3-(5-chloro-7-isopropyl-4-methylbenzoxazol-2-ylthio)propyl]piperazin-1-yl]-N-(2,6-diisopropyl-3-methoxyphenyl)-acetamide:

N,N-Diisopropylethylamine (513 mg, 3.97 mmol) and a solution of trimethylsilyl diazomethane in hexane (2.0 M, 2.0 ml, 3.97 mmol) were added to a solution of N-(2,6-diisopropyl-3-hydroxyphenyl)-2-[4-(3-hydroxypropyl) piperazin-1-yl]acetamide (500mg, 1.32 mmol) in a mixed solvent of methanol (8 ml) and acetonitrile (12 ml) and the mixture was stirred for four days. After the reaction, the solvent was evaporated. The residue was made basic by adding an aqueous solution of sodium bicarbonate and extracted with chloroform. The organic layer was washed with a saturated sodium chloride solution, dried over anhydrous sodium sulfate and the solvent was evaporated therefrom. The residue was purified by a silica gel column chromatography (developing solvent, chloroform : methanol = 10 : 1) to provide 449 mg (yield 87%) of N-(2,6-diisopropyl-3-methoxyphenyl)-2-[4-(3-hydroxypropyl)piperazin-1-yl]acetamide.

To a solution of the alcohol (150 mg, 0.38 mmol) in THF (3 ml) were added triethylamine (50 mg, 0.50 mmol) and 4-dimethylaminopyridine (5 mg, 0.04 mmol), then methanesulfonyl chloride (53 mg, 0.46 mmol) was dropped thereinto with ice cooling and stirring, the mixture was stirred for 30 minutes. After the reaction, the reaction solution was diluted with water and extracted with ethyl acetate. The organic layer was washed with a saturated sodium chloride solution, dried over anhydrous sodium sulfate and the solvent was evaporated. The resulting residue was dissolved in DMF (5 ml), then 5-chloro-7-isopropyl-2-mercapto-4-methylbenzoxazole (93 mg, 0.38 mmol), potassium carbonate (64 mg, 0.46 mmol) and 18-crown-6 (10 mg, 0.04 mmol) were added thereto and the mixture was stirred at 80°C for 1 hour. The reaction solution was diluted with water and extracted with ethyl acetate. The organic layer was washed with water and a saturated sodium chloride solution successively, dried over anhydrous sodium sulfate and the solvent was evaporated. The resulting residue was purified by a silica gel column chromatography (developing solvent, chloroform : methanol = 50 : 1) and the resulting crystals were recrystallized from ethyl acetate-hexane to provide 91 mg (yield 39%) of the desired compound as colorless powdery crystals.
Melting point: 126-127°C
IR (KBr) cm⁻¹: 3288, 2962, 1663, 1501, 1491.
1H-NMR (CDCl₃) δ :
1.18 (6H, d, J = 6.9 Hz), 1.30 (6H, d, J = 6.9 Hz), 1.33 (6H, d, J = 6.9 Hz), 2.92 (2H, quint, J = 7.0 Hz), 2.50-2.60 (4H, m), 2.52 (3H, s), 2.54 (2H, t, J = 7.0 Hz), 2.70 - 2.79 (4H, m), 2.92 (1H, sept, J = 6.9 Hz), 3.13 - 3.26 (2H, m), 3.21 (2H, s), 3.36 (2H, t, J = 7.0 Hz), 3.80 (3H, s), 6.84 (1H, d, J = 8.7 Hz), 7.07 (1H, s) 7.12 (1H, d, J = 8.7 Hz), 8.59 (1H, br s).
EIMS *m*/*z* (relative intensity): 616 (M⁺+1), 139 (100).

### Example 111

### Preparation of N-(2,6-diisopropyl-3-methoxyphenyl)-2- [4-[3-(7-trifuluoromethylbenzoxazol-2-ylthio)propyl]piperazin-1-yl]acetamide:

The same reaction and treatment as in Example 110 were conducted using 2-mercapto-7-trifluoromethylbenzoxazole instead of 5-chloro-7-isopropyl-2-mercapto-4-methylbenzoxazole to provide the desired compound as colorless powdery crystals.
Melting point: 139-141°C
IR (KBr) cm⁻¹: 3278, 2960, 1664, 1506, 1332.
1H-NMR (CDCl₃) δ :
1.17 (6H, d, J = 6.9 Hz), 1.30 (6H, d, J = 6.9 Hz), 2.06 (2H, quint, J = 6.9 Hz), 2.50-2.60 (4H, m), 2.55 (2H, t, J = 6.9 Hz), 2.71-2.78 (4H, m), 2.92 (1H, sept, J = 6.9 Hz), 3.18 (1H, sept, J = 6.9 Hz), 3.21 (2H, s), 3.39 (2H, t, J = 6.9 Hz), 3.80 (3H, s), 6.84 (1H, d, J = 8.6 Hz), 7.12 (1H, d, J = 8.6 Hz), 7.38 (1H, m), 7.47 (1H, d, J = 7.8 Hz), 7.75 (1H, d, J = 7.8 Hz), 8.60 (1H, br s).

### Example 112

### Preparation of 2-[4-[2-(7-methylthiobenzoxazol-2-ylthio)ethyl]piperazin-1-yl]-N-(2,6-diisopropylphenyl)-acetamide:

N-tert-Butoxycarbonyl-2-benzyloxy-3-nitroaniline (9.37 g, 27.2 mmol) was dissolved in methanol (150 ml), p-toluenesulfonic acid monohydrate (7.84 g, 45.5 mmol) was added thereto and the mixture was stirred at 50°C for 12 hours. The reaction solution was neutralized with a saturated aqueous solution of sodium bicarbonate and extracted with chloroform. The organic layer was washed with water and a saturated sodium chloride solution successively and dried over anhydrous sodium sulfate and the solvent was evaporated. The residue was purified by a silica gel column chromatography (50 g of silica gel; developing solvent, hexane:ethyl acetate = 6:1) to provide 6.44 g (yield 96.9%) of 2-benzyloxy-3-nitroaniline as pale yellowish brown oil.

To the resulting nitroaniline (5.80 g, 23.7 mmol) was added concentrated hydrochloric acid (10 ml) with ice cooling and, with stirring, a solution of sodium nitrite (4.27 g, 61.9 mmol) in water (5 ml) was dropped into the resulting suspension during 10 minutes. The mixture was stirred for 1 hour in an ice bath, adjusted to pH 7 with a saturated aqueous solution of sodium bicarbonate, then sodium thiomethoxide (2.00 g, 28.5 mmol) was added thereto, and the mixture was stirred for 5 minutes. This was stirred at 80°C for 10 minutes more, the reaction solution was allowed to cool to room temperature, extracted with chloroform, the organic layer was washed with water and a saturated sodium chloride solution successively and dried over anhydrous sodium sulfate and the solvent was evaporated. The residue was purified by a silica gel column chromatography (150 g of silica gel; developing solvent, hexane:benzene = 2:1) and the resulting crude crystals were recrystallized from ethyl acetate-hexane to provide 0.87 g (yield 19.8%) of 2-methylthio-6-nitrophenol as pale yellowish brown needles.

The nitrophenol (290 mg, 1.57 mmol) was dissolved in acetic acid (13 ml) and concentrated hydrochloric acid (0.3 ml) and, under cooling with water, zinc powder (411 mg, 6.28 mmol) was added thereto. The mixture was stirred at room temperature for 15 minutes, the reaction solution was filtered off and the filtrate was neutralized with 2N sodium hydroxide and a saturated aqueous solution of sodium bicarbonate and extracted with ethyl acetate. The organic layer was washed with water and a saturated sodium chloride solution successively and dried over anhydrous sodium sulfate and the solvent was evaporated. The residue was purified by a silica gel column chromatography (20 g of silica gel; developing solvent, hexane:ethyl acetate = 1:1) to provide 230 mg (yield 94%) of 2-amino-6-methylthiophenol as pale brown needles.

To a solution of the phenol compound (230 mg, 1.48 mmol) in ethanol (30 ml) was added potassium O-ethyldithiocarbonate (285 mg, 1.78 mmol) and the mixture was heated to reflux for 12 hours. The reaction solution was diluted with water, acidified with 2N hydrochloric acid and extracted with ethyl acetate. The organic layer was washed with water and a saturated sodium chloride solution successively and dried over anhydrous sodium sulfate and the solvent was evaporated. The residue was purified by a silica gel column chromatography (25 g of silica gel; developing solvent, hexane:ethyl acetate = 2:1) to provide 224 mg (yield 77%) of 2-mercapto-7-methylthiobenzoxazole as yellow crystals.

The same reaction and treatment as in Example 1 were conducted using 2-mercapto-7-methylthiobenzoxazole instead of 2-mercaptobenzooxazole to provide the desired compound as colorless needles.
Melting point: 120-121°C
IR (KBr) cm⁻¹: 3247, 2960, 1660, 1499, 1414.
1H-NMR (CDCl₃) δ :
1.20 (12H, d, J = 7.0 Hz), 2.59 (3H, s), 2.60-2.78 (8H, m), 2.85 (2H, t, J = 6.8 Hz), 3.00 (2H, sept, J = 7.0 Hz), 3.21 (2H, s), 3.49 (2H, t, J = 6.8 Hz), 7.13-7.31 (5H, m), 7.40 (1H, d, J = 7.8 Hz), 8.61 (1H, br s).
EIMS *m*/*z* (relative intensity): 526 (M⁺), 125 (100).

### Example 113

### Preparation of 2-[4-[2-(7-methanesulfonylbenzoxazol-2-ylthio)ethyl]piperazin-1-yl]-N-(2,6-diisopropylphenyl)acetamide:

Sodium perborate tetrahydrate (640 mg, 4.16 mmol) was added to a solution of 2-methylthio-6-nitrophenol (120 mg, 0.648 mmol) in acetic acid (6 ml) and the mixture was stirred at 55°C for 4 hours. The residue obtained by concentrating the reaction solution was purified by a silica gel column chromatography (50 g of silica gel; developing solvent, chloroform → chloroform:methanol = 50:1 1 → chloroform: methanol = 4:1), the resulting residue was suspended in acetic acid (12 ml), under cooling with ice water then zinc (450 mg, 6.88 mmol) and concentrated hydrochloric acid (0.2 ml) were added thereto and the mixture was returned to room temperature and stirred for 20 minutes. The reaction solution was neutralized by adding a saturated sodium bicarbonate solution thereto and extracted with chloroform. The organic layer was washed with a saturated sodium chloride solution and dried over anhydrous sodium sulfate and the solvent was evaporated. The residue was purified by a preparative thin layer chromatography (developing solvent, chloroform:methanol = 10:1) to provide 26 mg (yield 21%) of 2-amino-6-methanesulfonylphenol as brown oil.

Potassium O-ethyldithiocarbonate (67.3 mg, 0.420 mmol) was added to a solution of the phenol compound (25.5 mg, 0.136 mmol) in ethanol (8 ml) and the mixture was heated to reflux with stirring for 9 hours. The reaction solution was concentrated, 1N hydrochloric acid was added to the residue until pH became 4 and the mixture was extracted with chloroform. The organic layer was washed with a saturated sodium chloride solution and dried over anhydrous sodium sulfate and the solvent was evaporated. The residue was purified by a silica gel column chromatography (2.5 g of silica gel; developing solvent, chloroform:methanol = 10:1) to provide 29.4 mg (yield 94.4%) of 2-mercapto-7-methanesulfonylbenzoxazole as brown solid. This was recrystallized from methanol-chloroform-ether to provide pale brown crystals.

The same reaction and treatment as in Example 1 were conducted using 2-mercapto-7-methanesulfonylbenzoxazole instead of 2-mercaptobenzooxazole to provide the desired compound as colorless crystals.
Melting point: 125-128°C
IR (KBr) cm⁻¹: 3449, 1660, 1503, 1426, 1322.
1H-NMR (CDCl₃) δ :
1.21 (12H, d, J = 6.8 Hz), 2.66-2.71 (4H, m), 2.74-2.81 (4H, m), 2.86 (2H, t, J=6.8 Hz), 3.00 (2H, sept, J = 6.8 Hz), 3.22 (2H, s), 3.26 (3H, s), 3.53 (2H, t, J = 6.8 Hz), 7.18 (1H, d, J = 8.3 Hz), 7.19 (1H, d, J = 7.3 Hz), 7.29 (1H, dd, J = 8.3, 7.3 Hz), 7.45 (1H, t, J = 7.8 Hz), 7.78 (1H, dd, J = 7.8, 1.2 Hz), 7.84 (1H, dd, J = 7.8, 1.2 Hz), 8.60 (1H, br s).

### Industrial Applicability

The present invention offers an ACAT inhibitor, an agent for inhibiting the transportation of intracellular cholesterol, an agent for lowering the cholesterol in blood or an agent for suppressing the foaming of macrophage containing the compound represented by the above formula (I) or salt(s) or solvate(s) thereof and a pharmaceutically acceptable carrier. Thus, the present invention offers a pharmaceutical composition for therapy and prevention, an agent for therapy and prevention and a method for therapy and prevention of the diseases such as hyperlipemia, arteriosclerosis, cervical and cerebral arteriosclerosis, cerebrovascular disorder, ischemic cardiopathy, coronary arteriosclerosis, nephrosclerosis, arteriosclerotic nephrosclerosis, arteriocapillary sclerotic nephrosclerosis, malignant nephrosclerosis, ischemic entheropathy, acute occlusion of mesenteric vessel, chronic mesenteric angina, ischemic colitis, aortic aneurysm and arteriosclerosis obliterans (ASO).

## Claims

1. A compound of formula (I), or a salt or solvate of a compound of formula (I): 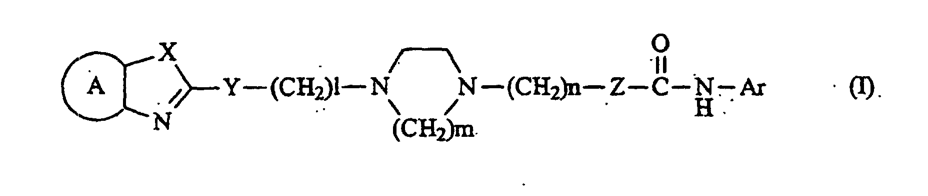 wherein
l is from 1 to 10;
m is 2 or 3;
n is from 1 to 3,
X is -NH-, oxygen atom or sulfur atom;
Y is -NR¹-, oxygen atom, sulfur atom, sulfoxide or sulfone;
Z is a single bond or -NR²-;
R¹ is hydrogen, optionally substituted straight or branched chain C₁ to C₈ alkyl, optionally substituted six-membered aromatic hydrocarbon group or five- to seven-membered heterocyclic group containing from one to three oxygen atom(s), nitrogen atom(s) or sulfur atom(s) as heteroatom(s), or optionally substituted silyl alkyl comprising a straight chain or a branched chain alkyl having 1 to 8 carbon atoms;
R² is hydrogen, optionally substituted straight or branched chain C₁ to C₈ alkyl, optionally substituted six-membered aromatic hydrocarbon group or five- to seven-membered heterocyclic group containing from one to three oxygen atom(s), nitrogen atom(s) or sulfur atom(s) as heteroatom(s), or optionally substituted silyl alkyl comprising a straight chain or a branched chain alkyl having 1 to 8 carbon atoms; 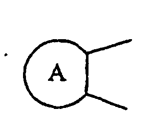 is
(i) a group 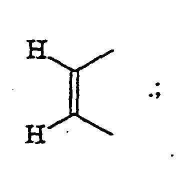
(ii) an unsubstituted divalent residue of benzene, pyridine, cyclohexane or naphthalene; or
(iii) a substituted divalent residue of benzene, pyridine, cyclohexane or naphthalene;
and wherein the optional substituents for the foregoing straight or branched C₁ to C₈ alkyl groups are selected from groups W1;
the optional substituents for the foregoing six-membered aromatic hydrocarbon and five- to seven- membered heterocyclic groups are selected from groups W2; and
the substituents for the foregoing substituted divalent residue are selected from groups W3;
each group W1 is hydroxyl, C₁ to C₈ straight or branched alkoxy, C₁ to C₈ straight or branched alkylthio, C₁ to C₈ straight or branched alkoxycarbonyl C₁ to C₈ straight or branched alkylcarbonyloxy, halogen, amino or nitro;
each group W2 is optionally substituted straight or branched chain C₁ to C₈ alkyl, optionally substituted straight or branched chain C, to C₈ alkoxy, optionally substituted straight or branched chain C₁ to C₈ alkylthio, optionally substituted straight or branched chain C₁ to C₈ alkylsulfinyl, optionally substituted straight or branched chain C, to C₈ alkylsulfonyl, optionally substituted straight or branched chain C₁ to C₈ alkylsulfonyloxy, optionally substituted straight or branched chain C₁ to C₈ alkylcarbonyl, halogen, hydroxyl, optionally substituted C₁ to C₈ alkylcarbonyloxy, nitro, phosphoric acid group, di-(straight or branched chain C, to C₈ alkoxy)-phosphoryloxy, sulfonamide, amino, substituted amino and straight or branched chain C₁ to C₈ alkylenedioxy;
each group W3 is optionally substituted straight or branched chain C₁ to C₈ alkyl, optionally substituted straight or branched chain C₁ to C₈ alkoxy, optionally substituted straight or branched chain C₁ to C₈ alkylcarbonyl, straight or branched chain C₁ to C₈ alkylthio, straight or branched chain C₁ to C₈ alkylsulfinyl, straight or branched chain C₁ to C₈ alkylsulfonyl, halogen, hydroxyl, carboxyl, straight or branched chain C₁ to C₈ alkoxycarbonyl, hydroxy-straight or branched chain C₁ to C₈ alkyl, phosphoric acid group, cyano, nitro, sulfonamide, optionally substituted amino, optionally substituted amino straight or branched chain C₁ to C₈ alkyl, optionally substituted silyl straight or branched chain C₁ to C₈ alkyl, or an optionally substituted residue of a saturated or unsaturated five to seven membered heterocyclic group containing one to four oxygen atom(s), nitrogen atom(s) or sulfur atom(s) as heteroatom(s); or straight or branched chain C₁ to C₈ alkylenedioxy group;
and wherein the optional substituents for groups W2 and W3 other than substituted amine, substituted aminoalkyl and substituted silylalkyl groups W2 and W3, are selected from groups W1;
the substituents for substituted amino and substituted aminoalkyl groups W2 and W3 are selected from groups W4; and
the substituents for substituted silylalkyl groups W2 and W3 are selected from groups W5;
each group W4 is selected from straight or branched C₁ to C₈ alkyl, optionally substituted six-membered aromatic hydrocarbon group or five- to seven- membered heterocyclic group containing from one to three oxygen atom(s), nitrogen atom(s) or sulfur atom(s) as heteroatom(s) and optionally substituted aralkyl group having 7 to 20 carbons, the two groups W4 of the amino group may form a five- to seven- membered optionally substituted ring together with the nitrogen atom of the amino group, one or more carbon atom(s) in that ring may be replaced by oxygen atom, sulfur atom or nitrogen atom, optional substituents for groups W4 being selected from straight or branched chain C₁ to C₈ alkyl, straight or branched chain C₁ to C₈ alkoxy, straight or branched chain C₁ to C₈ alkylthio, straight or branched chain C₁ to C₈ alkylsulfinyl, straight or branched chain C₁ to C₈ alkylsulfonyl, straight or branched chain C₁ to C₈ alkylsulfonyloxy, straight or branched chain C₁ to C₈ alkylcarbonyl, halogen, hydroxyl, nitro, phosphoric acid group, di-(straight or branched chain C₁ to C₈ alkoxy)-phosphoryloxy, sulfonamide, amino group and straight or branched chain C₁ to C₈ alkylenedioxy; and
each group W5 is selected from straight or branched chain C₁ to C₈ alkyl; six-membered aromatic hydrocarbon group or five- to seven- membered heterocyclic group containing from one to three oxygen atom(s), nitrogen atom(s) or sulfur atom(s) as heteroatom(s) or aralkyl of 7 to 20 carbons;
and Ar is optionally substituted six-membered aromatic hydrocarbon group or five- to seven- membered heterocyclic group containing from one to three oxygen atom(s), nitrogen atom(s) or sulfur atom(s) as heteroatom(s) wherein optional substituents for Ar are selected from groups W2 as defined above.

2. A compound according to claim 1, or a salt or solvate thereof, wherein Ar is phenyl, pyridyl or pyrimidyl group which may be substituted with from one to four group(s) selected from optionally-substituted alkyl, optionally substituted alkoxy, alkylthio, alkylsulfinyl, alkylsulfonyl, alkylsulfonyloxy, optionally substituted alkylcarbonyl, halogen, hydroxyl, optionally substituted alkylcarbonyloxy, nitro, phosphoric acid group, dialkoxy-phosphoryloxy, sulfonamide, optionally substituted amino and alkylenedioxy.

3. A compound according to claim 2, or a salt or solvate thereof, wherein and wherein, 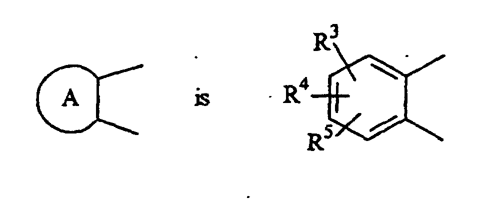 R³, R⁴ and R⁵ are the same or different and are each independently selected from hydrogen, optionally substituted alkyl, optionally substituted alkoxy, optionally substituted alkylcarbonyl, alkylthio, alkylsulfinyl, alkylsulfonyl, halogen, hydroxy, carboxy, alkoxycarbonyl, hydroxyalkyl, phosphoric acid group, cyano, nitro, sulfonamide, optionally substituted amino, optionally substituted aminoalkyl, optionally substituted silylalkyl and heterocyclic residues, or any two of R³, R⁴ and R⁵ together form an alkylenedioxy group.

4. A compound according to claim 2, or a salt or solvate thereof, wherein 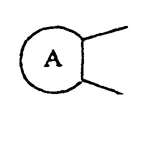 is 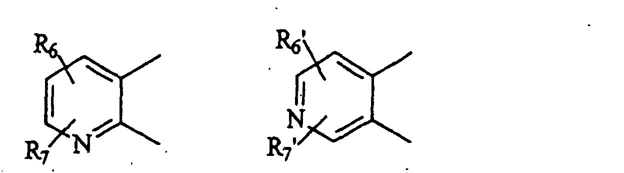 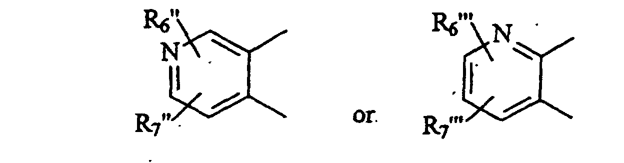 and wherein
R₆ and R₇, R₆' and R₇', R₆" and R₇", R₆''' and R₇''' are the same or different and are each independently selected from hydrogen, optionally substituted alkyl, optionally substituted alkoxy, halogen, hydroxyl, carboxyl, alkoxycarbonyl, hydroxyalkyl, phosphoric acid group, sulfonamide, optionally substituted amino, optionally substituted aminoalkyl, optionally substituted silylalkyl, and heterocyclic residues; or any two of R₆ and R₇; R₆' and R₇'; R₆" and R₇"; R₆''' and R₇''' may form an alkylenedioxy group.

5. A pharmaceutical composition comprising a compound, salt or solvate as claimed in any one of claims 1 to 4.

6. A pharmaceutical composition according to claim 5 comprising an effective amount of the compound, salt or solvate and a pharmaceutically acceptable carrier.

7. A compound, salt or solvate as claimed in any one of claims 1 to 4, for use as an ACAT inhibitor, an agent for inhibiting the transportation or intracellular cholesterol, an agent for lowering the cholesterol in blood or an agent for suppressing foaming of a macrophage.

8. A compound, salt or solvate as claimed in any one of claims 1 to 4, for use as an agent for therapy and prevention of hyperlipemia, arteriosclerosis, cervical and cerebral arteriosclerosis, cerebrovascular disorder, ischemic cardiopathy, coronary arteriosclerosis, nephrosclerosis, arteriosclerotic nephrosclerosis, arteriocapillary sclerotic nephrosclerosis, malignant nephrosclerosis, ischemic entheropathy, acute occlusion of mesenteric vessel, chronic mesenteric angina, ischemic colitis, aortic aneurysm and arteriosclerosis obliterans (ASO).

9. Use of a compound, salt or solvate as claimed in any one of claims 1 to 4, for the preparation of a medicament for use as an ACAT inhibitor, an agent for inhibiting the transportation of intracellular cholesterol, an agent for lowering cholesterol in blood or an agent for suppressing foaming of a macropage.

10. Use according to claim 9 wherein the medicament is for use as an agent for therapy or prevention of hyperlipemia, arteriosclerosis, cervical and cerebral arteriosclerosis, cerebrovascular disorder, ischemic cardiopathy, coronary arteriosclerosis, nephrosclerosis, arteriosclerotic nephrosclerosis, arteriocapillary sclerotic nephrosclerosis, malignant nephrosclerosis, ischemic entheropathy, acute occlusion ofmesenteric vessel, chronic mesenteric angina, ischemic colitis, aortic aneurysm and arteriosclerosis obliterans (ASO).

## Patentansprüche

1. Verbindung der Formel (I), oder ein Salz oder ein Solvat einer Verbindung der Formel (I): 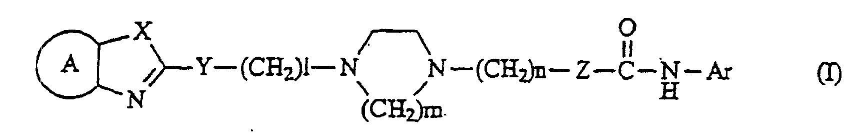 worin
I 1 bis 10 ist;
m 2 oder 3 ist;
n 1 bis 3 ist,
X -NH-, ein Sauerstoffatom oder Schwefelatom ist;
Y -NR¹-, ein Sauerstoffatom, Schwefelatom, Sulfoxid oder Sulfon ist;
Z eine Einfachbindung oder -NR²- ist;
R¹ Wasserstoff, ein gegebenenfalls substituiertes gerad- oder verzweigtkettiges C₁- bis C₈-Alkyl, eine gegebenenfalls substituierte, sechsgliedrige, aromatische Kohlenwasserstoffgruppe oder fünf- bis siebengliedrige heterocyclische Gruppe, enthaltend ein bis drei Sauerstoffatom(e), Stickstoffatom(e) oder Schwefelatom(e) als Heteroatom(e), oder ein gegebenenfalls substituiertes Silylalkyl, umfassend ein geradkettiges oder ein verzweigtkettiges Alkyl mit 1 bis 8 Kohlenstoffatomen ist;
R² Wasserstoff, ein gegebenenfalls substituiertes gerad- oder verzweigtkettiges C₁- bis C₈-Alkyl, eine gegebenenfalls substituierte, sechsgliedrige, aromatische Kohlenwasserstoffgruppe oder fünf- bis siebengliedrige heterocyclische Gruppe, enthaltend ein bis drei Sauerstoffatom(e), Stickstoffatom(e) oder Schwefelatom(e) als Heteroatom(e), oder ein gegebenenfalls substituiertes Silylalkyl, umfassend ein geradkettiges oder ein verzweigtkettiges Alkyl mit 1 bis 8 Kohlenstoffatomen, ist; 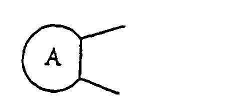
(i) eine Gruppe 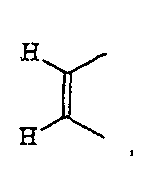
(ii) ein nicht substituierter zweiwertiger Rest aus Benzol, Pyridin, Cyclohexan oder Naphthalin; oder
(iii)ein substituierter zweiwertiger Rest aus Benzol, Pyridin, Cyclohexan oder Naphthalin ist;
und worin die optionalen Substituenten der vorstehenden geraden oder verzweigten C₁- bis C₈-Alkylgruppen aus den Gruppen W1 gewählt sind;
die optionalen Substituenten für die vorstehenden sechsgliedrigen aromatischen Kohlenwasserstoff- und fünf- bis siebengliedrigen heterocyclischen Gruppen aus den Gruppen W2 gewählt sind; und
die Substituenten für die die vorstehenden substituierten zweiwertigen Reste aus den Gruppen W3 gewählt sind;
jede Gruppe W1 Hydroxyl, gerades oder verzweigtes C₁- bis C₈-Alkoxy, gerades oder verzweigtes C₁- bis C₈-Alkylthio, gerades oder verzweigtes C₁- bis C₈-Alkoxycarbonyl, gerades oder verzweigtes C₁- bis C₈-Alkylcarbonyloxy, Halogen, Amino oder Nitro ist;
jede Gruppe W2 gegebenenfalls substituiertes, gerad- oder verzweigtkettiges C₁bis C₈-Alkyl, gegebenenfalls substituiertes gerad- oder verzweigtkettiges C₁- bis C₈-Alkoxy, gegebenenfalls substituiertes, gerad- oder verzweigtkettiges C₁- bis C₈-Alkylthio, gegebenenfalls substituiertes, gerad- oder verzweigtkettiges C₁- bis C₈-Alkylsulfinyl, gegebenenfalls substituiertes, gerad- oder verzweigtkettiges C₁bis C₈-Alkylsulfonyl, gegebenenfalls substituiertes, gerad- oder verzweigtkettiges C₁- bis C₈-Alkylsulfonyloxy, gegebenenfalls substituiertes, gerad- oder verzweigtkettiges C₁- bis C₈-Alkylcarbonyl, Halogen, Hydroxyl, gegebenenfalls substituiertes C₁- bis C₈-Alkylcarbonyloxy, Nitro, eine Phosphorsäuregruppe, di-(gerad- oder verzweigtkettiges C₁- bis C₈-Alkoxy-)phosphoryloxy, Sulfonamid, Amino, substituiertes Amino und gerad- oder verzweigtkettiges C₁- bis C₈-Alkylendioxy ist;
jede Gruppe W3 gegebenenfalls substituiertes, gerad- oder verzweigtkettiges C₁bis C₈-Alkyl, gegebenenfalls substituiertes gerad- oder verzweigtkettiges C₁- bis C₈-Alkoxy, gegebenenfalls substituiertes, gerad- oder verzweigtkettiges C₁- bis C₈-Alkylcarbonyl, gerad- oder verzweigtkettiges C₁- bis C₈-Alkylthio, gerad- oder verzweigtkettiges C₁- bis C₈-Alkylsulfinyl, gerad- oder verzweigtkettiges C₁- bis C₈-Alkylsulfonyl, Halogen, Hydroxyl, Carboxyl, gerad- oder verzweigtkettiges C₁bis C₈-Alkoxycarbonyl, gerad- oder verzweigtkettiges Hydroxy-C₁- bis C₈-Alkyl, Phosphorsäuregruppe, Cyano, Nitro, Sulfonamid, gegebenenfalls substituiertes Amino, gegebenenfalls substituiertes, gerad- oder verzweigtkettiges Amino-C₁bis C₈-Alkyl, gegebenenfalls substituiertes, gerad- oder verzweigtkettiges Silyl-C₁bis C₈-Alkyl oder ein gegebenenfalls substituierter Rest einer gesättigten oder ungesättigten, fünf- bis siebengliedrigen heterocyclischen Gruppe, die ein bis vier Sauerstoffatom(e), Stickstoff(e) oder Schwefelatom(e) als Heteroatom(e) enthält; oder eine gerad- oder verzweigtkettiges C₁- bis C₈-Alkylendioxygruppe ist;
und worindie optionalen Substituenten für die Gruppen W2 und W3, welche nicht substituiertes Amin, substituiertes Aminoalkyl und substituierte Silylalkylgruppen W2 und W3 sind, aus den Gruppen W1 gewählt sind;
die Substituenten für die substituierten Amino- und substituierten Aminoalkylgruppen W2 und W3 aus den Gruppen W4 gewählt sind; und
die Substituenten für substituierte Silylalkylgruppen W2 und W3 aus den Gruppen W5 gewählt sind;
jede Gruppe W4 gewählt wird aus geradem oder verzweigtem C₁- bis C₈-Alkyl, gegebenenfalls substituierter, sechsgliedriger aromatischer Kohlenwasserstoffgruppe oder fünf- bis siebengliedriger heterocyclischer Gruppe, enthaltend ein bis drei Sauerstoffatom(e), Stickstoffatom(e) oder Schwefelatom(e) als Heteroatom(e), und gegebenenfalls substituierter Aralkylgruppe mit 7 bis 20 Kohlenstoffatomen, wobei die zwei Gruppen W4 der Aminogruppe einen fünf- bis siebengliedrigen, gegebenenfalls substituierten Ring zusammen mit dem Stickstoffatom der Aminogruppe bilden können, wobei ein oder mehrere Kohlenstoffatom(e) in dem Ring durch ein Sauerstoffatom, Schwefelatom oder Stickstoffatom ersetzt werden können, wobei optionale Substituenten für die Gruppen W4 aus geradoder verzweigtkettigem C₁- bis C₈-Alkyl, gerad- oder verzweigtkettigem C₁- bis C₈-Alkoxy, gerad- oder verzweigtkettigem C₁- bis C₈-Alkylthio, gerad- oder verzweigtkettigen C₁- bis C₈-Alkylsulfinyl, gerad- oder verzweigtkettigem C₁- bis C₈-Alkylsulfonyl, gerad- oder verzweigtkettigem C₁- bis C₈-Alkylsulfonyloxy, geradoder verzweigtkettigem C₁- bis C₈-Alkylcarbonyl, Halogen, Hydroxyl, Nitro, Phosphorsäuregruppe, di-(gerad- oder verzweigtkettigem C₁- bis C₈-Alkoxy)-phosphoryloxy, Sulfonamid, Aminogruppe und gerad- oder verzweigtkettigem C₁bis C₈-Alkylendioxy gewählt ist; und
jede Gruppe W5 aus gerad- oder verzweigtkettigen C₁- bis C₈-Alkyl; sechsgliedriger, aromatischer Kohlenwasserstoffgruppe oder fünf- bis siebengliedriger heterocyclischer Gruppe, enthaltend ein bis drei Sauerstoffatom(e), Stickstoffatom(e) oder Schwefelatom(e) als Heteroatom(e), oder Aralkyl mit 7 bis 20 Kohlenstoffatomen gewählt wird;
und Ar ist eine gegebenenfalls substituierte, sechsgliedrige, aromatische Kohlenwasserstoffgruppe oder eine fünf- bis siebengliedrige heterocyclische Gruppe, enthaltend ein bis drei Sauerstoffatom(e), Stickstoffatom(e) oder Schwefelatom(e) als Heteroatom(e), wobei optionale Substituenten für Ar aus den Gruppen W2 wie oben definiert gewählt sind.

2. Verbindung gemäß Anspruch 1, oder ein Salz oder Solvat davon, worin Ar eine Phenyl-, Pyridyl- oder Pyrimidylgruppe ist, welche mit einer bis vier Gruppe(n) substituiert werden kann, gewählt aus gegebenenfalls substituiertem Alkyl, gegebenenfalls substituiertem Alkoxy, Alkylthio, Alkylsulfinyl, Alkylsulfonyl, Alkylsulfonyloxy, gegebenenfalls substituiertem Alkylcarbonyl, Halogen, Hydroxyl, gegebenenfalls substituiertem Alkylcarbonyloxy, Nitro, Phosphorsäuregruppe, Dialkoxy-phosphoryloxy, Sulfonamid, gegebenenfalls substituiertem Amino und Alkylendioxy.

3. Verbindung gemäß Anspruch 2, oder ein Salz oder Solvat davon, worin 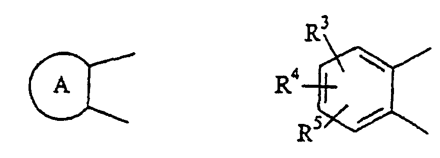 ist,
und worin R³, R⁴ und R⁵ gleich oder unterschiedlich sind und jeweils unabhängig gewählt werden aus Wasserstoff, gegebenenfalls substituiertem Alkyl, gegebenefalls substituiertem Alkoxy, gegebenenfalls substituiertem Alkylcarbonyl, Alkylthio, Alkylsulfinyl, Alkylsulfonyl, Halogen, Hydroxy, Carboxy, Alkoxycarbonyl, Hydroxyalkyl, Phosphorsäuregruppe, Cyano, Nitro, Sulfonamid, gegebenenfalls substituiertem Amino, gegebenenfalls substituiertem Aminoalkyl, gegebenenfalls substituiertem Silylalkyl und heterocyclischen Resten, oder wobei jegliche zwei von R³, R⁴ und R⁵ zusammen eine Alkylendioxygruppe bilden.

4. Verbindung gemäß Anspruch 2, oder ein Salz oder Solvat davon, worin 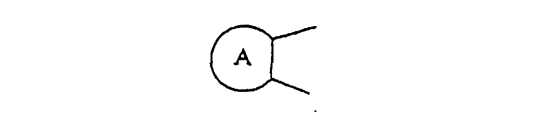 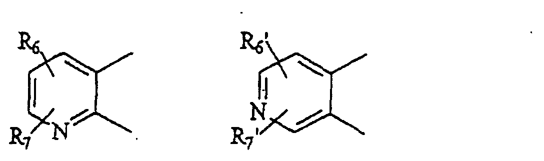 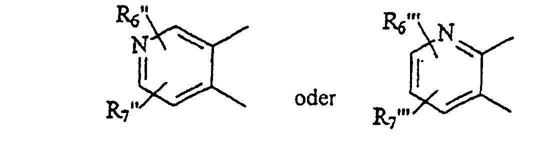 ist,
und worin
R₆ und R₇, R₆' und R₇', R₆" und R₇", R₆''' und R₇''' gleich oder unterschiedlich sind und unabhängig gewählt werden aus Wasserstoff, gegebenenfalls substituiertem Alkyl, gegebenenfalls substituiertem Alkoxy, Halogen, Hydroxyl, Carboxyl, Alkoxycarbonyl, Hydroxyalkyl, Phosphorsäuregruppe, Sulfonamid, gegebenenfalls substituiertem Amino, gegebenenfalls substituiertem Aminoalkyl, gegebenenfalls substituiertem Silylalkyl und heterocyclischen Resten; oder wobei jegliche zwei von R₆ und R₇; R₆' und R₇'; R₆" und R₇"; R₆''' und R₇''' eine Alkylendioxygruppe bilden können.

5. Pharmazeutische Zusammensetzung, umfassend eine Verbindung, ein Salz oder Solvat, wie in mindestens einem der Ansprüche 1 bis 4 beansprucht.

6. Pharmazeutische Zusammensetzung gemäß Anspruch 5, umfassend eine wirksame Menge der Verbindung, des Salzes oder Solvats und einen pharmazeutisch annehmbaren Träger.

7. Verbindung, Salz oder Solvat, wie in mindestens einem der Ansprüche 1 bis 4 beansprucht, zur Verwendung als ein ACAT-Inhibitor, ein Mittel zur Inhibition des Transports von intrazellulärem Cholesterol, ein Mittel zur Senkung des Cholesterols im Blut oder als ein Mittel für das Unterdrücken der Schäumung eines Makrophagen.

8. Verbindung, Salz oder Solvat, wie in mindestens einem der Ansprüche 1 bis 4 beansprucht, zur Verwendung als ein Mittel für die Therapie und zur Verhinderung von Hyperlipämie, Arteriosklerose, zervikaler oder zerebraler Arteriosklerose, zerebrovaskulärer Störung, ischämischer Kardiopathie, koronarer Arteriosklerose, Nephrosklerose, arteriosklerotischer Nephrosklerose, arteriokapillarer sklerotisclher Nephrosklerose, maligner Nephrosklerose, ischämischer Enteropathie, akuter Okklusion vom Mesenterialgefäß, chronischer Mesenterialangina, ischämische Colitis, Aortenaneurysma und Arteriosclerosis obliterans (ASO).

9. Verwendung einer Verbindung, eines Salzes oder Solvats, wie in mindestens einem der Ansprüche 1 bis 4 beansprucht, zur Herstellung eines Medikaments zur Verwendung als ein ACAT-Inhibitor, einem Mittel zur Inhibierung des Transports von intrazellulärem Cholesterol, einem Mittel zur Senkung von Cholesterol im Blut oder einem Mittel zur Unterdrückung des Schäumens eines Makrophagen.

10. Verwendung gemäß Anspruch 9, wobei das Medikament zur Verwendung als ein Mittel zur Therapie oder Verhinderung von Hyperlipämie, Arteriosklerose, zervikaler oder zerebraler Arteriosklerose, zerebrovaskulärer Störung, ischämischer Kardiopathie, koronarer Arteriosklerose, Nephrosklerose, arteriosklerotischer Nephrosklerose, arteriokapillarer sklerotischer Nephrosklerose, maligner Nephrosklerose, ischämischer Enteropathie, akuter Okklusion vom Mesenterialgefäß, chronischer Mesenterialangina, ischämische Colitis, Aortenaneurysma und Arteriosclerosis obliterans (ASO) ist.

## Revendications

1. Composé de formule (I), ou un sel ou solvate d'un composé de formule (I) : 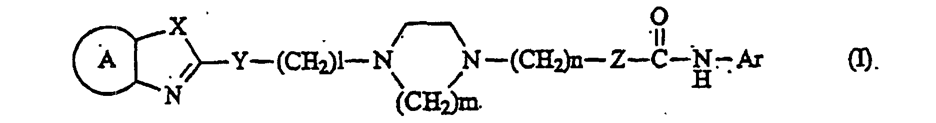 dans laquelle
l est de 1 à 10 ;
m est 2 ou 3 ;
n est de 1 à 3,
X est -NH-, un atome d'oxygène ou un atome de soufre ;
Y est -NR¹-, un atome d'oxygène, un atome de soufre, un sulfoxyde ou une sulfone ;
Z est une liaison simple ou -NR²- ;
R¹ est de l'hydrogène, un groupe alkyle en C₁ à C₈ à chaîne linéaire ou ramifiée facultativement substitué, un groupe hydrocarboné aromatique à six chaînons ou un groupe hétérocyclique à cinq à sept chaînons contenant de un à trois atome(s) d'oxygène, atome(s) d'azote ou atome(s) de soufre en tant qu'hétéroatome(s), facultativement substitués, ou un groupe silylalkyle facultativement substitué comprenant un alkyle à chaîne linéaire ou chaîne ramifiée ayant 1 à 8 atomes de carbone ;
R² est de l'hydrogène, un groupe alkyle en C₁ à C₈ à chaîne linéaire ou ramifiée éventuellement substitué, un groupe hydrocarboné aromatique à six chaînons ou un groupe hétérocyclique à cinq à sept chaînons contenant de un à trois atome(s) d'oxygène, atome(s) d'azote ou atome(s) de soufre, en tant qu'hétéroatomes, éventuellement substitués, ou un groupe silylalkyle éventuellement substitué comprenant un alkyle à chaîne linéaire ou à chaîne ramifiée ayant 1 à 8 atomes de carbone ; 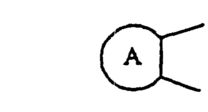 est
(i) un groupe 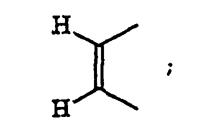
(ii) un résidu divalent non substitué de benzène, de pyridine, de cyclohexane ou de naphtalène ; ou
(iii) un résidu divalent substitué de benzène, de pyridine, de cyclohexane ou de naphtalène ;
et dans laquelle les substituants facultatifs pour les groupes alkyles en C₁ à C₈ linéaires ou ramifiés précités sont choisis parmi les groupes W1 ;
les substituants facultatifs pour les groupes hydrocarbonés aromatiques à six chaînons et hétérocycliques à cinq à sept chaînons précités sont choisis parmi les groupes W2 ; et
les substituants pour les résidus divalents substitués précités sont choisis parmi les groupes W3 ;
chaque groupe W1 est un groupe hydroxyle, alcoxy en C₁ à C₈ linéaire ou ramifié, alkylthio en C₁ à C₈ linéaire ou ramifié, alcoxycarbonyle en C₁ à C₈ linéaire ou ramifié, alkylcarbonyloxy en C₁ à C₈ linéaire ou ramifié, un halogène, un groupe amino ou nitro ;
chaque groupe W2 est un groupe alkyle en C₁ à C₈ à chaîne linéaire ou ramifiée facultativement substitué, alcoxy en C₁ à C₈ à chaîne linéaire ou ramifiée facultativement substitué, alkylthio en C₁ à C₈ à chaîne linéaire ou ramifiée facultativement substitué, alkylsulfinyle en C₁ à C₈ à chaîne linéaire ou ramifiée facultativement substitué, alkylsulfonyle en C₁ à C₈ à chaîne linéaire ou ramifiée facultativement substitué, alkylsulfonyloxy en C₁ à C₈ à chaîne linéaire ou ramifiée facultativement substitué, alkylcarbonyle en C₁ à C₈ à chaîne linéaire ou ramifiée facultativement substitué, un halogène, un groupe hydroxyle, alkylcarbonyloxy en C₁ à C₈ facultativement substitué, nitro, un groupe acide phosphorique, di(alcoxy en C₁ à C₈ à chaîne linéaire ou ramifiée)phosphoryloxy, sulfonamide, amino, amino substitué et alkylènedioxy en C₁ à C₈ à chaîne linéaire ou ramifiée ;
chaque groupe W3 est un groupe alkyle en C₁ à C₈ à chaîne linéaire ou ramifiée facultativement substitué, alcoxy en C₁ à C₈ à chaîne linéaire ou ramifiée facultativement substitué, alkylcarbonyle en C₁ à C₈ à chaîne linéaire ou ramifiée facultativement substitué, alkylthio en C₁ à C₈ à chaîne linéaire ou ramifiée, alkylsulfinyle en C₁ à C₈ à chaîne linéaire ou ramifiée, alkylsulfonyle en C₁ à C₈ à chaîne linéaire ou ramifiée, un halogène, un groupe hydroxyle, carboxyle, alkoxycarbonyle en C₁ à C₈ à chaîne linéaire ou ramifiée, hydroxy-alkyle en C₁ à C₈ à chaîne linéaire ou ramifiée, un groupe acide phosphorique, cyano, nitro, sulfonamide, amino facultativement substitué, amino-alkyle en C₁ à C₈ à chaîne linéaire ou ramifiée facultativement substitué, silyl-alkyle en C₁ à C₈ à chaîne linéaire ou ramifiée facultativement substitué ou un résidu facultativement substitué d'un groupe hétérocyclique à cinq à sept chaînons saturé ou insaturé contenant un à quatre atome(s) d'oxygène, atome(s) d'azote ou atome(s) de soufre en tant qu'hétéroatome(s) ; ou un groupe alkylènedioxy en C₁ à C₈ à chaîne linéaire ou ramifiée ;
et dans laquelle les substituants facultatifs pour les groupes W2 et W3, mis à part les groupes W2 et W3 amine substitué, aminoalkyle substitué et silylalkyle substitué, sont choisis parmi les groupes W1 ;
les substituants pour les groupes W2 et W3 amino substitué et aminoalkyle substitué sont choisis parmi les groupes W4 ; et
les substituants pour les groupes W2 et W3 silylalkyle substitué sont choisis parmi les groupes W5 ;
chaque groupe W4 est choisi parmi un groupe alkyle en C₁ à C₈ linéaire ou ramifié, un groupe hydrocarboné aromatique à six chaînons ou un groupe hétérocyclique à cinq à sept chaînons contenant de un à trois atome(s) d'oxygène, atome(s) d'azote ou atome(s) de soufre en tant qu'hétéro-atome(s), facultativement substitués, et un groupe aralkyle facultativement substitué ayant 7 à 20 carbones, les deux groupes W4 du groupe amino pouvant former un cycle à cinq à sept chaînons facultativement substitué àvec l'atome d'azote du groupe amino, un ou plusieurs atome(s) de carbone dans ce cycle pouvant être remplacé(s) par un atome d'oxygène, un atome de soufre ou un atome d'azote, des substituants facultatifs pour les groupes W4 étant choisis parmi un groupe alkyle en C₁ à C₈ à chaîne linéaire ou ramifiée, alcoxy en C₁ à C₈ à chaîne linéaire ou ramifiée, alkylthio en C₁ à C₈ à chaîne linéaire ou ramifiée, alkylsulfinyle en C₁ à C₈ à chaîne linéaire ou ramifiée, alkylsulfonyle en C₁ à C₈ à chaîne linéaire ou ramifiée, alkylsulfonyloxy en C₁ à C₈ à chaîne linéaire ou ramifiée, alkylcarbonyle en C₁ à C₈ à chaîne linéaire ou ramifiée, un halogène, un groupe hydroxyle, nitro, un groupe acide phosphorique, di(alcoxy en C₁ à C₈ à chaîne linéaire ou ramifiée)-phosphoryloxy, sulfonamide, un groupe amino et alkylènedioxy en C₁ à C₈ à chaîne linéaire ou ramifiée ; et
chaque groupe W5 est choisi parmi un groupe alkyle en C₁ à C₈ à chaîne linéaire ou ramifiée ; un groupe hydrocarboné aromatique à six chaînons ou un groupe hétérocyclique à cinq à sept chaînons contenant de un à trois atome(s) d'oxygène, atome(s) d'azote ou atome(s) de soufre en tant qu'hétéroatome(s) ou aralkyle de 7 à 20 carbones ;
et Ar est un groupe hydrocarboné aromatique à six chaînons ou un groupe hétérocyclique à cinq à sept chaînons contenant d'un à trois atome(s) d'oxygène, atome(s) d'azote ou atome(s) de soufre en tant qu'hétéroatome(s), facultativement substitués, dans lequel des substituants facultatifs pour Ar sont choisis parmi les groupes W2 tels que définis plus haut.

2. Composé selon la revendication 1, ou un sel ou solvate de celui-ci, dans lequel Ar est un groupe phényle, pyridyle ou pyrimidyle qui peut être substitué par un à quatre groupe(s) choisi(s) parmi un groupe alkyle facultativement substitué, alcoxy facultativement substitué, alkylthio, alkylsulfinyle, alkylsulfonyle, alkylsulfonyloxy, alkylcarbonyle facultativement substitué, un halogène, un groupe hydroxyle, alkylcarbonyloxy facultativement substitué, nitro, un groupe acide phosphorique, dialcoxyphosphoryloxy, sulfonamide, amino facultativement substitué et alkylènedioxy.

3. Composé selon la revendication 2, ou un sel ou solvate de celui-ci, dans lequel 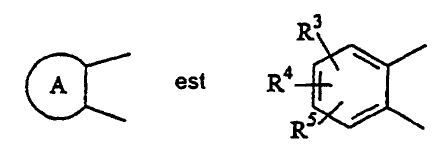 R³, R⁴ et R⁵ sont identiques ou différents et sont chacun indépendamment choisis parmi l'hydrogène, un groupe alkyle facultativement substitué, alcoxy facultativement substitué, alkylcarbonyle facultativement substitué, alkylthio, alkylsulfinyle, alkylsulfonyle, un halogène, un groupe hydroxy, carboxy, alcoxycarbonyle, hydroxyalkyle, un groupe acide phosphorique, cyano, nitro, sulfonamide, amino facultativement substitué, aminoalkyle facultativement substitué, silylalkyle facultativement substitué et des résidus hétérocycliques, ou deux quelconques parmi R³, R⁴ et R⁵ forment ensemble un groupe alkylènedioxy.

4. Composé selon la revendication 2, ou un sel ou solvate de celui-ci, dans lequel 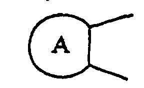 est 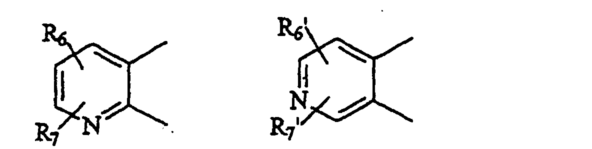 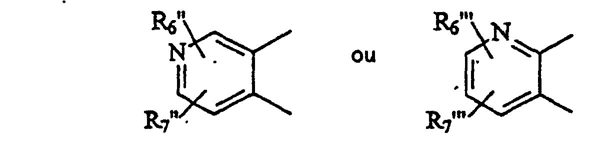 et dans lequel
R₆ et R₇, R₆' et R₇', R₆" et R₇", R₆''' et R₇''' sont identiques ou différents et sont chacun indépendamment choisis parmi l'hydrogène, un groupe alkyle facultativement substitué, alcoxy facultativement substitué, un halogène, un groupe hydroxyle, carboxyle, alcoxycarbonyle, hydroxyalkyle, un groupe acide phosphorique, sulfonamide, amino facultativement substitué, aminoalkyle facultativement substitué, silylalkyle facultativement substitué et des résidus hétérocycliques ; ou deux quelconques parmi R₆ et R₇ ; R₆' et R₇' ; R₆" et R₇" ; R₆''' et R₇''' peuvent former un groupe alkylènedioxy.

5. Composition pharmaceutique comprenant un composé, sel ou solvate tel que revendiqué dans l'une quelconque des revendications 1 à 4.

6. Composition pharmaceutique selon la revendication 5, comprenant une quantité efficace du composé, sel ou solvate et un véhicule pharmaceutiquement acceptable.

7. Composé, sel ou solvate tel que revendiqué dans l'une quelconque des revendications 1 à 4, pour l'utilisation en tant qu'inhibiteur ACAT, agent pour inhiber le transport du cholestérol intracellulaire, agent pour abaisser le cholestérol dans le sang ou agent pour supprimer la transformation des macrophages en cellules spumeuses.

8. Composé, sel ou solvate tel que revendiqué dans l'une quelconque des revendications 1 à 4, pour l'utilisation en tant qu'agent pour la thérapie et la prévention d'une hyperlipémie, d'une artériosclérose, d'une artériosclérose cervicale et cérébrale, d'un trouble cérébrovasculaire, d'une cardiopathie ischémique, d'une artériosclérose coronaire, d'une néphrosclérose, d'une néphrosclérose artérioscléreuse, d'une néphrosclérose sclérotique artériocapillaire, d'une néphrosclérose maligne, d'une enthéropathie ischémique, d'une occlusion aiguë d'un vaisseau mésentérique, d'une angine mésentérique chronique, d'une colite ischémique, d'un anévrisme aortique et d'une artériosclérose obliterans (ASO).

9. Utilisation d'un composé, sel ou solvate tel que revendiqué dans l'une quelconque des revendications 1 à 4, pour la préparation d'un médicament pour utilisation en tant qu'inhibiteur ACAT, agent pour inhiber le transport du cholestérol intracellulaire, agent pour abaisser le cholestérol dans le sang ou agent pour supprimer la transformation des macrophages en cellules spumeuses.

10. Utilisation selon la revendication 9, dans laquelle le médicament est destiné à une utilisation en tant qu'agent pour la thérapie ou la prévention d'une hyperlipémie, d'une artériosclérose, d'une artériosclérose cervicale et cérébrale, d'un trouble cérébrovasculaire, d'une cardiopathie ischémique, d'une artériosclérose coronaire, d'une néphrosclérose, d'une néphrosclérose artérioscléreuse, d'une néphrosclérose sclérotique artériocapillaire, d'une néphrosclérose maligne, d'une enthéropathie ischémique, d'une occlusion aiguë d'un vaisseau mésentérique, d'une angine mésentérique chronique, d'une colite ischémique, d'un anévrisme aortique et d'une artériosclérose obliterans (ASO).
